# EUROPEAN PATENT APPLICATION

(11) **EP 3 662 903 A2**
(43) Date of publication of application: **10.06.2020**
(21) Application number: 19206634.8
(22) Date of filing: 02.10.2015
(51) Int. Cl.: A61K 31/00, A61K 39/395, A61P 35/00, C07K 16/28

(54) **COMBINATION THERAPIES**

(30) Priority: 03.10.2014 US 201462059832 P
(62) Divisional of application: 15782164.6
(71) Applicant: Novartis AG, 4056 Basel (CH)
(72) Inventor: CAO, Zhu, Alexander, Cambridge, MA 02139 (US); RONG, Xianhui, Cambridge, MA 02139 (US); PINZON-ORTIZ, Maria, Consuelo, Cambridge, MA 02139 (US); LONGMIRE, Tyler, Cambridge, MA 02139 (US); LEE, Benjamin, Hyun, Cambridge, MA 02139 (US)
(74) Representative: Elkington and Fife LLP

(57) **Abstract**

Combination therapies are disclosed. The combination therapies can be used to treat or prevent cancerous conditions and/or disorders.

SEQUENCE LISTING

<110> NOVARTIS AG

<120> COMBINATION THERAPIES

<130> C2160-7006WO

<140>
<141>

<150> 62/059,832
<151> 2014-10-03

<160> 7

<170> PatentIn version 3.5

<210> 1
<211> 5
<212> PRT
<213> Unknown

<220>
<221> source
<223> /note="Description of Unknown:
      PD-1 motif peptide"

<400> 1
Met Tyr Pro Pro Tyr
1               5


<210> 2
<211> 440
<212> PRT
<213> Artificial Sequence

<220>
<221> source
<223> /note="Description of Artificial Sequence: Synthetic
      polypeptide"

<400> 2
Gln Val Gln Leu Val Glu Ser Gly Gly Gly Val Val Gln Pro Gly Arg
1               5                   10                  15


Ser Leu Arg Leu Asp Cys Lys Ala Ser Gly Ile Thr Phe Ser Asn Ser
            20                  25                  30


Gly Met His Trp Val Arg Gln Ala Pro Gly Lys Gly Leu Glu Trp Val
        35                  40                  45


Ala Val Ile Trp Tyr Asp Gly Ser Lys Arg Tyr Tyr Ala Asp Ser Val
    50                  55                  60


Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Lys
        435                 440

<210> 3
<211> 214
<212> PRT
<213> Artificial Sequence

<220>
<221> source
<223> /note="Description of Artificial Sequence: Synthetic
polypeptide"

<400> 3
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 4
<211> 447
<212> PRT
<213> Artificial Sequence

<220>
<221> source
<223> /note="Description of Artificial Sequence: Synthetic
polypeptide"

<400> 4
Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Met Tyr Trp Val Arg Gln Ala Pro Gly Gln Gly Leu Glu Trp Met
        35                  40                  45

Gly Gly Ile Asn Pro Ser Asn Gly Gly Thr Asn Phe Asn Glu Lys Phe
    50                  55                  60

Lys Asn Arg Val Thr Leu Thr Thr Asp Ser Ser Thr Thr Thr Ala Tyr
65                  70                  75                  80

Met Glu Leu Lys Ser Leu Gln Phe Asp Asp Thr Ala Val Tyr Tyr Cys
                85                  90                  95

Ala Arg Arg Asp Tyr Arg Phe Asp Met Gly Phe Asp Tyr Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile

325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

<210> 5
<211> 218
<212> PRT
<213> Artificial Sequence

<220>
<221> source
<223> /note="Description of Artificial Sequence: Synthetic
      polypeptide"

<400> 5
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg

                85                  90                  95

Asp Leu Pro Leu Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210> 6
<211> 118
<212> PRT
<213> Artificial Sequence

<220>
<221> source
<223> /note="Description of Artificial Sequence: Synthetic
polypeptide"

<400> 6
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ile Met Met Trp Val Arg Gln Ala Pro Gly Lys Gly Leu Glu Trp Val
        35                  40                  45

Ser Ser Ile Tyr Pro Ser Gly Gly Ile Thr Phe Tyr Ala Asp Lys Gly
    50                  55                  60

Arg Phe Thr Ile Ser Arg Asp Asn Ser Lys Asn Thr Leu Tyr Leu Gln

65                  70                  75                  80

Met Asn Ser Leu Arg Ala Glu Asp Thr Ala Val Tyr Tyr Cys Ala Arg
                85                  90                  95

Ile Lys Leu Gly Thr Val Thr Thr Val Asp Tyr Trp Gly Gln Gly Thr
            100                 105                 110

Leu Val Thr Val Ser Ser
        115

<210> 7
<211> 110
<212> PRT
<213> Artificial Sequence

<220>
<221> source
<223> /note="Description of Artificial Sequence: Synthetic
      polypeptide"

<400> 7
Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu
            100                 105                 110

## Description

### CROSS REFERENCE TO RELATED APPLICATIONS

This application claims the benefit of U.S. Provisional Application No. 62/059,832, filed October 3, 2014, the contents of the aforementioned application are hereby incorporated by reference in their entirety.

### SEQUENCE LISTING

The instant application contains a Sequence Listing which has been submitted electronically in ASCII format and is hereby incorporated by reference in its entirety. Said ASCII copy, created on October 1, 2015, is named C2160-7006WO_SL.txt and is 14,618 bytes in size.

### BACKGROUND

The ability of T cells to mediate an immune response against an antigen requires two distinct signaling interactions (Viglietta, V. et al. (2007) Neurotherapeutics 4:666-675; Korman, A. J. et al. (2007) Adv. Immunol. 90:297-339). First, an antigen that has been arrayed on the surface of antigen-presenting cells (APC) is presented to an antigen-specific naive CD4⁺ T cell. Such presentation delivers a signal via the T cell receptor (TCR) that directs the T cell to initiate an immune response specific to the presented antigen. Second, various co-stimulatory and inhibitory signals mediated through interactions between the APC and distinct T cell surface molecules trigger the activation and proliferation of the T cells and ultimately their inhibition.

The immune system is tightly controlled by a network of costimulatory and co-inhibitory ligands and receptors. These molecules provide the second signal for T cell activation and provide a balanced network of positive and negative signals to maximize immune responses against infection, while limiting immunity to self (Wang, L. *et al.* (Epub Mar. 7, 2011) J. Exp. Med. 208(3):577-92; Lepenies, B. et al. (2008) Endocrine, Metabolic & Immune Disorders-Drug Targets 8:279-288). Examples of costimulatory signals include the binding between the B7.1 (CD80) and B7.2 (CD86) ligands of the APC and the CD28 and CTLA-4 receptors of the CD4⁺ T-lymphocyte (Sharpe, A. H. et al. (2002) Nature Rev. Immunol. 2:116-126; Lindley, P. S. et al. (2009) Immunol. Rev. 229:307-321). Binding of B7.1 or B7.2 to CD28 stimulates T cell activation, whereas binding of B7.1 or B7.2 to CTLA-4 inhibits such activation (Dong, C. et al. (2003) Immunolog. Res. 28(1):39-48; Greenwald, R. J. et al. (2005) Ann. Rev. Immunol. 23:515-548). CD28 is constitutively expressed on the surface of T cells (Gross, J., et al. (1992) J. Immunol. 149:380-388), whereas CTLA-4 expression is rapidly up-regulated following T-cell activation (Linsley, P. et al. (1996) Immunity 4:535-543).

Other ligands of the CD28 receptor include a group of related B7 molecules, also known as the "B7 Superfamily" (Coyle, A. J. et al. (2001) Nature Immunol. 2(3):203-209; Sharpe, A. H. et al. (2002) Nature Rev. Immunol. 2:116-126; Collins, M. et al. (2005) Genome Biol. 6:223.1-223.7; Korman, A. J. et al. (2007) Adv. Immunol. 90:297-339). Several members of the B7 Superfamily are known, including B7.1 (CD80), B7.2 (CD86), the inducible co-stimulator ligand (ICOS-L), the programmed death-1 ligand (PD-L1; B7-H1), the programmed death-2 ligand (PD-L2; B7-DC), B7-H3, B7-H4 and B7-H6 (Collins, M. et al. (2005) Genome Biol. 6:223.1-223.7).

The Programmed Death 1 (PD-1) protein is an inhibitory member of the extended CD28/CTLA-4 family of T cell regulators (Okazaki et al. (2002) Curr Opin Immunol 14: 391779-82; Bennett et al. (2003) J. Immunol. 170:711-8). Other members of the CD28 family include CD28, CTLA-4, ICOS and BTLA. PD-1 is suggested to exist as a monomer, lacking the unpaired cysteine residue characteristic of other CD28 family members. PD-1 is expressed on activated B cells, T cells, and monocytes.

The PD-1 gene encodes a 55 kDa type I transmembrane protein (Agata et al. (1996) Int Immunol. 8:765-72). Although structurally similar to CTLA-4, PD-1 lacks the MYPPY motif (SEQ ID NO: 1) that is important for B7-1 and B7-2 binding. Two ligands for PD-1 have been identified, PD-L1 (B7-H1) and PD-L2 (B7-DC), that have been shown to downregulate T cell activation upon binding to PD-1 (Freeman et al. (2000) J. Exp. Med. 192:1027-34; Carter et al. (2002) Eur. J. Immunol. 32:634-43). Both PD-L1 and PD-L2 are B7 homologs that bind to PD-1, but do not bind to other CD28 family members. PD-L1 is abundant in a variety of human cancers (Dong et al. (2002) Nat. Med. 8:787-9).

PD-1 is known as an immunoinhibitory protein that negatively regulates TCR signals (Ishida, Y. et al. (1992) EMBO J. 11:3887-3895; Blank, C. *et al.* (Epub 2006 Dec. 29) Immunol. Immunother. 56(5):739-745). The interaction between PD-1 and PD-L1 can act as an immune checkpoint, which can lead to, *e.g.,* a decrease in tumor infiltrating lymphocytes, a decrease in T-cell receptor mediated proliferation, and/or immune evasion by cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1 or PD-L2; the effect is additive when the interaction of PD-1 with PD-L2 is blocked as well (Iwai et al. (2002) Proc. Nat'l. Acad. Sci. USA 99:12293-7; Brown et al. (2003) J. Immunol. 170:1257-66).

Given the importance of immune checkpoint pathways in regulating an immune response, the need exists for developing novel combination therapies that activate the immune system.

### SUMMARY

The present invention provides, at least in part, methods and compositions comprising an immunomodulator (*e.g.,* one or more of: an activator of a costimulatory molecule or an inhibitor of an immune checkpoint molecule) in combination with a second therapeutic agent chosen from one or more of the agents listed in Table 1. In one embodiment, an inhibitor of an immune checkpoint molecule (*e.g.,* one or more inhibitors of PD-1, PD-L1, LAG-3, TIM-3, CEACAM (*e.g.,* CEACAM-1, -3, and/or -5) or CTLA-4) can be combined with a second therapeutic agent chosen from one or more agents listed in Table 1 (*e.g.,* one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor; 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor, or 17) a BCR-ABL inhibitor). The combinations described herein can provide a beneficial effect, *e.g.,* in the treatment of a cancer, such as an enhanced anti-cancer effect, reduced toxicity and/or reduced side effects. For example, the immunomodulator, the second therapeutic agent, or both, can be administered at a lower dosage than would be required to achieve the same therapeutic effect compared to a monotherapy dose. Thus, compositions and methods for treating proliferative disorders, including cancer, using the aforesaid combination therapies are disclosed.

Accordingly, in one aspect, the invention features a method of treating (*e.g.,* inhibiting, reducing, ameliorating, or preventing) a proliferative condition or disorder (*e.g.,* a cancer) in a subject. The method includes administering to the subject an immunomodulator (*e.g.,* one or more of: an activator of a costimulatory molecule or an inhibitor of an immune checkpoint molecule) and a second therapeutic agent, *e.g.,* a second therapeutic agent chosen from one or more of the agents listed in Table 1, thereby treating the proliferative condition or disorder (*e.g.,* the cancer). In certain embodiments, the immunomodulator is an inhibitor of an immune checkpoint molecule (*e.g.,* an inhibitor of PD-1, PD-L1, LAG-3, TIM-3, CEACAM (*e.g.,* CEACAM-1, -3, and/or -5) or CTLA-4, or any combination thereof). In other embodiments, the second therapeutic agent is chosen from one or more of the agents listed in Table 1, *e.g.,* one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor); 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor, or 17) a BCR-ABL inhibitor). The combination of the immunomodulator and the second agent can be administered together in a single composition or administered separately in two or more different compositions, *e.g.,* one or more compositions or dosage forms as described herein. The administration of the immunomodulator and the second agent can be in any order. For example, the immunomodulator can be administered concurrently with, prior to, or subsequent to, the second agent.

In another aspect, the invention features a method of reducing an activity (*e.g.,* growth, survival, or viability, or all), of a proliferative (*e.g.,* a cancer) cell. The method includes contacting the cell with an immunomodulator (*e.g.,* one or more of: an activator of a costimulatory molecule or an inhibitor of an immune checkpoint molecule) and a second therapeutic agent, *e.g.,* a second therapeutic agent chosen from one or more of the agents listed in Table 1, thereby reducing an activity in the cell. In certain embodiments, the immunomodulator is an inhibitor of an immune checkpoint molecule (*e.g.,* an inhibitor of PD-1, PD-L1, LAG-3, TIM-3, CEACAM (*e.g.,* CEACAM-1, -3, and/or -5) or CTLA-4, or any combination thereof). In other embodiments, the second therapeutic agent is chosen from one or more of the agents listed in Table 1, *e.g.,* one or more: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor); 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell *(e.g.,* a CAR T cell targeting CD19); 16) a MEK inhibitor, or 17) a BCR-ABL inhibitor).

In some embodiments, the methods described herein can be used *in vitro.* For example, *in vitro* hPBMC-based assays can be used to screen for combination signals of immunomodulators and second therapeutic agents, as disclosed, *e.g.,* in Wang, C. et al. (2014) Cancer Immunology Research 2:846-856. In some embodiments, the methods described herein can be used *in vivo, e.g.,* in an animal subject or model or as part of a therapeutic protocol. The contacting of the cell with the immunomodulator and the second agent can be in any order. In certain embodiments, the cell is contacted with the immunomodulator concurrently, prior to, or subsequent to, the second agent. In some embodiments, the method described herein is used to measure tumor lymphocyte infiltration (TLI) *in vitro* or *in vivo,* as disclosed, *e.g.,* in Frederick, D.T. et al. (2013) Clinical Cancer Research 19:1225-31.

In some embodiments, the method includes contacting the cell with an immunomodulator (*e.g.,* one or more of: an activator of a costimulatory molecule or an inhibitor of an immune checkpoint molecule) and/or a second therapeutic agent, *e.g.,* a second therapeutic agent chosen from one or more of the agents listed in Table 1, in an animal model. In some embodiments, the animal model has a mutation that inhibits or activates IAP, EGF receptor, cMET, ALK, CDK4/6, PI3K, BRAF, FGF receptor, MEK, and/or BCR-ABL. In one exemplary embodiment, an animal model is a mouse model implanted with MC38 murine colon carcinoma. In another exemplary embodiment, an animal model is a mouse model with an inactivated p110δ isoform of PI3 kinase (*e.g.,* p110δ^{D910A}) as disclosed, *e.g.,* in Ali, K., et al., (2014) Nature 510:407-411. In some embodiments, an immune phenotype is determined by measuring one or more of expression, activation, signalling, flow cytometry, mRNA analysis, cytokine levels and/or immunohistochemisty. In some embodiments, the immune phenotype is determined systemically, *e.g.,* in PBMCs. In some embodiments, the immune phenotype is determined *in situ, e.g,* in tumor cells. In some embodiments, one or more of the following parameters is characterized to determine an immune phenotype: checkpoint induction; level of M1 macrophages relative to level of M2 macrophages; level of effector T cells relative to level of regulatory T cells; and/or level of T_{H1} cells relative to T_{H2/H17} cells.

In another aspect, the invention features a composition (*e.g.,* one or more compositions, formulations or dosage formulations) or a pharmaceutical combination, comprising an immunomodulator (*e.g.,* one or more of: an activator of a costimulatory molecule or an inhibitor of an immune checkpoint molecule) and a second therapeutic agent, *e.g.,* a second therapeutic agent chosen from one or more of the agents listed in Table 1. In certain embodiments, the immunomodulator is an inhibitor of an immune checkpoint molecule (*e.g.,* an inhibitor of PD-1, PD-L1, LAG-3, TIM-3, CEACAM (*e.g.,* CEACAM-1, -3 and/or -5) or CTLA-4, or any combination thereof). In other embodiments, the second therapeutic agent is chosen from one or more of the agents listed in Table 1, *e.g.,* one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor); 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell *(e.g.,* a CAR T cell targeting CD19); 16) a MEK inhibitor, or 17) a BCR-ABL inhibitor). In one embodiment, the composition comprises a pharmaceutically acceptable carrier. The immunomodulator and the second agent can be present in a single composition or as two or more different compositions. The immunomodulator and the second agent can be administered via the same administration route or via different administration routes. In one embodiment, the pharmaceutical combination comprises the immunomodulator and the second agent separately or together.

In one embodiment, the composition, formulation or pharmaceutical combination is for use as a medicine, *e.g.,* for the treatment of a proliferative disease (*e.g.,* a cancer as described herein). In some embodiments, the immunomodulator and the second agent are administered concurrently, *e.g.,* independently at the same time or within an overlapping time interval, or separately within time intervals. In certain embodiment, the time interval allows the immunomodulator and the second agent to be jointly active. In one embodiment, the composition, formulation or pharmaceutical combination includes an amount which is jointly therapeutically effective for the treatment of a proliferative disease, *e.g.,* a cancer as described herein.

In another aspect, the invention features a use of a composition (*e.g.,* one or more compositions, formulations or dosage formulations) or a pharmaceutical combination, comprising an immunomodulator (*e.g.,* one or more of: an activator of a costimulatory molecule or an inhibitor of an immune checkpoint molecule) and a second therapeutic agent, *e.g.,* a second therapeutic agent chosen from one or more of the agents listed in Table 1, for the manufacture of a medicament for treating a proliferative disease, *e.g.,* a cancer. In certain embodiments, the immunomodulator is an inhibitor of an immune checkpoint molecule (*e.g.,* an inhibitor of PD-1, PD-L1, LAG-3, TIM-3, CEACAM (*e.g.,* CEACAM-1, -3 and/or -5) or CTLA-4, or any combination thereof). In other embodiments, the second therapeutic agent is chosen from one or more of the agents listed in Table 1, *e.g.,* one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor); 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor, or 17) a BCR-ABL inhibitor).

Kits, *e.g.,* therapeutic kits, that include the immunomodulator (*e.g.,* one or more of: an activator of a costimulatory molecule or an inhibitor of an immune checkpoint molecule as described herein) and the second therapeutic agent, *e.g.,* a second therapeutic agent chosen from one or more of the agents listed in Table 1, and instructions for use, are also disclosed.

Additional features or embodiments of the methods, compositions, dosage formulations, and kits described herein include one or more of the following:
In certain embodiments, the immunomodulator is an activator of a costimulatory molecule. In one embodiment, the agonist of the costimulatory molecule is chosen from an agonist (*e.g.,* an agonistic antibody or antigen-binding fragment thereof, or a soluble fusion) of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand, or any combination thereof.

In certain embodiments, the immunomodulator is an inhibitor of an immune checkpoint molecule. In one embodiment, the immunomodulator is an inhibitor of PD-1, PD-L1, PD-L2, CTLA-4, TIM-3, LAG-3, CEACAM (*e.g.,* CEACAM-1, -3 and/or -5), VISTA, BTLA, TIGIT, LAIR1, CD160, 2B4 and/or TGFR beta. In one embodiment, the inhibitor of an immune checkpoint molecule inhibits PD-1, PD-L1, LAG-3, TIM-3, CEACAM (e.g., CEACAM-1, -3 and/or -5) or CTLA-4, or any combination thereof.

Inhibition of an inhibitory molecule can be performed at the DNA, RNA or protein level. In embodiments, an inhibitory nucleic acid (*e.g.,* a dsRNA, siRNA or shRNA), can be used to inhibit expression of an inhibitory molecule. In other embodiments, the inhibitor of an inhibitory signal is, a polypeptide *e.g.,* a soluble ligand (*e.g.,* PD-1-Ig or CTLA-4 Ig). In other embodiments, the inhibitor of the inhibitory signal is an antibody or antigen-binding fragment thereof, that binds to the inhibitory molecule; *e.g.,* an antibody or fragment thereof (also referred to herein as "an antibody molecule") that binds to PD-1, PD-L1, PD-L2, CTLA-4, TIM-3, LAG-3, CEACAM (*e.g.,* CEACAM-1, -3 and/or -5), VISTA, BTLA, TIGIT, LAIR1, CD160, 2B4 and/or TGFR beta, or a combination thereof.

In one embodiment, the antibody molecule is a full antibody or fragment thereof (*e.g.,* a Fab, F(ab')₂, Fv, or a single chain Fv fragment (scFv)). In yet other embodiments, the antibody molecule has a heavy chain constant region (Fc) chosen from, *e.g.,* the heavy chain constant regions of IgG1, IgG2, IgG3, IgG4, IgM, IgA1, IgA2, IgD, and IgE; particularly, chosen from, *e.g.,* the heavy chain constant regions of IgG1, IgG2, IgG3, and IgG4, more particularly, the heavy chain constant region of IgG1 or IgG4 (*e.g.,* human IgG1 or IgG4). In one embodiment, the heavy chain constant region is human IgG1 or human IgG4. In one embodiment, the constant region is altered, *e.g.,* mutated, to modify the properties of the antibody molecule (*e.g.,* to increase or decrease one or more of: Fc receptor binding, antibody glycosylation, the number of cysteine residues, effector cell function, or complement function).

In certain embodiments, the antibody molecule is in the form of a bispecific or multispecific antibody molecule. In one embodiment, the bispecific antibody molecule has a first binding specificity to PD-1 or PD-L1 and a second binding specifity, *e.g.,* a second binding specificity to TIM-3, LAG-3, or PD-L2. In one embodiment, the bispecific antibody molecule binds to PD-1 or PD-L1 and TIM-3. In another embodiment, the bispecific antibody molecule binds to PD-1 or PD-L1 and LAG-3. In another embodiment, the bispecific antibody molecule binds to PD-1 or PD-L1 and CEACAM (*e.g.,* CEACAM-1, -3 and/or -5). In another embodiment, the bispecific antibody molecule binds to PD-1 or PD-L1 and CEACAM-1. In still another embodiment, the bispecific antibody molecule binds to PD-1 or PD-L1 and CEACAM-3. In yet another embodiment, the bispecific antibody molecule binds to PD-1 or PD-L1 and CEACAM-1. In another embodiment, the bispecific antibody molecule binds to PD-1 or PD-L1. In yet another embodiment, the bispecific antibody molecule binds to PD-1 and PD-L2. In another embodiment, the bispecific antibody molecule binds to TIM-3 and LAG-3. In another embodiment, the bispecific antibody molecule binds to CEACAM (*e.g.,* CEACAM-1, -3 and/or - 5) and LAG-3. In another embodiment, the bispecific antibody molecule binds to CEACAM (*e.g.,* CEACAM-1, -3 and/or -5) and TIM-3. Any combination of the aforesaid molecules can be made in a multispecific antibody molecule, *e.g.,* a trispecific antibody that includes a first binding specificity to PD-1 or PD-1, and a second and third binding specifities to two or more of: TIM-3, CEACAM (*e.g.,* CEACAM-1, -3 and/or -5), LAG-3, or PD-L2.

In certain embodiments, the immunomodulator is an inhibitor of PD-1, *e.g.,* human PD-1. In another embodiment, the immunomodulator is an inhibitor of PD-L1, *e.g.,* human PD-L1. In one embodiment, the inhibitor of PD-1 or PD-L1 is an antibody molecule to PD-1 or PD-L1. The PD-1 or PD-L1 inhibitor can be administered alone, or in combination with other immunomodulators, *e.g.,* in combination with an inhibitor of LAG-3, TIM-3, CEACAM (*e.g.,* CEACAM-1, -3 and/or -5) or CTLA-4. In an exemplary embodiment, the inhibitor of PD-1 or PD-L1, *e.g.,* the anti-PD-1 or PD-L1 antibody molecule, is administered in combination with a LAG-3 inhibitor, *e.g.,* an anti-LAG-3 antibody molecule. In another embodiment, the inhibitor of PD-1 or PD-L1, *e.g.,* the anti-PD-1 or PD-L1 antibody molecule, is administered in combination with a TIM-3 inhibitor, *e.g.,* an anti-TIM-3 antibody molecule. In yet other embodiments, the inhibitor of PD-1 or PD-L1, *e.g.,* the anti-PD-1 antibody molecule, is administered in combination with a LAG-3 inhibitor, *e.g.,* an anti-LAG-3 antibody molecule, and a TIM-3 inhibitor, *e.g.,* an anti-TIM-3 antibody molecule. In another embodiment, the inhibitor of PD-1 or PD-L1, *e.g.,* the anti-PD-1 or PD-L1 antibody molecule, is administered in combination with a CEACAM (*e.g.,* CEACAM-1, -3 and/or -5) inhibitor, *e.g.,* an anti-CEACAM antibody molecule. In another embodiment, the inhibitor of PD-1 or PD-L1, *e.g.,* the anti-PD-1 or PD-L1 antibody molecule, is administered in combination with a CEACAM-1 inhibitor, *e.g.,* an anti-CEACAM-1 antibody molecule. In another embodiment, the inhibitor of PD-1 or PD-L1, *e.g.,* the anti-PD-1 or PD-L1 antibody molecule, is administered in combination with a CEACAM-3 inhibitor, *e.g.,* an anti-CEACAM-3 antibody molecule. In another embodiment, the inhibitor of PD-1 or PD-L1, *e.g.,* the anti-PD-1 or PD-L1 antibody molecule, is administered in combination with a CEACAM-5 inhibitor, *e.g.,* an anti-CEACAM-5 antibody molecule. Other combinations of immunomodulators with a PD-1 inhibitor (*e.g.,* one or more of PD-L2, CTLA-4, TIM-3, LAG-3, VISTA, BTLA, TIGIT, LAIR1, CD160, 2B4 and/or TGFR) are also within the present invention. Any of the antibody molecules known in the art or disclosed herein can be used in the aforesaid combinations of inhibitors of checkpoint molecule.

### Exemplary Inhibitors of Immune Checkpoint Molecules

In one embodiment, the PD-1 inhibitor is an anti-PD-1 antibody chosen from Nivolumab, Pembrolizumab or Pidilizumab.

In some embodiments, the anti-PD-1 antibody is Nivolumab. Alternative names for Nivolumab include MDX- 1106, MDX-1106-04, ONO-4538, or BMS-936558. In some embodiments, the anti-PD-1 antibody is Nivolumab (CAS Registry Number: 946414-94-4). Nivolumab is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168.

In other embodiments, the anti-PD-1 antibody is Pembrolizumab. Pembrolizumab (Trade name KEYTRUDA formerly Lambrolizumab, also known as Merck 3745, MK-3475 or SCH-900475) is a humanized IgG4 monoclonal antibody that binds to PD-1. Pembrolizumab is disclosed, *e.g.,* in Hamid, O. et al. (2013) New England Journal of Medicine 369 (2): 134-44, WO2009/114335, and US 8,354,509.

In some embodiments, the anti-PD-1 antibody is Pidilizumab. Pidilizumab (CT-011; Cure Tech) is a humanized IgGlk monoclonal antibody that binds to PD-1. Pidilizumab and other humanized anti-PD-1 monoclonal antibodies are disclosed in WO2009/101611. Other anti-PD-1 antibodies are disclosed in US 8,609,089, US 2010028330, and/or US 20120114649. Other anti-PD-1 antibodies include AMP 514 (Amplimmune).

In some embodiments, the PD-1 inhibitor is an immunoadhesin (*e.g.,* an immunoadhesin comprising an extracellular or PD-1 binding portion of PD-L1 or PD-L2 fused to a constant region (*e.g.,* an Fc region of an immunoglobulin sequence)). In some embodiments, the PD-1 inhibitor is AMP-224.

In some embodiments, the PD-L1 inhibitor is anti-PD-Ll antibody. In some embodiments, the anti-PD-Ll inhibitor is chosen from YW243.55.S70, MPDL3280A, MEDI-4736, MSB-0010718C, or MDX-1105.

In one embodiment, the PD-L1 inhibitor is MDX-1105. MDX-1105, also known as BMS-936559, is an anti-PD-Ll antibody described in WO2007/005874.

In one embodiment, the PD-L1 inhibitor is YW243.55.S70. The YW243.55.S70 antibody is an anti-PD-Ll described in WO 2010/077634 (heavy and light chain variable region sequences shown in SEQ ID Nos. 20 and 21, respectively, of WO 2010/077634).

In one embodiment, the PD-L1 inhibitor is MDPL3280A (Genentech / Roche). MDPL3280A is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906.

In other embodiments, the PD-L2 inhibitor is AMP-224. AMP-224 is a PD-L2 Fc fusion soluble receptor that blocks the interaction between PD-1 and B7-H1 (B7-DCIg; Amplimmune; e.g., disclosed in WO2010/027827 and WO2011/066342).

In one embodiment, the LAG-3 inhibitor is an anti-LAG-3 antibody molecule. In one embodiment, the LAG-3 inhibitor is BMS-986016, disclosed in more detail herein below.

In one embodiment, the TIM-3 inhibitor is an anti-TIM-3 antibody molecule, *e.g.,* an anti-TIM-3 antibody molecule as described herein.

One or more of the aforesaid inhibitors of immune checkpoint molecules can be used in combination with one or more of the second agents disclosed in Table 1, as more specifically exemplified below. In embodiments, the second agent is chosen from one or more of:
1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
2) ((1R, 9S, 12S, 15R, 16E, 18R, 19R, 21R, 23S, 24E, 26E, 28E, 30S, 32S, 35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone);
3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
4) N-(4-((1R,3S,5S)-3-amino-5-methylcyclohexyl)pyridin-3-yl)-6-(2,6-difluorophenyl)-5-fluoropicolinamide;
5) anti-HER3 monoclonal antibody or antigen binding fragment thereof, that comprises a VH of SEQ ID NO: 141 and VL of SEQ ID NO: 140, as described in U.S. 8,735,551;
6) (E)-N-hydroxy-3-(4-(((2-(2-methyl-1H-indol-3-yl)ethyl)amino)methyl)phenyl) acrylamide;
7) (3R)-3-cyclopentyl-3-[4-(7H-pyrrolo-[2,3-d]pyrimidin-4-yl)-1H-pyrazol-1-yl]propanenitrile; and/or
8) 8-(2,6-difluoro-3,5-dimethoxy-phenyl)-quinoxaline-5-carboxylic acid (4-dimethylaminomethyl-1H-imidazol-2-yl)-amide.

### Exemplary Combination Therapies

In one embodiment, the inhibitor of PD-1 is Nivolumab (CAS Registry No: 946414-94-4) disclosed in *e.g.,* US 8,008,449, and having a sequence disclosed herein, *e.g.,* a heavy chain sequence of SEQ ID NO: 2 and a light chain sequence of SEQ ID NO: 3 (or a sequence substantially identical or similar thereto, *e.g.,* a sequence at least 85%, 90%, 95% identical or higher to the sequence specified).
In another embodiment, the inhibitor of PD-1 is Pembrolizumab disclosed in, *e.g.,* US 8,354,509 and WO 2009/114335, and having a sequence disclosed herein, *e.g.,* a heavy cahin sequence of SEQ ID NO: 4 and a light chain sequence of SEQ ID NO: 5 (or a sequence substantially identical or similar thereto, *e.g.,* a sequence at least 85%, 90%, 95% identical or higher to the sequence specified).

In another embodiment, the inhibitor of PD-L1 is MSB0010718C (also referred to as A09-246-2) disclosed in, *e.g.,* WO 2013/0179174, and having a sequence disclosed herein, *e.g.,* a heavy cahin sequence of SEQ ID NO: 6 and a light chain sequence of SEQ ID NO: 7 (or a sequence substantially identical or similar thereto, *e.g.,* a sequence at least 85%, 90%, 95% identical or higher to the sequence specified).

In certain embodiments, the PD-1 inhibitor, *e.g.,* the anti-PD-1 antibody (*e.g.,* Nivolumab) is used in a method or composition described herein. For example, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C) (alone or in combination with other immunomodulators) is used in combination with one or more of the agents described herein, e.g., listed in Table 1, or disclosed in a publication listed in Table 1, *e.g.,* one or more of: 1) an Inhibitor of Apoptosis (IAP) inhibitor; 2) an inhibitor of a Target of Rapamycin (TOR) kinase; 3) an inhibitor of a human homolog of mouse double minute 2 E3 ubiquitin ligase (HDM2); 4) a PIM kinase inhibitor; 5) an inhibitor of Human epidermal growth factor 3 (HER3) kinase; 6) a Histone Deacetylase (HDAC) inhibitor; 7) a Janus kinase inhibitor; 8) an fibroblast growth factor receptor (FGF) receptor inhibitor); 9) an epidermal growth factor (EGF) receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor, or 17) a BCR-ABL inhibitor. In one embodiment, one or more of the aforesaid combinations is used to treat a disorder, *e.g.,* a disorder described herein (*e.g.,* a disorder disclosed in Table 1). In one embodiment, one or more of the aforesaid combinations is used to treat a cancer, *e.g.,* a cancer described herein (*e.g.,* a cancer disclosed in Table 1). Each of these combinations is discussed in more detail below.

In one embodiment, the inhibitor of the immune checkpoint molecule (alone or in combination with other immunomodulators) is used in combination with an IAP inhibitor to treat a cancer, *e.g.,* a cancer described herein (*e.g.,* a cancer disclosed in Table 1). In one embodiment, the IAP inhibitor is disclosed herein, *e.g.,* in Table 1. In one embodiment, the IAP inhibitor is LCL161 as disclosed herein, or in a publication recited in Table 1. In certain embodiments, the IAP inhibitor is disclosed, *e.g.,* in U.S. Patent No. 8,546,336. In one embodiment, LCL161 has the structure provided in Table 1, or as disclosed in the publication recited in Table 1. In one embodiment, the inhibitor of the immune checkpoint molecule (*e.g.,* one of Nivolumab, Pembrolizumab or MSB0010718C) is used in combination with LCL161 to treat a cancer or disorder described herein, *e.g.,* in Table 1, *e.g.,* a solid tumor, *e.g.,* a breast cancer, colon cancer, or a pancreatic cancer; or a hematological malignancy, *e.g.,* multiple myeloma or a hematopoeisis disorder.

In an embodiment, the inhibitor of the immune checkpoint molecule (alone or in combination with other immunomodulators) is used in combination with LCL161, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide.

In an embodiment, LCL161 is administered at a dose (*e.g.,* oral dose) of about 10-3000 mg, *e.g.,* about 20-2400 mg, about 50-1800 mg, about 100-1500 mg, about 200-1200 mg, about 300-900 mg, *e.g.,* about 600 mg, about 900 mg, about 1200 mg, about 1500 mg, about 1800 mg, about 2100 mg, or about 2400 mg. In an embodiment, LCL161 is administered once a week or once every two weeks. In an embodiment, LCL161 is administered prior to administration of the immune checkpoint inhibitor *(e.g.,* the anti-PD-1 antibody). For example, LCL161 can be administered one, two, three, four or five days or more before the anti-PD-1 antibody is administered. In another embodiment, LCL161 is administred concurrently or substantially concurrently (*e.g.,* on the same day) with the anti-PD-1 antibody. In yet another embodiment, LCL161 is administered after administration of the immune checkpoint inhibitor (*e.g.,* the anti-PD-1 antibody).

In one embodiment, the inhibitor of the immune checkpoint molecule (alone or in combination with other immunomodulators) is used in combination with a TOR kinase inhibitor to treat a cancer, *e.g.,* a cancer described herein (*e.g.,* a cancer disclosed in Table 1). In one embodiment, the TOR kinase inhibitor is disclosed herein, *e.g.,* in Table 1. In one embodiment, the TOR kinase inhibitor is Rad-001 as disclosed herein, or in a publication recited in Table 1. In certain embodiments, the TOR kinase inhibitor is disclosed, *e.g.,* in International Patent Publication No. 2014/085318. In one embodiment, Rad-001 has the structure provided in Table 1, or as disclosed in the publication recited in Table 1. In one embodiment, the inhibitor of the immune checkpoint molecule (*e.g.,* one of Nivolumab, Pembrolizumab or MSB0010718C) is used in combination with Rad-001 to treat a cancer or disorder described herein, *e.g.,* in Table 1, *e.g.,* a solid tumor, *e.g.,* a sarcoma, a lung cancer (*e.g.,* a non-small cell lung cancer (NSCLC) (*e.g.,* a NSCLC with squamous and/or non-squamous histology)), a melanoma (*e.g.,* an advanced melanoma), a digestive/gastrointestinal cancer, a gastric cancer, a neurologic cancer, a prostate cancer, a bladder cancer, a breast cancer; or a hematological malignancy, *e.g.,* a lymphoma or leukemia.

In an embodiment, the inhibitor of the immune checkpoint molecule (alone or in combination with other immunomodulators) is used in combination with Rad-001, wherein Rad-001 is ((1R, 9S, 12S, 15R, 16E, 18R, 19R, 21R, 23S, 24E, 26E, 28E, 30S, 32S, 35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone).

In one embodiment, the inhibitor of the immune checkpoint molecule (alone or in combination with other immunomodulators) is used in combination with a HDM2 ligase inhibitor to treat a cancer, *e.g.,* a cancer described herein (*e.g.,* a cancer disclosed in Table 1). In one embodiment, the HDM2 ligase inhibitor is disclosed herein, *e.g.,* in Table 1. In one embodiment, the HDM2 ligase inhibitor is CGM097 as disclosed herein, or in a publication recited in Table 1. In certain embodiments, the HDM2 ligase inhibitor is disclosed, *e.g.,* in International Patent Publication No. 2011/076786. In one embodiment, CGM097 has the structure provided herein, *e.g.,* in Table 1, or as disclosed in the publication recited in Table 1. In one embodiment, the inhibitor of the immune checkpoint molecule (*e.g.,* one of Nivolumab, Pembrolizumab or MSB0010718C) is used in combination with CGM097 to treat a cancer or disorder described herein, *e.g.,* in Table 1, *e.g.,* a solid tumor.

In an embodiment, the inhibitor of the immune checkpoint molecule (alone or in combination with other immunomodulators) is used in combination with CGM097, wherein CGM097 is (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one.

In one embodiment, the inhibitor of the immune checkpoint molecule (alone or in combination with other immunomodulators) is used in combination with a PIM kinase inhibitor to treat a cancer, *e.g.,* a cancer described herein (*e.g.,* a cancer disclosed in Table 1). In one embodiment, the PIM kinase inhibitor is LGH447 (also known as PIM447) disclosed herein, *e.g.,* in Table 1. In one embodiment, the PIM kinase inhibitor is disclosed in a publication recited in Table 1. In certain embodiments, the PIM kinase inhibitor is disclosed, *e.g.,* in International Patent Publication No. 2010/026124, European Patent Application No. EP2344474, and U.S. Patent Publication No. 2010/0056576. In one embodiment, the PIM kinase inhibitor has the structure provided in Table 1, or as disclosed in the publication recited in Table 1. In one embodiment, the inhibitor of the immune checkpoint molecule (*e.g.,* one of Nivolumab, Pembrolizumab or MSB0010718C) is used in combination with the PIM kinase inhibitor to treat a cancer or disorder described herein, *e.g.,* in Table 1, *e.g.,* hematological malignancy, *e.g.,* multiple myeloma, myelodysplastic syndrome, myeloid leukemia, or non-Hodgkin's lymphoma.

In an embodiment, the inhibitor of the immune checkpoint molecule (alone or in combination with other immunomodulators) is used in combination with LGH447, wherein LGH447 is N-(4-((1R,3S,5S)-3-amino-5-methylcyclohexyl)pyridin-3-yl)-6-(2,6-difluorophenyl)-5-fluoropicolinamide.

In one embodiment, the inhibitor of the immune checkpoint molecule (alone or in combination with other immunomodulators) is used in combination with a HER3 kinase inhibitor to treat a cancer, *e.g.,* a cancer described herein (*e.g.,* a cancer disclosed in Table 1). In one embodiment, the HER3 kinase inhibitor is disclosed herein, *e.g.,* in Table 1. In one embodiment, the HER3 kinase inhibitor is LJM716 as disclosed herein, or in a publication recited in Table 1. In certain embodiments, the HER3 kinase inhibitor is disclosed, *e.g.,* in International Patent Publication No. 2012/022814 and U.S. Patent No. 8,735,551. In one embodiment, LJM716 has the structure provided in Table 1, or as disclosed in the publication recited in Table 1. In one embodiment, the anti-HER3 monoclonal antibody or antigen binding fragment thereof, comprises a VH of SEQ ID NO: 141 and VL of SEQ ID NO: 140, as described in U.S. 8,735,551. In one embodiment, the inhibitor of the immune checkpoint molecule (*e.g.,* one of Nivolumab, Pembrolizumab or MSB0010718C) is used in combination with LJM716 to treat a cancer or disorder described herein, *e.g.,* in Table 1, *e.g.,* a solid tumor, *e.g.* a gastric cancer, an esophageal cancer, a breast cancer, a head and neck cancer, a stomach cancer, or a digestive/gastrointestinal cancer therapy.

In one embodiment, the inhibitor of the immune checkpoint molecule (alone or in combination with other immunomodulators) is used in combination with a HDAC inhibitor to treat a cancer, *e.g.,* a cancer described herein (*e.g.,* a cancer disclosed in Table 1). In one embodiment, the HDAC inhibitor is disclosed herein, *e.g.,* in Table 1. In one embodiment, the HDAC inhibitor is LBH589 as disclosed herein, or in a publication recited in Table 1. In certain embodiments, the HDAC inhibitor is disclosed, *e.g.,* in International Patent Publication Nos. 2014/072493 and 2002/022577 and European Patent Application No. EP1870399. In one embodiment, LBH589 has the structure provided in Table 1, or as disclosed in the publication recited in Table 1. In one embodiment, the inhibitor of the immune checkpoint molecule *(e.g.,* one of Nivolumab, Pembrolizumab or MSB0010718C) is used in combination with LBH589 to treat a cancer or disorder described herein, *e.g.,* in Table 1, *e.g.,* a solid tumor,*e.g.*, a bone cancer, a small cell lung cancer, a respiratory/thoracic cancer a prostate cancer, a non-small cell lung cancer (NSCLC), a nerologic cancer, a gastric cancer, a melanoma, a breast cancer, a pancreatic cancer, a colorectal cancer, a renal cancer, or a head and neck cancer, or a liver cancer; or a hematological malignancy, *e.g.,* multiple myeloma, a hematopoeisis disorder, myelodysplastic syndrome, lymphoma (*e.g.,* non-Hodgkin's lymphoma), or leukemia (*e.g.,* myeloid leukemia).

In an embodiment, the inhibitor of the immune checkpoint molecule (alone or in combination with other immunomodulators) is used in combination with LBH589, wherein LBH589 is (E)-N-hydroxy-3-(4-(((2-(2-methyl-1H-indol-3-yl)ethyl)amino)methyl)phenyl) acrylamide.

In one embodiment, the inhibitor of the immune checkpoint molecule (alone or in combination with other immunomodulators) is used in combination with a Janus kinase inhibitor to treat a cancer, *e.g.,* a cancer described herein (*e.g.,* a cancer disclosed in Table 1). In one embodiment, the Janus kinase inhibitor is disclosed herein, *e.g.,* in Table 1. In one embodiment, the Janus kinase inhibitor is INC424 as disclosed herein, or in a publication recited in Table 1. In certain embodiments, the Janus kinase inhibitor is disclosed, *e.g.,* in International Patent Publication Nos. 2007/070514 and 2014/018632, European Patent Application No. EP2474545, and U.S. Patent No. 7,598,257. In one embodiment, INC424 has the structure provided herein, *e.g.,* in Table 1, or as disclosed in the publication recited in Table 1. In one embodiment, the inhibitor of the immune checkpoint molecule (*e.g.,* one of Nivolumab, Pembrolizumab or MSB0010718C) is used in combination with INC424 to treat a cancer or disorder described herein, *e.g.,* in Table 1, *e.g.,* a solid tumor, *e.g.*, a prostate cancer, a lung cancer, a breast cancer, a pancreatic cancer, a colorectal cancer; or a hematological malignancy, *e.g.,* multiple myeloma, lymphoma (*e.g.,* non-Hodgkin lymphoma), or leukemia (*e.g.,* myeloid leukemia, lymphocytic leukemia). In some embodiments, the cancer has, or is identified as having, a JAK mutation. In some embodiments, the JAK mutation is a JAK2 V617F mutation.

In an embodiment, the inhibitor of the immune checkpoint molecule (alone or in combination with other immunomodulators) is used in combination with INC424, wherein INC424 is (3R)-3-cyclopentyl-3-[4-(7H-pyrrolo-[2,3-d]pyrimidin-4-yl)-1H-pyrazol-1-yl]propanenitrile.

In one embodiment, the inhibitor of the immune checkpoint molecule (alone or in combination with other immunomodulators) is used in combination with an FGF receptor inhibitor to treat a cancer, *e.g.,* a cancer described herein (*e.g.,* a cancer disclosed in Table 1). In one embodiment, the FGF receptor inhibitor is disclosed herein, *e.g.,* in Table 1. In one embodiment, the FGF receptor inhibitor is BUW078 or BGJ398 as disclosed herein, or in a publication recited in Table 1. In one embodiment, the FGF receptor inhibitor, *e.g.,* BUW078 or BGJ398, has the structure (compound or generic structure) provided herein, *e.g.,* in Table 1, or as disclosed in the publication recited in Table 1. In one embodiment, one of Nivolumab, Pembrolizumab or MSB0010718C is used in combination with BUW078 or BGJ398 to treat a cancer described herein, *e.g.,* in Table 1, *e.g.,* a solid tumor, *e.g.,* a digestive/gastrointestinal cancer; or a hematological cancer.

In an embodiment, the inhibitor of the immune checkpoint molecule (alone or in combination with other immunomodulators) is used in combination with BUW078, wherein BUW078 is 8-(2,6-difluoro-3,5-dimethoxy-phenyl)-quinoxaline-5-carboxylic acid (4-dimethylaminomethyl-1H-imidazol-2-yl)-amide.

In some embodiments, any of the aforesaid combinations can further include one or more of the second agents described herein below, *e.g.,* one or more of the additional compounds shown in Table 1 (*e.g.,* one or more of: an EGF receptor inhibitor, a c-MET inhibitor, an ALK inhibitor, a CDK4/6 inhibitor, a PI3K inhibitor, a BRAF inhibitor, a CAR T cell inhibitor, a MEK inhibitor or a BCR-ABL inhibitor as described herein).

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with an EGF receptor inhibitor to treat a cancer, *e.g.,* a cancer described herein (*e.g.,* a cancer disclosed in Table 1). In one embodiment, the EGF receptor inhibitor is disclosed herein, *e.g.,* in Table 1. In one embodiment, the EGF receptor inhibitor is EGF816, or as provided herein (*e.g.,* a publication recited in Table 1). In one embodiment, the EGF receptor inhibitor, *e.g.,* EGF816, has the structure (compound or generic structure) provided herein, *e.g.,* in Table 1, or as disclosed in the publication recited in Table 1. In one embodiment, one of Nivolumab, Pembrolizumab or MSB0010718C is used in combination with EGF816 to treat a cancer described herein, *e.g.,* in Table 1, *e.g.,* a solid tumor, *e.g.,* a lung cancer *(e.g.,* non-small cell lung cancer (NSCLC)), a lymphoma, or a neuroblastoma.

In one embodiment, the cancer is NSCLC and is characterized by one or more of: aberrant activation, amplification, or a mutation of epidermal growth factor receptor (EGFR). In certain embodiments the cancer is NSCLC wherein the NSCLC is characterized by harbouring an EGFR exon 20 insertion, an EGFR exon 19 deletion, EGFR L858R mutation, EGFR T790M, or any combination thereof. In some embodiments, the combination is for use in the treatment of NSCLC, wherein the NSCLC is characterized by harboring an EGFR exon 20 insertion, an EGFR exon 19 deletion, EGFR L858R mutation, EGFR T790M, or any combination thereof. In some embodiments, the NSCLC is characterized by harboring L858R and T790M mutations of EGFR. In other embodiments, the NSCLC is characterized by harboring an EGFR exon 20 insertion and T790M mutations of EGFR. In yet other embodiments, the NSCLC is characterized by harboring an EGFR exon 19 deletion and T790M mutations of EGFR. In other embodiments, the NSCLC is characterized by harboring EGFR mutation selected from the group consisting of an exon 20 insertion, an exon 19 deletion, L858R mutation, T790M mutation, and any combination thereof.

In some embodiments, the lymphoma (*e.g.,* an anaplastic large-cell lymphoma or non-Hodgkin lymphoma) has, or is identified as having, an ALK translocation, *e.g.,* an EML4-ALK fusion.

In certain embodiments, EGF816 is administered at an oral dose of about 50 to 500 mg, *e.g.,* about 100 mg to 400 mg, about 150 mg to 350 mg, or about 200 mg to 300 mg, *e.g.,* about 100 mg, 150 mg or 200 mg. The dosing schedule can vary from *e.g.,* every other day to daily, twice or three times a day. In one embodiment, EGF816 is administered at an oral dose from about 100 to 200 mg, *e.g.,* about 150 mg, once a day. In some embodiments, EGF816 isadministered at a dose of 75, 100, 150, 225, 150, 200, 225, 300 or 350 mg. These doses may be administered once daily. *E.g.* EGF816 may be administered at a dose of 100 or 150 mg once daily. In embodiments of the combination, Nivolumab is administered in an amount from about 1 mg/kg to 5 mg/kg, *e.g.,* 3 mg/kg, and may be administered over a period of 60 minutes, ca. once a week to once every 2, 3 or 4 weeks.

In another embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with a c-MET inhibitor to treat a cancer, *e.g.,* a cancer described herein (*e.g.,* a cancer disclosed in Table 1). In one embodiment, the c-MET inhibitor is disclosed herein, *e.g.,* in Table 1. In one embodiment, the c-MET inhibitor is INC280 (formerly known as INCB28060) as disclosed herein, or in a publication recited in Table 1. In one embodiment, the c-MET inhibitor, *e.g.,* INC280, has the structure (compound or generic structure) provided herein, *e.g.,* in Table 1, or as disclosed in the publication recited in Table 1. In one embodiment, one of Nivolumab, Pembrolizumab or MSB0010718C is used in combination with INC280 to treat a cancer described in Table 1, *e.g.,* a solid tumor, *e.g.,* a lung cancer (*e.g.,* non-small cell lung cancer (NSCLC)), glioblastoma multiforme (GBM), a renal cancer, a liver cancer (*e.g.,* a hepatocellular carcinoma) or a gastric cancer. In some embodiments, the cancer has, or is identified as having, a c-MET mutation (*e.g.,* a c-MET mutation or a c-MET amplification).

In certain embodiments, INC280 is administered at an oral dose of about 100 to 1000 mg, *e.g.,* about 200 mg to 900 mg, about 300 mg to 800 mg, or about 400 mg to 700 mg, *e.g.,* about 400 mg, 500 mg or 600 mg. The dosing schedule can vary from *e.g.,* every other day to daily, twice or three times a day. In one embodiment, INC280 is administered at an oral dose from about 400 to 600 mg twice a day.

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with an Alk inhibitor to treat a cancer, *e.g.,* a cancer described herein (*e.g.,* a cancer disclosed in Table 1). In one embodiment, the Alk inhibitor is disclosed herein, *e.g.,* in Table 1. In one embodiment, the Alk inhibitor is LDK378 (also known as ceritinib (Zykadia®), *e.g.,* as described herein or in a publication recited in Table 1. In one embodiment, the Alk inhibitor, *e.g.,* LDK378, has the structure (compound or generic structure) provided herein, *e.g.,* in Table 1, or as disclosed in the publication recited in Table 1.

In one embodiment, one of Nivolumab, Pembrolizumab or MSB0010718C is used in combination with LDK378 to treat a cancer described in Table 1, *e.g.,* a solid tumor, *e.g.,* a lung cancer (*e.g.,* non-small cell lung cancer (NSCLC)), a lymphoma (*e.g.,* an anaplastic large-cell lymphoma or non-Hodgkin lymphoma), an inflammatory myofibroblastic tumor (IMT), or a neuroblastoma. In some embodiments, the NSCLC is a stage IIIB or IV NSCLC, or a relapsed locally advanced or metastic NSCLC. In some embodiments, the cancer (*e.g.,* the lung cancer, lymphoma, inflammatory myofibroblastic tumor, or neuroblastoma) has, or is identified as having, an ALK rearrangement or translocation, *e.g.,* an ALK fusion. In one embodiment, the ALK fusion is an EML4-ALK fusion, *e.g.,* an EML4-ALK fusion described herein. In another embodiment, the ALK fusion is an ALK-ROS1 fusion. In certain embodiments, the cancer has progressed on, or is resistant or tolerant to, a ROS1 inhibitor, or an ALK inhibitor, *e.g.,* an ALK inhibitor other than LDK378. In some embodiments, the cancer has progressed on, or is resistant or tolerant to, crizotinib. In one embodiment, the subject is an ALK-naïve patient, *e.g.,* a human patient. In another embodiment, the subject is a patient, *e.g.,* a human patient, that has been pretreated with an ALK inhibitor. In another embodiment, the subject is a patient, *e.g.,* a human patient, that has been pretreated with LDK378.

In one embodiment, LDK378 and Nivolumab are administered to an ALK-naïve patient. In another embodiment, LDK378 and Nivolumab are administered to a patient that has been pretreated with an ALK inhibitor. In yet another embodiment, LDK378 and Nivolumab are administered to a patient that has been pretreated with LDK378.

In certain embodiments, LDK378 is administered at an oral dose of about 100 to 1000 mg, *e.g.,* about 150 mg to 900 mg, about 200 mg to 800 mg, about 300 mg to 700 mg, or about 400 mg to 600 mg, *e.g.,* about 150 mg, 300 mg, 450 mg, 600 mg or 750 mg. In certain embodiment, LDK378 is administered at an oral dose of about 750 mg or lower, *e.g.,* about 600 mg or lower, *e.g.,* about 450 mg or lower. In certain embodiments, LDK378 is administered with food. In other embodiments, the dose is under fasting condition. The dosing schedule can vary from *e.g.,* every other day to daily, twice or three times a day. In one embodiment, LDK378 is administered daily. In one embodiment, LDK378 is administered at an oral dose from about 150 mg to 750 mg daily, either with food or in a fasting condition. In one embodiment, LDK378 is administered at an oral dose of about 750 mg daily, in a fasting condition. In one embodiment, LDK378 is administered at an oral dose of about 750 mg daily, via capsule or tablet. In another embodiment, LDK378 is administered at an oral dose of about 600 mg daily, via capsule or tablet. In one embodiment, LDK378 is administered at an oral dose of about 450 mg daily, via capsule or tablet.

In one embodiment, LDK378 is administered at a dose of about 450 mg and nivolumab is administered at a dose of about 3 mg/kg. In another embodiment, the LDK378 dose is 600 mg and the nivolumab dose is 3 mg/kg. In one embodiment, LDK378 is administered with a low fat meal.

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with a CDK4/6 inhibitor to treat a cancer, *e.g.,* a cancer described herein (*e.g.,* a cancer disclosed in Table 1). In one embodiment, the CDK4/6 inhibitor is disclosed herein, *e.g.,* in Table 1. In one embodiment, LEE011 (also knows as Ribociclib®), *e.g.,* as described herein or in a publication recited in Table 1. In one embodiment, the CDK4/6 inhibitor, *e.g.,* LEE011, has the structure (compound or generic structure) provided herein, *e.g.,* in Table 1, or as disclosed in the publication recited in Table 1. In one embodiment, one of Nivolumab, Pembrolizumab or MSB0010718C is used in combination with LEE011 to treat a cancer described in Table 1, *e.g.,* a solid tumor, *e.g.,* a lung cancer (*e.g.,* non-small cell lung cancer (NSCLC)), a neurologic cancer, melanoma or a breast cancer, or a hematological malignancy, *e.g.,* lymphoma.

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with a PI3K-inhibitor to treat a cancer, *e.g.,* a cancer described herein (*e.g.,* a cancer disclosed in Table 1). In one embodiment, the PI3K inhibitor is disclosed herein, *e.g.,* in Table 1. In one embodiment, the PI3K inhibitor is BKM120 or BYL719, *e.g.,* disclosed herein or in a publication recited in Table 1. In one embodiment, the PI3K-inhibitor, *e.g.,* BKM120 or BYL719, has the structure (compound or generic structure) provided herein, *e.g.,* in Table 1, or as disclosed in the publication recited in Table 1. In one embodiment, one of Nivolumab, Pembrolizumab or MSB0010718C is used in combination with BKM120 or BYL719 to treat a cancer or disorder described herein, *e.g.,* in Table 1. In some embodiments, the cancer or disorder is chosen from, *e.g.,* a solid tumor, *e.g.,* a lung cancer (*e.g.,* non-small cell lung cancer (NSCLC)), a prostate cancer, an endocrine cancer, an ovarian cancer, a melanoma, a bladder cancer, a female reproductive system cancer, a digestive/gastrointestinal cancer, a colorectal cancer, glioblastoma multiforme (GBM), a head and neck cancer, a gastric cancer, a pancreatic cancer or a breast cancer; or a hematological malignancy, *e.g.,* leukemia, non-Hodgkin lymphoma; or a hematopoiesis disorder.

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with a BRAF inhibitor to treat a cancer, *e.g.,* a cancer described herein (*e.g.,* a cancer disclosed in Table 1). In one embodiment, the BRAF inhibitor is disclosed herein, *e.g.,* in Table 1. In one embodiment, the BRAF inhibitor is LGX818, *e.g.,* as described herein or in a publication recited in Table 1. In one embodiment, the BRAF inhibitor, *e.g.,* LGX818, has the structure (compound or generic structure) provided herein, *e.g.,* in Table 1, or as disclosed in the publication recited in Table 1. In one embodiment, one of Nivolumab, Pembrolizumab or MSB0010718C is used in combination with LGX818 to treat a cancer described in Table 1, *e.g.,* a solid tumor, *e.g.,* a lung cancer (*e.g.,* non-small cell lung cancer (NSCLC)), a melanoma, *e.g.,* advanced melanoma, a thyroid cancer, *e.g,* papillary thyroid cancer, or a colorectal cancer. In some embodiments, the cancer has, or is identified as having, a BRAF mutation (*e.g.,* a BRAF V600E mutation), a BRAF wildtype, a KRAS wildtype or an activating KRAS mutation. The cancer may be at an early, intermediate or late stage.

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with a CAR T cell targeting CD19 to treat a cancer, *e.g.,* a cancer described herein (*e.g.,* a cancer disclosed in Table 1). In one embodiment, the CAR T cell targeting CD19 is disclosed in Table 1, *e.g.,* CTL019, or in a publication recited in Table 1. In one embodiment, the CAR T cell targeting CD19, *e.g.,* CTL019, has the structure (compound or generic structure) provided herein, *e.g.,* in Table 1, or as disclosed in the publication recited in Table 1. In one embodiment, one of Nivolumab, Pembrolizumab or MSB0010718C is used in combination with CTL019 to treat a cancer described in Table 1, *e.g.,* a solid tumor, or a hematological malignancy, *e.g.,* a lymphocytic leukemia or a non-Hodgkin lymphoma.

In one embodiment, the CAR T cell targeting CD19 has the USAN designation TISAGENLECLEUCEL-T. CTL019 is made by a gene modification of T cells is mediated by stable insertion via transduction with a self-inactivating, replicationdeficient Lentiviral (LV) vector containing the CTL019 transgene under the control of the EF-1 alpha promoter. CTL019 is a mixture of transgene positive and negative T cells that are delivered to the subject on the basis of percent transgene positive T cells.

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with a MEK inhibitor to treat a cancer, *e.g.,* a cancer described herein (*e.g.,* a cancer disclosed in Table 1). In one embodiment, the MEK inhibitor is disclosed herein, *e.g.,* in Table 1. In one embodiment, the MEK inhibitor is MEK162, *e.g.,* disclosed herein or in a publication recited in Table 1. In one embodiment, the MEK inhibitor, *e.g.,* MEK162, has the structure (compound or generic structure) provided herein, *e.g.,* in Table 1, or as disclosed in the publication recited in Table 1. In one embodiment, one of Nivolumab, Pembrolizumab or MSB0010718C is used in combination with MEK162 to treat a cancer described in Table 1. In other embodiments, the cancer or disorder treated with the combination is chosen from a melanoma, a colorectal cancer, a non-small cell lung cancer, an ovarian cancer, a breast cancer, a prostate cancer, a pancreatic cancer, a hematological malignancy or a renal cell carcinoma, a multisystem genetic disorder, a digestive/gastrointestinal cancer, a gastric cancer, or a colorectal cancer; or rheumatoid arthritis. In some embodiments, the cancer has, or is identified as having, a KRAS mutation.

In one embodiment, the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C), (alone or in combination with other immunomodulators) is used in combination with a BCR-ABL inhibitor to treat a cancer, *e.g.,* a cancer described herein (*e.g.,* a cancer disclosed in Table 1). In one embodiment, the BCR-ABL inhibitor is disclosed herein, *e.g.,* in Table 1. In one embodiment, the BCR-ABL inhibitor is AMN-107 (also known as Nilotinib, trade name Tasigna), *e.g.,* disclosed herein or in a publication recited in Table 1. In one embodiment, AMN-107 has the structure (compound or generic structure) provided herein, *e.g.,* in Table 1, or as disclosed in the publication recited in Table 1. In one embodiment, one of Nivolumab, Pembrolizumab or MSB0010718C is used in combination with AMN-107 to treat a cancer or disorder described in Table 1, *e.g.,* a solid tumor, *e.g.,* a neurologic cancer, a melanoma, a digestive/gastrointestinal cancer, a colorectal cancer, a head and neck cancer; or a hematological malignancy, *e.g.,* chronic myelogenous leukemia (CML), a lymphocytic leukemia, a myeloid leukemia; Parkinson's disease; or pulmonary hypertension.

### Cancers and Subjects

In certain embodiments of the compositions and methods described herein, the proliferative disorder or condition, *e.g.,* the cancer, includes but is not limited to, a solid tumor, a soft tissue tumor (*e.g.,* a hematological cancer, leukemia, lymphoma, or myeloma), and a metastatic lesion of any of the aforesaid cancers. In one embodiment, the cancer is a solid tumor. Examples of solid tumors include malignancies, *e.g.,* sarcomas, adenocarcinomas, and carcinomas, of the various organ systems, such as those affecting the lung, breast, ovarian, lymphoid, gastrointestinal (*e.g.,* colon), anal, genitals and genitourinary tract (*e.g.,* renal, urothelial, bladder cells, prostate), pharynx, CNS (*e.g.,* brain, neural or glial cells), head and neck, skin (*e.g.,* melanoma), and pancreas, as well as adenocarcinomas which include malignancies such as colon cancers, rectal cancer, renal-cell carcinoma, liver cancer, non-small cell lung cancer, cancer of the small intestine and cancer of the esophagus. The cancer may be at an early, intermediate, late stage or metastatic cancer.

In one embodiment, the cancer is chosen from a cancer disclosed in Table 1. For example, the cancer can be chosen from a solid tumor, *e.g.,* a lung cancer (*e.g.,* a non-small cell lung cancer (NSCLC) (*e.g.,* a NSCLC with squamous and/or non-squamous histology)), a colorectal cancer, a melanoma (*e.g.,* an advanced melanoma), a head and neck cancer (*e.g.,* head and neck squamous cell carcinoma (HNSCC), a digestive/gastrointestinal cancer, a gastric cancer, a neurologic cancer, a glioblastoma (*e.g.,* glioblastoma multiforme), an ovarian cancer, a renal cancer, a liver cancer, a pancreatic cancer, a prostate cancer, a liver cancer; a breast cancer, an anal cancer, a gastro-esophageal cancer, a thyroid cancer, a cervical cancer; or a hematological cancer (*e.g.,* chosen from a Hogdkin lymphoma, a non-Hodgkin lymphoma, a lymphocytic leukemia, or a myeloid leukemia).

In one embodiment, the cancer is a colon cancer, *e.g.,* a colon cancer that expresses an IAP, *e.g.,* a human IAP. The human IAP family includes, *e.g.,* NAIP, XIAP, cIAP1, cIAP2, ILP2, BRUCE, surviving, and livin.

In one embodiment, the cancer is a non-small cell lung cancer (NSCLC), *e.g.,* an ALK+ NSCLC. As used herein, the term "ALK+ non-small cell lung cancer" or "ALK+ NSCLC" refers to an NSCLC that has an activated (*e.g.,* constitutively activated) anaplastic lymphoma kinase activity or has a rearrangement or translocation of an Anaplastic Lymphoma Kinase (ALK) gene. Typically, compared with the general NSCLC population, patients with ALK+ NSCLC are generally younger, have light (*e.g.,* < 10 pack years) or no smoking history, present with lower Eastern Cooperative Oncology Group performance status, or may have more aggressive disease and, therefore, experience earlier disease progression (Shaw et al. J Clin Oncol. 2009; 27(26):4247-4253; Sasaki et al. Eur J Cancer. 2010; 46(10):1773-1780; Shaw et al. N Engl J Med. 2013;368(25):2385-2394; Socinski et al. J Clin Oncol. 2012; 30(17):2055-2062; Yang et al. J Thorac Oncol. 2012;7(1):90-97).

In one embodiment, the cancer, *e.g.,* an NSCLC, has a rearrangement or translocation of an ALK gene. In one embodiment, the rearrangement or translocation of the ALK gene leads to a fusion (*e.g.,* fusion upstream of the ALK promoter region). In certain embodiments, the fusion results in constitutive activation of the kinase activity.

In one embodiment, the fusion is an EML4-ALK fusion. Exemplary EML4-ALK fusion proteins include, but are not limited to, E13;A20 (V1), E20;A20 (V2), E6a/b;A20 (V3a/b), E14;A20 (V4), E2a/b;A20 (V5a/b), E13b;A20 (V6), E14;A20(V7), E15;A20("V4"), or E18;A20 (V5) (Choi et al. Cancer Res. 2008; 68(13):4971-6; Horn et al. J Clin Oncol. 2009; 27(26):4232-5; Koivunen et al. Clin Cancer Res. 2008; 14(13):4275-83; Soda et al. Nature. 2007; 448(7153):561-6; Takeuchi et al. Clin Cancer Res. 2008; 14(20):6618-24; Takeuchi et al. Clin Cancer Res. 2009; 15(9):3143-9; Wong et al. Cancer. 2009 Apr 15;115(8):1723-33).

In certain embodiments, the ALK gene is fused to a non-EML4 partner. In one embodiment, the fusion is a KIF5B-ALK fusion. In another embodiment, the fusion is a TFG-ALK fusion. Exemplary KIF5B-ALK and TFG-ALK fusions are described, *e.g.,* in Takeuchi et al. Clin Cancer Res. 2009; 15(9):3143-9, Rikova et al. Cell. 2007; 131(6):1190-203.

ALK gene rearrangements or translocations, or cancer cells that has an ALK gene rearrangement or translocation, can be detected, *e.g.,* using fluorescence *in situ* hybridization (FISH), *e.g.,* with an ALK break apart probe.

Methods and compositions disclosed herein are useful for treating metastatic lesions associated with the aforementioned cancers

In other embodiments, the subject is a mammal, *e.g.,* a primate, preferably a higher primate, *e.g.,* a human (*e.g.,* a patient having, or at risk of having, a disorder described herein). In one embodiment, the subject is in need of enhancing an immune response. In one embodiment, the subject has, or is at risk of, having a disorder described herein, *e.g.,* a cancer as described herein. In certain embodiments, the subject is, or is at risk of being, immunocompromised. For example, the subject is undergoing or has undergone a chemotherapeutic treatment and/or radiation therapy. Alternatively, or in combination, the subject is, or is at risk of being, immunocompromised as a result of an infection.

In one embodiment, the subject (*e.g.,* a subject having a lung cancer (*e.g.,* a non-small cell lung cancer), a lymphoma (*e.g.,* an anaplastic large-cell lymphoma or non-Hodgkin lymphoma), an inflammatory myofibroblastic tumor, or a neuroblastoma) is being treated, or has been treated, with another ALK inhibitor and/or a ROS1 inhibitor, *e.g.,* crizotinib. For example, crizotinib can be administered at a daily oral dose of 750 mg or lower, *e.g.,* 600 mg or lower, *e.g.,* 450 mg or lower.

In another embodiment, the subject or cancer (*e.g.,* a lung cancer (*e.g.,* a non-small cell lung cancer), a lymphoma (*e.g.,* an anaplastic large-cell lymphoma or non-Hodgkin lymphoma), an inflammatory myofibroblastic tumor, or a neuroblastoma) has progressed on, or is resistant or tolerant to, another ALK inhibitor and/or a ROS1 inhibitor, *e.g.,* crizotinib.

In yet another embodiment, the subject or cancer (*e.g.,* a lung cancer (*e.g.,* a non-small cell lung cancer), a lymphoma (*e.g.,* an anaplastic large-cell lymphoma or non-Hodgkin lymphoma), an inflammatory myofibroblastic tumor, or a neuroblastoma) is at risk of progression on, or developing resistance or tolerance to, another ALK inhibitor and/or a ROS1 inhibitor, *e.g.,* crizotinib.

In other embodiments, the subject or cancer is resistant or tolerant, or is at risk of developing resistance or tolerance, to a tyrosine kinase inhibitor (TKI), *e.g.,* an EGFR tyrosine kinase inhibitor.

In some embodiments, the subject or cancer has no detectable EGFR mutation, KRAS mutation, or both.

In some embodiments, the subject has previously been treated with PD-1.

In some embodiments, the subject has or is identified as having a tumor that has one or more of high PD-L1 level or expression and/or Tumor Infiltrating Lymphocyte (TIL)+. In certain embodiments, the subject has or is identified as having a tumor that has high PD-L1 level or expression and TIL+. In some embodiments, the methods described herein further describe identifying a subject based on having a tumor that has one or more of high PD-L1 level or expression and/or TIL+. In certain embodiments, the methods described herein further describe identifying a subject based on having a tumor that has high PD-L1 level or expression and TIL+. In some embodiments, tumors that are TIL+ are positive for CD8 and IFNγ. In some embodiments, the subject has or is identified as having a high percentage of cells that are positive for one or more of PD-L1, CD8, and/or IFNγ. In certain embodiments, the subject has or is identified as having a high percentage of cells that are positive for all of PD-L1, CD8, and IFNγ.

In some embodiments, the methods described herein further describe identifying a subject based on having a high percentage of cells that are positive for one or more of PD-L1, CD8, and/or IFNγ. In certain embodiments, the methods described herein further describe identifying a subject based on having a high percentage of cells that are positive for all of PD-L1, CD8, and IFNγ. In some embodiments, the subject has or is identified as having one or more of PD-L1, CD8, and/or IFNγ, and one or more of a lung cancer, *e.g.,* squamous cell lung cancer or lung adenocarcinoma; a head and neck cancer; a squamous cell cervical cancer; a stomach cancer; a thyroid cancer; and/or a melanoma. In certain embodiments, the methods described herein further describe identifying a subject based on having one or more of PD-L1, CD8, and/or IFNγ, and one or more of a lung cancer, *e.g.,* squamous cell lung cancer or lung adenocarcinoma; a head and neck cancer; a squamous cell cervical cancer; a stomach cancer; a thyroid cancer; and/or a melanoma.

### Dosages and Administration

Dosages and therapeutic regimens of the agents described herein can be determined by a skilled artisan.

In certain embodiments, the anti-PD-1 antibody molecule is administered by injection (*e.g.,* subcutaneously or intravenously) at a dose of about 1 to 30 mg/kg, *e.g.,* about 5 to 25 mg/kg, about 10 to 20 mg/kg, about 1 to 5 mg/kg, or about 3 mg/kg. The dosing schedule can vary from *e.g.,* once a week to once every 2, 3, or 4 weeks. In one embodiment, the anti-PD-1 antibody molecule is administered at a dose from about 10 to 20 mg/kg every other week.

In one embodiment, the anti-PD-1 antibody molecule, *e.g.,* Nivolumab, is administered intravenously at a dose from about 1 mg/kg to 3 mg/kg, *e.g.,* about 1 mg/kg, 2 mg/kg or 3 mg/kg, every two weeks. In one embodiment, the anti-PD-1 antibody molecule, *e.g.,* Nivolumab, is administered intravenously at a dose of about 2 mg/kg at 3-week intervals. In one embodiment, Nivolumab is administered in an amount from about 1 mg/kg to 5 mg/kg, *e.g.,* 3 mg/kg, and may be administered over a period of 60 minutes, ca. once a week to once every 2, 3 or 4 weeks.

The combination therapies described herein can be administered to the subject systemically (*e.g.,* orally, parenterally, subcutaneously, intravenously, rectally, intramuscularly, intraperitoneally, intranasally, transdermally, or by inhalation or intracavitary installation), topically, or by application to mucous membranes, such as the nose, throat and bronchial tubes.

In one embodiment, the anti-PD-1 antibody molecule is administered intravenously. In one embodiment, in a combination therapy, one or more of the agents listed in Table 1, *e.g.,* an IAP inhibitor or LCL161, is administered orally. In one embodiment, the anti-PD-1 antibody molecule is administered, *e.g.,* intravenously, at least one, two, three, four, five, six, or seven days, *e.g.,* three days, after an agent listed in Table 1, *e.g.,* an IAP inhibitor or LCL161, is administered, *e.g.,* orally. In one embodiment, the anti-PD-1 antibody molecule is administered, *e.g.,* intravenously, at least one, two, three, four, five, six, or seven days, *e.g.,* three days, before an agent listed in Table 1, *e.g.,* an IAP inhibitor or LCL161, is administered, *e.g.,* orally. In yet another embodiment, the anti-PD-1 antibody molecule is administered, *e.g.,* intravenously, on the same day, as the one or more agents listed in Table 1, *e.g.,* an IAP inhibitor or LCL161, is administered, e.g., orally. In one embodiment, the administration of the anti-PD-1 antibody molecule and one or more of the agents listed in Table 1, *e.g.,* an IAP inhibitor or LCL161, results in an enhanced reduction of a solid tumor, *e.g.,* colon cancer, relative to administration of each of these agents as a monotherapy. In certain embodiments, in a combination therapy, the concentration of an agent listed in Table 1, *e.g.,* an IAP inhibitor or LCL161, that is required to achieve inhibition, *e.g.,* growth inhibition, is lower than the therapeutic dose of the agent as a monotherapy, *e.g.,* 10-20%, 20-30%, 30-40%, 40-50%, 50-60%, 60-70%, 70-80%, or 80-90% lower. In other embodiments, in a combination therapy, the concentration of the anti-PD-1 antibody molecule that is required to achieve inhibition, *e.g.,* growth inhibition, is lower than the therapeutic dose of the anti-PD-1 antibody molecule as a monotherapy, *e.g.,* 10-20%, 20-30%, 30-40%, 40-50%, 50-60%, 60-70%, 70-80%, or 80-90% lower.

The methods and compositions described herein can be used in combination with further agents or therapeutic modalities. The combination therapies can be administered simultaneously or sequentially in any order. Any combination and sequence of the anti-PD-1 or PD-L1 antibody molecules and other therapeutic agents, procedures or modalities (*e.g.,* as described herein) can be used. The combination therapies can be administered during periods of active disorder, or during a period of remission or less active disease. The combination therapies can be administered before the other treatment, concurrently with the treatment, post-treatment, or during remission of the disorder.

In certain embodiments, the methods and compositions described herein are administered in combination with one or more of other antibody molecules, chemotherapy, other anti-cancer therapy (*e.g.,* targeted anti-cancer therapies, gene therapy, viral therapy, RNA therapy bone marrow transplantation, nanotherapy, or oncolytic drugs), cytotoxic agents, immune-based therapies (*e.g.,* cytokines or cell-based immune therapies), surgical procedures (*e.g.,* lumpectomy or mastectomy) or radiation procedures, or a combination of any of the foregoing. The additional therapy may be in the form of adjuvant or neoadjuvant therapy. In some embodiments, the additional therapy is an enzymatic inhibitor (*e.g.,* a small molecule enzymatic inhibitor) or a metastatic inhibitor. Exemplary cytotoxic agents that can be administered in combination with include antimicrotubule agents, topoisomerase inhibitors, anti-metabolites, mitotic inhibitors, alkylating agents, anthracyclines, vinca alkaloids, intercalating agents, agents capable of interfering with a signal transduction pathway, agents that promote apoptosis, proteosome inhibitors, and radiation (*e.g.,* local or whole body irradiation (*e.g.,* gamma irradiation). In other embodiments, the additional therapy is surgery or radiation, or a combination thereof. In other embodiments, the additional therapy is a therapy targeting an mTOR pathway, an HSP90 inhibitor, or a tubulin inhibitor.

Alternatively, or in combination with the aforesaid combinations, the methods and compositions described herein can be administered in combination with one or more of: a vaccine, *e.g.,* a therapeutic cancer vaccine; or other forms of cellular immunotherapy.

In another embodiment, the combination therapy is used in combination with one, two or all of oxaliplatin, leucovorin or 5-FU (*e.g.,* a FOLFOX co-treatment). Alternatively or in combination, combination further includes a VEGF inhibitor (*e.g.,* a VEGF inhibitor as disclosed herein). In some embodiments, the cancer treated with the combination is chosen from a melanoma, a colorectal cancer, a non-small cell lung cancer, an ovarian cancer, a breast cancer, a prostate cancer, a pancreatic cancer, a hematological malignancy or a renal cell carcinoma. The cancer may be at an early, intermediate or late stage.

In other embodiments, the combination therapy is administered with a tyrosine kinase inhibitor (*e.g.,* axitinib) to treat renal cell carcinoma and other solid tumors.

In other embodiments, the combination therapy is administered with a 4-1BB receptor targeting agent (*e.g.,* an antibody that stimulates signaling through 4-1BB (CD-137), *e.g.,* PF-2566). In one embodiment, the combination therapy is administered in combination with a tyrosine kinase inhibitor (*e.g.,* axitinib) and a 4-1BB receptor targeting agent.

All publications, patent applications, patents, and other references mentioned herein are incorporated by reference in their entirety.

Other features, objects, and advantages of the invention will be apparent from the description and drawings, and from the claims.

### BRIEF DESCRIPTION OF THE DRAWINGS

**Figure 1** shows a graphical representation of flow cytometry of PD-L1 surface expression in EBC-1 cells *in vitro* with or without INC280 treatment. EBC-1 cells are non-small cell lung cancer cells with a cMET amplification.
**Figure 2** shows a graphical representation of PD-L1 mRNA expression in Hs.746.T cells in a tumor xenograft model with or without INC280 treatment. Hs.746.T cells are gastric cancer cells with a c-MET amplification and a c-MET mutation.
**Figure 3** shows a graphical representation of PD-L1 mRNA expression in H3122 cells *in vitro* with or without LDK378. H3122 cells are non-small cell lung cancer (NSCLC) cells with an ALK translocation.
**Figure 4** shows a graphical representation of PD-L1 mRNA expression in LOXIMV1 cells (BRAF mutant melanoma cells) in a tumor xenograft model with or without LGX818 treatment.
**Figure 5** shows a graphical representation of PD-L1 mRNA expression in HEYA8 cells (KRAS mutant ovarian cancer cells) in a tumor xenograft model with or without MEK162 treatment.
**Figure 6** shows a graphical representation of PD-L1 mRNA expression in UKE-1 cells (JAK2 V617F mutant myeloproliferative neoplasm cells) in a tumor xenograft model with or without INC424 treatment.
**Figure 7A** shows a graphical representation of IFN-γ production in unstimulated PBMCs or stimulated PBMCs treated with different concentrations of LCL161 or DMSO control.
**Figure 7B** shows a graphical representation of IL-10 production in unstimulated PBMCs or stimulated PBMCs treated with different concentrations of LCL161 or DMSO control.
**Figure 8A** shows a graphical representation of FACS analysis of CD4+ T cells from unstimulated PBMCs or PMBCs stimulated in the presence of different concentrations of LCL161 or DMSO control.
**Figure 8B** shows a graphical representation of FACS analysis of CD8+ T cells from unstimulated PBMCs or PMBCs stimulated in the presence of different concentrations of LCL161 or DMSO control.
**Figure 9** shows a graphical representation of CyTOF mass cytometry of unstimulated PBMCs or stimulated PBMCs treated with LCL161 or DMSO control.
**Figure 10A** shows a graphical representation of expression signatures related to T cells from mice implanted with MC38 cells. The mice were treated with LCL161, anti-mouse PD-1, or both. In the control group, mice were dosed with vehicle and isotype (mIgG1).
**Figure 10B** shows a graphical representation of expression signatures related to dendritic cells from mice implanted with MC38 cells. The mice were treated with LCL161, anti-mouse PD-1, or both. In the control group, mice were dosed with vehicle and isotype (mIgG1).
**Figure 10C** shows a graphical representation of expression signatures related to macrophages from mice implanted with MC38 cells. The mice were treated with LCL161, anti-mouse PD-1, or both. In the control group, mice were dosed with vehicle and isotype (mIgG1).
**Figure 10D** shows a graphical representation of chemokine expression signatures from mice implanted with MC38 cells. The mice were treated with LCL161, anti-mouse PD-1, or both. In the control group, mice were dosed with vehicle and isotype (mIgG1).
**Figure 11A** shows an exemplary treatment schedule and a graphical representation of tumor volumes in mice implanted with MC38 cells. The mice were treated with LCL161, anti-mouse PD-1, or both. In this treatment schedule, anti-mouse PD-1 was administered three days after LCL161 was administered. In the control group, mice were dosed with vehicle and isotype (mIgG1).
**Figure 11B** shows another exemplary treatment schedule and a graphical representation of tumor volumes in mice implanted with MC38 cells. The mice were treated with LCL161, anti-mouse PD-1, or both. In this treatment schedule, LCL161 and anti-mouse PD-1 were administered concurrently. In the control group, mice were dosed with vehicle and isotype (mIgG1).
**Figure 12** is a representation of the sequence of drug administration for patients enrolled in the Phase II trial that will be treated with EGF816 and Nivolumab.

### BRIEF DESCRIPTION OF THE TABLE

**Table 1** is a summary of selected therapeutic agents that can be administered in combination with the immunomodulators (e.g., one or more of: an activator of a costimulatory molecule and/or an inhibitor of an immune checkpoint molecule) described herein. **Table 1** provides from left to right the following: the Name and/or Designation of the second therapeutic agent, the Compound structure, a Patent publication disclosing the Compound, Exemplary Indications/Uses, and Generic structure.
**Table 2** shows the trial objectives and related endpoints in a phase II, multicenter, open-label study of EGF816 in combination with nivolumab in adult patients with EGFR mutated non-small cell lung cancer.
**Table 3** shows the dose and treatment schedule in a phase II, multicenter, open-label study of EGF816 in combination with nivolumab in adult patients with EGFR mutated non-small cell lung cancer.

### DETAILED DESCRIPTION

Methods and compositions are disclosed, which comprise an immunomodulator (*e.g.,* one or more of: an activator of a costimulatory molecule and/or an inhibitor of an immune checkpoint molecule) in combination with a second therapeutic agent chosen from one or more of the agents listed in Table 1. Immune therapy alone can be effective in a number of indications (*e.g.,* melanoma). However, for most patients, it is not a cure. In one embodiment, an inhibitor of an immune checkpoint molecule (*e.g.,* one or more of inhibitors to PD-1, PD-L1, LAG-3, TIM-3, CEACAM (*e.g.,* CEACAM-1, -3 and/or -5) or CTLA-4) can be combined with a second therapeutic agent chosen from one or more of the agents listed in Table 1 (*e.g.,* chosen from one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor; 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor; or 17) a BCR-ABL inhibitor),. The combinations described herein can provide a beneficial effect, *e.g.,* in the treatment of a cancer, such as an enhanced anti-cancer effect, reduced toxicity and/or reduced side effects. For example, the immunomodulator, the second therapeutic agent, or both, can be administered at a lower dosage than would be required to achieve the same therapeutic effect compared to a monotherapy dose.

The term "inhibition" or "inhibitor" includes a reduction in a certain parameter, *e.g.,* an activity, of a given molecule, *e.g.,* an immune checkpoint inhibitor. For example, inhibition of an activity, *e.g.,* an activity of, *e.g.,* PD-1, PD-L1, c-MET, ALK, CDK4/6, PI3K, BRAF, FGFR, MET or BCR-ABL, of at least 5%, 10%, 20%, 30%, 40% or more is included by this term. Thus, inhibition need not be 100%.

The term "Programmed Death 1" or "PD-1" include isoforms, mammalian, *e.g.,* human PD-1, species homologs of human PD-1, and analogs comprising at least one common epitope with PD-1. The amino acid sequence of PD-1, *e.g.,* human PD-1, is known in the art, *e.g.,* Shinohara T et al. (1994) Genomics 23(3):704-6; Finger LR, et al. Gene (1997) 197(1-2):177-87.

The term or "PD-Ligand 1" or "PD-L1" include isoforms, mammalian, *e.g.,* human PD-1, species homologs of human PD-L1, and analogs comprising at least one common epitope with PD-L1. The amino acid sequence of PD-L1, *e.g.,* human PD-L1, is known in the art

The term "Lymphocyte Activation Gene-3" or "LAG-3" include all isoforms, mammalian, *e.g.,* human LAG-3, species homologs of human LAG-3, and analogs comprising at least one common epitope with LAG-3. The amino acid and nucleotide sequences of LAG-3, *e.g.,* human LAG-3, is known in the art, *e.g.,* Triebel et al. (1990) J. Exp. Med. 171:1393-1405.

As used herein, "TIM-3" refers to a transmembrane receptor protein that is expressed on Th1 (T helper 1) cells. TIM-3 has a role in regulating immunity and tolerance *in vivo* (see Hastings et al., Eur J Immunol. 2009 Sep;39(9):2492-501).

The term "Carcinoembryonic Antigen-related Cell Adhesion Molecule" or "CEACAM" includes all family members (*e.g.,* CEACAM-1, CEACAM-3, or CEACAM-5), isoforms, mammalian, *e.g.,* human CEACAM, species homologs of human CEACAM, and analogs comprising at least one common epitope with CEACAM. The amino acid sequence of CEACAM, *e.g.,* human CEACAM, is known in the art, *e.g.,* Hinoda et al. (1988) Proc. Natl. Acad. Sci. U.S.A. 85 (18), 6959-6963; Zimmermann W. et al. (1987) Proc. Natl. Acad. Sci. U.S.A. 84 (9), 2960-2964; Thompson J. et al. (1989) Biochem. Biophys. Res. Commun. 158 (3), 996-1004.

Additional terms are defined below and throughout the application.

As used herein, the articles "a" and "an" refer to one or to more than one (*e.g.,* to at least one) of the grammatical object of the article.

The term "or" is used herein to mean, and is used interchangeably with, the term "and/or", unless context clearly indicates otherwise.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

The compositions and methods of the present invention encompass polypeptides and nucleic acids having the sequences specified, or sequences substantially identical or similar thereto, e.g., sequences at least 85%, 90%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 85%, 90%. 91%, 92%, 93%, 94%, 95%, 96%, 97%, 98% or 99% identity to a reference sequence, *e.g.,* a sequence provided herein.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 85%, 90%, 91%, 92%, 93%, 94%, 95%, 96%, 97%, 98% or 99% identity to a reference sequence, *e.g.,* a sequence provided herein.

The term "functional variant" refers polypeptides that have a substantially identical amino acid sequence to the naturally-occurring sequence, or are encoded by a substantially identical nucleotide sequence, and are capable of having one or more activities of the naturally-occurring sequence.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (*e.g.,* gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453) algorithm which has been incorporated into the GAP program in the GCG software package (available at www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to a nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (*e.g.,* XBLAST and NBLAST) can be used. See http://www.ncbi.nlm.nih.gov.

As used herein, the term "hybridizes under low stringency, medium stringency, high stringency, or very high stringency conditions" describes conditions for hybridization and washing. Guidance for performing hybridization reactions can be found in Current Protocols in Molecular Biology, John Wiley & Sons, N.Y. (1989), 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

It is understood that the molecules of the present invention may have additional conservative or non-essential amino acid substitutions, which do not have a substantial effect on their functions.

The term "amino acid" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing. As used herein the term "amino acid" includes both the D- or L- optical isomers and peptidomimetics.

A "conservative amino acid substitution" is one in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine).

The terms "polypeptide", "peptide" and "protein" (if single chain) are used interchangeably herein to refer to polymers of amino acids of any length. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. The polypeptide can be isolated from natural sources, can be a produced by recombinant techniques from a eukaryotic or prokaryotic host, or can be a product of synthetic procedures.

The terms "nucleic acid," "nucleic acid sequence," "nucleotide sequence," or "polynucleotide sequence," and "polynucleotide" are used interchangeably. They refer to a polymeric form of nucleotides of any length, either deoxyribonucleotides or ribonucleotides, or analogs thereof. The polynucleotide may be either single-stranded or double-stranded, and if single-stranded may be the coding strand or non-coding (antisense) strand. A polynucleotide may comprise modified nucleotides, such as methylated nucleotides and nucleotide analogs. The sequence of nucleotides may be interrupted by non-nucleotide components. A polynucleotide may be further modified after polymerization, such as by conjugation with a labeling component. The nucleic acid may be a recombinant polynucleotide, or a polynucleotide of genomic, cDNA, semisynthetic, or synthetic origin which either does not occur in nature or is linked to another polynucleotide in a nonnatural arrangement.

The term "isolated," as used herein, refers to material that is removed from its original or native environment (*e.g.,* the natural environment if it is naturally occurring). For example, a naturally-occurring polynucleotide or polypeptide present in a living animal is not isolated, but the same polynucleotide or polypeptide, separated by human intervention from some or all of the co-existing materials in the natural system, is isolated. Such polynucleotides could be part of a vector and/or such polynucleotides or polypeptides could be part of a composition, and still be isolated in that such vector or composition is not part of the environment in which it is found in nature.

Various aspects of the invention are described in further detail below. Additional definitions are set out throughout the specification.

### Antibody Molecules

In one embodiment, the antibody molecule binds to a mammalian, *e.g.,* human, checkpoint molecule, *e.g.,* PD-1, PD-L1, LAG-3, CEACAM (*e.g.,* CEACAM-1, -3 and/or -5) or TIM-3. For example, the antibody molecule binds specifically to an epitope, *e.g.,* linear or conformational epitope, (*e.g.,* an epitope as described herein) on PD-1, PD-L1, LAG-3, CEACAM (*e.g.,* CEACAM-1, -3 and/or -5) or TIM-3.

As used herein, the term "antibody molecule" refers to a protein comprising at least one immunoglobulin variable domain sequence. The term antibody molecule includes, for example, full-length, mature antibodies and antigen-binding fragments of an antibody. For example, an antibody molecule can include a heavy (H) chain variable domain sequence (abbreviated herein as VH), and a light (L) chain variable domain sequence (abbreviated herein as VL). In another example, an antibody molecule includes two heavy (H) chain variable domain sequences and two light (L) chain variable domain sequence, thereby forming two antigen binding sites, such as Fab, Fab', F(ab')₂, Fc, Fd, Fd', Fv, single chain antibodies (scFv for example), single variable domain antibodies, diabodies (Dab) (bivalent and bispecific), and chimeric (*e.g.,* humanized) antibodies, which may be produced by the modification of whole antibodies or those synthesized de novo using recombinant DNA technologies. These functional antibody fragments retain the ability to selectively bind with their respective antigen or receptor. Antibodies and antibody fragments can be from any class of antibodies including, but not limited to, IgG, IgA, IgM, IgD, and IgE, and from any subclass (*e.g.,* IgG1, IgG2, IgG3, and IgG4) of antibodies. The antibodies of the present invention can be monoclonal or polyclonal. The antibody can also be a human, humanized, CDR-grafted, or *in vitro* generated antibody. The antibody can have a heavy chain constant region chosen from, *e.g.,* IgG1, IgG2, IgG3, or IgG4. The antibody can also have a light chain chosen from, *e.g.,* kappa or lambda.

Examples of antigen-binding fragments include: (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a diabody (dAb) fragment, which consists of a VH domain; (vi) a camelid or camelized variable domain; (vii) a single chain Fv (scFv), *see e.g.,* Bird et al. (1988) Science 242:423-426; and Huston et al. (1988) Proc. Natl. Acad. Sci. USA 85:5879-5883); (viii) a single domain antibody. These antibody fragments are obtained using conventional techniques known to those with skill in the art, and the fragments are screened for utility in the same manner as are intact antibodies.

The term "antibody" includes intact molecules as well as functional fragments thereof. Constant regions of the antibodies can be altered, *e.g.,* mutated, to modify the properties of the antibody (*e.g.,* to increase or decrease one or more of: Fc receptor binding, antibody glycosylation, the number of cysteine residues, effector cell function, or complement function).

Antibody molecules can also be single domain antibodies. Single domain antibodies can include antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, fish, shark, goat, rabbit, and bovine. According to another aspect of the invention, a single domain antibody is a naturally occurring single domain antibody known as heavy chain antibody devoid of light chains. Such single domain antibodies are disclosed in WO 9404678, for example. For clarity reasons, this variable domain derived from a heavy chain antibody naturally devoid of light chain is known herein as a VHH or nanobody to distinguish it from the conventional VH of four chain immunoglobulins. Such a VHH molecule can be derived from antibodies raised in *Camelidae* species, for example in camel, llama, dromedary, alpaca and guanaco. Other species besides *Camelidae* may produce heavy chain antibodies naturally devoid of light chain; such VHHs are within the scope of the invention.

The VH and VL regions can be subdivided into regions of hypervariability, termed "complementarity determining regions" (CDR), interspersed with regions that are more conserved, termed "framework regions" (FR or FW).

The extent of the framework region and CDRs has been precisely defined by a number of methods (*see,* Kabat, E. A., et al. (1991) Sequences of Proteins of Immunological Interest, Fifth Edition, U.S. Department of Health and Human Services, NIH Publication No. 91-3242; Chothia, C. et al. (1987) J. Mol. Biol. 196:901-917; and the AbM definition used by Oxford Molecular's AbM antibody modeling software. *See,* generally, *e.g.,* Protein Sequence and Structure Analysis of Antibody Variable Domains. In: Antibody Engineering Lab Manual (Ed.: Duebel, S. and Kontermann, R., Springer-Verlag, Heidelberg).

The terms "complementarity determining region," and "CDR," as used herein refer to the sequences of amino acids within antibody variable regions which confer antigen specificity and binding affinity. In general, there are three CDRs in each heavy chain variable region (HCDR1, HCDR2, HCDR3) and three CDRs in each light chain variable region (LCDR1, LCDR2, LCDR3).

The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme). As used herein, the CDRs defined according the "Chothia" number scheme are also sometimes referred to as "hypervariable loops."

For example, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 26-32 (LCDR1), 50-52 (LCDR2), and 91-96 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL.

As used herein, an "immunoglobulin variable domain sequence" refers to an amino acid sequence which can form the structure of an immunoglobulin variable domain. For example, the sequence may include all or part of the amino acid sequence of a naturally-occurring variable domain. For example, the sequence may or may not include one, two, or more N- or C-terminal amino acids, or may include other alterations that are compatible with formation of the protein structure.

The term "antigen-binding site" refers to the part of an antibody molecule that comprises determinants that form an interface that binds to the PD-1 polypeptide, or an epitope thereof. With respect to proteins (or protein mimetics), the antigen-binding site typically includes one or more loops (of at least four amino acids or amino acid mimics) that form an interface that binds to the PD-1 polypeptide. Typically, the antigen-binding site of an antibody molecule includes at least one or two CDRs and/or hypervariable loops, or more typically at least three, four, five or six CDRs and/or hypervariable loops.

The terms "monoclonal antibody" or "monoclonal antibody composition" as used herein refer to a preparation of antibody molecules of single molecular composition. A monoclonal antibody composition displays a single binding specificity and affinity for a particular epitope. A monoclonal antibody can be made by hybridoma technology or by methods that do not use hybridoma technology (*e.g.,* recombinant methods).

An "effectively human" protein is a protein that does not evoke a neutralizing antibody response, *e.g.,* the human anti-murine antibody (HAMA) response. HAMA can be problematic in a number of circumstances, *e.g.,* if the antibody molecule is administered repeatedly, *e.g.,* in treatment of a chronic or recurrent disease condition. A HAMA response can make repeated antibody administration potentially ineffective because of an increased antibody clearance from the serum (*see, e.g.,* Saleh et al., Cancer Immunol. Immunother., 32:180-190 (1990)) and also because of potential allergic reactions (*see, e.g.,* LoBuglio et al., Hybridoma, 5:5117-5123 (1986)).

The antibody molecule can be a polyclonal or a monoclonal antibody. In other embodiments, the antibody can be recombinantly produced, *e.g.,* produced by phage display or by combinatorial methods.

Phage display and combinatorial methods for generating antibodies are known in the art (as described in, *e.g.,* Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982, the contents of all of which are incorporated by reference herein).

In one embodiment, the antibody is a fully human antibody (*e.g.,* an antibody made in a mouse which has been genetically engineered to produce an antibody from a human immunoglobulin sequence), or a non-human antibody, *e.g.,* a rodent (mouse or rat), goat, primate (*e.g.,* monkey), camel antibody. Preferably, the non-human antibody is a rodent (mouse or rat antibody). Methods of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, *e.g.,* Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L.L. et al. 1994 Nature Genet. 7:13-21; Morrison, S.L. et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326).

An antibody can be one in which the variable region, or a portion thereof, *e.g.,* the CDRs, are generated in a non-human organism, *e.g.,* a rat or mouse. Chimeric, CDR-grafted, and humanized antibodies are within the invention. Antibodies generated in a non-human organism, *e.g.,* a rat or mouse, and then modified, *e.g.,* in the variable framework or constant region, to decrease antigenicity in a human are within the invention.

Chimeric antibodies can be produced by recombinant DNA techniques known in the art (see Robinson et al., International Patent Publication PCT/US86/02269; Akira, *et al.,* European Patent Application 184,187; Taniguchi, M., European Patent Application 171,496; Morrison *et al.,* European Patent Application 173,494; Neuberger et al., International Application WO 86/01533; Cabilly et al. U.S. Patent No. 4,816,567; Cabilly *et al.,* European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. (1985) Nature 314:446-449; and Shaw et al., 1988, J. Natl Cancer Inst. 80:1553-1559).

A humanized or CDR-grafted antibody will have at least one or two but generally all three recipient CDRs (of heavy and or light immuoglobulin chains) replaced with a donor CDR. The antibody may be replaced with at least a portion of a non-human CDR or only some of the CDRs may be replaced with non-human CDRs. It is only necessary to replace the number of CDRs required for binding of the humanized antibody to PD-1. Preferably, the donor will be a rodent antibody, *e.g.,* a rat or mouse antibody, and the recipient will be a human framework or a human consensus framework. Typically, the immunoglobulin providing the CDRs is called the "donor" and the immunoglobulin providing the framework is called the "acceptor." In one embodiment, the donor immunoglobulin is a non-human (*e.g.,* rodent). The acceptor framework is a naturally-occurring (*e.g.,* a human) framework or a consensus framework, or a sequence about 85% or higher, preferably 90%, 95%, 99% or higher identical thereto.

As used herein, the term "consensus sequence" refers to the sequence formed from the most frequently occurring amino acids (or nucleotides) in a family of related sequences (See *e.g.,* Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody can be humanized by methods known in the art (*see e.g.,* Morrison, S. L., 1985, Science 229:1202-1207, by Oi et al., 1986, BioTechniques 4:214, and by Queen et al. US 5,585,089, US 5,693,761 and US 5,693,762, the contents of all of which are hereby incorporated by reference).

Humanized or CDR-grafted antibodies can be produced by CDR-grafting or CDR substitution, wherein one, two, or all CDRs of an immunoglobulin chain can be replaced. *See e.g.,* U.S. Patent 5,225,539; Jones et al. 1986 Nature 321:552-525; Verhoeyan et al. 1988 Science 239:1534; Beidler et al. 1988 J. Immunol. 141:4053-4060; Winter US 5,225,539, the contents of all of which are hereby expressly incorporated by reference. Winter describes a CDR-grafting method which may be used to prepare the humanized antibodies of the present invention (UK Patent Application GB 2188638A, filed on March 26, 1987; Winter US 5,225,539), the contents of which is expressly incorporated by reference.

Also within the scope of the invention are humanized antibodies in which specific amino acids have been substituted, deleted or added. Criteria for selecting amino acids from the donor are described in US 5,585,089, *e.g.,* columns 12-16 of US 5,585,089, *e.g.,* columns 12-16 of US 5,585,089, the contents of which are hereby incorporated by reference. Other techniques for humanizing antibodies are described in Padlan et al. EP 519596 A1, published on December 23, 1992.

The antibody molecule can be a single chain antibody. A single-chain antibody (scFV) may be engineered (see, for example, Colcher, D. et al. (1999) Ann N Y Acad Sci 880:263-80; and Reiter, Y. (1996) Clin Cancer Res 2:245-52). The single chain antibody can be dimerized or multimerized to generate multivalent antibodies having specificities for different epitopes of the same target protein.

In yet other embodiments, the antibody molecule has a heavy chain constant region chosen from, *e.g.,* the heavy chain constant regions of IgG1, IgG2, IgG3, IgG4, IgM, IgA1, IgA2, IgD, and IgE; particularly, chosen from, *e.g.,* the (*e.g.,* human) heavy chain constant regions of IgG1, IgG2, IgG3, and IgG4. In another embodiment, the antibody molecule has a light chain constant region chosen from, *e*.g., the *(e.g.,* human) light chain constant regions of kappa or lambda. The constant region can be altered, *e.g.,* mutated, to modify the properties of the antibody (*e.g.,* to increase or decrease one or more of: Fc receptor binding, antibody glycosylation, the number of cysteine residues, effector cell function, and/or complement function). In one embodiment the antibody has: effector function; and can fix complement. In other embodiments the antibody does not; recruit effector cells; or fix complement. In another embodiment, the antibody has reduced or no ability to bind an Fc receptor. For example, it is a isotype or subtype, fragment or other mutant, which does not support binding to an Fc receptor, *e.g.,* it has a mutagenized or deleted Fc receptor binding region.

Methods for altering an antibody constant region are known in the art. Antibodies with altered function, *e.g.* altered affinity for an effector ligand, such as FcR on a cell, or the C1 component of complement can be produced by replacing at least one amino acid residue in the constant portion of the antibody with a different residue (*see e.g.,* EP 388,151 A1, U.S. Pat. No. 5,624,821 and U.S. Pat. No. 5,648,260, the contents of all of which are hereby incorporated by reference). Similar type of alterations could be described which if applied to the murine, or other species immunoglobulin would reduce or eliminate these functions.

An antibody molecule can be derivatized or linked to another functional molecule (*e.g.,* another peptide or protein). As used herein, a "derivatized" antibody molecule is one that has been modified. Methods of derivatization include but are not limited to the addition of a fluorescent moiety, a radionucleotide, a toxin, an enzyme or an affinity ligand such as biotin. Accordingly, the antibody molecules of the invention are intended to include derivatized and otherwise modified forms of the antibodies described herein, including immunoadhesion molecules. For example, an antibody molecule can be functionally linked (by chemical coupling, genetic fusion, noncovalent association or otherwise) to one or more other molecular entities, such as another antibody (*e.g.,* a bispecific antibody or a diabody), a detectable agent, a cytotoxic agent, a pharmaceutical agent, and/or a protein or peptide that can mediate association of the antibody or antibody portion with another molecule (such as a streptavidin core region or a polyhistidine tag).

One type of derivatized antibody molecule is produced by crosslinking two or more antibodies (of the same type or of different types, *e.g.,* to create bispecific antibodies). Suitable crosslinkers include those that are heterobifunctional, having two distinctly reactive groups separated by an appropriate spacer (*e.g.,* m-maleimidobenzoyl-N-hydroxysuccinimide ester) or homobifunctional (*e.g.,* disuccinimidyl suberate). Such linkers are available from Pierce Chemical Company, Rockford, Ill.

An antibody molecules may be conjugated to another molecular entity, typically a label or a therapeutic (*e.g.,* a cytotoxic or cytostatic) agent or moiety. Radioactive isotopes can be used in diagnostic or therapeutic applications. Radioactive isotopes that can be coupled to the anti-PSMA antibodies include, but are not limited to α-, β-, or γ-emitters, or β-and γ-emitters. Such radioactive isotopes include, but are not limited to iodine (¹³¹I or ¹²⁵I), yttrium (⁹⁰Y), lutetium ( ¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (⁹⁹mTc), phosphorus (³²P), rhodium (¹⁸⁸Rh), sulfur (³⁵S) , carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), or gallium (⁶⁷Ga). Radioisotopes useful as therapeutic agents include yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), and rhodium (¹⁸⁸Rh). Radioisotopes useful as labels, *e.g.,* for use in diagnostics, include iodine (¹³¹I or ¹²⁵I), indium (¹¹¹In), technetium (⁹⁹mTc), phosphorus (³²P), carbon (¹⁴C), and tritium (³H), or one or more of the therapeutic isotopes listed above.

The invention provides radiolabeled antibody molecules and methods of labeling the same. In one embodiment, a method of labeling an antibody molecule is disclosed. The method includes contacting an antibody molecule, with a chelating agent, to thereby produce a conjugated antibody. The conjugated antibody is radiolabeled with a radioisotope, *e.g.,* ¹¹¹Indium, ⁹⁰Yttrium and ¹⁷⁷Lutetium, to thereby produce a labeled antibody molecule.

As is discussed above, the antibody molecule can be conjugated to a therapeutic agent. Therapeutically active radioisotopes have already been mentioned. Examples of other therapeutic agents include taxol, cytochalasin B, gramicidin D, ethidium bromide, emetine, mitomycin, etoposide, tenoposide, vincristine, vinblastine, colchicine, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, *e.g.,* maytansinol (*see* U.S. Pat. No. 5,208,020), CC-1065 (see U.S. Pat. Nos. 5,475,092, 5,585,499, 5,846, 545) and analogs or homologs thereof. Therapeutic agents include, but are not limited to, antimetabolites (*e.g.,* methotrexate, 6-mercaptopurine, 6-thioguanine, cytarabine, 5-fluorouracil decarbazine), alkylating agents (*e.g.,* mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclinies (*e.g.,* daunorubicin (formerly daunomycin) and doxorubicin), antibiotics (*e.g.,* dactinomycin (formerly actinomycin), bleomycin, mithramycin, and anthramycin (AMC)), and anti-mitotic agents (*e.g.,* vincristine, vinblastine, taxol and maytansinoids).

### Combination Therapies

The combination therapies (*e.g.,* methods and compositions described herein) can include an immunomodulator (*e.g.,* one or more of: an activator of a costimulatory molecule or an inhibitor of an immune checkpoint molecule) and a second therapeutic agent, *e.g.,* a second therapeutic agent chosen from one or more of the agents listed in Table 1.

By "combination" or "in combination with," it is not intended to imply that the therapy or the therapeutic agents must be administered at the same time and/or formulated for delivery together (*e.g.,* in the same composition), although these methods and compositions are within the scope described herein. The immunomodulator and the second therapeutic agent can be administered concurrently with, prior to, or subsequent to, one or more other additional therapies or therapeutic agents. The agents in the combination can be administered in any order. In general, each agent will be administered at a dose and/or on a time schedule determined for that agent. In will further be appreciated that the additional therapeutic agent utilized in this combination may be administered together in a single composition or administered separately in different compositions. In general, it is expected that additional therapeutic agents utilized in combination be utilized at levels that do not exceed the levels at which they are utilized individually. In some embodiments, the levels utilized in combination will be lower than those utilized individually.

In some embodiments, a combination includes a formulation of the immunomodulator and the second therapeutic agent, with or without instructions for combined use or to combination products. The combined compounds can be manufactured and/or formulated by the same or different manufacturers. The combination partners may thus be entirely separate pharmaceutical dosage forms or pharmaceutical compositions that are also sold independently of each other. In embodiments, instructions for their combined use are provided: (i) prior to release to physicians (*e.g.* in the case of a "kit of part" comprising the compound of the disclosure and the other therapeutic agent); (ii) by the physicians themselves (or under the guidance of a physician) shortly before administration; (iii) the patient themselves by a physician or medical staff.

### Immunomodulators

The combination therapies disclosed herein can include an inhibitor of an inhibitory molecule of an immune checkpoint molecule. The term "immune checkpoints" refers to a group of molecules on the cell surface of CD4 and CD8 T cells. These molecules can effectively serve as "brakes" to down-modulate or inhibit an anti-tumor immune response. Inhibition of an inhibitory molecule can be performed by inhibition at the DNA, RNA or protein level. In embodiments, an inhibitory nucleic acid (*e.g.,* a dsRNA, siRNA or shRNA), can be used to inhibit expression of an inhibitory molecule. In other embodiments, the inhibitor of an inhibitory signal is, a polypeptide *e.g.,* a soluble ligand, or an antibody or antigen-binding fragment thereof, that binds to the inhibitory molecule.

Immune checkpoint molecules useful in the methods and compositions of the present invention include, but are not limited to, Programmed Death 1 (PD-1), PD-1, PD-L1, PD-L2, Cytotoxic T-Lymphocyte Antigen 4 (CTLA-4), TIM-3, CEACAM (*e.g.,* CEACAM-1, CEACAM-3 and/or CEACAM-5), LAG-3, VISTA, BTLA, TIGIT, LAIR1, CD160, 2B4, CD80, CD86, B7-H1, B7-H3 (CD276), B7-H4 (VTCN1), HVEM (TNFRSF14 or CD270), KIR, A2aR, MHC class I, MHC class II, GAL9, adenosine, TGFR (*e.g.,* TGFR beta). In certain embodiments, the immunomodulator is an inhibitor of an immune checkpoint molecule (*e.g.,* an inhibitor of PD-1, PD-L1, LAG-3, TIM-3, CEACAM (*e.g.,* CEACAM-1, -3 and/or -5) or CTLA-4, or any combination thereof).

In other embodiments, the PD-1 inhibitor is an anti-PD-1 antibody chosen from Nivolumab, Pembrolizumab or Pidilizumab.

In some embodiments, the anti-PD-1 antibody is Nivolumab. Alternative names for Nivolumab include MDX-1106, MDX-1106-04, ONO-4538, or BMS-936558. In some embodiments, the anti-PD-1 antibody is Nivolumab (CAS Registry Number: 946414-94-4). Nivolumab is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168. In one embodiment, the inhibitor of PD-1 is Nivolumab, and having a sequence disclosed herein (or a sequence substantially identical or similar thereto, *e.g.,* a sequence at least 85%, 90%, 95% identical or higher to the sequence specified).

The heavy and light chain amino acid sequences of Nivolumab are as follows:

Heavy chain (SEQ ID NO: 2)

QVQLVESGGGVVQPGRSLRLDCKASGITFSNSGMHWVRQAPGKGLEWVAVIWYDGSKRYYADSV
KGRFTISRDNSKNTLFLQMNSLRAEDTAVYYCATNDDYWGQGTLVTVSSASTKGPSVFPLAPCS
RSTSESTAALGCLVKDYFPEPVTVSWNSGALTSGVHTFPAVLQSSGLYSLSSVVTVPSSSLGTK
TYTCNVDHKPSNTKVDKRVESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWLNGKEYKCKVSNKG
LPSSIEKTISKAKGQPREPQVYTLPPSQEEMTKNQVSLTCLVKGFYPSDIAVEWESNGQPENNY
KTTPPVLDSDGSFFLYSRLTVDKSRWQEGNVFSCSVMHEALHNHYTQKSLSLSLGK

Light chain (SEQ ID NO: 3)
EIVLTQSPATLSLSPGERATLSCRASQSVSSYLAWYQQKPGQAPRLLIYDASNRATGIPARFSG
SGSGTDFTLTISSLEPEDFAVYYCQQSSNWPRTFGQGTKVEIKRTVAAPSVFIFPPSDEQLKSG
TASVVCLLNNFYPREAKVQWKVDNALQSGNSQESVTEQDSKDSTYSLSSTLTLSKADYEKHKVY
ACEVTHQGLSSPVTKSFNRGEC

In some embodiments, the anti-PD-1 antibody is Pembrolizumab. Pembrolizumab (also referred to as Lambrolizumab, MK-3475, MK03475, SCH-900475 or KEYTRUDA®; Merck) is a humanized IgG4 monoclonal antibody that binds to PD-1. Pembrolizumab and other humanized anti-PD-1 antibodies are disclosed in Hamid, O. et al. (2013) New England Journal of Medicine 369 (2): 134-44, US 8,354,509 and WO2009/114335. In one embodiment, the inhibitor of PD-1 is Pembrolizumab disclosed in, *e.g.,* US 8,354,509 and WO 2009/114335, and having a sequence disclosed herein (or a sequence substantially identical or similar thereto, *e.g.,* a sequence at least 85%, 90%, 95% identical or higher to the sequence specified).

The heavy and light chain amino acid sequences of Pembrolizumab are as follows:

Heavy chain (SEQ ID NO: 4)

QVQLVQSGVE VKKPGASVKV SCKASGYTFT NYYMYWVRQA PGQGLEWMGG 50
INPSNGGTNF NEKFKNRVTL TTDSSTTTAY MELKSLQFDD TAVYYCARRD 100
YRFDMGFDYW GQGTTVTVSS ASTKGPSVFP LAPCSRSTSE STAALGCLVK 150
DYFPEPVTVS WNSGALTSGV HTFPAVLQSS GLYSLSSVVT VPSSSLGTKT 200
YTCNVDHKPS NTKVDKRVES KYGPPCPPCP APEFLGGPSV FLFPPKPKDT 250
LMISRTPEVT CVVVDVSQED PEVQFNWYVD GVEVHNAKTK PREEQFNSTY 300
RVVSVLTVLH QDWLNGKEYK CKVSNKGLPS SIEKTISKAK GQPREPQVYT 350
LPPSQEEMTK NQVSLTCLVK GFYPSDIAVE WESNGQPENN YKTTPPVLDS 400
DGSFFLYSRL TVDKSRWQEG NVFSCSVMHE ALHNHYTQKS LSLSLGK 447

Light chain (SEQ ID NO: 5)

EIVLTQSPAT LSLSPGERAT LSCRASKGVS TSGYSYLHWY QQKPGQAPRL 50
LIYLASYLES GVPARFSGSG SGTDFTLTIS SLEPEDFAVY YCQHSRDLPL 100
TFGGGTKVEI KRTVAAPSVF IFPPSDEQLK SGTASVVCLL NNFYPREAKV 150
QWKVDNALQS GNSQESVTEQ DSKDSTYSLS STLTLSKADY EKHKVYACEV 200
THQGLSSPVT KSFNRGEC 218

In some embodiments, the anti-PD-1 antibody is Pidilizumab. Pidilizumab (CT-011; Cure Tech) is a humanized IgGlk monoclonal antibody that binds to PD-1. Pidilizumab and other humanized anti-PD-1 monoclonal antibodies are disclosed in WO2009/101611.

Other anti-PD-1 antibodies include AMP 514 (Amplimmune), among others, *e.g.,* anti-PD-1 antibodies disclosed in US 8,609,089, US 2010028330, and/or US 20120114649.

### Exemplary PD-L1 or PD-L2 Inhibitors

In some embodiments, the PD-L1 inhibitor is an antibody molecule. In some embodiments, the anti-PD-Ll inhibitor is chosen from YW243.55.S70, MPDL3280A, MEDI-4736, MSB-0010718C, or MDX-1105.

In some embodiments, the anti-PD-Ll antibody is MSB0010718C. MSB0010718C (also referred to as A09-246-2; Merck Serono) is a monoclonal antibody that binds to PD-L1. Pembrolizumab and other humanized anti-PD-L1 antibodies are disclosed in WO2013/079174, and having a sequence disclosed herein (or a sequence substantially identical or similar thereto, *e.g.,* a sequence at least 85%, 90%, 95% identical or higher to the sequence specified). The heavy and light chain amino acid sequences of MSB0010718C include at least the following:

Heavy chain variable region (SEQ ID NO: 24 as disclosed in WO2013/079174) (SEQ ID NO: 6)
EVQLLESGGGLVQPGGSLRLSCAASGFTFSSYIMMWVRQAPGKGLEWVSSIYPSGGITFYADKG
RFTISRDNSKNTLYLQMNSLRAEDTAVYYCARIKLGTVTTVDYWGQGTLVTVSS

Light chain variable region (SEQ ID NO: 25 as disclosed in WO2013/079174) (SEQ ID NO: 7)
QSALTQPASVSGSPGQSITISCTGTSSDVGGYNYVSWYQQHPGKAPKLMIYDVSN
RPSGVSNRFSGSKSGNTASLTISGLQAEDEADYYCSSYTSSSTRVFGTGTKVTVL

In one embodiment, the PD-L1 inhibitor is YW243.55.S70. The YW243.55.S70 antibody is an anti-PD-Ll described in WO 2010/077634 (heavy and light chain variable region sequences shown in SEQ ID Nos. 20 and 21, respectively, of WO 2010/077634), and having a sequence disclosed therein (or a sequence substantially identical or similar thereto, *e.g.,* a sequence at least 85%, 90%, 95% identical or higher to the sequence specified).

In one embodiment, the PD-L1 inhibitor is MDX-1105. MDX-1105, also known as BMS-936559, is an anti-PD-Ll antibody described in WO2007/005874, and having a sequence disclosed therein (or a sequence substantially identical or similar thereto, *e.g.,* a sequence at least 85%, 90%, 95% identical or higher to the sequence specified).

In one embodiment, the PD-L1 inhibitor is MDPL3280A (Genentech / Roche). MDPL3280A is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906.

In other embodiments, the PD-L2 inhibitor is AMP-224. AMP-224 is a PD-L2 Fc fusion soluble receptor that blocks the interaction between PD-1 and B7-H1 (B7-DCIg; Amplimmune; *e.g*., disclosed in WO2010/027827 and WO2011/066342).

### Exemplary TIM-3 Inhibitors

In one embodiment, a combination described herein includes a TIM-3 inhibitor. In some embodiments, the combination is used to treat a cancer, *e.g.,* a cancer described herein, *e.g.,* a solid tumor or a hematologic malignancy.

Exemplary anti-TIM-3 antibodies are disclosed in U.S. Patent No.: 8,552,156, WO 2011/155607, EP 2581113 and U.S Publication No.: 2014/044728.

### Exemplary LAG-3 Inhibitors

In one embodiment, a combination described herein includes a LAG-3 inhibitor. In some embodiments, the combination is used to treat a cancer, *e.g.,* a cancer described herein, *e.g.,* a solid tumor or a hematologic malignancy.

In some embodiments, the anti-LAG-3 antibody is BMS-986016. BMS-986016 (also referred to as BMS986016; Bristol-Myers Squibb) is a monoclonal antibody that binds to LAG-3. BMS-986016 and other humanized anti-LAG-3 antibodies are disclosed in US 2011/0150892, WO2010/019570, and WO2014/008218.

### Exemplary CTLA-4 Inhibitors

In one embodiment, a combination described herein includes a CTLA-4 inhibitor. In some embodiments, the combination is used to treat a cancer, *e.g.,* a cancer described herein, *e.g.,* a solid tumor or a hematologic malignancy.

Exemplary anti-CTLA-4 antibodies include Tremelimumab (IgG2 monoclonal antibody available from Pfizer, formerly known as ticilimumab, CP-675,206); and Ipilimumab (CTLA-4 antibody, also known as MDX-010, CAS No. 477202-00-9).

In one embodiment, the combination includes an anti-PD-1 antibody molecule, *e.g.,* as described herein, and an anti-CTLA-4 antibody, *e.g.,* ipilimumab. Exemplary doses that can be use include a dose of anti-PD-1 antibody molecule of about 1 to 10 mg/kg, *e.g.,* 3 mg/kg, and a dose of an anti-CTLA-4 antibody, *e.g.,* ipilimumab, of about 3 mg/kg. In one embodiment, the anti-PD-1 antibody molecule is administered after treatment, *e.g.,* after treatment of a melanoma, with an anti-CTLA-4 antibody *(e.g.,* ipilimumab) with or without a BRAF inhibitor (*e.g.,* vemurafenib or dabrafenib).

Other exemplary anti-CTLA-4 antibodies are disclosed, *e.g.,* in U.S. Pat. No. 5,811,097.

In one embodiment, the inhibitor is a soluble ligand (*e.g.,* a CTLA-4-Ig), or an antibody or antibody fragment that binds to PD-L1, PD-L2 or CTLA-4. For example, the anti-PD-1 antibody molecule can be administered in combination with an anti-CTLA-4 antibody, *e.g.,* ipilimumab, for example, to treat a cancer (*e.g.,* a cancer chosen from: a melanoma, *e.g.,* a metastatic melanoma; a lung cancer, *e.g.,* a non-small cell lung carcinoma; or a prostate cancer).

### Additional Combinations of Inhibitors

In certain embodiments, the anti-PD-1 molecules described herein are administered in combination with one or more other inhibitors of PD-1, PD-L1 and/or PD-L2, *e.g.,* as described herein. The antagonist may be an antibody, an antigen binding fragment thereof, an immunoadhesin, a fusion protein, or oligopeptide.

In one embodiment, the anti-PD-1 or PD-L1 antibody molecule is administered in combination with an anti-LAG-3 antibody or an antigen-binding fragment thereof. In another embodiment, the anti-PD-1 or PD-L1 antibody molecule is administered in combination with an anti-TIM-3 antibody or antigen-binding fragment thereof. In yet other embodiments, the anti-PD-1 or PD-L1 antibody molecule is administered in combination with an anti-LAG-3 antibody and an anti-TIM-3 antibody, or antigen-binding fragments thereof. The combination of antibodies recited herein can be administered separately, *e.g.,* as separate antibodies, or linked, *e.g.,* as a bispecific or trispecific antibody molecule. In one embodiment, a bispecific antibody that includes an anti-PD-1 or PD-L1 antibody molecule and an anti-TIM-3 or anti-LAG-3 antibody, or antigen-binding fragment thereof, is administered. In certain embodiments, the combination of antibodies recited herein is used to treat a cancer, *e.g.,* a cancer as described herein (*e.g.,* a solid tumor). The efficacy of the aforesaid combinations can be tested in animal models known in the art. For example, the animal models to test the synergistic effect of anti-PD-1 and anti-LAG-3 are described, *e.g.,* in Woo et al. (2012) Cancer Res. 72(4):917-27).

In another embodiment, the anti-PD-1 or PD-L1 antibody molecule is administered in combination with an inhibitor of CEACAM (*e.g.,* CEACAM-1, -3 and/or -5). In one embodiment, the inhibitor of CEACAM (*e.g.,* CEACAM-1, -3 and/or -5) is an anti-CEACAM antibody molecule. Without wishing to be bound by theory, carcinoembryonic antigen cell adhesion molecules (CEACAM), such as CEACAM-1 and CEACAM-5, are believed to mediate, at least in part, inhibition of an anti-tumor immune response (*see e.g.,* Markel et al. J Immunol. 2002 Mar 15;168(6):2803-10; Markel et al. J Immunol. 2006 Nov 1;177(9):6062-71; Markel et al. Immunology. 2009 Feb;126(2):186-200; Markel et al. Cancer Immunol Immunother. 2010 Feb;59(2):215-30; Ortenberg et al. Mol Cancer Ther. 2012 Jun;11(6):1300-10; Stern et al. J Immunol. 2005 Jun 1;174(11):6692-701; Zheng et al. PLoS One. 2010 Sep 2;5(9). pii: e12529). For example, CEACAM-1 has been described as a heterophilic ligand for TIM-3 and as playing a role in TIM-3-mediated T cell tolerance and exhaustion (*see e.g.,* WO 2014/022332; Huang, et al. (2014) Nature doi:10.1038/nature13848). In embodiments, co-blockade of CEACAM-1 and TIM-3 has been shown to enhance an anti-tumor immune response in xenograft colorectal cancer models (*see e.g.,* WO 2014/022332; Huang, *et al.* (2014), *supra*). In other embodiments, co-blockade of CEACAM-1 and PD-1 reduce T cell tolerance as described, *e.g.,* in WO 2014/059251. Thus, CEACAM inhibitors can be used with the other immunomodulators described herein (*e.g.,* anti-PD-1 and/or anti-TIM-3 inhibitors) to enhance an immune response against a cancer, *e.g.,* a melanoma, a lung cancer (*e.g.,* NSCLC), a bladder cancer, a colon cancer an ovarian cancer, and other cancers as described herein.

Accordingly, in some embodiments, the anti-PD-1 antibody molecule is administered in combination with a CEACAM inhibitor (*e.g.,* CEACAM-1, CEACAM-3, and/or CEACAM-5 inhibitor). In one embodiment, the inhibitor of CEACAM is an anti-CEACAM antibody molecule. In one embodiment, the anti-PD-1 antibody molecule is administered in combination with a CEACAM-1 inhibitor, *e.g.,* an anti- CEACAM-1 antibody molecule. In another embodiment, the anti-PD-1 antibody molecule is administered in combination with a CEACAM-3 inhibitor, *e.g.,* an anti- CEACAM-3 antibody molecule. In another embodiment, the anti-PD-1 antibody molecule is administered in combination with a CEACAM-5 inhibitor, *e.g.,* an anti-CEACAM-5 antibody molecule. Exemplary anti-CEACAM-1 antibodies are described in WO 2010/125571, WO 2013/082366 and WO 2014/022332, *e.g.,* a monoclonal antibody 34B1, 26H7, and 5F4; or a recombinant form thereof, as described in, *e.g.,* US 2004/0047858, US 7,132,255 and WO 99/052552. In other embodiments, the anti-CEACAM antibody binds to CEACAM-5 as described in, *e.g.,* Zheng et al. PLoS One. 2010 Sep 2;5(9). pii: e12529 (DOI:10:1371/journal.pone.0021146), or crossreacts with CEACAM-1 and CEACAM-5 as described in, *e.g.,* WO 2013/054331 and US 2014/0271618.

### Costimulatory Modulators

In certain embodiments, the combination therapies disclosed herein include a modulator of a costimulatory molecule. In one embodiment, the costimulatory modulator, *e.g.,* agonist, of a costimulatory molecule is chosen from an agonist (*e.g.,* an agonistic antibody or antigen-binding fragment thereof, or soluble fusion) of an MHC class I molecule, a TNF receptor protein, an Immunoglobulin-like proteins, a cytokine receptor, an integrin, a signaling lymphocytic activation molecules (SLAM proteins), an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, ITGAM, CDllb, ITGAX, CD11c, ITGB1, CD29, ITGB2, CD18, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), SLAM7, BLAME (SLAMF8), SELPLG (CD162), LTBR, LAT, GADS, SLP-76, PAG/Cbp, CD19a, and a ligand that specifically binds with CD83.

In one embodiment, the combination therapies disclosed herein include a costimulatory molecule, *e.g.,* an agonist associated with a positive signal that includes a costimulatory domain of CD28, CD27, ICOS and GITR.

### Exemplary GITR agonist

In one embodiment, a combination described herein includes a GITR agonist. In some embodiments, the combination is used to treat a cancer, *e.g.,* a cancer described herein, *e.g.,* a solid tumor or a hematologic malignancy.

Exemplary GITR agonists include, *e.g.,* GITR fusion proteins and anti-GITR antibodies (e.g., bivalent anti-GITR antibodies), such as, a GITR fusion protein described in U.S. Patent No.: 6,111,090, European Patent No.: 0920505B1, U.S Patent No.: 8,586,023, PCT Publication Nos.: WO 2010/003118 and 2011/090754, or an anti-GITR antibody described, *e.g.,* in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, U.S. Patent No.: 8,709,424, PCT Publication No.:WO 2013/039954, International Publication No.: WO2013/039954, U.S. Publication No.: US2014/0072566, International Publication NO.: WO2015/026684, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, U.S. Patent No.: 6,689,607, PCT Publication No.: WO2006/083289, PCT Publication No.: WO 2005/115451, U.S. Patent No.: 7,618,632, PCT Publication No.: WO 2011/051726, International Publication No.: WO2004060319, and International Publication No.: WO2014012479.

In one embodiment, the GITR agonist is used in combination with a PD-1 inhibitor, *e.g.,* as described in WO2015/026684.

In another embodiment, the GITR agonist is used in combination with a TLR agonist, *e.g.,* as described in WO2004060319, and International Publication No.: WO2014012479.

### Additional Combinations

In another embodiment, the combination therapies include a modified T-cell, *e.g.,* in combination with an adoptive T-cell immunotherapy using chimeric antigen receptor (CAR) T cells (*e.g.,* as described by John LB, et al. (2013) Clin. Cancer Res. 19(20): 5636-46).

In other embodiments, the combination therapies disclosed herein can also include a cytokine, *e.g.,* interleukin-21 or interleukin-2. In certain embodiments, the combination described herein is used to treat a cancer, *e.g.,* a cancer as described herein (*e.g.,* a solid tumor or melanoma).

Exemplary immunomodulators that can be used in the combination therapies include, but are not limited to, *e.g.,* afutuzumab (available from Roche®); pegfilgrastim (Neulasta®); lenalidomide (CC-5013, Revlimid®); thalidomide (Thalomid®), actimid (CC4047); and cytokines, *e.g.,* IL-21 or IRX-2 (mixture of human cytokines including interleukin 1, interleukin 2, and interferon γ, CAS 951209-71-5, available from IRX Therapeutics).

In other embodiments, the combination therapies can be administered to a subject in conjunction with (*e.g.,* before, simultaneously or following) one or more of: bone marrow transplantation, T cell ablative therapy using chemotherapy agents such as, fludarabine, external-beam radiation therapy (XRT), cyclophosphamide, and/or antibodies such as OKT3 or CAMPATH. In one embodiment, the anti-PD-1 or PD-L1 antibody molecules are administered following B-cell ablative therapy such as agents that react with CD20, *e.g.,* Rituxan. For example, in one embodiment, subjects may undergo standard treatment with high dose chemotherapy followed by peripheral blood stem cell transplantation. In certain embodiments, following the transplant, subjects receive the anti-PD-1 or PD-L1 antibody molecules. In an additional embodiment, the anti-PD-1 or PD-L1 antibody molecules are administered before or following surgery.

Another example of a further combination therapy includes decarbazine for the treatment of melanoma. Without being bound by theory, the combined use of PD-1 blockade and chemotherapy is believed to be facilitated by cell death, that is a consequence of the cytotoxic action of most chemotherapeutic compounds, which can result in increased levels of tumor antigen in the antigen presentation pathway. Other combination therapies that may result in synergy with PD-1 blockade through cell death are radiation, surgery, and hormone deprivation. Each of these protocols creates a source of tumor antigen in the host. Angiogenesis inhibitors may also be combined with PD-1 blockade. Inhibition of angiogenesis leads to tumor cell death which may feed tumor antigen into host antigen presentation pathways.

Combination therapies can also be used in combination with bispecific antibodies. Bispecific antibodies can be used to target two separate antigens. For example anti-Fc receptor/anti tumor antigen (*e.g.,* Her-2/neu) bispecific antibodies have been used to target macrophages to sites of tumor. This targeting may more effectively activate tumor specific responses. The T cell arm of these responses would by augmented by the use of PD-1 blockade. Alternatively, antigen may be delivered directly to DCs by the use of bispecific antibodies which bind to tumor antigen and a dendritic cell specific cell surface marker.

Tumors evade host immune surveillance by a large variety of mechanisms. Many of these mechanisms may be overcome by the inactivation of proteins which are expressed by the tumors and which are immunosuppressive. These include among others TGF-beta (Kehrl, J. et al. (1986) J. Exp. Med. 163: 1037-1050), IL-10 (Howard, M. & O'Garra, A. (1992) Immunology Today 13: 198-200), and Fas ligand (Hahne, M. et al. (1996) Science 274: 1363-1365). Antibodies or antigen-binding fragments thereof to each of these entities may be used in combination with anti-PD-1 to counteract the effects of the immunosuppressive agent and favor tumor immune responses by the host.

Other antibodies which may be used to activate host immune responsiveness can be used in combination with the combination therapies described herein. These include molecules on the surface of dendritic cells which activate DC function and antigen presentation. Anti-CD40 antibodies are able to substitute effectively for T cell helper activity (Ridge, J. et al. (1998) Nature 393: 474-478) and can be used in conjunction with PD-1 antibodies (Ito, N. et al. (2000) Immunobiology 201 (5) 527-40). Antibodies to T cell costimulatory molecules such as CTLA-4 (*e.g.,* U.S. Pat. No. 5,811,097), OX-40 (Weinberg, A. et al. (2000) Immunol 164: 2160-2169), 4-1BB (Melero, I. et al. (1997) Nature Medicine 3: 682-685 (1997), and ICOS (Hutloff, A. et al. (1999) Nature 397: 262-266) may also provide for increased levels of T cell activation.

In all of the methods described herein, PD-1 blockade can be combined with other forms of immunotherapy such as cytokine treatment (*e.g.,* interferons, GM-CSF, G-CSF, IL-2, IL-21), or bispecific antibody therapy, which provides for enhanced presentation of tumor antigens (*see e.g.,* Holliger (1993) Proc. Natl. Acad. Sci. USA 90:6444-6448; Poljak (1994) Structure 2:1121-1123).

The combination therapies disclosed herein can be further combined with an immunogenic agent, such as cancerous cells, purified tumor antigens (including recombinant proteins, peptides, and carbohydrate molecules), cells, and cells transfected with genes encoding immune stimulating cytokines (He et al. (2004) J. Immunol. 173:4919-28). Non-limiting examples of tumor vaccines that can be used include peptides of melanoma antigens, such as peptides of gp100, MAGE antigens, Trp-2, MARTI and/or tyrosinase, or tumor cells transfected to express the cytokine GM-CSF.

PD-1 blockade can be combined with a vaccination protocol. Many experimental strategies for vaccination against tumors have been devised (*see* Rosenberg, S., 2000, Development of Cancer Vaccines, ASCO Educational Book Spring: 60-62; Logothetis, C., 2000, ASCO Educational Book Spring: 300-302; Khayat, D. 2000, ASCO Educational Book Spring: 414-428; Foon, K. 2000, ASCO Educational Book Spring: 730-738; *see* also Restifo, N. and Sznol, M., Cancer Vaccines, Ch. 61, pp. 3023-3043 in DeVita, V. et al. (eds.), 1997, Cancer: Principles and Practice of Oncology. Fifth Edition). In one of these strategies, a vaccine is prepared using autologous or allogeneic tumor cells. These cellular vaccines have been shown to be most effective when the tumor cells are transduced to express GM-CSF. GM-CSF has been shown to be a potent activator of antigen presentation for tumor vaccination (Dranoff et al. (1993) Proc. Natl. Acad. Sci. U.S.A. 90: 3539-43).

PD-1 blockade can be used in conjunction with a collection of recombinant proteins and/or peptides expressed in a tumor in order to generate an immune response to these proteins. These proteins are normally viewed by the immune system as self antigens and are therefore tolerant to them. The tumor antigen may also include the protein telomerase, which is required for the synthesis of telomeres of chromosomes and which is expressed in more than 85% of human cancers and in only a limited number of somatic tissues (Kim, N et al. (1994) Science 266: 2011-2013). (These somatic tissues may be protected from immune attack by various means). Tumor antigen may also be "neo-antigens" expressed in cancer cells because of somatic mutations that alter protein sequence or create fusion proteins between two unrelated sequences (ie. bcr-abl in the Philadelphia chromosome), or idiotype from B cell tumors.

Other tumor vaccines may include the proteins from viruses implicated in human cancers such a Human Papilloma Viruses (HPV), Hepatitis Viruses (HBV and HCV) and Kaposi's Herpes Sarcoma Virus (KHSV). Another form of tumor specific antigen which may be used in conjunction with PD-1 blockade is purified heat shock proteins (HSP) isolated from the tumor tissue itself. These heat shock proteins contain fragments of proteins from the tumor cells and these HSPs are highly efficient at delivery to antigen presenting cells for eliciting tumor immunity (Suot, R & Srivastava, P (1995) Science 269:1585-1588; Tamura, Y. et al. (1997) Science 278:117-120).

Dendritic cells (DC) are potent antigen presenting cells that can be used to prime antigen-specific responses. DC's can be produced *ex vivo* and loaded with various protein and peptide antigens as well as tumor cell extracts (Nestle, F. et al. (1998) Nature Medicine 4: 328-332). DCs may also be transduced by genetic means to express these tumor antigens as well. DCs have also been fused directly to tumor cells for the purposes of immunization (Kugler, A. et al. (2000) Nature Medicine 6:332-336). As a method of vaccination, DC immunization may be effectively combined with PD-1 blockade to activate more potent anti-tumor responses.

### Second Therapeutic Agents

The second therapeutic agent can be chosen from one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor; 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor; or 17) a BCR-ABL inhibitor; *e.g.,* chosen from one or more of the agents listed in Table 1.

**Table 1**

| Structure Name | Name | Compound Structure | Patent Publications | Exemplary Indication/Uses | Generic structure |
|---|---|---|---|---|---|
| | | 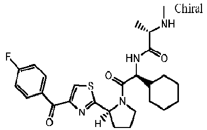 | WO 2008/016893 | | 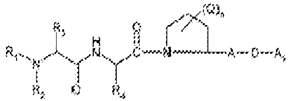 |
| | | | EP 2051990 | | |
| | | | US 8,546,336 | | |
| | | | (see, e.g., in WO2668/016893 (pgs. 2-4); Compound of formula (I), wherein: | Multiple Myeloma Therapy | |
| LCL161 | | | R₁ is H; | Breast Cancer Therapy | |
| | | | R₂ is C₁-C₄ alkyl; | Pancreatic Cancer Therapy | |
| | | | R₃ is C₁-C₄ alkyl; | Hematopoiesis Disorders Therapy | |
| | | | R₄ is C₃-C₁₀ cycloalkyl; | | |
| | | | A is het; | | |
| | | | D is C(O); | | |
| | | | A1 is substituted aryl; and n is 0. | | |
| | | | Specific compound: Compound A in Example 1. paragraph [122], pg. 29. | | |
| | | | Preparation of specific compound: Example 1). | | |
| | | 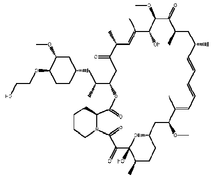 | WO 2014/085318 | Interstitial Lung Diseases, Treatment of Small Cell Lung Cancer Therapy | |
| | | | | Respiratory/Thoracic Cancer Therapy | |
| | | | | Prostate Cancer Therapy | |
| | | | | Multiple Myeloma Therapy | |
| | | | | Sarcoma Therapy | |
| Rad-001 | Everolimus, Afinitor | | | Age-Related Macular Degeneration, Treatment of Bone Cancer Therapy | |
| | | | | Tuberous Sclerosis, Treatment of Non-Small Cell Lung Cancer Therapy | |
| | | | | Endocrine Cancer Therapy | |
| | | | | Lymphoma Therapy | |
| | | | | Neurologic Drugs (Miscellaneous) | |
| | | | | Astrocytoma Therapy | |
| | | | | Cervical Cancer Therapy | |
| | | | | Neurologic Cancer Therapy | |
| | | | | Leukemia Therapy | |
| | | | | Immunosuppressants | |
| | | | | Treatment of Transplant Rejection | |
| | | | | Gastric Cancer Therapy | |
| | | | | Melanoma Therapy | |
| | | | | Antiepileptic Drugs | |
| | | | | Breast Cancer Therapy | |
| | | | | Bladder Cancer Therapy | |
| | | | | Oncolytic Drugs | |
| | | 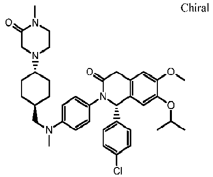 | WO 2011/076786 | Solid Tumor Therapy | 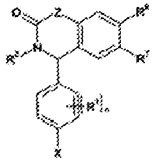 |
| | | | (see, e.g., pgs. 2-11); Compound of formula (I), wherein: Z is CH₂; | | |
| | | | X is halogen; | | |
| CGM097 | | | each of R⁶ and R⁷ is R'O-, wherein R' is C₁-C₇ alkyl; R² is phenyl, substituted in the para position by (R³)₂N-Y-, wherein Y is a bond, one R³ is C₁-C₇ alkyl; and the second R³ is (R⁵)₂-N-C₃-C₁₂ cycloalkyl-C₁-C₇ alkyl, wherein both R5, together with the N to which they are attached, form a 6-membered heterocyclic ring containing 1 N atom, wherein said heterocyclic ring is substituted with oxo and C₁-C₇ alkyl; and n is 0. | | |
| | | | Specific compound and preparation thereof: Example 106, pg. 265). | | |
| | | 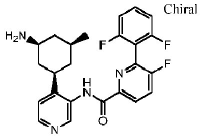 | WO 2010/026124 | Multiple Myeloma Therapy | 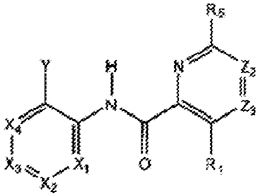 |
| PIM kinase inhibitor | | | EP 2344474 | Myelodysplastic Syndrome Therapy | |
| | | | US 2010/0056576 | Myeloid Leukemia Therapy | |
| | | | (see, e.g., WO2010/026124 (pgs. 9-10); Compound of Formula I, wherein: | Non-Hodgkin's Lymphoma Therapy | |
| | | | X₁, X₃, and X₄ are CR₂, wherein R₂ is hydrogen; | | |
| | | | X₂ is N; | | |
| | | | Y is substituted cycloalkyl; | | |
| | | | Z₂ and Z₃ are CR₁₂, wherein R₁₂ is hydrogen or halo; and | | |
| | | | R₅ is substituted aryl. | | |
| | | | Specific compound and preparation thereof: Example 70, pg. 132) | | |
| | | | | Gastric Cancer Therapy | |
| LJM716 | | Human monoclonal antibody | WO 2012/022814 | Esophageal Cancer | |
| | | | EP 2606070 | Stomach Cancer | |
| | | | US 8,735,551 | Oncolytic Drugs | |
| | | | | Breast Cancer Therapy | |
| | | | | Digestive/Gastrointestinal Cancer Therapy | |
| | | | | Head and Neck Cancer Therapy | |
| | | 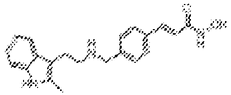 | | Small Cell Lung Cancer Therapy | |
| LBH589 | Panobinostat | | WO 2014/072493 | Respiratory/Thoracic Cancer Therapy | |
| | | | WO 2002/022577 | Prostate Cancer Therapy | |
| | | | EP 1870399 | Multiple Myeloma Therapy | |
| | | | | Myelodysplastic Syndrome Therapy | |
| | | | (see, e.g., WO2002/022577 (pgs. 4-6); Compound of formula (I), wherein: | Bone Cancer Therapy | |
| | | | | Non-Small Cell Lung Cancer Therapy | 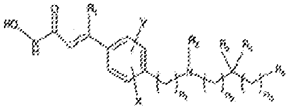 |
| | | | R¹ is H; | Endocrine Cancer Therapy | |
| | | | R² is H; | Lymphoma Therapy | |
| | | | R³ and R ⁴ are H; | Neurologic Cancer Therapy | |
| | | | R⁵ is substituted heteroaryl; | Leukemia Therapy | |
| | | | X and Y are H; | Anti-HIV Agents | |
| | | | n¹ is 1; n² is 1; and n³ is 0. | Immunosuppressants | |
| | | | Specific compound is Example 200, pg. 63). | Treatment of Transplant Rejection Gastric Cancer Therapy | |
| | | | | Melanoma Therapy | |
| | | | | Breast Cancer Therapy | |
| | | | | Pancreatic Cancer Therapy | |
| | | | | Colorectal Cancer Therapy | |
| | | | | Glioblastoma Multiforme Therapy | |
| | | | | Myeloid Leukemia Therapy | |
| | | | | Hematological Cancer Therapy | |
| | | | | Renal Cancer Therapy | |
| | | | | Non-Hodgkin's Lymphoma Therapy | |
| | | | | Head and Neck Cancer Therapy | |
| | | | | Hematopoiesis Disorders Therapy | |
| | | | | Liver Cancer Therapy | |
| | | 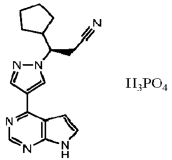 | | Prostate Cancer Therapy | |
| INC424 | Ruxolitinib Phosphate Jakavi | | WO 2007/070514 | Lymphocytic Leukemia Therapy | |
| | | | EP 2474545 | Multiple Myeloma Therapy | |
| | | | US 7,598,257 | Lymphoma Therapy | |
| | | | WO 2014/018632 | Lung Cancer Therapy | |
| | | | | Leukemia Therapy | |
| | | | (see, e.g., in WO2007/070514 (pgs. 8-12); Compound of | Treatment of Cachexia | 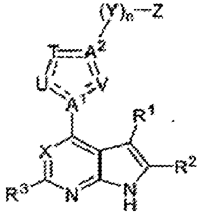 |
| | | | Formula I, wherein: | Breast Cancer Therapy | |
| | | | A₁ is C; | Pancreatic Cancer Therapy | |
| | | | A₂ and T are N; | Rheumatoid Arthritis, Treatment of | |
| | | | U and V are CR₅; wherein R₅ is H; | Antipsoriatics | |
| | | | X is N; | Colorectal Cancer Therapy | |
| | | | Y is C₁-₈ alkylene, substituted with -D¹-D²-D³-D⁴, wherein | Myeloid Leukemia Therapy | |
| | | | D¹, D², and D3 are absent, and D⁴ is CN; | Hematological Cancer Therapy | |
| | | | Z is Cy¹, wherein Cy¹ is cycloalkyl; | Treatment of Autoimmune Diseases | |
| | | | R¹ and R ² are H; and n is 1. | Non-Hodgkin's Lymphoma Therapy | |
| | | | | Antithrombocythemic | |
| | | | Specific compound and preparation thereof: Example 67, pgs. 91-93). | Hematologic Agents (Miscellaneous) | |
| | | 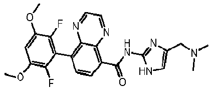 | WO 2009/141386 | Angiogenesis Inhibition | 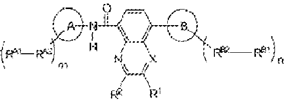 |
| BUW078 | | | US 2010/0105667 | Signal Transduction Modulation | |
| | | | (see, e.g., WO2009/141386 (pgs. 9-10); Compound of Formula (I), wherein: | | |
| | | | X is N; | | |
| | | | R¹ and R ² are hydrogen; | | |
| | | | A is heteroaryl; | | |
| | | | R^{A1} is a -NR^{A 3} R^{A4}, wherein R^{A3} and R^{A4} are each C₁₋₇ alkyl, and R^{A2} is a alkanediyl; | | |
| | | | B is aryl; | | |
| | | | R^{B1} is halo or straight-chain C₁₋₇ alkoxy, and R^{B1} is a direct bond; | | |
| | | | m is 1, and n is 4. | | |
| | | | Specific compound and preparation thereof: Example 127, pg. 146) | | |
| BGJ398 | | | US 8,552,002 | Digestive/Gastrointestinal Cancer Therapy | |
| | | | | Hematological Cancer Therapy | |
| | | 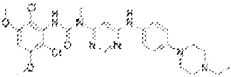 | (see e.g., Example 145, col 171 of US 8,552,002; e.g., encompassed by Formula (I) found in col 6. | Solid Tumors Therapy | 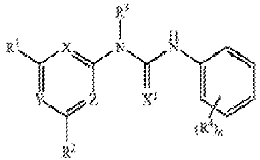 |
| | | | X is CR⁵, wherein R⁵ is H | | |
| | | | Y is N | | |
| | | | Z is N | | |
| | | | X¹ is O | | |
| | | | R¹ is a substituted organic moiety attached via a linker (L1), | | |
| | | | -L1-, wherein the organic moiety is a cyclic group (specifically phenyl) substituted by 4-ethylpiperazinyl and | | |
| | | | -L1- is NR^{a} wherein R^{a} is H | | |
| | | | R² is an organic moiety, specifically H | | |
| | | | R³ is an organic moiety, specifically lower aliphatic, e.g.. methyl | | |
| | | | n is 4 | | |
| | | | R⁴ is specifically chloro, chloro, methoxy, or methoxy). | | |
| EGF816 | | 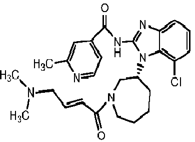 | WO2013/184757 | Cancer Therapy | 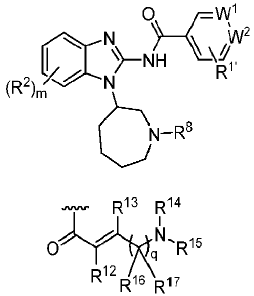 |
| | | | | Solid Tumor Therapy | |
| | | | (see e.g., Example 5; generically disclosed by Formula (5) -see claims 7, 10, 11 and 12. | | |
| | | | • W¹ is CR¹; | | |
| | | | • W² is N; | | |
| | | | • R¹ is methyl and R¹' is hydrogen; | | |
| | | | • R² is chloro; m=1 | | |
| | | | • R⁸ is substructure (h), q=1 | | |
| | | | • R¹², R¹³, R¹⁶ and R¹⁷ are hydrogen | | |
| | | | • R¹⁴ and R¹⁵ are methyl). | | |
| INC280 | | 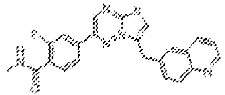 | | Non-Small Cell Lung Cancer Therapy | 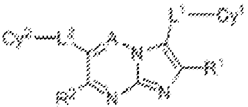 |
| | | | EP2099447; | Glioblastoma Multiforme Therapy | |
| | | | US7,767,675 | Renal Cancer Therapy | |
| | | | | Solid Tumors Therapy | |
| | | | (see e.g., for a generic in Claim 1 of EP2099447; species in claim 53 of EP2099447, and claim 4 of US patent 7,767,675). | Liver Cancer Therapy | |
| LDK378 | Zykadia | 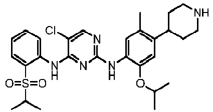 | WO2008/073687; US 8,039,479 | Non-Small Cell Lung Cancer Therapy | 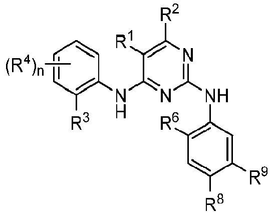 |
| | | | (see e.g., Example 7, compound 66 of WO2008/073687; | Solid Tumors Therapy | |
| | | | US 8,039,479: genus in claim 1; species in claim 5 Subgenus Formula (2) | | |
| | | | R¹ is halo; | | |
| | | | R² is H; | | |
| | | | R³ is SO₂R¹² and R¹² is C₁₋₆ alkyl; | | |
| | | | R⁴ is H (n=1); | | |
| | | | R⁶ is isopropoxy; and | | |
| | | | one of R⁸ and R⁹ is (CR²)_{q}Y wherein q is 0, Y is | | |
| | | | piperidinyl and the other is C₁₋₆ alkyl). | | |
| | | 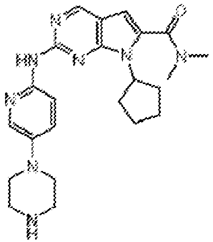 | US 8,415,355 | Lymphoma Therapy | 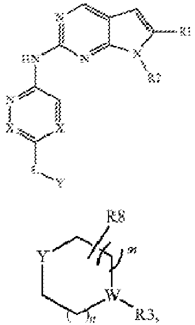 |
| LEE011 | | | US 8,685,980 | Neurologic Cancer Therapy | |
| | | | | Melanoma Therapy | |
| | | | (see e.g., Example 74, col 66 of US 8,415,355; | Breast Cancer Therapy | |
| | | | generically disclosed by Formula (I) | Solid Tumors Therapy | |
| | | | found in col 3-4 of of US 8,415,355:. | | |
| | | | X is CR9, wherein R9 is H | | |
| | | | R1 is CONR5R6, wherein R5 and R6 are both C₁₋₈ | | |
| | | | alkyl, specifically methyl | | |
| | | | R2 is C₃₋₁₄ cycloalkyl, specifically cyclopentyl L is a bond | | |
| | | | Y is part of the disclosed group, wherein Y is N, zero R8 are present, W is N, m and n are both 1, and R3 is H). | | |
| | | | See also, US 8,685,980 | | |
| BMK120 | Buparlisib | 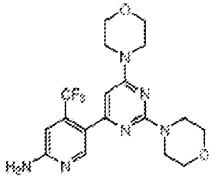 | WO2007/084786 | Prostate Cancer Therapy | 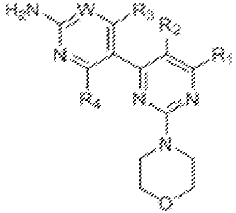 |
| | | | | Non-Small Cell Lung Cancer Therapy | |
| | | | Chemical name: 4-(trifluoromethyl)-5-(2,6-dimorpholinopyrimidin-4-yl)pyridin-2-amine | Endocrine Cancer Therapy, | |
| | | | | Leukemia Therapy, Ovarian Cancer Therapy | |
| | | | | Melanoma Therapy, Bladder Cancer Therapy | |
| | | | (see e.g., WO20071084786 (on pages 21-22); Compound of formula (I), wherein | Oncolytic Drugs Breast Cancer Therapy | |
| | | | | Female Reproductive System Cancer Therapy | |
| | | | W is CRw and Rw is hydrogen | | |
| | | | R₁ is unsubstituted heterocyclyl | Digestive/Gastrointestinal Cancer Therapy | |
| | | | | Colorectal Cancer Therapy | |
| | | | R₂ is hydrogen R₃ is substituted alkyl | Glioblastoma Multiforme Therapy | |
| | | | R₄ is hydrogen | Solid Tumors Therapy | |
| | | | | Non-Hodgkin's Lymphoma Therapy | |
| | | | Specific compound: Example 10 (in paragraph [0389] on page 140) | Hematopoiesis Disorders Therapy | |
| | | | | Head and Neck Cancer Therapy | |
| | | | Preparation of specific compound: Example 10). | | |
| BYL719 | | 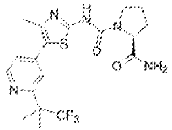 | WO 2010/029082; | Gastric Cancer Therapy | 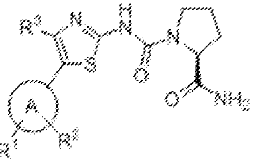 |
| | | | Chemical name: (S)-Pyrrolidine-1,2- dicarboxylic acid 2-amide 1-({4-methyl-5-[2-(2,2,2-trifluoro-1,1-dimethyl-ethyl)-pyridin-4-yl]-thiazol-2-yl}-amide) | Breast Cancer Therapy | |
| | | | | Pancreatic Cancer Therapy | |
| | | | | Digestive/Gastrointestinal Cancer Therapy | |
| | | | | Solid Tumors Therapy | |
| | | | (see e.g., in WO2010/029082: | Head and Neck Cancer Therapy | |
| | | | • Specific compound: Example 15 on page 55 | | |
| | | | • Preparation of specific compound: Example 15 on pages 55-56 | | |
| | | | Genus disclosed (see e.g., claim 1 on page 138-139); Compound of formula I, wherein | | |
| | | | A is pyridyl, pyrimidinyl, pyrazinyl, 1H-benzo[d]imidazolyl; R¹ is substituted C₁-C₇ alkyl, wherein said substituents are independently selected from one or more, preferably one to nine of the following deuterium, fluoro, or one or two of the following moieties C₃-C₅ cycloalkyl | | |
| | | | R² is hydrogen | | |
| | | | R³ is methyl). | | |
| LGX818 | Encorafenib | 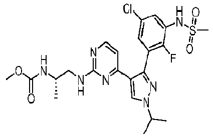 | WO2611/025927; | Non-Small Cell Lung Cancer Therapy | 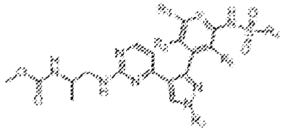 |
| | | | US 8,501,758 | Melanoma Therapy | |
| | | | (see e.g., Compound Structure: See page 59 (Example 6/compound 9) of WO2011025927; | Colorectal Cancer Therapy | |
| | | | See col 45 in US 8,501,758 | | |
| | | | R₂ is H; | | |
| | | | R₃ is halo (chloro) | | |
| | | | R₄ is R₉, and R₉ is C₁₋₆ alkyl (methyl) R₅ is halo (fluoro) | | |
| | | | R₇ is C₁₋₄alkyl (isopropyl); Y is CR₆ and R₆ is H | | |
| | | | WO2011/025927: generic structure on p. 6 and structure on p. 59). | | |
| CTL019 | Tisagenlecleucel-T | CART-19 | WO2012/079000 | Lymphocytic Leukemia Therapy | |
| | | | (see e.g., page 58, 65, SEQ ID NO: 12 is full CAR, and SEQ ID NO: 14 is CD19 scFv). | Non-Hodgkin's Lymphoma Therapy | |
| MEK162 | Binimetinib | 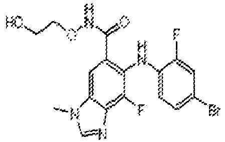 | WO03/077914 | Non-Small Cell Lung Cancer Therapy | 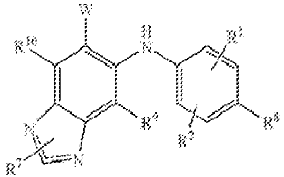 |
| | | | (see e.g., | Multisystem Genetic Disorders, Treatment of Melanoma Therapy | |
| | | | Generic structure: See page 8-10 of WO03/077914; | Ovarian Cancer Therapy | |
| | | | specific structure: See page 70 (Example 18/compound | Digestive/Gastrointestinal Cancer Therapy | |
| | | | 29III) of WO03/077914; R¹ is halogen; R² is hydrogen | Treatment of Rheumatoid Arthritis | |
| | | | R³ is C₁-C₁₀ alkyl substituted with OR' and R' is hydrogen | Colorectal Cancer Therapy | |
| | | | R⁴ is hydrogen; R⁷ is C₁-C₁₀ alkyl | | |
| | | | R⁸ is -Br; R⁹ is halogen | | |
| | | | R¹⁰ is hydrogen; W is -C(O)NR⁴OR³). | | |
| AMN107 | Nilotinib HCl monohydrate, Tasignia | 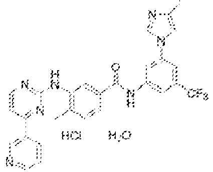 | WO2004/005281 | Lymphocytic Leukemia Therapy | 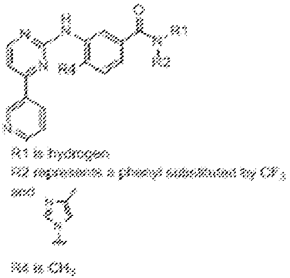 |
| | | | US 7,169,791 | Antiparkinsonian Drugs | |
| | | | | Neurologic Cancer Therapy | |
| | | | (see e.g., Example 92 of WO2004/005281; and | Melanoma Therapy | |
| | | | Formula (I), claim 1 and claim 8 of US 7,169,791). | Digestive/Gastrointestinal Cancer Therapy | |
| | | | | Colorectal Cancer Therapy | |
| | | | | Myeloid Leukemia Therapy | |
| | | | | Head and Neck Cancer Therapy | |
| | | | | Treatment of Pulmonary Hypertension | |

### Exemplary Combination Therapies

In certain embodiments, an inhibitor of the immune checkpoint molecule is used in a method or composition described herein. For example, an inhibitor of the immune checkpoint molecule described herein, *e.g.,* the PD-1 inhibitor, *e.g.,* the anti-PD-1 antibody (*e.g.,* Nivolumab or Pembrolizumab); or the PD-L1 inhibitor, *e.g.,* the anti-PD-Ll antibody (*e.g.,* MSB0010718C) (alone or in combination with other immunomodulators) is used in combination with one or more of the agents listed in Table 1; *e.g.,* 1) an Inhibitor of Apoptosis (IAP) inhibitor; 2) an inhibitor of a Target of Rapamycin (TOR) kinase; 3) an inhibitor of a human homolog of mouse double minute 2 E3 ubiquitin ligase (HDM2); 4) a PIM kinase inhibitor; 5) an inhibitor of Human epidermal growth factor 3 (HER3) kinase; 6) a Histone Deacetylase (HDAC) inhibitor; 7) a Janus kinase inhibitor; 8) an fibroblast growth factor receptor (FGF) receptor inhibitor; 9) an epidermal growth factor (EGF) receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor, or 17) a BCR-ABL inhibitor. In one embodiment, one or more of the aforesaid combinations is used to treat a disorder, *e.g.,* a disorder described herein (*e.g.,* a disorder disclosed in Table 1). In one embodiment, one or more of the aforesaid combinations is used to treat a cancer, *e.g.,* a cancer described herein (*e.g.,* a cancer disclosed in Table 1).

In some embodiments, one or more of the immunomodulators described herein are used in combination with:
1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone);
3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
4) N-(4-((1R,3S,5S)-3-amino-5-methylcyclohexyl)pyridin-3-yl)-6-(2,6-difluorophenyl)-5-fluoropicolinamide;
5) anti-HER3 monoclonal antibody or antigen binding fragment thereof, that comprises a VH of SEQ ID NO: 141 and VL of SEQ ID NO: 140, as described in U.S. 8,735,551;
6) (E)-N-hydroxy-3-(4-(((2-(2-methyl-1H-indol-3-yl)ethyl)amino)methyl)phenyl) acrylamide;
7) (3R)-3-cyclopentyl-3-[4-(7H-pyrrolo-[2,3-d]pyrimidin-4-yl)-1H-pyrazol-1-yl]propanenitrile; and/or
8) 8-(2,6-difluoro-3,5-dimethoxy-phenyl)-quinoxaline-5-carboxylic acid (4-dimethylaminomethyl-1H-imidazol-2-yl)-amide.

Each of these combinations is discussed in more detail below.

In one embodiment, the inhibitor of the immune checkpoint molecule (alone or in combination with other immunomodulators) is used in combination with an IAP inhibitor to treat a cancer, *e.g.,* a cancer described herein (*e.g.,* a cancer disclosed in Table 1). In one embodiment, the IAP inhibitor is disclosed in Table 1, *e.g.,* LCL161, or in a publication recited in Table 1, *e.g.,* International Patent Publication No. WO2668/016893 (*e.g.,* Formula (I), Example 1, and Compound A), European Patent No. 2051990, and U.S. Patent No. 8,546,336. In certain embodiments, the IAP inhibitor is disclosed, *e.g.,* in International Patent Publication No. WO2668/016893 (*e.g*., Formula (I), Example 1, and Compound A), European Patent No. 2051990, and U.S. Patent No. 8,546,336. In one embodiment, the IAP inhibitor, *e.g.,* LCL161, has the structure (compound or generic) provided in Table 1, or as disclosed in the publication recited in Table 1, *e.g.,* International Patent Publication No. WO2668/016893 (*e.g.,* Formula (I), Example 1, and Compound A), European Patent No. 2051990, and U.S. Patent No. 8,546,336. In one embodiment, the inhibitor of the immune checkpoint molecule (*e.g.,* one of Nivolumab, Pembrolizumab or MSB0010718C) is used in combination with LCL161 to treat a cancer or disorder described in Table 1, *e.g.,* a solid tumor, *e.g.,* a breast cancer or a pancreatic cancer; or a hematological malignancy, *e.g.,* multiple myeloma or a hematopoeisis disorder.

In one embodiment, the IAP inhibitor is a compound of Formula (I): 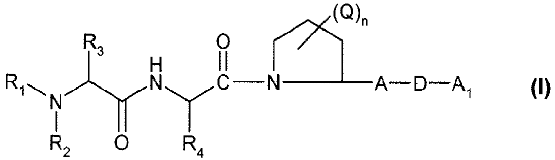 or a pharmaceutically acceptable salt thereof, wherein
R₁ is H, C₁-C₄ alkyl, C₂-C₄ alkenyl, C₂-C₄ alkynyl or C₃-C₁₀ cycloalkyl, which R₁ may be unsubstituted or substituted;
R₂ is H, C₁-C₄ alkyl, C₂-C₄ alkenyl, C₂-C₄ alkynyl, C₃-C₁₀ cycloalkyl which R₂ may be unsubstituted or substituted;
R₃ is H, CF₃, C₂F₆, C₁-C₄ alkyl, C₂-C₄ alkenyl, C₂-C₄ alkynyl, CH₂-Z, or
R₂ and R₃, taken together with the nitrogen atom to which they are attached, form a heterocyclic ring, which alkyl, alkenyl, alkynyl or het ring may be unsubstituted or substituted;
Z is H, OH, F, Cl, CH₃, CH₂CI, CH₂F or CH₂OH;
R₄ is C₀₋₁₀ alkyl, C₀₋₁₀ alkenyl, C₀₋₁₀ alkynyl, C₃-C₁₀ cycloalkyl, wherein the C₀₋₁₀ alkyl, or cycloalkyl group is unsubstituted or substituted;
A is het, which may be substituted or unsubstituted;
D is C₁-C₇ alkylene or C₂-C₉ alkenylene, C(O), O, NR₇, S(O)r, C(O)-C₁-C₁₀ alkyl, O-C₁-C₁₀ alkyl, S(O)r-CᵣC₁₀ alkyl, C (O) C₀-C₁₀ arylalkyl, OC₀-C₁₀ arylalkyl, or S(O)r C₀-C₁₀ arylalkyl, which alkyl and aryl groups may be unsubstituted or substituted;
r is O, 1 or 2;
A₁ is a substituted or unsubstituted aryl or unsubstituted or substituted het which substituents on aryl and het are halo, alkyl, lower alkoxy, NR₅R₆, CN, NO₂ or SR₅;
each Q is independently H, C₁-C₁₀ alkyl, C₁-C₁₀ alkoxy, aryl C₁-C₁₀ alkoxy, OH, O-C₁-C₁₀ alkyl, (CH₂)₀-₆-C₃-C₇ cycloalkyl, aryl, aryl C₁-C₁₀ alkyl, O-(CH₂)₀-₆ aryl, (CH₂)₁-₆ het, het, O-(CH₂)₁₋₆ het, -OR₁₁, C(O)R₁₁, -C(O)N(R₁₁)(R₁₂), N(R₁₁)(R₁₂J₁SR₁₁, S(O)R₁₁₁S(O)₂R₁₁, S(O)₂-N(R₁₁)(R₁₂), or NR₁₁-S(O)₂-(R₁₂), wherein alkyl, cycloalkyl and aryl are unsubstituted or substituted;
n is 0, 1 ,2 or 3, 4, 5, 6 or 7;
het is a 5- to 7-membered monocyclic heterocyclic ring containing 1-4 heteroring atoms selected from N, O and S or an 8- to 12-membered fused ring system that includes one 5- to 7-membered monocyclic heterocyclic ring containing 1, 2 or 3 heteroring atoms selected from N, O and S, which het is unsubstituted or substituted;
R₁₁ and R₁₂ are independently H, C₁-C₁₀ alkyl, (CH₂)₀-₆-C₃-C₇ cycloalkyl, (CH₂)₀-₆-(CH)₀-₁(aryl)_{1·2},C(O)-C₁-C₁₀ alkyl, -C(O)-(CH₂)₁₋₆-C₃-C₇ cycloalkyl, -C(O)-O-(CH₂)₀₋₆-aryl,-C(O)-(CH₂)₀₋₆-O-fluorenyl, C(O)-NH-(CH₂)₀₋₆-aryl, C(O)-(CH₂)₀₋₆-aryl, C(O)- (CH₂)₁₋₆-het,-C(S)-CᵣC₁₀alkyl, -C(S)-(CH₂)_{L6}-C₃-C₇ cycloalkyl, -C(S)-O-(CH₂W aryl, -C(S)-(CH₂)₀₋₆-O-fluorenyl, C(S)-NH-(CH₂)₀₋₆-aryl, -C(S)-(CH₂)₀₋₆-aryl or C(S)-(CH₂)₁₋₆-het, C(O)R₁₁, C(O)NR₁₁R₁₂, C(O)OR_{11,} S(O)ₙR₁₁, S(O)₁ₙNR₁₁R₁₂, m = 1 or 2, C(S)R₁₁, C(S)NR₁₁R₁₂, C(S)OR₁₁, wherein alkyl, cycloalkyl and aryl are unsubstituted or substituted; or R₁₁ and R₁₂ are a substituent that facilitates transport of the molecule across a cell membrane,
or R₁₁ and R₁₂ together with the nitrogen atom form het,
wherein the alkyl substituents of R₁₁ and R₁₂ may be unsubstituted or substituted by one or more substituents selected from C₁-C₁₀ alkyl, halogen, OH, O-C₁-C₆ alkyl, -S-C₁-C₆ alkyl, CF₃ or NR₁₁R₁₂;
substituted cycloalkyl substituents of R₁₁ and R₁₂ are substituted by one or more substituents selected from a C₂-C₁₀ alkene; C₁-C₆ alkyl; halogen; OH; 0-C₁-C₆ alkyl; S-C₁-C₆ alkyl,CF₃; or NR₁₁R₁₂;
substituted het or substituted aryl of R₁₁ and R₁₂ are substituted by one or more substituents selected from halogen, hydroxy, C₁-C₄ alkyl, C₁-C₄ alkoxy, nitro, CNO-C(O)-CᵣC₄alkyl and C(O)-O-CᵣC₄-alkyl;
R₅, R₆ and R₇ are independently hydrogen, lower alkyl, aryl, aryl lower alkyl, cycloalkyl, or cycloalkyl lower alkyl, C(O)R₅; S(O)R₅C(O)OR₅C(O)NR₅R₆, and
the substituents on R₁, R₂, R₃, R₄, Q, and A and A₁ groups are independently halo, hydroxy, lower alkyl, lower alkenyl, lower alkynyl, lower alkanoyl, lower alkoxy, aryl, aryl lower alkyl, amino, amino lower alkyl, diloweralkylamino, lower alkanoyl, amino lower alkoxy, nitro, cyano, cyano lower alkyl, carboxy, lower carbalkoxy, lower alkanoyl, aryloyl, lower arylalkanoyl, carbamoyl, N-mono- or N,N-di lower alkyl carbamoyl, lower alkyl carbamic acid ester, amidino, guanidine, ureido, mercapto, sulfo, lower alkylthio, sulfoamino, sulfonamide, benzosulfonamide, sulfonate, sulfanyl lower alkyl, aryl sulfonamide, halogen substituted aryl sulfonate, lower alkylsulfinyl, arylsulfinyl; aryl-lower alkylsulfinyl, lower alkylarylsulfinyl, lower alkylsulfonyl, arylsulfonyl, aryl-lower alkylsulfonyl, lower aryl alkyl lower alkylarylsulfonyl, halogen- lower alkylmercapto, halogen-lower alkylsulfonyl, phosphono (-P(=O)(OH)₂), hydroxy-lower alkoxy phosphoryl or di-lower alkoxyphosphoryl, (R₉)NC(O)-NR₁₀R₁₃, lower alkyl carbamic acid ester or carbamates or -NR₈R₁₄, wherein
R₈ and R₁₄ can be the same or different and are independently H or lower alkyl, or
R₈ and R₁₄, together with the N atom, form a 3- to 8-membered heterocyclic ring containing a nitrogen heteroring atoms and may optionally contain one or two additional heteroring atoms selected from nitrogen, oxygen and sulfur, which heterocyclic ring may be unsubstituted or substituted with lower alkyl, halo, lower alkenyl, lower alkynyl, hydroxy, lower alkoxy, nitro, amino, lower alkyl, amino, diloweralkyl amino, cyano, carboxy, lower carbalkoxy, formyl, lower alkanoyl, oxo, carbarmoyl, Λ/-lower or Λ/,Λ/-dilower alkyl carbamoyl, mercapto, or lower alkylthio; and
R₉, R₁₀ and R₁₃ are independently hydrogen, lower alkyl, halogen substituted lower alkyl, aryl, aryl lower alkyl, halogen substituted aryl, halogen substituted aryl lower alkyl.

In one embodiment, LCL161 has the following structure:

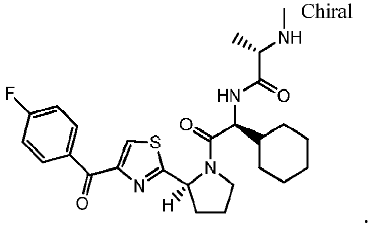

In one embodiment, LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide.

In one embodiment, the inhibitor of the immune checkpoint molecule (alone or in combination with other immunomodulators) is used in combination with a TOR kinase inhibitor to treat a cancer, *e.g.,* a cancer described herein (*e.g.,* a cancer disclosed in Table 1). In one embodiment, the TOR kinase inhibitor is disclosed in Table 1, *e.g.,* Rad-001, or in a publication recited in Table 1, *e.g.,* in International Patent Publication No. WO 2014/085318 (*e.g.,* Compound B). In one embodiment, the TOR kinase inhibitor, *e.g.,* Rad-001, has the structure (compound or generic structure) provided in Table 1, or as disclosed in the publication recited in Table 1, *e.g.,* International Patent Publication No. WO 2014/085318 (*e.g.,* Compound B). In one embodiment, the inhibitor of the immune checkpoint molecule (*e.g.,* one of Nivolumab, Pembrolizumab or MSB0010718C) is used in combination with Rad-001 to treat a cancer or disorder described in Table 1, *e.g.,* a solid tumor, *e.g.,* a sarcoma, a lung cancer (*e.g.,* a non-small cell lung cancer (NSCLC) (*e.g.,* a NSCLC with squamous and/or non-squamous histology)), a melanoma (*e.g.,* an advanced melanoma), a digestive/gastrointestinal cancer, a gastric cancer, a neurologic cancer, a prostate cancer, a bladder cancer, a breast cancer; or a hematological malignancy, *e.g.,* a lymphoma or leukemia.

In one embodiment, Rad-001 has the following structure:

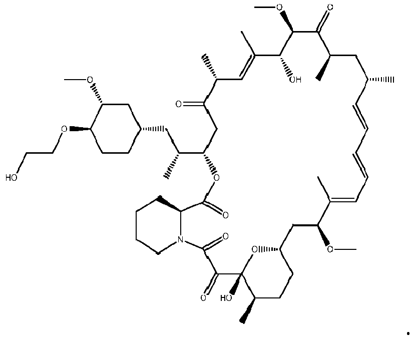

In one embodiment, Rad-001 is ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone).

In one embodiment, the inhibitor of the immune checkpoint molecule (alone or in combination with other immunomodulators) is used in combination with a HDM2 ligase inhibitor to treat a cancer, *e.g.,* a cancer described herein (*e.g.,* a cancer disclosed in Table 1). In one embodiment, the HDM2 ligase inhibitor is disclosed in Table 1, *e.g.,* CGM097, or in a publication recited in Table 1, *e.g.,* International Patent Publication No. WO2011/076786 (*e.g.,* Formula (I) or Example 106). In certain embodiments, the HDM2 ligase inhibitor is disclosed, *e.g.,* in International Patent Publication No. WO2011/076786 (*e.g.,* formula (I) or Example 106). In one embodiment, the HDM2 ligase inhibitor, *e.g.,* CGM097, has the structure provided in Table 1 (compound or generic structure), or as disclosed in the publication recited in Table 1, *e.g.,* International Patent Publication No. WO2011/076786 (*e.g.,* Formula (I) or Example 106). In one embodiment, the inhibitor of the immune checkpoint molecule (*e.g.,* one of Nivolumab, Pembrolizumab or MSB0010718C) is used in combination with CGM097 to treat a cancer or disorder described in Table 1, *e.g.,* a solid tumor.

In one embodiment, the HDM2 ligase inhibitor is a compound of formula (I), or a tautomer or N-oxide or pharmaceutically acceptable salt or solvate thereof, 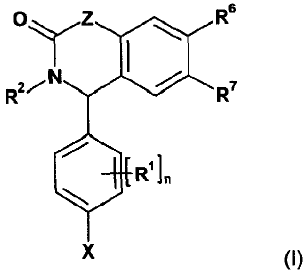 wherein
Z is CH₂ or N-R⁴;
X is halogen;
R⁴ is selected from the group consisting of H and C₁-C₇-alkyl;
R⁶ is independently selected from the group consisting of H, R'O, and (R')₂N;
R⁷ is independently selected from the group consisting of R'O and (R')₂N;
each R' is independently selected from the group consisting of H, C₁-C₇-alkyl, C₁-C₇-alkenyl, halo- C₁-C₇-alkyl, halo-C₁-C₇-alkenyl, C₃-C₁₂-cycloalkyl, heterocyclyl, aryl, hydroxy-C₁-C₇-alkyl, C₁-C₇-alkoxy-C₁-C₇ alkyl, amino-C₁-C₇-alkyl, N- C₁-C₇-alkyl-amino- C₁-C₇-alkyl, N,N-di- C₁-C₇-alkyl-amino- C₁-C₇-alkyl, C₃-C₁₂-cycloalkyl- C₁-C₇-alkyl, heterocyclyl- C₁-C₇-alkyl, aryl- C₁-C₇-alkyl, C₁-C₇-alkyl-carbonyl, halo- C₁-C₇-alkyl-carbonyl, hydroxy- C₁-C₇-alkyl-carbonyl-, C₁-C₇-alkoxy- C₁-C₇-alkyl-carbonyl, amino-C₁-C₇-alkyl-carbonyl, N- C₁-C₇-alkyl-amino- C₁-C₇-alkyl -carbonyl, C₃-C₁₂-cycloalkyl carbonyl, heterocyclyl- C₁-C₇-alkylcarbonyl, aryl- C₁-C₇-alkyl-carbonyl, C₃-C₁₂-cycloalkyl- C₁-C₇-alkyl-carbonyl, heterocyclyl-carbonyl, aryl-carbonyl, C₁-C₇-alkyl-carbonyl- C₁-C₇-alkyl, halo- C₁-C₇-alkyl-carbonyl, hydroxy- C₁-C₇-alkyl-carbonyl- C₁-C₇-alkyl, C₁-C₇-alkoxy- C₁-C₇-alkyl-carbonyl- C₁-C₇-alkyl, amino- C₁-C₇-alkyl-carbonyl- C₁-C₇-alkyl, heterocyclyl-carbonyl- C₁-C₇-alkyl, aryl-carbonyl-C₁-C₇-alkyl, carbonyl-C₁-C₇-alkyl, hydroxyl-carbonyl- C₁-C₇-alkyl, C₁-C₇-alkoxy-carbonyl- C₁-C₇-alkyl, amino-carbonyl- C₁-C₇-alkyl, N- C₁-C₇-alkyl-amino-carbonyl- C₁-C₇-alkyl, N,N-di-C₁-C₇-alkyl-amino-carbonyl- C₁-C₇-alkyl, C₃-C₁₂-cycloalkyl-carbonyl- C₁-C₇-alkyl, heterocyclyl-carbonyl- C₁-C₇-alkyl, aryl-carbonyl- C₁-C₇-alkyl, C₁-C₇-alkyl-carbonyl-amino-C₁-C₇-alkyl, C₁-C₇-alkyl-carbonyl-N-C₁-C₇-alkyl-amino-C₁-C₇-alkyl, halo-C₁-C₇-alkyl-carbonyl-amino-C₁-C₇-alkyl, halo- C₁-C₇-alkyl-carbonyl-N-C₁-C₇-alkyl-amino-C₁-C₇-alkyl, wherein aryl, heterocyclyl and C₃-C₁₂-cycloalkyl are unsubstituted or substituted by 1-4 substituents selected from C₁-C₇-alkyl, halo- C₁-C₇-alkyl, halogen, hydroxy, C₁-C₇-alkoxy, amino, nitro or cyano;
each R¹ is independently selected from the group consisting of halogen, cyano, nitro, C d-alkyl, C₁-C₇-alkenyl, halo- C₁-C₇-alkyl, hydroxyl, C₁-C₇-alkoxy, amino, N- C₁-C₇-alkyl-amino, N,N-di-C₁-C₇-alkyl-amino, amino-carbonyl-amino, N-C₁-C₇-alkyl-amino-carbonyl-amino, N.N-di-C₁-C₇-alkyl-amino-carbonyl-amino, C₁-C₇-alkyl-carbonyl-amino, amino-carbonyl, N-C₁-C₇-alkyl-amino-carbonyl, N,N-di- C₁-C₇-alkyl-amino-carbonyl, hydroxy- C₁-C₇-alkyl, amino-C₁-C₇-alkyl, N-C₁-C₇-alkyl-amino-C₁-C₇-alkyl, N,N-di-C₁-C₇-alkyl-amino-C₁-C₇-alkyl, C₁-C₇-alkyl-carbonyl-amino-C₁-C₇-alkyl, C₁-C₇-alkyl-carbonyl-N-C₁-C₇-alkyl-amino-C₁-C₇-alkyl; n is 0, 1 or 2;
   R² is selected from
   (A) phenyl, 2-pyridyl or 3-pyridyl, said phenyl, 2-pyridyl or 3-pyridyl being substituted in para-position (relative to the isoquinolinone or quinazolinone), by (R³)₂N-Y- wherein Y is absent (a bond) or (R₃)₂N-Y- is selected from 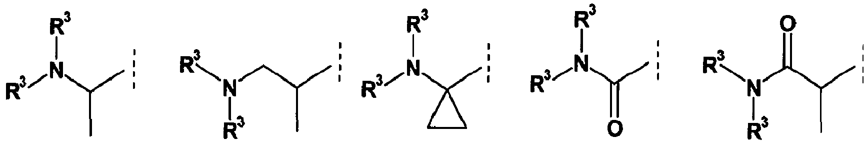 and said phenyl, 2-pyridyl or 3-pyridyl being optionally substituted by 1-2 additional substituents selected from halogen, cyano, C₁-C₇-alkyl, halo- C₁-C₇-alkyl, hydroxyl, C₁-C₇-alkoxy, or hydroxy- C₁-C₇-alkyl;
   (B) phenyl, 2-pyridyl or 3-pyridyl, said phenyl, 2-pyridyl or 3-pyridyl being substituted in para-position (relative to the isoquinolinone or quinazolinone), by a substituent selected from cyano, halogen, nitro, C₁-C₇-alkyl, halo-C₁-C₇-alkyl, hydroxyl- C₁-C₇-alkyl, C₁-C₇-alkoxycarbonyl, C₁-C₇-alkyl-carbonyl, C₁-C₇-alkoxy, or (C-bound)-heterocyclyl, wherein (C-bound)-heterocyclyl is unsubstituted or substituted by 1-4 substituents selected from C₁-C₇-alkyl, halo-C₁-C₇-alkyl, halogen, hydroxy, C₁-C₇-alkoxy, amino, nitro or cyano; and wherein said phenyl, 2-pyridyl and 3-pyridyl are optionally substituted by 1-2 additional substituents independently selected from halogen, cyano, C₁-C₇-alkyl, halo- C₁-C₇-alkyl, hydroxyl, C₁-C₇-alkoxy, (C-bound or N-bound)heterocyclyl- C₁-C₇-alkyl, and hydroxyl- C₁-C₇-alkyl; or
   (C) phenyl, substituted in ortho-position (relative to the isoquinolinone or quinazolinone), by R³O and substituted in para- or meta-position by a substituent selected from methyl, chloro, C₁-C₇-alkyl-carbonyl, or C₁-C₇-alkoxy-carbonyl- ;
   (D) (C-bound)-heterocycle selected from 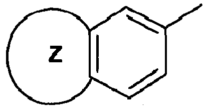 wherein Z is a 4-6 membered heterocyclic ring, annulated to phenyl in para and meta position, containing 1-3 heteroatoms selected from N,O, S,which is optionally substituted by 1-2 additional substituents selected from halogen, cyano, C₁-C₇-alkyl, halo- C₁-C₇-alkyl, hydroxyl, C₁-C₇-alkoxy, hydroxyl-C₁-C₇-alkyl;
   (E) pyrazin-2-yl (relative to the isoquinolinone or quinazolinone), substituted at the 5 position by: 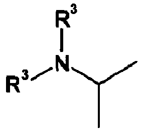
   (F) pyridazin-3-yl (relative to the isoquinolinone or quinazolinone), substituted at the 6 position by: 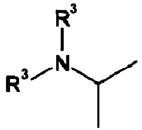 or
   (G) pyrimidin-2-yl (relative to the isoquinolinone or quinazolinone), substituted at the 5 position by: 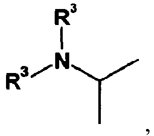 wherein each R³ is independently selected from H, C₁-C₇-alkyl, hydroxy-C₁-C₇-alkyl, C₃-C₁₂-cycloalkyl, C₁-C₇-alkoxy- C₁-C₇-alkyl-carbonyl, amino-C₁-C₇-alkyl-carbonyl, N-C₁-C₇-alkyl-amino- C₁-C₇-alkyl-carbonyl, N, N-di-C₁-C₇-alkyl-amino-C₁-C₇-alkyl-carbonyl, (R⁵)₂N-C₃-C₁₂-cycloalkyl, (R⁵)₂N-C₁-C₇-alkyl, (R⁵)₂N-C₃-C₁₂-cycloalkyl-C₁-C₇-alkyl, (R⁵)₂N-C₃-C₂-cycloalkyl-carbonyl, R⁵O-C₃-C₁₂-cycloalkyl, R⁵O-C₁-C₇-alkyl, R⁵O-C₃-C₁₂-cycloalkyl-C₁-C₇-alkyl, R⁵O-(C₁-C₇-alkyl)-C₃-C₁₂-cycloalkyl-C₁-C₇-alkyl, R⁵O-(hydroxy-C₁-C₇-alkyl)-C₃-C₁₂-cycloalkyl-C₁-C₇-alkyl, (R⁵)₂N-CO-C₃-C₁₂-cycloalkyl-C₁-C₇-alkyl, C₁-C₇-alkoxycarbonyl-C₃-C₁₂-cycloalkyl-C₁-C₇-alkyl, hydroxycarbonyl-C₃-C₁₂-cycloalkyl-C₁-C₇-alkyl, amino-carbonyl-C₃-C₁₂-cycloalkyl-C₁-C₇-alkyl, R⁵O-C₃-C₁₂-cycloalkyl-carbonyl, (R⁵)₂N-carbonyl-C₁-C₇-alkyl, R⁵O-carbonyl-C₁-C₇-alkyl, aryl- C₁-C₇-alkyl, heterocyclyl- C₁-C₇-alkyl, C₁-C₇-alkyl-carbonyl, halo-C₁-C₇-alkyl-carbonyl, heterocyclyl-carbonyl, aryl-carbonyl, C₃-C₁₂-cycloalkyl-carbonyl, C₃-C₁₂-cycloalkyl-C₁-C₇-alkyl, heterocyclyl, aryl, wherein aryl, heterocyclyl and C₃-C₁₂-cycloalkyl are unsubstituted or substituted by 1-4 substituents selected from halogen, C₁-C₇-alkyl, halo- C₁-C₇-alkyl, C₁-C₇-alkyl-carbonyl, C₃-C₁₂-cycloalkyl-carbonyl, C₁-C₇-alkylsulfonyl, amino-sulfonyl, N- C₁-C₇-alkyl-amino-sulfonyl, N,N-di-C₁-C₇-alkyl-amino-sulfonyl, amino-carbonyl, N- C₁-C₇-alkyl-amino-carbonyl, N,N-di- C₁-C₇-alkyl-amino-carbonyl, oxo=,
or two R³, together with the N to which they are attached my form a 3-9 membered heterocyclic ring, optionally containing 1-4 additional heteroatoms selected from N, O or S, said heterocyclic ring is unsubstituted or substituted by 1-3 substituents selected from halogen, hydroxy-C₁-C₇-alkyl, C₁-C₇-alkyl, halo- C₁-C₇-alkyl, oxo=, hydroxyl. C₁-C₇-alkoxy, amino, N-C₁-C₇-alkyl-amino, N,N-di- C₁-C₇-alkyl-amino, hydroxy-carbonyl, C₁-C₇-alkoxy-carbonyl, amino-carbonyl, N- C₁-C₇-alkyl-amino-carbonyl, N,N-di-C₁-C₇-alkyl-amino-carbonyl, C₁-C₇-alkyl-carbonyl, C₁-C₇-alkyl-sulphonyl, heterocyclyl, C₁-C₇-alkyl-carbonyl-amino, C₁-C₇-alkylcarbonyl-N- C₁-C₇-alkyl-amino, and
each R⁵ is independently selected from H, C₁-C₇-alkyl, hydroxy- C₁-C₇-alkyl, C₁-C₇-alkyl-carbonyl, C₁-C₇-alkyl-carbonyl, C₁-C₇-alkyl-carbonyl-C₁-C₇-alkyl, amino-carbonyl- C₁-C₇-alkyl, N-C₁-C₇-alkyl-amino-carbonyl-C₁-C₇-alkyl, N,N-di-C₁-C₇-alkyl-amino-carbonyl-C₁-C₇-alkyl, C₁-C₇-alkyl-sulfonyl, amino-sulfonyl, N-C₁-C₇-alkyl-amino-sulfonyl, N,N-di- C₁-C₇-alkyl-amino-sulfonyl, heterocyclyl-carbonyl, amino-carbonyl, N- C₁-C₇-alkyl-amino-carbonyl, N.N-di-C₁-C₇-alkyl-amino-carbonyl, C₃-C₁₂-cycloalkyl-carbonyl, C₁-C₇-alkoxy-carbonylamino- C₁-C₇-alkyl, C₁-C₇-alkoxy-carbonyl-N-C₁-C₇-alkyl-amino-C₁-C₇-alkyl, C₁-C₇-alkoxycarbonyl, C₃-C₁₂-cycloalkyl, hydroxy-C₃-C₁₂-cycloalkyl,
or two R⁵, together with the N to which they are attached may form a 3, 4, 5, 6, 7, 8 or 9 membered heterocyclic ring, optionally containing , 2, 3 or 4 additional heteroatoms selected from N, O or S, said heterocyclic ring is unsubstituted or substituted by from 1 to 3 substituents independently selected from C₁-C₇-alkyl, oxo=, C₁-C₇-alkyl-carbonyl, C₁-C₇-alkyl-sulphonyl, hydroxy-C₁-C₇-alkyl;
with the proviso that if Z is CH₂, n is 0 or 1 , and when present, R¹ is ortho-chloro, and R² is selected from para- C₁-C₇-alkyl-phenyl, para-(halo- C₁-C₇-alkyl)-phenyl, para- C₁-C₇-alkoxyphenyl, para-halo-phenyl, para-nitro-phenyl, para-(C₁-C₇-alkoxy-carbonyl)-phenyl, para-(hydroxy-carbonyl)-phenyl, wherein the phenyl is optionally substituted by 1-2 additional substituents, said substituents being independently selected from halo and methyl, then R⁶ and R⁷ are not both ethoxy or methoxy.

In one embodiment, CGM097 has the following structure:

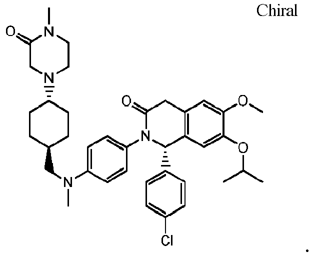

In one embodiment, CGM097 is (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one.

In one embodiment, the inhibitor of the immune checkpoint molecule (alone or in combination with other immunomodulators) is used in combination with a PIM kinase inhibitor to treat a cancer, *e.g.,* a cancer described herein (*e.g.,* a cancer disclosed in Table 1). In one embodiment, the PIM kinase inhibitor is disclosed in Table 1, or in a publication recited in Table 1, *e.g*., International Patent Publication No. WO2010/026124 (*e.g.,* Formula I or Example 70), European Patent Application No. EP2344474, and U.S. Patent Publication No. 2010/0056576. In certain embodiments, the PIM kinase inhibitor is disclosed, *e.g*., in International Patent Publication No. WO2010/026124 (*e.g.,* Formula I or Example 70), European Patent Application No. EP2344474, and U.S. Patent Publication No. 2010/0056576. In one embodiment, the PIM kinase inhibitor, *e.g*., LGH447, has the structure (compound or generic structure) provided in Table 1, or as disclosed in the publication recited in Table 1, *e.g*.. International Patent Publication No. WO2010/026124 (*e.g.,* Formula I or Example 70), European Patent Application No. EP2344474, and U.S. Patent Publication No. 2010/0056576. In one embodiment, the inhibitor of the immune checkpoint molecule (*e.g.,* one of Nivolumab, Pembrolizumab or MSB0010718C) is used in combination with the PIM kinase inhibitor to treat a cancer or disorder described in Table 1, *e.g*., hematological malignancy, *e.g*., multiple myeloma, myelodysplastic syndrome, myeloid leukemia, or non-Hodgkin lymphoma.

In one embodiment, the PIM kinase inhibitor is a compound of formula (I), 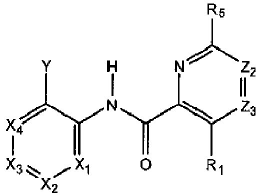 or a pharmaceutically acceptable salt thereof, wherein:
X₁, X₂, X₃ and X₄ are independently selected from CR2 and N; provided that at least one but not more than two of X₁, X₂, X₃ and X₄ are N;
Y is selected from a group consisting of cycloalkyl, partially unsaturated cycloalkyl, andiieterocycloalkyl, wherein each member of said group may be substituted with up to four substituents;
Z₂ and Z₃ are independently selected from CR₁₂ and N; provided that not more than one of Z₂ and Z₃ can be N;
R₁ is selected from the group consisting of hydrogen, -NHR₃ halo, hydroxyl, alkyl, cyano, and nitro;
R₂ and R₁₂ independently at each occurrence are selected from the group consisting of hydrogen, halo, hydroxyl, nitro, cyano, SO₃H and substituted or unsubstituted alkyl, alkenyl, alkynyl, alkoxy, amino, cycloalkyl, hetero cycloalkyl, and partially saturated cycloalkyl;
R₃ is selected from the group consisting of hydrogen, -CO-R₄ and substituted or unsubstituted alkyl, cycloalkyl, heterocyclyl, aryl, and heteroaryl;
R₄ is selected from the group consisting of alkyl, substituted alkyl, alkoxy, substituted alkoxy, amino, substituted amino, and alkylamino; and
R₅ represents a group selected from substituted or unsubstituted aryl, C₃-C₇ cycloalkyl, heteroaryl, partially unsaturated cycloalkyl and alkyl, wherein each said substituted R₅ group may be substituted with up to four substituents selected from halo, cyano, amino, C₁₋₄ alkyl, C₃₋₆ cycloalkyl, alkoxy, nitro, carboxy, carbonyl, carboalkoxy, aminocarboxy, substituted aminocarbonyl, aminosulfonyl, substituted aminosulfonyl and alkoxy alkyl.

In one embodiment, LGH447 has the following structure:

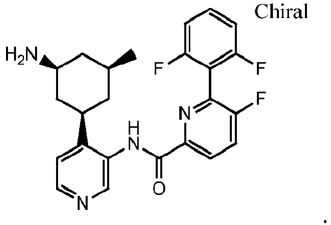

In one embodiment, LGH447 is N-(4-((1R,3S,5S)-3-amino-5-methylcyclohexyl)pyridine-3-yl)-6-(2,6-difluorophenyl)-3-fluoropicolinamide.

In one embodiment, the inhibitor of the immune checkpoint molecule (alone or in combination with other immunomodulators) is used in combination with a HER3 kinase inhibitor to treat a cancer, *e.g.,* a cancer described herein (*e.g.,* a cancer disclosed in Table 1). In one embodiment, the HER3 kinase inhibitor is disclosed in Table 1, *e.g*., LJM716, or in a publication recited in Table 1. In certain embodiments, the HER3 kinase inhibitor is disclosed, *e.g*., in International Patent Publication No. 2012/022814 and U.S. Patent No. 8,735,551. In one embodiment, LJM716 is a monoclonal antibody provided in Table 1, or as disclosed in the publication recited in Table 1. In one embodiment, the inhibitor of the immune checkpoint molecule (*e.g.,* one of Nivolumab, Pembrolizumab or MSB0010718C) is used in combination with LJM716 to treat a cancer or disorder described in Table 1, *e.g.,* a solid tumor, *e.g.* a gastric cancer, an esophageal cancer, a breast cancer, a head and neck cancer, a stomach cancer, or a digestive/gastrointestinal cancer therapy.

In some embodiments, the HER3 kinase inhibitor, *e.g*., LJM716, is an anti-HER3 monoclonal antibody or antigen binding fragment thereof, that comprises a VH of SEQ ID NO: 141 and VL of SEQ ID NO: 140, as described in U.S. 8,735,551. In other embodiments, the HER3 kinase inhibitor, *e.g*., LJM716, is an anti-HER3 monoclonal antibody or antigen binding fragment thereof, that comprises a heavy chain variable region CDR1 of SEQ ID NO: 128; CDR2 of SEQ ID NO: 129; CDR3 of SEQ ID NO: 130; and a light chain variable region CDR1 of SEQ ID NO: 131; CDR2 of SEQ ID NO: 132; and CDR3 of SEQ ID NO: 133, as described in U.S. 8,735,551, *e.g.,* the sequences underlined in the heavy and light chain variable region sequences of LJM716 below. In certain embodiments, the HER3 kinase inhibitor, *e.g*., LJM716, is an anti-HER3 monoclonal antibody or antigen binding fragment thereof, that recognizes a conformational epitope of a HER3 receptor, *e.g.,* the conformational epitope comprises amino acid residues 265-277, and 315 within domain 2 and amino acid residues 571, 582-584, 596-597, 600-602, and 609-615 within domain 4 of the HER3 receptor of SEQ ID NO: 1 of U.S. 8,735,551.

The amino acid sequences of the heavy and light chain variable regions of LJM716 include at least the following:

Heavy chain variable region (SEQ ID NO: 141 as disclosed in U.S. 8,735,551) (SEQ ID NO: 8)
EVQLLESGGGLVQPGGSLRLSCAASGFTFSSYAMSWVRQAPGKGLEWVSAINSQGKSTYYADSV
KGRFTISRDNSKNTLYLQMNSLRAEDTAVYYCARWGDEGFDIWGQGTLVTVSS

Light chain variable region (SEQ ID NO: 140 as disclosed in U.S. 8,735,551) (SEQ ID NO: 9)
DIQMTQSPSSLSASVGDRVTITCRASQGISNWLAWYQQKPGKAPKLLIYGASSLQSGVPSRFSG
SGSGTDFTLTISSLQPEDFATYYCQQYSSFPTTFGQGTKVEIK

In one embodiment, the inhibitor of the immune checkpoint molecule (alone or in combination with other immunomodulators) is used in combination with a HDAC inhibitor to treat a cancer, *e.g.,* a cancer described herein (*e.g.,* a cancer disclosed in Table 1). In one embodiment, the HDAC inhibitor is disclosed in Table 1, *e.g*., LBH589, or in a publication recited in Table 1, *e.g.,* in International Patent Publication Nos. 2014/072493 and 2002/022577 (*e.g.,* formula (I) and Example 200) and European Patent Application No. EP1870399. In certain embodiments, the HDAC inhibitor is disclosed, *e.g*., in International Patent Publication Nos. 2014/072493 and 2002/022577 (*e.g.,* formula (I) and Example 200) and European Patent Application No. EP1870399. In one embodiment, LBH589 has the structure (compound or generic) provided in Table 1, or as disclosed in the publication recited in Table 1, *e.g.,* in International Patent Publication Nos. 2014/072493 and 2002/022577 (*e.g.,* formula (I) and Example 200) and European Patent Application No. EP1870399. In one embodiment, the inhibitor of the immune checkpoint molecule (*e.g.,* one of Nivolumab, Pembrolizumab or MSB0010718C) is used in combination with LBH589 to treat a cancer or disorder described in Table 1, *e.g.,* a solid tumor, *e.g.,* a bone cancer, a small cell lung cancer, a respiratory/thoracic cancer a prostate cancer, a non-small cell lung cancer (NSCLC), a nerologic cancer, a gastric cancer, a melanoma, a breast cancer, a pancreatic cancer, a colorectal cancer, a renal cancer, or a head and neck cancer, or a liver cancer; or a hematological malignancy, *e.g*., multiple myeloma, a hematopoeisis disorder, myelodysplastic syndrome, lymphoma (*e.g.,* non-Hodgkin lymphoma), or leukemia (*e.g.,* myeloid leukemia).

In one embodiment, the HDAC inhibitor is a compound of formula (I): 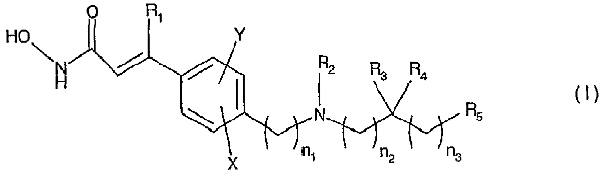 wherein
R₁ is H, halo, or a straight chain C₁-C₆ alkyl (especially methyl, ethyl or n-propyl, which methyl, ethyl and n-propyl substituents are unsubstituted or substituted by one or more substituents described below for alkyl substituents);
R₂ is selected from H, C₁-C₁₀ alkyl, (*e.g.* methyl, ethyl or -CH₂CH₂-OH), C₄ - C₉ cycloalkyl, C₄-C₉ heterocycloalkyl, C₄-C₉ heterocycloalkylalkyl, cycloalkylalkyl (*e.g.,* cyclopropylmethyl), aryl, heteroaryl, arylalkyl (*e.g.* benzyl), heteroarylalkyl (*e.g.* pyridylmethyl), -(CH₂)ₙC(O)R₆, -(CH₂)ₙOC(O)R₆, amino acyl, HON-C(O)-CH=C(R₁)- aryl-alkyl- and-(CH₂)ₙR₇;
R₃ and R₄ are the same or different and independently H, C₁-C₆ alkyl, acyl or acylamino, or R₃ and R₄ together with the carbon to which they are bound represent C=O, C=S, or C=NR₈, or R₂ together with the nitrogen to which it is bound and R₃ together with the carbon to which it is bound can form a C₄-C₉ heterocycloalkyl, a heteroaryl, a polyheteroaryl, a non-aromatic polyheterocycle, or a mixed aryl and non-aryl polyheterocycle ring;
R₅ is selected from H, C₁-C₆ alkyl, C₄-C₉ cycloalkyl, C₄-C₉ heterocycloalkyl, acyl, aryl, heteroaryl, arylalkyl (*e.g.,* benzyl), heteroarylalkyl (*e.g.,* pyridylmethyl), aromatic polycycles, non-aromatic polycycles, mixed aryl and non-aryl polycycles, polyheteroaryl, non-aromatic polyheterocycles, and mixed aryl and non-aryl polyheterocycles;
n, n₁, n₂, and n₃ are the same or different and independently selected from 0 - 6, when n1 is 1-6, each carbon atom can be optionally and independently substituted with R₃ and/or R₄;
X and Y are the same or different and independently selected from H, halo, C₁-C₄ alkyl, such as CH₃ and CF₃, NO₂, C(O)R₁, OR₉, SR₉, CN, and NR₁₀R₁₁;
R₆ is selected from H, C₁-C₆ alkyl, C₄ - C₉ cycloalkyl, C₄ - C₉ heterocycloalkyl, cycloalkylalkyl (*e.g.,* cyclopropylmethyl), aryl, heteroaryl, arylalkyl (*e.g.,* benzyl, 2-phenylethenyl), heteroarylalkyl (*e.g.,* pyridylmethyl), OR₁₂, and NR₁₃R₁₄;
R₇ is selected from OR₁₅, SRιₛ, S(O)R₁₆, SO₂R₁₇, NR₁₃Rι₄, and NR₁₂SO₂R₆;
R₈ is selected from H, OR₁₅, NR₁₃R₁₄, C₁-C₆ alkyl, C₄ - C₉ cycloalkyl, C₄-C₉ heterocycloalkyl, aryl, heteroaryl, arylalkyl (*e.g.,* benzyl), and heteroarylalkyl (*e.g.,* pyridylmethyl);
R₉ is selected from C₁-C₆ alkyl, for example, CH₃ and CF₃, C(O)-alkyl, for example
   C(O)CH₃, and C(O)CF₃;
R₁₀ and R₁₁ are the same or different and independently selected from H, C₁-C₄ alkyl, and -C(O)-alkyl;
R₁₂ is selected from H, C₁-C₆ alkyl, C₄ - C₉ cycloalkyl, C₄ - C₉ heterocycloalkyl, C₄ - C₉ heterocycloalkylalkyl, aryl, mixed aryl and non-aryl polycycle, heteroaryl, arylalkyl (*e.g.,* benzyl), and heteroarylalkyl (*e.g.,* pyridylmethyl);
R₁₃ and R₁₄ are the same or different and independently selected from H, C₁-C₆ alkyl,
C₄-C₉ cycloalkyl, C₄ - C₉ heterocycloalkyl, aryl, heteroaryl, arylalkyl (*e.g.,* benzyl), heteroarylalkyl (*e.g.,* pyridylmethyl), amino acyl, or R₁₃ and R₁₄ together with the nitrogen to which they are bound are C₄ - C₉ heterocycloalkyl, heteroaryl, polyheteroaryl, non-aromatic polyheterocycle or mixed aryl and non-aryl polyheterocycle;
R₁₅ is selected from H, Ci-Ce alkyl, C₄ - C₉ cycloalkyl, C₄ - C₉ heterocycloalkyl, aryl, heteroaryl, arylalkyl, heteroarylalkyl and (CH₂)ₘZR₁₂;
R₁₆ is selected from C₁-C₆ alkyl, C₄-C₉ cycloalkyl, C₄-C₉ heterocycloalkyl, aryl, heteroaryl, polyheteroaryl, arylalkyl, heteroarylalkyl and (CH₂)ₘZRι₂;
R₁₇ is selected from C₁-C₆ alkyl, C₄ - C₉ cycloalkyl, C₄ - C₉ heterocycloalkyl, aryl, aromatic polycycles, heteroaryl, arylalkyl, heteroarylalkyl, polyheteroaryl and
m is an integer selected from 0 to 6;
and Z is selected from O, NR₁₃, S and S(O), or a pharmaceutically acceptable salt thereof.

In one embodiment, LBH589 has the following structure:

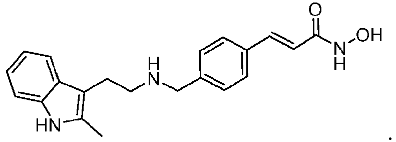

In one embodiment, LBH589 is (E)-N-hydroxy-3-(4-(((2-(2-methyl-1H-indol-3-yl)ethyl)amino)methyl)phenyl)acrylamide.

In one embodiment, the inhibitor of the immune checkpoint molecule (alone or in combination with other immunomodulators) is used in combination with a Janus kinase inhibitor to treat a cancer, *e.g.,* a cancer described herein (*e.g.,* a cancer disclosed in Table 1). In one embodiment, the Janus kinase inhibitor is disclosed in Table 1, *e.g.,* INC424, or in a publication recited in Table 1, *e.g.,* in International Patent Publication Nos. WO2007/070514 (*e.g.,* Formula (I) or Example 67) and WO2014/018632, European Patent Application No. EP2474545, and U.S. Patent No. 7,598,257. In certain embodiments, the Janus kinase inhibitor is disclosed, *e.g*., in International Patent Publication Nos. 2007/070514 (*e.g.,* Formula (I) or Example 67) and 2014/018632, European Patent Application No. EP2474545, and U.S. Patent No. 7,598,257. In one embodiment, the Janus kinase inhibitor, *e.g*., INC424, has the structure (compound or generic) provided in Table 1, or as disclosed in the publication recited in Table 1, *e.g.,* in International Patent Publication Nos. WO2007/070514 (*e.g.,* Formula (I) or Example 67) and WO2014/018632, European Patent Application No. EP2474545, and U.S. Patent No. 7,598,257. In one embodiment, the inhibitor of the immune checkpoint molecule (*e.g.,* one of Nivolumab, Pembrolizumab or MSB0010718C) is used in combination with INC424 to treat a cancer or disorder described in Table 1, *e.g.,* a solid tumor, *e.g.,* a prostate cancer, a lung cancer, a breast cancer, a pancreatic cancer, a colorectal cancer; or a hematological malignancy, *e.g*., multiple myeloma, lymphoma (*e.g.,* non-Hodgkin's lymphoma), or leukemia (*e.g.,* myeloid leukemia, lymphocytic leukemia). In some embodiments, the cancer has, or is identified as having, a JAK mutation. In some embodiments, the JAK mutation is a JAK2 V617F mutation.

In one embodiment, the Janus kinase inhibitor is a compound of Formula (I): 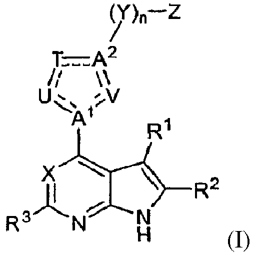 or a pharmaceutically acceptable salt or prodrug thereof, wherein
A¹ and A² are independently selected from C and N;
T, U, and V are independently selected from O, S, N, CR⁵, and NR⁶; wherein the 5-membered ring formed by A¹, A², U, T, and V is aromatic;
X is N or CR⁴;
Y is C₁₋₈ alkylene, C₂₋₈ alkenylene, C₂₋₈ alkynylene, (CR¹¹R¹²)p-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)p-(heteroarylene)-(CR¹¹'R¹²)_{q}, (CR¹¹R¹²)pO(CR¹¹R¹²), (CR¹¹R¹²)ₚS(CR¹¹R¹²), (CR¹¹R¹²)ₚC(O)(CR¹¹R¹²)_{q}, (CR¹(CR^{1I}R¹²)ₚC(O)NR^{c}(CR^{I1}R¹²)_{q}, (CR¹¹R^{I2})ₚC(O)O(CR¹¹R^{I2})_{q}, (CR¹¹R¹²)ₚOC(O)(CR¹¹R¹²)_{q}, (CR¹¹R^{I2})ₚNR^{c}(CR¹¹R^{I2})_{q}, (CR¹¹R¹² )ₚNR^{c}C(O)NR^{d}(CR¹¹R¹²)_{q}, (CR¹¹R¹²)pS(O)(CR¹¹R¹²)_{q}, (CR"R¹²)ₚS(O)NR^{c}(CR¹¹R¹²)_{q}, (CR¹¹R¹²)pS(O)₂(CR¹¹R¹²)_{q}, or (CR¹¹R¹²)pS(O)₂NR^{c}(CR¹¹R¹²)_{q}, wherein said C₁₋₈ alkylene, C_{2·8} alkenylene, C₂₋₈ alkynylene, cycloalkylene, arylene, heterocycloalkylene, or heteroarylene, is optionally substituted with 1, 2, or 3 substituents independently selected from -D'-D²-D³-D⁴;
Z is H, halo, C₁₋₄ alkyl, C₂₋₄ alkenyl, C₂₋₄ alkynyl, Ci₋₄ haloalkyl, halosulfanyl, C₁₋₄ hydroxyalkyl, C₁₋₄ cyanoalkyl, =C-R^{!}, =N-R^{!}, Cy¹, CN, NO₂, OR\ SR^{a}, C(O)R^{b}, C(O)NR^{c}R^{d}, C(O)OR³, OC(O)R⁶, OC(O)NR^{c}R^{d}, NR^{c}R^{d}, NR^{c}C(O)R^{b}, NR^{c}C(O)NR^{c}R^{d}, NR⁰C(O)OR³, C(=NR')NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, :5 alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C_{w} alkyl, C₂₋₄ alkenyl, C₂₋₄ alkynyl, Ci₋₄ haloalkyl, halosulfanyl, C₁₋₄ hydroxyalkyl, Ci₋₄ cyanoalkyl, Cy¹, CN, NO₂, OR", SR", C(O)R^{b}, C(O)NR^{c}R^{d}, C(O)OR", OC(O)R^{b}, OC(O)NR^{c}R^{d}, NR^{c}R^{d}, NR^{c}C(O)R^{b}, NR^{∘}C(O)NR^{c}R^{d}, NR^{c}C(O)OR^{a}, C(=NRⁱ)NR^{c}R^{d}, NR^{c}C(=NR')NR^{∘}R^{d}, S(O)R^{b}, S(O)NR^{∘}R^{d}, S(O)₂R^{b}, O NR^{c}S(O)₂R^{b}, C(=NOH)R^{b}, C(=NO(C₁₋₆ alkyl))R^{b}, and S(O)₂NR^{c}R^{d}; wherein when Z is H, n is 1 ;
or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from -(W)ₘ-Q;
W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c"};
Q is H, halo, CN, NO₂, Ci₋₈ alkyl, C₂₋S alkenyl, C₂₋₈ alkynyl, d.₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, Ci₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{3'}, SR^{a'}, C(O)R^{b"}, C(O)NR^{c'}R^{d'}, C(O)OR^{3'}, OC(O)R"^{'}, OC(O)NR⁰R"^{'}, NR^{∘}R^{d'}, NR^{∘'}C(O)R^{b'}, NR^{c"}C(O)NR^{∘'}R^{d"}, NR^{∘'}C(O)OR^{a'}, S(O)R^{b"}, S(O)NR^{c'}R^{d"}, S(O)₂R^{b>}, NR^{∘'}S(O)₂R^{b}\ and S(O)₂NR^{∘'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, 0R^{a"}, SR^{a"}, C(O)R^{b"}, C(O)NR^{c"}R^{d"}, C(O)OR^{3"}, OC(O)R^{b"}, OC(O)NR^{∘"}R^{d"}, NR^{c"}R^{d"}, NR^{c"}C(O)R^{b"}, NR^{c"}C(O)OR^{a"}, NR^{∘"}S(O)R^{b"}, NR^{∘"}S(O)₂R^{b"}, S(O)R^{b"}, S(O)NR^{c"}R^{d"}, S(O)₂R^{b"}, and S(O)₂NR^{∘"}R^{d"};
R¹, R², R³, and R⁴ are independently selected from H, halo, C₁₋₆ alkyl, C₂₋₄ alkenyl, C₂₋₄ alkynyl, C₁₋₆ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷OC(O)R⁸, OC(O)NR⁹R¹⁰, NR⁹R¹⁰, NR⁹C(O)R⁸, NR⁰C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₎₋₄ alkyl, C₂₋₄ alkenyl, C₂₋₄ alkynyl, C_{N4} haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, Ci₋₆ haloalkyl, C₂₋₆ alkenyl, C_{2·6} alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₆ alkyl, C₁₋₆ haloalkyl, C₂₋₆ alkenyl, C_{2·6} alkynyl, C₁₋₆ alkylcarbonyl, arylcarbonyl, C₁₋₆ alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H and -E^{ι}-E²-E³-E⁴;
D¹ and E¹ are independently absent or independently selected from C₁₋₆ alkylene, C₂₋₆ alkenylene, C_{2·6} alkynylene, arylene, cycloalkylene, heteroarylene, and heterocycloalkylene, wherein each of the Ci₋₆ alkylene, C₂₋₆ alkenylene, C_{2·6} alkynylene, arylene, cycloalkylene, heteroarylene, and heterocycloalkylene is optionally substituted by 1, 2 or 3 substituents independently selected from halo, CN, NO₂, N₃, SCN, OH, Ci.₆alkyl, C₁₋₆ haloalkyl, C_{2·8} alkoxyalkyl, C₁₋₆ alkoxy, C₁₋₆ haloalkoxy, amino, C₁₋₆ alkylamino, and C₂₋₈ dialkylamino;
D² and E² are independently absent or independently selected from C₁₋₆ alkylene, C₂₋₆ alkenylene, C₂₋₆ alkynylene, (C₁₋₆ alkylene)ᵣ-O-(C₁₋₆ alkylene)ₛ, (C₁₋₆ alkylene)ᵣ-S-(C₁₋₆ alkylene)ₛ, (C₁₋₆ alkylene)ᵣ-NR^{e}-(C₁₋₆ alkylene)ₛ, (C ,_{·6} alkylene)ᵣ-CO-(C₁₋₆ alkylene)ₛ, (C₁₋₆ alkylene)ᵣ-COO-(C₁₋₆ alkylene)ₛ, (C₁₋₆ alkylene)ᵣ-CONR^{e}-(C₁₋₆ alkylene)ₛ, (C₁₋₆ alkylene)ᵣ-SO-( C₁₋₆ alkylene)ₛ, (C₁₋₆ alkylene)ᵣ-SO₂-(C₁₋₆ alkylene)ₛ, (C₁₋₆ alkylene)ᵣ-SONR^{c}-(C₁₋₆ alkylene)ₛ, and (C₁₋₆ alkylene)ᵣ- NR^{e}CONR^{f}-(C₁₋₆ alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C_{]-6} alkylamino, and C_{2·8} dialkylamino;
D³ and E³ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₎₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆ alkoxy, C₁₋₆ haloalkyl, amino, C₁₋₆ alkylamino, and C₂₋₈ dialkylamino;
D⁴ and E⁴ are independently selected from H, halo, C₁₋₄ alkyl, C₂₋₄ alkenyl, C₂₋₄ alkynyl, C)₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR⁰, OC(O)R⁶, OC(O)NR^{c}R^{d}, NR^{c}R^{d}, NR^{∘}C(O)R^{b}, NR^{c}C(O)NR^{c}R^{d}, NR⁰C(O)OR⁰, C(=NR')NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(Ci₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NR')NR^{c}R^{d}, S(O)R^{b}, S(O)NR^{c}R^{d}, S(O)₂R⁶, NR⁰S(O)₂R", C(=NOH)R^{b}, C(=NO(C,_{·6} alkyl))R^{b}, and S(O)₂NR^{c}R^{d};
R^{a} is H, Cy¹, -(C₁₋₆ alkyl)-Cy¹, C₋₆ haloalkyl, C₂₋₆ alkenyl, C₂₋₆ alkynyl, wherein said C₁₋₆ alkyl, C₁₋₆ haloalkyl, C₂₋₆ alkenyl, or C_{2·6} alkynyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b} is H, Cy¹, -(C₁₋₆ alkyl)-Cy', C₁₋₆ alkyl, C₁₋₆ haloalkyl, C_{2·6} alkenyl, C₂₋₆ alkynyl, wherein said C₁₋₆ alkyl, C₁₋₆ haloalkyl, C_{2·6} alkenyl, or C_{2·6} alkynyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{a'} and R^{a"} are independently selected from H, Ci₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1 , 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{c} and R^{d} are independently selected from H, Cy¹, -(C₁₋₆ alkyl)-Cy', C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl, is optionally substituted with 1, 2, or 3 substituents independently selected from Cy¹,-(C₁₋₆ alkyl)-Cy', OH, CN, amino, halo, C₁₋₆ alkyl, C₁₋₆ haloalkyl, and halosulfanyl;
or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆ alkyl)-Cy¹, OH, CN, amino, halo, C₁₋₆ alkyl, C₁₋₆ alkyl, and halosulfanyl;
R^{c'} and R^{d'} are independently selected from H, C₁₋₆ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1 , 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{c"} and R^{d"} are independently selected from H, C₁₋₆ alkyl, C₁₋₆ haloalkyl, C₂₋₆ alkenyl, C₂₋6 alkynyl, aryl, heteroaryl, cycloalkyl, heterocycloalkyl, arylalkyl, heteroarylalkyl, cycloalkylalkyl and heterocycloalkylalkyl, wherein said C₁₋₆ alkyl, C₁₋₆ haloalkyl, C₂-₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1 , 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
or R^{c"} and R^{d"} together with the N atom to which they are attached form a 4-, 5-, 6- or 7-membered heterocycloalkyl group optionally substituted with 1 , 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl; R
R^{j} is H, CN, NO₂, or C₁₋₆ alkyl;
R^{e} and R^{f} are independently selected from H and C₁₋₆ alkyl;
Rⁱ is H, CN, or NO₂;
m is 0 or 1 ;
n is 0 or 1;
p is 0, 1, 2, 3, 4, 5, or 6;
q is 0, 1, 2, 3, 4, 5 or 6;
r is 0 or 1;
and s is 0 or 1.

In one embodiment, INC424 has the following structure:

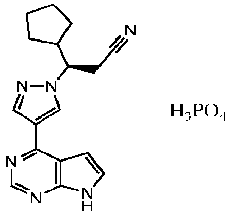

In one embodiment, INC424 is (3R)-3-cyclopentyl-3-[4-(7H-pyrrolo-[2,3-d]pyrimidin-4-yl)-1H-pyrazol-1-yl]propanenitrile.

In one embodiment, the inhibitor of the immune checkpoint molecule (alone or in combination with other immunomodulators) is used in combination with an FGF receptor inhibitor to treat a cancer, *e.g.,* a cancer described herein (*e.g.,* a cancer disclosed in Table 1). In one embodiment, the FGF receptor inhibitor is disclosed in Table 1, *e.g*., BUW078, or in a publication recited in Table 1, *e.g*., International Patent Publication No. WO2009/141386 (*e.g.,* Formula (I) and Example 127) and U.S. Patent Publication No. 2010/0105667). In one embodiment, the FGF receptor inhibitor, *e.g*., BUW078, has the structure (compound or generic structure) provided in Table 1, or as disclosed in the publication recited in Table 1, *e.g.,* International Patent Publication No. WO 2009/141386 (*e.g.,* Formula (I) and Example 127) and U.S. Patent Publication No. 2010/0105667. In one embodiment, the FGF receptor inhibitor is disclosed in Table 1, *e.g.,* BGJ398, or in a publication recited in Table 1, *e.g.,* U.S. 8,552,002 (*e.g.,* Example 145 or Formula (I) in column 6). In one embodiment, the FGF receptor inhibitor, *e.g.,* BGJ398, has the structure (compound or generic structure) provided in Table 1, or as disclosed in the publication recited in Table 1, *e.g.,* U.S. 8,552,002 (*e.g.,* Example 145 or Formula (I) in column 6). In one embodiment, one of Nivolumab, Pembrolizumab or MSB0010718C is used in combination with BUW078 or BGJ398 to treat a cancer described in Table 1, *e.g.,* a solid tumor, *e.g.,* a digestive/gastrointestinal cancer; or a hematological cancer.

In one embodiment, the FGF receptor inhibitor is a compound of Formula (I): 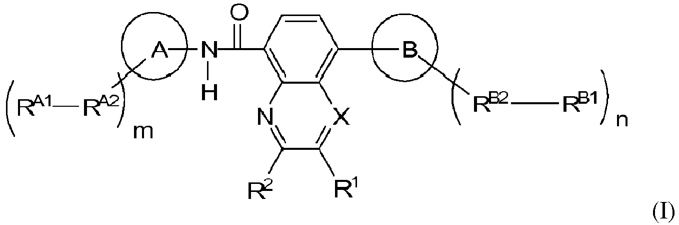
wherein X represents N or CH;
R¹ represents hydrogen, halogen, alkyl, alkyl substituted with saturated heterocyclyl which is unsubstituted or substituted by alkyl, amino, mono-substituted amino wherein the substituent is selected from the group consisting of alkyl, aminoalkyl, alkylaminoalkyl, dialkylaminoalkyl, di-substituted amino wherein the substituents are selected from the group consisting of alkyl, aminoalkyl, alkylaminoalkyl, dialkylaminoalkyl, alkoxy, substituted alkoxy wherein the substituents are selected from the group consisting of halo and alkoxy;
R² represents hydrogen, halogen, alkyl, alkyl substituted with saturated heterocyclyl which is unsubstituted or substituted by alkyl, amino, mono-substituted amino wherein the substituent is selected from the group consisting of alkyl, aminoalkyl, alkylaminoalkyl, dialkylaminoalkyl, di-substituted amino wherein the substituents are selected from the group consisting of alkyl, aminoalkyl, alkylaminoalkyl, dialkylaminoalkyl, alkoxy, substituted alkoxy wherein the substituents are selected from the group consisting of halo and alkoxy;
A represents aryl or heteroaryl;
B represents aryl or heteroaryl;
R^{A1} represents hydrogen or a substituent different from hydrogen;
R^{A2} represents a direct bond or an alkanediyl;
R^{B1} represents hydrogen or a substituent different from hydrogen;
R represents a direct bond or aminocarbonyl;
m represents an integer selected from 0 to 3;
n represents an integer selected from 0 to 5;
or a salt, solvate, ester, N-oxide thereof.

In one embodiment, BUW078 has the following structure:

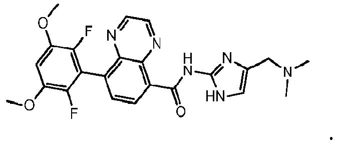

In one embodiment, BUW078 is 8-(2,6-difluoro-3,5-dimethoxy-phenyl)-quinoxaline-5-carboxylic acid (4-dimethylaminomethyl-1H-imidazol-2-yl)-amide.

In one embodiment, the FGF receptor inhibitor has the following structure: 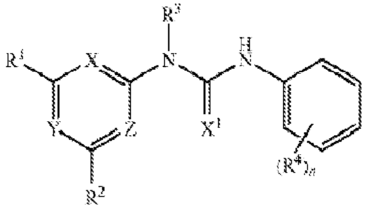
where n is 0, 1, 2, 3, 4 or 5;
X, Y and Z are each independently selected from N or C-R⁵, wherein at least two of X, Y and Z are N; and
X¹ is oxygen,
R¹, R², R³ and R⁴ if present, are each independently selected from an organic or inorganic moiety, where the inorganic moiety is especially selected from halo, especially chloro, hydroxyl, cyano, azo (N=N=N), nitro; and
where the organic moiety is substituted or unsubstituted and may be attached via a linker, -L¹-, the organic moiety being especially selected from hydrogen; lower aliphatic (especially C₁, C₂, C₃ or C₄ aliphatic) *e.g.* lower alkyl, lower alkenyl, lower alkynyl; amino; guanidino; hydroxyguanidino; formamidino; isothioureido; ureido; mercapto; C(O)H or other acyl; acyloxy; substituted hydroxy; carboxy; sulfo; sulfamoyl; carbamoyl; a substituted or unsubstituted cyclic group, for example the cyclic group (whether substituted or unsubstituted) may be cycloalkyl, *e.g.* cyclohexyl, phenyl, pyrrole, imidazole, pyrazole, isoxazole, oxazole, thiazole, pyridazine, pyrimidine, pyrazine, pyridyl, indole, isoindole, indazole, purine, indolizidine, quinoline, isoquinoline, quinazoline, pteridine, quinolizidine, piperidyl, piperazinyl, pyrollidine, morpholinyl or thiomorpholinyl and, for example, substituted lower aliphatic or substituted hydroxy may be substituted by such substituted or unsubstituted cyclic groups;
and -L¹- having 1, 2, 3, 4 or 5 in-chain atoms (*e.g*. selected from C, N, O and S) and optionally being selected from (i) C₁, C₂, C₃ or C₄ alkyl, such an alkyl group optionally being interrupted and/or terminated by
an -O-, -C(O)- or --NRₐ-- linkage; -O-; -S-; -C(O)-; cyclopropyl (regarded as having two in-chain atoms) and chemically appropriate combinations thereof; and --NR^{a}--, wherein R^{a} is hydrogen, hydroxy, hydrocarbyloxy or hydrocarbyl, wherein hydrocarbyl is optionally interrupted by an -O- or -NH- linkage and may be, for example, selected from an aliphatic group (*e.g.,* having 1 to 7 carbon atoms, for example 1, 2, 3, or 4), cycloalkyl, especially cyclohexyl, cycloalkenyl, especially cyclohexenyl, or another carbocyclic group, for example phenyl; where the hydrocarbyl moiety is substituted or unsubstituted;
each R⁴ is the same or different and selected from an organic or inorganic moiety, for example, each R⁴ is the same or different and selected from halogen; hydroxy; protected hydroxy for example trialkylsilylhydroxy; amino; amidino; guanidino; hydroxyguanidino; formamidino; isothioureido; ureido; mercapto; C(O)H or other acyl; acyloxy; carboxy; sulfo; sulfamoyl; carbamoyl; cyano; azo; nitro; C₁-C₇ aliphatic optionally substituted by one or more halogens and/or one or two functional groups selected from hydroxy, protected hydroxy for example trialkylsilylhydroxy, amino, amidino, guanidino, hydroxyguanidino, formamidino, isothioureido, ureido, mercapto, C(O)H or other acyl, acyloxy, carboxy, sulfo, sulfamoyl, carbamoyl, cyano, azo, or nitro; all of the aforesaid hydroxy, amino, amidino, guanidino, hydroxyguanidino, formamidino, isothioureido, ureido, mercapto, carboxy, sulfo, sulfamoyl and carbamoyl groups in turn optionally being substituted on at least one heteroatom by one or more C₁-C₇ aliphatic groups; or salts, esters, N-oxides or prodrugs thereof.

In one embodiment, X is CR⁵, wherein R⁵ is H; X1 is oxygen; Y is N; Z is N; R¹ is a substituted organic moiety is a cyclic group (*e.g.,* phenyl) substituted with 4-ethylpiperazinyl and -L¹- is N^{Ra}, wherein N^{Ra} is H; R² is an organic moiety (*e.g.,* H); R³ is an organic moiety (*e.g.,* lower aliphatic, *e.g*., methyl); R⁴ is chloro or methoxy; and n is 4.

In one embodiment, BGJ398 has the following structure:

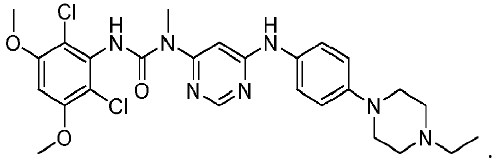

In one embodiment, BGJ398 is 3-(2,6-dichloro-3,5-dimethoxyphenyl)-1-(6-((4-(4-ethylpiperazin-1-yl)phenyl)amino)pyrimidin-4-yl)-1-methylurea.

In one embodiment, the the inhibitor of the immune checkpoint molecule (alone or in combination with other immunomodulators) is used in combination with an EGF receptor inhibitor to treat a cancer, *e.g.,* a cancer described herein (*e.g.,* a cancer disclosed in Table 1). In one embodiment, the EGF receptor inhibitor is disclosed in Table 1, *e.g*., EGF816, or in a publication recited in Table 1, *e.g.,* in WO 2013/184757 (*e.g*., Formula (5), in claims 7, 10, 11 and 12, or in Example 5). In one embodiment, the EGF receptor inhibitor, *e.g*., EGF816, has the structure (compound or generic structure) provided in Table 1, or as disclosed in the publication recited in Table 1, *e.g.,* in WO 2013/184757 (*e.g.,* Formula (5), in claims 7, 10, 11 and 12, or in Example 5). In one embodiment, one of Nivolumab, Pembrolizumab or MSB0010718C is used in combination with EGF816 to treat a cancer described in Table 1, *e.g.,* a solid tumor, *e.g.,* a lung cancer (*e.g.,* non-small cell lung cancer (NSCLC)).

In certain embodiments, EGF816 is administered at an oral dose of about 50 to 500 mg, *e.g.,* about 100 mg to 400 mg, about 150 mg to 350 mg, or about 200 mg to 300 mg, *e.g.,* about 100 mg, 150 mg or 200 mg. The dosing schedule can vary from *e.g.,* every other day to daily, twice or three times a day. In one embodiment, EGF816 is administered at an oral dose from about 100 to 200 mg, *e.g*., about 150 mg, once a day.

In one embodiment, the EGF receptor inhibitor is of formula: 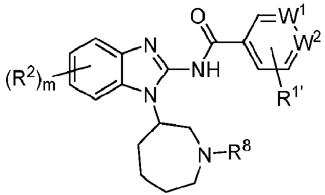
wherein W¹ and W² are independently CR¹ or N; and
R¹, R^{1'} and R² are independently hydrogen; halo; cyano; C₁₋₆ alkyl; C₁₋₆ haloalkyl; 5-6 membered heteroaryl comprising 1-4 heteroatoms selected from N, O and S; phenyl, 5-6 membered heterocyclyl comprising 1-2 heteroatoms selected from N, O, S and P, and optionally substituted by oxo; -X¹-C(O)OR³; -X¹-O-C(O)R³; -X¹-C(O)R³; -X¹-C(O)NR⁴R⁵; -X¹-C(O)NR⁴-X³-C(O)OR³; -X¹-C(O)NR⁴-X³-S(O)₀₋₂R⁶; -X¹-NR⁴R⁵; -X¹NR⁴-X²-C(O)R³; -X¹-NR⁴-X²-C(O)OR³; -X¹-NR⁴-X²-C(O)NR⁴R⁵; -X¹-NR⁴-X³-S(O)₀-₂R⁶; -X¹-NR⁴S(O)₂R⁶; -X¹-OS(O)₂R⁶;-X¹-OR³; -X¹-O-X⁴-OR³; -X¹-O-X⁴-S(O)₀-₂R⁶; -X¹-O-X⁴-NR⁴R⁵; -X¹-S(O)₀₋₂R⁶; -X¹-S(O)₀-₂-X³-NR⁴R⁵; -X¹-C(O)NR⁴-X³-P(O)R^{6a}R^{6b}; -X¹-NR⁴-X¹-P(O)R^{6a}R^{6b}; -X¹-O-X¹-P(O)R^{6a}R^{6b}; -X¹-P(O)R^{6a}-X¹-NR⁴R⁵; -X¹-P(O)R^{6a}R^{6b} or -X¹-S(O)₂NR⁴R⁵; wherein each phenyl, heteroaryl, or heterocyclyl in R¹ or R² is unsubstituted or substituted by 1-3 groups selected from OH, halo, C₁₋₆ alkyl, C₁₋₆ haloalkyl and C₁₋₆ haloalkoxy;
R³, R⁴ and R⁵ are independently hydrogen, C₁₋₆ alkyl or C₁₋₆ haloalkyl; or wherein R⁴ and R⁵ together with N in NR⁴R⁵ may form a 4-7 membered ring containing 1-2 heteroatoms selected from N, O, S and P, and optionally substituted with 1-4 R⁷;
R⁶ is C₁₋₆ alkyl or C₁₋₆ haloalkyl;
R^{6a} and R^{6b} are independently hydroxy, C₁₋₆ alkyl, C₁₋₆ haloalkyl, C₁₋₆ alkoxy, C₁₋₆ haloalkoxy, 6-10 membered monocyclic or bicyclic aryl; a 5-10 membered heteroaryl comprising 1-4 heteroatoms selected from N, O and S; or a 4-12 membered monocyclic or bicyclic heterocyclyl comprising 1-4 heteroatoms selected from N, O and S, and optionally substituted with oxo;
R⁸ is 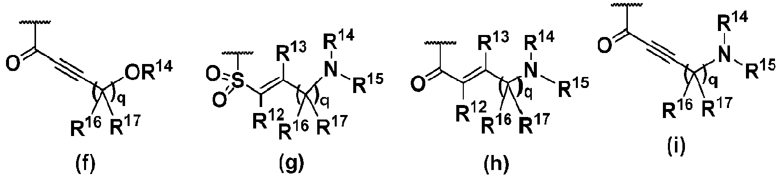 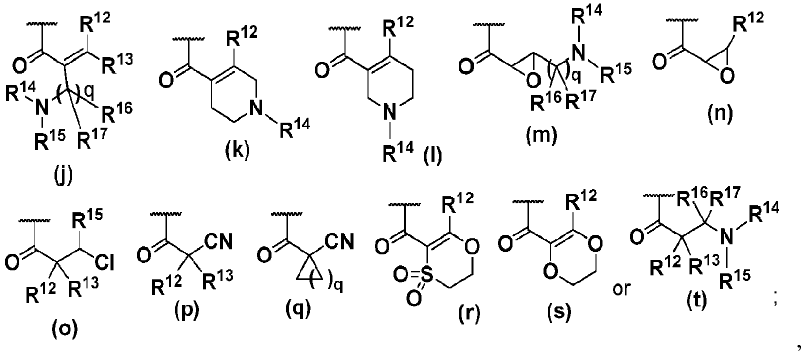
X¹ and X² are independently a bond or C₁₋₆ alkyl;
X³ is C₁₋₆ alkyl;
X⁴ is C₂₋₆ alkyl;
R¹², R¹³, R¹⁶ and R¹⁷ are independently hydrogen or C₁₋₆ alkyl;
R¹⁴ and R¹⁵ are independently hydrogen; C₁₋₆ alkyl; -C(O)O-(C₁₋₆ alkyl); C₃₋₇ cycloalkyl unsubstituted or substituted with C₁₋₆ alkyl; or R¹⁴ and R¹⁵ together with N in NR¹⁴R¹⁵ may form a 4-7 membered ring containing 1-2 heteroatoms selected from N, O, S, and P, and optionally substituted with 1-4 R¹⁸ groups;
R⁷ and R¹⁸ are independently oxo, halo, hydroxyl, C₁₋₆ alkyl or C₁₋₆ alkoxy; and
m and q are independently 1-2;
or a pharmaceutically acceptable salt thereof.

In one embodiment, R¹ and R^{1'} are independently hydrogen; methyl; t-butyl; trifluoromethyl; methoxy; ethoxy; trifluoromethoxy; difluoromethoxy; fluoro; chloro; cyano; dimethylamino; methylsulfonyl; dimethylphosphoryl; tetrazolyl; pyrrolyl; phenyl unsubstituted or substituted by methyl; or piperidinyl.

In one embodiment, R² is hydrogen; chloro; methyl; trifluoromethyl; methoxy; isoproproxy; cyano; hydroxy methyl; methoxy methyl; ethoxymethyl; methylsulfonyl; methylcarbonyl; carboxy; methoxycarbonyl; carbamoyl; dimethylaminomethyl; pyrrolidinylmethyl unsubstituted or substituted by 1-2 hydroxy, halo or methoxy; morpholinomethyl; azeditinylmethyl unsubstituted or substituted by 1-2 halo or methoxy; piperidinylmethyl; ((4-methyl-3-oxo-piperazin-lyl)methyl); ((4-acetylpiperazin-1-yl)methyl); (1,1-dioxidothiomorpholine-4-carbonyl); pyrrolidinyl carbonyl unsubstituted or substituted by 1-2 hydroxy; pyrrolidinylethoxy; (1,1-dioxidothiomorpholino)methyl; or 1,2,4-oxadiazolyl unsubstituted or substituted by C₁₋₆ alkyl;
alternatively, R² is -CH₂-N(CH₃)-C(O)-CH₃; -CH₂-O-(CH₂)₂-OCH₃; -CH₂-N(CH₃)-(CH₂)₂-SO₂(CH₃); -C(O)NH-(CH₂)_{1·2}-C(O)-OCH₃; -C(O)NH-(CH₂)_{1·2}-C(O)OH; or -C(O)NH-(CH₂)₂-SO₂(CH₃).

In one embodiment, R⁸ is 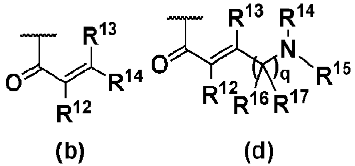 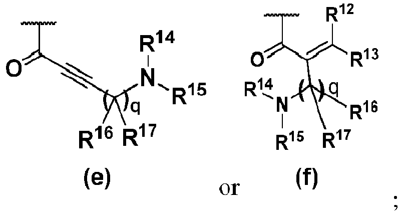
wherein R¹⁴ and R¹⁵ are independently hydrogen, C₁₋₆ alkyl or C₃₋₇ cycloalkyl; or R¹⁴ and R¹⁵ together with N in NR¹⁴R¹⁵ may form an azetidinyl, piperidyl, pyrrolidinyl or morpholinyl; where said azetidinyl or pyrrolidinyl can be optionally substituted with 1-2 halo, methoxy or hydroxyl; and
R¹² and R¹³ are independently hydrogen, halo, cyano, C₁₋₆ alkyl or C₁₋₆ haloalkyl;
R¹⁶ and R¹⁷ are independently hydrogen or C₁₋₆ alkyl; or R¹⁶ and R¹⁷ together with the carbon to which they are attached may form a C₃₋₆ cycloalkyl.

In one embodiment, W¹ is CR¹; W² is N; R¹ is methyl and R^{1'} is hydrogen; R² is chloro; m=1; R⁸ is substructure (h), q=1; R¹², R¹³, R¹⁶ and R¹⁷ are hydrogen; R¹⁴ and R¹⁵ are methyl.

In one embodiment, the EGF receptor inhibitor has the following structure: 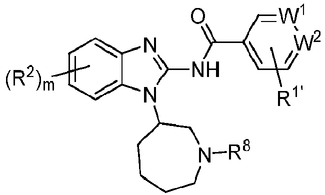 or

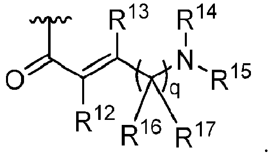

In one embodiment, EGF816 has the following structure:

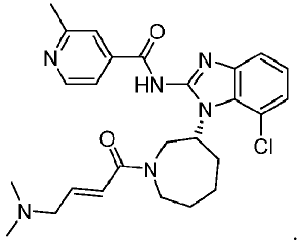

In one embodiment, EGF816 is (R,E)-N-(7-chloro-1-(1-(4-(dimethylamino)but-2-enoyl)azepan-3-yl)-1H-benzo[d]imidazol-2-yl)-2-methylisonicotinamide.

In another embodiment, the inhibitor of the immune checkpoint molecule (alone or in combination with other immunomodulators) is used in combination with a c-MET inhibitor to treat a cancer, *e.g.,* a cancer described herein *(e.g.,* a cancer disclosed in Table 1). In one embodiment, the c-MET inhibitor is disclosed in Table 1, *e.g.,* INC280, or in a publication recited in Table 1, *e.g.,* in EP 2099447 (*e.g.,* in claim 1 or 53) or US 7,767,675 (*e.g.,* in claim 4). In one embodiment, the c-MET inhibitor, *e.g*., INC280, has the structure (compound or generic structure) provided in Table 1, or as disclosed in the publication recited in Table 1. In one embodiment, one of Nivolumab, Pembrolizumab or MSB0010718C is used in combination with INC280 to treat a cancer described in Table 1, *e.g.,* a solid tumor, *e.g.,* a lung cancer (*e.g.,* non-small cell lung cancer (NSCLC)), glioblastoma multiforme (GBM), a renal cancer, a liver cancer or a gastric cancer. In some embodiments, the cancer has, or is identified as having, a c-MET mutation (*e.g*., a c-MET mutation or a c-MET amplification).

In certain embodiments, INC280 is administered at an oral dose of about 100 to 1000 mg, *e.g.,* about 200 mg to 900 mg, about 300 mg to 800 mg, or about 400 mg to 700 mg, *e.g.,* about 400 mg, 500 mg or 600 mg. The dosing schedule can vary from *e.g.,* every other day to daily, twice or three times a day. In one embodiment, INC280 is administered at an oral dose from about 400 to 600 mg twice a day.

In one embodiment, the c-MET inhibitor has the following structure: 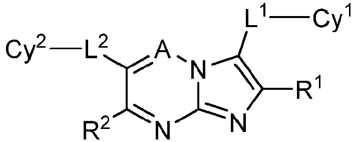 or pharmaceutically acceptable salt thereof or prodrug thereof, wherein:
A is N or CR³; and
Cy¹ is aryl, heteroaryl, cycloalkyl, or heterocycloalkyl, each optionally substituted by 1, 2, 3, 4, or 5 -W-X-Y-Z;
Cy² is aryl, heteroaryl, cycloalkyl, or heterocycloalkyl, each optionally substituted by 1, 2, 3, 4, or 5 -W'-X'-Y'-Z';
L¹ is (CR⁴R⁵)ₘ, (CR⁴R⁵)ₚ-(cycloalkylene)-(CR⁴R⁵)_{q}, (CR⁴R⁵)ₚ-(arylene)-(CR⁴R⁵)_{q}, (CR⁴R⁵)ₚ-(heterocycloalkylene)-(CR⁴R⁵)_{q}, (CR⁴R⁵)ₚ-(heteroarylene)-(CR⁴R⁵)_{q}, (CR⁴R⁵)ₚO(CR⁴R⁵)_{q}, (CR⁴R⁵)ₚS(CR⁴R⁵)_{q}, (CR⁴R⁵)ₚC(O)(CR⁴R⁵)_{q}, (CR⁴R⁵)ₛC(O)NR⁶(CR⁴R⁵)_{q}, (CR⁴R⁵)ₚC(O)O(CR⁴R⁵)_{q}, (CR⁴R⁵)ₚOC(O)(CR⁴R⁵)_{q}, (CR⁴R⁵)ₚOC(O)NR⁶(CR⁴R⁵)_{q}, (CR⁴R⁵)ₚ NR⁶(CR⁴R⁵)_{q}, (CR⁴R⁵)ₚNR⁶C(O)NR⁶(CR⁴R⁵)_{q}, (CR⁴R⁵)ₚS(O)(CR⁴R⁵)_{q}, (CR⁴R⁵)ₚS(O)NR⁴(CR⁵R⁶)_{q}, (CR⁴R⁵)ₚS(O)₂(CR⁴R⁵)_{q}, or (CR⁴R⁵)ₚS(O)₂NR⁶(CR⁴R⁵)_{q}, wherein said cycloalkylene, arylene, heterocycloalkylene, or heteroarylene is optionally substituted with 1, 2, or 3 substituents independently selected from Cy³, halo, C₁₋₆ alkyl, C₂₋₆ alkenyl, C₂₋₆ alkynyl, C₁₋₆ haloalkyl, halosulfanyl, CN, NO₂, N₃, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, P(R^{f})₂, P(OR^{e})₂, P(O)R^{e}R^{f}, P(O)OR^{e}OR^{f}, S(O)R^{b}, S(O)NR^{c}R^{d}, S(O)₂R^{b}, NR^{c}S(O)₂R^{b}, and S(O)₂NR^{c}R^{d};
L² is (CR⁷R⁸)ᵣ, (CR⁷R⁸)ₛ-(cycloalkylene)-(CR⁷R⁸)ₜ, (CR⁷R⁸)ₛ-(arylene)-(CR⁷R⁸)ₜ, (CR⁷R⁸)ₛ-(heterocycloalkylene)-(CR⁷R⁸)ₜ, (CR⁷R⁸)ₛ-(heteroarylene)-(CR⁷R⁸)ₜ, (CR⁷R⁸)ₛO(CR⁷R⁸)ₜ, (CR⁷R⁸)ₛS(CR⁷R⁸)ₜ, (CR⁷R⁸)ₛC(O)(CR⁷R⁸)ₜ, (CR⁷R⁸)ₛC(O)NR⁹(CR⁷R⁸)ₜ, (CR7R⁸)ₛC(O)O(CR⁷R⁸)ₜ, (CR⁷R⁸)ₛOC(O)(CR⁷R⁸)ₜ, (CR⁷R⁸)ₛOC(O)NR⁹(CR⁷R⁸)ₜ, (CR⁷R⁸), NR⁹CR⁷R⁸)ₜ, (CR⁷R⁸)ₛNR⁹C(O)NR⁹(CR⁷R⁸)ₜ, (CR⁷R⁸)ₛS(O)(CR⁷R⁸)ₜ, (CR⁷R⁸)ₛS(O)NR⁷(CR⁸R⁹)ₜ, (CR⁷R⁸)ₛS(O)₂(CR⁷R⁸)ₜ, or (CR⁷R⁸)ₛS(O)₂NR⁹(CR⁷R⁸)ₜ, wherein said cycloalkylene, arylene, heterocycloalkylene, or heteroarylene is optionally substituted with 1, 2, or 3 substituents independently selected from Cy⁴, halo, C₁₋₆ alkyl, C₂₋₆ alkenyl, C₂₋₆ alkynyl, C₁₋₆ haloalkyl, halosulfanyl, CN, NO₂, N₃, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, P(R^{f1})₂, P(OR^{e1})₂, P(O)R^{e1}R^{f1}, P(O)OR^{e1}OR^{f1}, S(O)R^{b1}, S(O)NR^{c1}R^{d1}, S(O)₂R^{b1}, NR^{c1}S(O)₂R^{b1}, and S(O)₂NR^{c1}R^{d1};
R¹ is H or -W"-X"-Y"-Z";
R² is H, halo, C₁₋₆ alkyl, C₂₋₆ alkenyl, C₂₋₆ alkynyl, C₁₋₆ haloalkyl, CN, NO₂, OR^{A}, SR^{A}, C(O)R^{B}, C(O)NR^{C}R^{D}, C(O)OR^{A}, OC(O)R^{B}, OC(O)NR^{C}R^{D}, NR^{C}R^{D}, NR^{C}C(O)R^{B} NR^{C}C(O)NR^{C}R^{D}, NR^{C}C(O)OR^{A}, S(O)R^{B}, S(O)NR^{C}R^{D}, S(O)₂R^{B}, NR^{C}S(O)₂R^{B}, or S(O)₂NR^{C}R^{D};
R³ is H, cycloalkyl, aryl, heterocycloalkyl, heteroaryl, halo, C₁₋₆ alkyl, C₂₋₆ alkenyl, C₂₋₆ alkynyl, C₁₋₆ haloalkyl,, CN, NO₂, OR^{A}, SR^{A}, C(O)R^{B}, C(O)NR^{C}R^{D}, C(O)OR^{A}, OC(O)R^{B}, OC(O)NR^{C}R^{D}, NR^{C}R^{D}, NR^{C}C(O)R^{B}, NR^{C}C(O)NR^{C}R^{D}, NR^{C}C(O)OR^{A}, S(O)R^{B}, S(O)NR^{C}R^{D}, S(O)₂R^{B}, NR^{C}S(O)₂R^{B} and S(O)₂NR^{C}R^{D}; wherein said cycloalkyl, aryl, heterocycloalkyl, heteroaryl, or C₁₋₆ alkyl is optionally substituted with 1, 2, or 3 substituents independently selected from Cy⁵, halo, C₁₋₆ alkyl, C₂₋₆ alkenyl, C₂₋₆ alkynyl, C₁₋₆ haloalkyl, halosulfanyl, CN, NO₂, N₃, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, P(R^{f1})₂, P(OR^{e1})₂, P(O)R^{e1}R^{f1}, P(O)OR^{e1}OR^{f1}, S(O)R^{b1}, S(O)NR^{c1}R^{d1}, S(O)₂R^{b1}, NR^{c1}S(O)₂R^{b1}, and S(O)₂NR^{c1}R^{d1};
or R² and -L²-Cy² are linked together to form a group of formula: 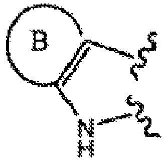
wherein ring B is a fused aryl or fused heteroaryl ring, each optionally substituted with 1, 2, or 3 -W'-X'-Y'-Z';
R⁴ and R⁵ are independently selected from H, halo, OH, C₁₋₆ alkyl, C₂₋₆ alkenyl, C₂₋₆ alkynyl, C₁₋₆ alkoxy, alkoxyalkyl, cyanoalkyl, heterocycloalkyl, cycloalkyl, C₁₋₆ haloalkyl, CN, and NO₂;
R⁷ and R⁸ are independently selected from H, halo, OH, C₁₋₆ alkyl, C₂₋₆ alkenyl, C₂₋₆ alkynyl, C₁₋₆ alkoxy, C₁₋₆ haloalkyl, CN, and NO₂;
or R⁷ and R⁸ together with the C atom to which they are attached form a 3, 4, 5, 6, or 7-membered cycloalkyl or heterocycloalkyl ring, each optionally substituted by 1, 2, or 3 substituent independently selected from halo, OH, C₁₋₆ alkyl, C₂₋₆ alkenyl, C₂₋₆ alkynyl, C₁₋₆ alkoxy, C₁₋₆ haloalkyl, CN, and NO₂;
R⁹ is H, C₁₋₆ alkyl, C₂₋₆ alkenyl, or C₂₋₆ alkynyl;
W, W', and W" are independently absent or independently selected from C₁₋₆ alkylene, C₂₋₆ alkenylene, C₂₋₆ alkynylene, O, S, NR^{h}, CO, COO, CONR^{h}, SO, SO₂, SONR^{h} and NR^{h}CONRⁱ, wherein each of the C₁₋₆ alkylene, C₂₋₆ alkenylene, and C₂₋₆ alkynylene is optionally substituted by 1, 2 or 3 substituents independently selected from halo, C₁₋₆ alkyl, C₁₋₆ haloalkyl, OH, C₁₋₆ alkoxy, C₁₋₆ haloalkoxy, amino, C₁₋₆ alkylamino, and C₂₋₈ dialkylamino;
X, X', and X" are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, OH, C₁₋₆ alkyl, C₁₋₆ haloalkyl, C₂₋₈ alkoxyalkyl, C₁₋₆ alkoxy, C₁₋₆ haloalkoxy, C₂₋₈ alkoxyalkoxy, cycloalkyl, heterocycloalkyl, C(O)OR^{j}, C(O)NR^{h}Rⁱ, amino, C₁₋₆ alkylamino, and C₂₋₈ dialkylamino;
Y, Y', and Y" are independently absent or independently selected from C₁₋₆ alkylene, C₂₋₆ alkenylene, C₂₋₆ alkynylene, O, S, NR^{h}, CO, COO, CONR^{h}, SO, SO₂, SONR^{h}, and NR^{h}CONRⁱ, wherein each of the C₁₋₆ alkylene, C₂₋₆ alkenylene, and C₂₋₆ alkynylene is optionally substituted by 1, 2 or 3 substituents independently selected from halo, C₁₋₆ alkyl, C₁₋₆ haloalkyl, OH, C₁₋₆ alkoxy, C₁₋₆ haloalkoxy, amino, C₁₋₆ alkylamino, and C₂₋₈ dialkylamino;
Z, Z', and Z" are independently selected from H, halo, C₁₋₆ alkyl, C₂₋₆ alkenyl, C₂₋₆ alkynyl, C₁₋₆ haloalkyl, halosulfanyl, CN, NO₂, N₃, OR^{a2}, SR^{a2}, C(O)R¹², C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)NR^{c2}R^{d2}, NR^{c2}C(O)OR^{a2}, C(=NR⁹)NR^{c2}R^{d2}, NR^{c2}C(=NR^{g})NR^{c2}R^{d2}, P(R^{f2})₂, P(OR^{e2})₂, P(O)R^{e2}R^{f2}, P(O)OR^{e2}OR^{f2}, S(O)R^{b2}, S(O)NR^{c2}R^{d2}, S(O)₂R^{b2}, NR^{c2}S(O)₂R^{b2}, S(O)₂NR^{c2}R^{d2}, aryl, cycloalkyl, heteroaryl, and heterocycloalkyl, wherein said C₁₋₆ alkyl, C₂₋₆ alkenyl, C₂₋₆ alkynyl, aryl, cycloalkyl, heteroaryl, and heterocycloalkyl are optionally substituted by 1, 2, 3, 4 or 5 substituents independently selected from halo, C₁₋₆ alkyl, C₂₋₆ alkenyl, C₂₋₆ alkynyl, C₁₋₆ haloalkyl, halosulfanyl, CN, NO₂, N₃, OR^{a2}, SR^{a2}, C(O)R¹², C(O)NR^{c2}R^{d2}, C(O)OR^{a2}, OC(O)R¹², OC(O)NR^{c2}R^{d2}, NR^{c2}R^{d2}, NR^{c2}C(O)R^{b2}, NR^{c2}C(O)NR^{c2}R^{d2}, NR^{c2}C(O)OR^{a2}, C(=NR^{g})NR^{c2}R^{d2}, NR^{c2}C(=NR⁹)NR^{c2}R^{d2}, P(R²)₂, P(OR^{e2})₂, P(O)R^{e2}R², P(O)OR^{e2}OR^{f2}, S(O)R^{b2}, S(O)NR^{c2}R^{d2}, S(O)₂R^{b2}, NR^{c2}S(O)₂R^{b2}, and S(O)₂NR^{c2}R^{d2};
wherein two adjacent -W-X-Y-Z, together with the atoms to which they are attached, optionally form a fused 4-20 membered cycloalkyl ring or a fused 4-20 membered heterocycloalkyl ring, each optionally substituted by 1, 2, or 3 substituents independently selected from halo, C₁₋₆ alkyl, C₂₋₆ alkenyl, C₂₋₆ alkynyl, C₁₋₆ haloalkyl, halosulfanyl, CN, NO₂, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{d3}, NR^{c3}C(O)R^{b3}, NR^{c3}C(O)NR^{c3}R^{d3}, NR^{c3}C(O)OR^{a3}, C(=NR^{g})NR^{c3}R^{d3}, NR^{c3}C(=NR^{g})NR^{c3}R^{d3}, S(O)R^{b3}, S(O)NR^{c3}R^{d3}, S(O)₂R^{b3}, NR^{c3}S(O)₂R^{b3}, S(O)₂NR^{c3}R^{d3}, aryl, cycloalkyl, heteroaryl, and heterocycloalkyl;
wherein two adjacent -W'-X'-Y'-Z', together with the atoms to which they are attached, optionally form a fused 4-20 membered cycloalkyl ring or a fused 4-20 membered heterocycloalkyl ring, each optionally substituted by 1, 2, or 3 substituents independently selected from halo, C₁₋₆ alkyl, C₂₋₆ alkenyl, C₂₋₆ alkynyl, C₁₋₆ haloalkyl, halosulfanyl, CN, NO₂, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{d3}, NR^{c3}C(O)R^{b3}, NR^{c3}C(O)NR^{c3}R^{d3} NR^{c3}C(O)OR^{a3}, C(=NR^{g})NR^{c3}R^{d3}, NR^{c3}C(=NR^{g})NR^{c3}R^{d3}, S(O)R^{b3}, S(O)NR^{c3}R^{d3}, S(O)₂R^{b3} NR^{c3}S(O)₂R^{b3}, S(O)₂NR^{c3}R^{d3}, aryl, cycloalkyl, heteroaryl, and heterocycloalkyl;
Cy⁴, and Cy⁵ are independently selected from aryl, cycloalkyl, heteroaryl, and heteorcycloalkyl, each optionally substituted by 1, 2, 3, 4, or 5 substituents independently selected from halo, C₁₋₆ alkyl, C₂₋₆ alkenyl, C₂₋₆ alkynyl, C₁₋₆ haloalkyl, halosulfanyl, CN, NO₂, N₃, OR^{a4}, SR^{a4}, C(O)R^{b4}, C(O)NR^{c4}R^{d4}, C(O)OR^{a4}, OC(O)R^{b4}, OC(O)NR^{c4}R^{d4}, NR^{c4}R^{d4}, NR^{c4}C(O)R^{b4}, NR^{c4}C(O)NR^{c4}R^{d4}, NR^{c4}C(O)OR^{a4}, C(=NR^{g})NR^{c4}R^{d4}, NR^{c4}C(=NR^{g})NR^{c4}R^{d4}, P(R^{f4})₂, P(OR⁴)₂, P(O)R^{e4}R^{f4}, P(O)OR^{e4}OR^{f4}, S(O)^{b4} S(O)NR^{c4}R^{d4}, S(O)₂R^{b4}, NR^{c4}S(O)₂R^{b4}, and S(O)₂NR^{c4}R^{d4};
R^{A} is H, C₁₋₄ alkyl, C₂₋₄ alkenyl, C₂₋₄ alkynyl, cycloalkyl, heterocycloalkyl, aryl, or heteroaryl wherein said C₁₋₄ alkyl, C₂₋₄ alkenyl, C₂₋₄ alkynyl, cycloalkyl, heterocycloalkyl, aryl or heteroaryl is optionally substituted with 1, 2, or 3 substituents independently selected from OH, CN, amino, halo, and C₁₋₄ alkyl;
R^{B} is H, C₁₋₄ alkyl, C₂₋₄ alkenyl, C₂₋₄ alkynyl, cycloalkyl, heterocycloalkyl, aryl, or heteroaryl wherein said C₁₋₄ alkyl, C₂₋₄ alkenyl, or C₂₋₄ alkynyl, cycloalkyl, heterocycloalkyl, aryl, or heteroaryl is optionally substituted with 1, 2, or 3 substituents independently selected from OH, CN, amino, halo, and C₁₋₄ alkyl;
R^{C} and R^{D} are independently selected from H, C₁₋₄ alkyl, C₂₋₄ alkenyl, or C₂₋₄ alkynyl, wherein said C₁₋₄ alkyl, C₂₋₄ alkenyl, or C₂₋₄ alkynyl, is optionally substituted with 1, 2, or 3 substituents independently selected from OH, CN, amino, halo, and C₁₋₄ alkyl;
or R^{C} and R^{D} together with the N atom to which they are attached form a 4-, 5-, 6- or 7 - membered heterocycloalkyl group or heteroaryl group, each optionally substituted with 1, 2, or 3 substituents independently selected from OH, CN, amino, halo, and C₁₋₄ alkyl;
R^{a}, R^{a1}, R^{a2}, R^{a3}, and R^{a4} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{b}, R^{b1}, R^{b2}, R^{b3}, and R^{b4} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{c} and R^{d} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkyl;
or R^{c} and R^{d} together with the N atom to which they are attached form a 4-, 5-, 6- or 7-membered heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{c1} and R^{d1} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
or R^{c1} and R^{d1} together with the N atom to which they are attached form a 4-, 5-, 6- or 7-membered heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{c2} and R^{d2} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl, heterocycloalkylalkyl, arylcycloalkyl, arylheterocycloalkyl, arylheteroaryl, biaryl, heteroarylcycloalkyl, heteroarylheterocycloalkyl, heteroarylaryl, and biheteroaryl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl, heterocycloalkylalkyl, arylcycloalkyl, arylheterocycloalkyl, arylheteroaryl, biaryl, heteroarylcycloalkyl, heteroarylheterocycloalkyl, heteroarylaryl, and biheteroaryl are each optionally substituted with 1, 2, or 3 substituents independently selected from OH, CN, amino, halo, C₁₋₆ alkyl, C₁₋₆ alkoxy, C₁₋₆ haloalkyl, C₁₋₆ haloalkoxy, hydroxyalkyl, cyanoalkyl, aryl, heteroaryl, C(O)OR^{a4}, C(O)R^{b4}, S(O)₂R^{b3}, alkoxyalkyl, and alkoxyalkoxy;
or R^{c2} and R^{d2} together with the N atom to which they are attached form a 4-, 5-, 6- or 7-membered heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ baloalkyl, C₁₋₆ haloalkoxy, hydroxyalkyl, cyanoalkyl, aryl, heteroaryl, C(O)OR^{a4}, C(O)R^{b4}, S(O)₂R^{b3}, alkoxyalkyl, and alkoxyalkoxy;
R^{c3} and R^{d3} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloatkoxy;
or R^{c3} and R^{d3} together with the N atom to which they are attached form a 4-, 5-, 6- or 7-membered heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{c4} and R^{d4} are independently selected from H, C₁₋₁₀alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
or R^{c4} and R^{d4} together with the N atom to which they are attached form a 4-, 5-, 6- or 7-membered heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{e}, R^{e1}, R^{e2}, and R^{e4} are independently selected from H, C₁₋₆ alkyl, C₁₋₆ haloalkyl, C₂₋₆ alkenyl, (C₁₋₆ alkoxy)-C₁₋₆ alkyl, C₂₋₆ alkynyl, aryl, cycloalkyl, heteroaryl, heterocycloalkyl, arylalkyl, cycloalkylalkyl, heteroarylalkyl, and heterocycloalkylalkyl;
R^{f}, R^{f1}, R^{f2}, and R^{f4} are independently selected from H, C₁₋₆ alkyl, C₁₋₆ haloalkyl, C₂₋₆ alkenyl, C₂₋₆ alkynyl, aryl, cycloalkyl, heteroaryl, and heterocycloalkyl;
R^{g} is H, CN, and NO₂;
R^{h} and Rⁱ are independently selected from H and C₁₋₆ alkyl;
R^{j} is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl;
m is 0, 1, 2, 3, 4, 5, or 6;
p is 0, 1, 2, 3, or 4;
q is 0, 1, 2, 3, or 4;
r is 0, 1, 2, 3, 4, 5, or 6;
s is 0, 1, 2, 3, or 4; and
t is 0, 1, 2, 3, or 4;
with the proviso that when A is CH, then L1 is other than CO or (CR⁴R⁵)ᵤ wherein u is 1.

In one embodiment, L¹ is (CR⁴R⁵)ₘ, wherein R⁴ and R⁵ are independently H and m is 1; Cy¹ is heteroaryl; R¹ is H; A is N; R² is H; L² is (CR⁷R⁸)ᵣ,wherein r is 0; and Cy² is aryl substituted with 2 W'-X'-Y'-Z'.

In one embodiment, INC280 has the following structure:

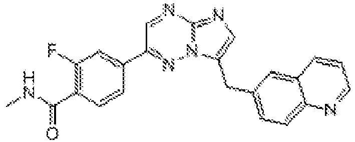

In one embodiment, INC280 is 2-fluoro-N-methyl-4-[7-(quinolin-6-ylmethyl)imidazo[1,2-b][1,2,4]triazin-2-yl]benzamide, or a pharmaceutically acceptable salt thereof.

In one embodiment, the the inhibitor of the immune checkpoint molecule (alone or in combination with other immunomodulators) is used in combination with an Alk inhibitor to treat a cancer, *e.g.,* a cancer described herein (*e.g.,* a cancer disclosed in Table 1). In one embodiment, the Alk inhibitor is disclosed in Table 1, *e.g*., LDK378, or in a publication recited in Table 1, *e.g.,* in WO 2008/073687 (*e.g.,* Example 7/Compound 66) or US 8,039,479 (*e.g.,* claim 1 or 5) (also known as ceritinib (Zykadia®). In one embodiment, the Alk inhibitor, *e.g*., LDK378, has the structure (compound or generic structure) provided in Table 1, or as disclosed in the publication recited in Table 1, *e.g.,* in WO 2008/073687 (*e.g*., Example 7/Compound 66) or US 8,039,479 (*e.g*., claim 1 or 5).

In one embodiment, one of Nivolumab, Pembrolizumab or MSB0010718C is used in combination with LDK378 to treat a cancer described in Table 1, *e.g.,* a solid tumor, *e.g.,* a lung cancer (*e.g.,* non-small cell lung cancer (NSCLC)), a lymphoma (*e.g.,* an anaplastic large-cell lymphoma or non-Hodgkin lymphoma), an inflammatory myofibroblastic tumor (IMT), or a neuroblastoma. In some embodiments, the NSCLC is a stage IIIB or IV NSCLC, or a relapsed locally advanced or metastic NSCLC. In some embodiments, the cancer (*e.g.,* the lung cancer, lymphoma, inflammatory myofibroblastic tumor, or neuroblastoma) has, or is identified as having, an ALK rearrangement or translocation, *e.g*., an ALK fusion. In one embodiment, the ALK fusion is an EML4-ALK fusion, *e.g.,* an EML4-ALK fusion described herein. In another embodiment, the ALK fusion is an ALK-ROS1 fusion. In certain embodiments, the cancer has progressed on, or is resistant or tolerant to, a ROS1 inhibitor, or an ALK inhibitor, *e.g.,* an ALK inhibitor other than LDK378. In some embodiments, the cancer has progressed on, or is resistant or tolerant to, crizotinib. In one embodiment, the subject is an ALK-naïve patient, *e.g.,* a human patient. In another embodiment, the subject is a patient, *e.g.,* a human patient, that has been pretreated with an ALK inhibitor. In another embodiment, the subject is a patient, *e.g.,* a human patient, that has been pretreated with LDK378.

In one embodiment, LDK378 and Nivolumab are administered to an ALK-naïve patient. In another embodiment, LDK378 and Nivolumab are administered to a patient that has been pretreated with an ALK inhibitor. In yet another embodiment, LDK378 and Nivolumab are administered to a patient that has been pretreated with LDK378.

In certain embodiments, LDK378 is administered at an oral dose of about 100 to 1000 mg, *e.g.,* about 150 mg to 900 mg, about 200 mg to 800 mg, about 300 mg to 700 mg, or about 400 mg to 600 mg, *e.g.,* about 150 mg, 300 mg, 450 mg, 600 mg or 750 mg. In certain embodiment, LDK378 is administered at an oral dose of about 750 mg or lower, *e.g.,* about 600 mg or lower, *e.g.,* about 450 mg or lower. In certain embodiments, LDK378 is administered with food. In other embodiments, the dose is under fasting condition. The dosing schedule can vary from *e.g.,* every other day to daily, twice or three times a day. In one embodiment, LDK378 is administered daily. In one embodiment, LDK378 is administered at an oral dose from about 150 mg to 750 mg daily, either with food or in a fasting condition. In one embodiment, LDK378 is administered at an oral dose of about 750 mg daily, in a fasting condition. In one embodiment, LDK378 is administered at an oral dose of about 750 mg daily, via capsule or tablet. In another embodiment, LDK378 is administered at an oral dose of about 600 mg daily, via capsule or tablet. In one embodiment, LDK378 is administered at an oral dose of about 450 mg daily, via capsule or tablet.

In one embodiment, LDK378 is administered at a dose of about 450 mg and nivolumab is administered at a dose of about 3 mg/kg. In another embodiment, the LDK378 dose is 600 mg and the nivolumab dose is 3 mg/kg. In one embodiment, LDK378 is administered with a low fat meal.

In one embodiment, the Alk inhibitor has the following structure: 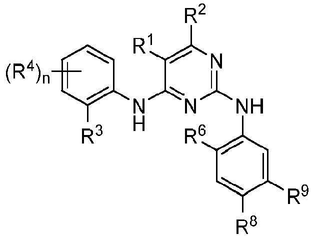 or pharmaceutically acceptable salts thereof;
wherein R¹ is halo or C₁₋₆ alkyl;
R² is H;
R³ is (CR₂)₀₋₂SO₂R¹²;
R⁴ is C₁₋₆ alkyl, C₂₋₆ alkenyl or C₂₋₆ alkynyl; OR¹², NR(R¹²), halo, nitro, SO₂R¹², (CR₂)ₚR¹³ or X; or R⁴ is H;
R⁶ is isopropoxy or methoxy;
   one of R⁸ and R⁹ is (CR₂)_{q}Y and the other is C₁₋₆ alkyl, cyano, C(O)O₀₋₁R¹², CONR(R¹²) or CONR(CR₂)ₚNR(R¹²);
X is (CR₂)_{q}Y, cyano, C(O)O₀₋₁R¹², CONR(R¹²), CONR(CR₂)ₚNR(R¹²), CONR(CR₂)ₚOR¹², CONR(CR₂)ₚSR¹², CONR(CR₂)ₚS(O)₁₋₂R¹² or (CR₂)₁₋₆NR(CR₂)ₚOR¹²;
Y is pyrrolidinyl, piperidinyl or azetidinyl, each of which is attached to the phenyl ring via a carbon atom;
R¹² and R¹³ are independently 3-7 membered saturated or partially unsaturated carbocyclic ring, or a 5-7 membered heterocyclic ring comprising N, O and/or S; aryl or heteroaryl; or R¹² is H or C₁₋₆ alkyl;
R is H or C₁₋₆ alkyl;
n is 0-1;
p is 0-4; and
q is 0.

In one embodiment, R² is H; R³ is SO₂R¹² and R¹² is C₁₋₆ alkyl; R⁴ is H (n=1); R⁶ is isopropoxy; and one of R⁸ and R⁹ is (CR²)qY wherein q=0, Y is piperidinyl and the other is C₁₋₆ alkyl.

In one embodiment, LDK378 has the following structure:

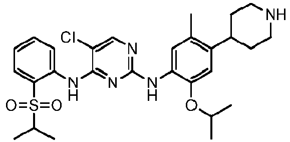

In one embodiment, LDK378 is 5-chloro-N2-(2-isopropoxy-5-methyl-4-(piperidin-4-yl)-phenyl)-N4-[2-(propane-2-sulfonyl)-phenyl]-pyrimidine-2,4-diamine, or a pharmaceutically acceptable salt thereof.

In one embodiment, the inhibitor of the immune checkpoint molecule (alone or in combination with other immunomodulators) is used in combination with a CDK4/6 inhibitor to treat a cancer, *e.g.,* a cancer described herein (*e.g.,* a cancer disclosed in Table 1). In one embodiment, the CDK4/6 inhibitor is disclosed in Table 1, *e.g.,* LEE011, or in a publication recited in Table 1, *e.g.,* in US 8,685,980 or US 8,415,355 (*e.g*., Formula (I) in columns 3-4 or in Example 74 at column 66). In one embodiment, the CDK4/6 inhibitor, *e.g*., LEE011, has the structure (compound or generic structure) provided in Table 1, or as disclosed in the publication recited in Table 1, *e.g.,* in US 8,685,980 or US 8,415,355 (*e.g*., Formula (I) in columns 3-4 or in Example 74 at column 66). In one embodiment, one of Nivolumab, Pembrolizumab or MSB0010718C is used in combination with LEE011 to treat a cancer described in Table 1, *e.g.,* a solid tumor, *e.g.,* a lung cancer (*e.g.,* non-small cell lung cancer (NSCLC)), a neurologic cancer, melanoma or a breast cancer, or a hematological malignancy, *e.g.,* lymphoma.

In one embodiment, the CDK4/6 inhibitor has the following structure: 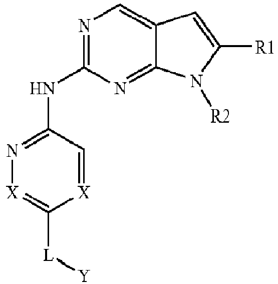
X is CR⁹ or N;
R¹ is C₁₋₈ alkyl, CN, C(O)OR⁴ or CONR⁵R⁶, a 5-14 membered heteroaryl group, or a 3-14 membered cycloheteroalkyl group;
R² is C₁₋₈alkyl, C₃₋₁₄ cycloalkyl, or a 5-14 membered heteroaryl group, and wherein R² may be substituted with one or more C₁₋₈alkyl, or OH;
L is a bond, C₁₋₈ alkylene, C(O), or C(O)NR¹⁰, and wherein L may be substituted or unsubstituted;
Y is H, R¹¹, NR¹²R¹³, OH, or Y is part of the following group 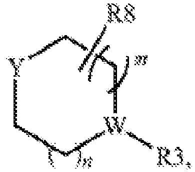
where Y is CR⁹ or N; where 0-3 R⁸ may be present, and R⁸ is C₁₋₈ alkyl, oxo, halogen, or two or more R⁸ may form a bridged alkyl group;
W is CR⁹, or N, or O (where W is O, R³ is absent);
R³ is H, C₁₋₈alkyl, C₁₋₈ alkylR¹⁴, C₃₋₁₄ cycloalkyl, C(O)C₁₋₈ alkyl, C₁₋₈ haloalkyl, C₁₋₈ alkylOH, C(O)NR¹⁴R¹⁵, C₁₋₈ cyanoalkyl, C(O)R¹⁴, C₀₋₈ alkylC(O)C₀₋₈ alkylNR¹⁴R¹⁵, C₀₋₈ alkylC(O)OR¹⁴, NR¹⁴R¹⁵, SO₂C₁₋₈ alkyl, C₁₋₈ alkylC₃₋₁₄ cycloalkyl, C(O)C₁₋₈ alkylC₃₋₁₄ cycloalkyl, C₁₋₈ alkoxy, or OH which may be substituted or unsubstituted when R³ is not H.
R⁹ is H or halogen;
R⁴, R⁵, R⁶, R⁷, R¹⁰, R¹¹, R¹², R¹³, R¹⁴, and R¹⁵ are each independently selected from H, C₁₋₈ alkyl, C₃₋₁₄ cycloalkyl, a 3-14 membered cycloheteroalkyl group, a C₆₋₁₄ aryl group, a 5-14 membered heteroaryl group, alkoxy, C(O)H, C(N)OH, C(N)OCH₃, C(O)C₁₋₃ alkyl, C₁₋₈ alkylNH₂, C₁₋₆ alkylOH, and wherein R⁴, R⁵, R⁶, R⁷, R¹⁰, R¹¹, R¹², and R¹³, R¹⁴, and R¹⁵ when not H may be substituted or unsubstituted;
m and n are independently 0-2; and
wherein L, R³, R⁴, R⁵, R⁶, R⁷, R¹⁰, R¹¹, R¹², and R¹³, R¹⁴, and R¹⁵ may be substituted with one or more of C₁₋₈ alkyl, C₂₋₈ alkenyl, C₂₋₈ alkynyl, C₃₋₁₄ cycloalkyl, 5-14 membered heteroaryl group, C₆₋₁₄ aryl group, a 3-14 membered cycloheteroalkyl group, OH, (O), CN, alkoxy, halogen, or NH₂.

In one embodiment, X is CR⁹, wherein R⁹ is H; R¹ is CONR⁵R⁶, wherein R⁵ and R⁶ are both C₁₋₈ alkyl, specifically methyl; R² is C₃₋₁₄ cycloalkyl, specifically cyclopentyl; L is a bond; and Y is part of the group 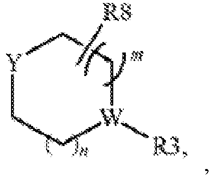 wherein Y is N, zero R⁸ are present, W is N, m and n are both 1, and R³ is H.

In one embodiment, LEE011 has the following structure:

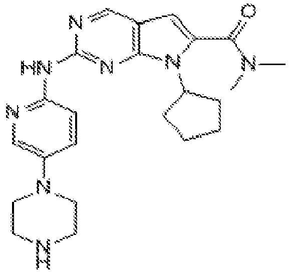

In one embodiment, LEE011 is 7-cyclopentyl-2-(5-piperazin-1-yl-pyridin-2-ylamino)-7H-pyrrolo[2,3-d]pyrimidine-6-carboxylic acid dimethylamide or a pharmaceutically acceptable salt thereof.

In one embodiment, the inhibitor of the immune checkpoint molecule (alone or in combination with other immunomodulators) is used in combination with a PI3K-inhibitor to treat a cancer, *e.g.,* a cancer described herein (*e.g.,* a cancer disclosed in Table 1). In one embodiment, the PI3K-inhibitor is disclosed in Table 1, e.g., BKM120 or BYL719, or in a publications recited in Table 1, *e.g.,* in WO2007/084786 (*e.g*., Example 10 in [0389] or Formula (I) in [0048]) or WO2010/029082 (*e.g*., Example 15 or Formula (I)). In one embodiment, the PI3K-inhibitor, *e.g.,* BKM120 or BYL719, has the structure (compound or generic structure) provided in Table 1, or as disclosed in the publications recited in Table 1 *e.g.,* in WO2007/084786 (*e.g.,* Example 10 in [0389] or Formula (I) in [0048]) or WO2010/029082 (*e.g.,* Example 15 or Formula (I)). In one embodiment, one of Nivolumab, Pembrolizumab or MSB0010718C is used in combination with BKM120 or BYL719 to treat a cancer or disorder described in Table 1, *e.g.,* a solid tumor, *e.g.,* a lung cancer (*e.g.,* non-small cell lung cancer (NSCLC)), a prostate cancer, an endocrine cancer, an ovarian cancer, a melanoma, a bladder cancer, a female reproductive system cancer, a digestive/gastrointestinal cancer, a colorectal cancer, glioblastoma multiforme (GBM), a head and neck cancer, a gastric cancer, a pancreatic cancer or a breast cancer; or a hematological malignancy, *e.g.,* leukemia, non-Hodgkin lymphoma; or a hematopoiesis disorder.

In one embodiment, the PI3K-inhibitor has the following structure: 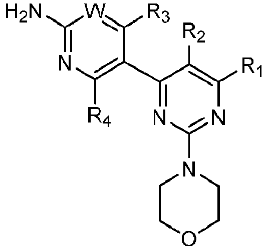 or a stereoisomer, tautomer, or pharmaceutically acceptable salt thereof,
wherein, W is CRWor N, wherein Rw is selected from the group consisting of (1) hydrogen, (2) cyano, (3) halogen, (4) methyl, (5) trifluoromethyl, and (6) sulfonamido; R₁ is selected from the group consisting of (1) hydrogen, (2) cyano, (3) nitro, (4) halogen, (5) substituted and unsubstituted alkyl, (6) substituted and unsubstituted alkenyl, (7) substituted and unsubstituted alkynyl, (8) substituted and unsubstituted aryl, (9) substituted and unsubstituted heteroaryl, (10) substituted and unsubstituted heterocyclyl, (11) substituted and unsubstituted cycloalkyl, (12) -COR₁ₐ, (13)-CO₂R₁ₐ(14)-CONR₁ₐR_{1b}, (15) -NR₁ₐR_{1b},, (16)-NR₁ₐCOR_{1b}, (17) -NR₁ₐSO₂R_{1b}, (18) -OCOR₁ₐ, (19) -OR₁ₐ, (20)-SR₁ₐ(21)-SOR₁ₐ, (22)-SO₂R₁ₐ, and (23)-SO₂NR₁ₐR_{1b}, wherein R₁ₐ, and R_{1b} are independently selected from the group consisting of (a) hydrogen, (b) substituted or unsubstituted alkyl, (c) substituted and unsubstituted aryl, (d) substituted and unsubstituted heteroaryl, (e) substituted and unsubstituted heterocyclyl, and (f) substituted and unsubstituted cycloalkyl; R is selected from the group consisting (1) hydrogen, (2) cyano, (3) nitro, (4) halogen, (5) hydroxy, (6) amino, (7) substituted and unsubstituted alkyl, (8) -COR2a , and wherein R₂ₐ- and R_{2b} are independently selected from the group consisting of (a) hydrogen, and (b) substituted or unsubstituted alkyl; R₃ is selected from the group consisting of (1) hydrogen, (2) cyano, (3) nitro, (4) halogen, (5) substituted and unsubstituted alkyl, (6) substituted and unsubstituted alkenyl, (7) substituted and unsubstituted alkynyl, (8) substituted and unsubstituted aryl, (9) substituted and unsubstituted heteroaryl, (10) substituted and unsubstituted heterocyclyl, (11) substituted and unsubstituted cycloalkyl, (12)-COR₃ₐ, (13)-NR₃ₐR_{3b}, (16)-OR₃ₐ, (17)-SR₃ₐ, (18)-SOR₃ₐ, (19)-SO₂R₃, and (20)-SO₂NR₃ₐR_{3b}, wherein R₃ₐ, and R_{3b} are independently selected from the group consisting of (a) hydrogen, (b) substituted or unsubstituted alkyl, (c) substituted and unsubstituted aryl, (d) substituted and unsubstituted heteroaryl, (e) substituted and unsubstituted heterocyclyl, and (f) substituted and unsubstituted cycloalkyl; and R₄ is selected from the group consisting of (1) hydrogen, and (2) halogen.

In one embodiment, W is CRw and Rw is hydrogen, R₁ is unsubstituted heterocyclyl, R₂ is hydrogen, R₃ is substituted alkyl, and R₄ is hydrogen.

In one embodiment, BKM120 has the following structure:

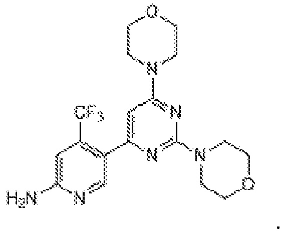

In one embodiment, BKM120 is 4-(trifluoromethyl)-5-(2,6-dimorpholinopyrimidin-4-yl)pyridine-2-amine or a pharmaceutically acceptable salt thereof.

In one embodiment, the PI3K-inhibitor has the following structure: 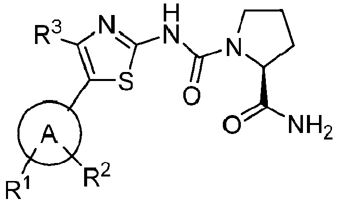 or salt thereof, wherein

A represents a heteroaryl selected from the group consisting of:

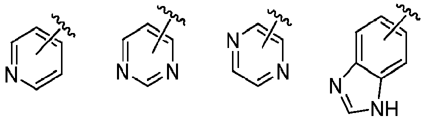

R¹ represents one of the following substituents: (1) unsubstituted or substituted, preferably substituted C₁-C₇ alkyl, wherein said substituents are independently selected from one or more, preferably one to nine of the following moieties: deuterium, fluoro, or one to two of the following moietiesC₃-C₅ cycloalkyl; (2) optionally substituted C₃-C₅ cycloalkyl wherein said substituents are independently substituted C₃-C₅ cycloalkyl wherein said substituents are independently selected from one or more, preferably one to four of the following moieties: deuterium, C₁-C₄ alkyl (preferably methyl), fluoro, cyano, aminocarbonyl; (3) optionally substituted phenyl wherein said substituents are independently selected from one or more, preferably one to two of the following moieties: deuterium, halo, cyano, C₁-C₇ alkyl, C₁-C₇ alkylamino, di(C₁-C₇ alkyl)amino, C₁-C₇ alkylaminocarbonyl, di(C₁-C₇-alkyl)aminocarbonyl, C₁-C₇ alkoxy; (4) optionally mono- or di-substituted amine; wherein said substituents are independently selected from the following moieties: deuterium, C₁-C₇ alkyl (which is unsubstituted or substituted by one or more substituents selected from the group of deuterium, fluoro, chloro, hydroxyl), phenylsulfonyl (which is unsubstituted or substituted by one or more, preferably one, C₁-C₇ alkyl, C₁-C₇ alkoxy, di(C₁-C₇-alkyl)amino-C₁-C₇-alkoxy); (5) substituted sulfonyl; wherein said substituent is selected from the following moieties: C₁-C₇ alkyl (which is unsubstituted or substituted by one or more substituents selected from the group of deuterium, fluoro), pyrrolidino, (which is unsubstituted or substituted by one or more substituents selected from the group of deuterium, hydroxyl, oxo; particularly one oxo); (6) fluoro, chloro; R2 represents hydrogen; R3 represents (1) hydrogen, (2) fluoro, chloro, (3) optionally substituted methyl, wherein said substituents are independently selected from one or more, preferably one to three of the following moireites: deuterium, fluoro, chloro, dimethylamino: with the exception of (S)-pyrrolidine-1,2-dicarboxylic acid 2-amide 1-({5-[2-(tert-butyl)-pyrimidin-4-yl]-4-methyl-thiazol-2-yl}-amide).

In one embodiments, A is 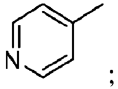 R¹ is substituted C₁-C₇ alkyl, wherein said substituents are independently selected from one or more, preferably one to nine of deuterium, fluoro, or C₃-C₅ cycloalkyl; R² is hydrogen, and R³ is methyl.

In one embodiment, BYL719 has the following structure:

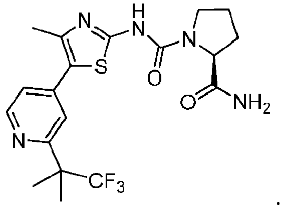

In one embodiment, BYL719 is (S)-pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof.

In one embodiment, the inhibitor of the immune checkpoint molecule (alone or in combination with other immunomodulators) is used in combination with a BRAF inhibitor to treat a cancer, *e.g.,* a cancer described herein (*e.g.,* a cancer disclosed in Table 1). In one embodiment, the BRAF inhibitor is disclosed in Table 1, *e.g.,* LGX818, or in a publication recited in Table 1, *e.g.,* WO2011/025927 (*e.g.,* Example 6/Compound 6 or Formula (Ia) in [0030]) or US 8,501,758 (*e.g.,* Example 5 in column 45). In one embodiment, the BRAF inhibitor, *e.g.,* LGX818, has the structure (compound or generic structure) provided in Table 1, or as disclosed in the publication recited in Table 1. In one embodiment, one of Nivolumab, Pembrolizumab or MSB0010718C is used in combination with LGX818 to treat a cancer described in Table 1, *e.g.,* a solid tumor, *e.g.,* a lung cancer (*e.g.,* non-small cell lung cancer (NSCLC)), a melanoma, *e.g.,* advanced melanoma, a thyroid cancer, *e.g,* papillary thyroid cancer, or a colorectal cancer. In some embodiments, the cancer has, or is identified as having, a BRAF mutation (*e.g*., a BRAF V600E mutation), a BRAF wildtype, a KRAS wildtype or an activating KRAS mutation. The cancer may be at an early, intermediate or late stage.

In one embodiment, the BRAF inhibitor has the following structure: 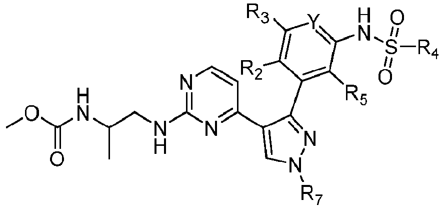 in Y is selected from N and CR₆; R₂, R₃, R₅, and R₆ are independently selected from hydrogen, halo, cyano, C₁₋₄ alkyl, halo-substituted C₁₋₄ alkyl, C₁₋₄ alkoxy and halo-substituted C₁₋₄ alkoxy; with the proviso that when R₅ is fluoro and R₁ is selected from hydrogen, -X1R8a,-X₁C(O)NR₈ₐR_{8b}, -XNR₈ₐX₂R_{8b}, -X₁NR₈ₐC(O)X₂OR_{8b} and -X₁NR₈ₐS(O)₀₋₂R_{8b}, R₃ and R₆ are not both hydrogen; R₄ is selected from -R₉ and -NR₁₀R₁₁; wherein R₉ is selected from C₁₋₆ alkyl, C₃₋₈ cycloalkyl, C₃₋₈ heterocycloalkyl, aryl, and heteroaryl; wherein said aryl, cycloalkyl, heterocycloalkyl, aryl, or heteroaryl of R₉ is optionally substituted with 1 to 3 radicals independently selected from halo, cyano, C₁₋₄ alkyl, halo-substituted C₁₋₄ alkyl, C₁₋₄ alkoxy and halo-substituted C₁₋₄ alkoxy; and R10 and R11 are independently selected from hydrogen and R9; and R7 is selected from hydrogen, C₁₋₄ alkyl, C3-5 cycloalkyl and C3-5 heterocycloalkyl; wherein said alkyl, cycloalkyl, or heterocycloalkyl of R7 is optionally substituted with 1 to 3 radicals independently selected from halo, cyano, hydroxyl, C₁₋₄ alkyl, halo-substituted C₁₋₄ alkyl, _{C1-4} alkoxy and halo-substitured C₁₋₄ alkoxy.

In one embodiment, R₃ is halo (*e.g.,* chloro); R₄ is R₉; R₉ is C₁₋₆ alkyl (*e.g.,* methyl), R₅ is halo (*e.g.,* fluoro), R₇ is C₁₋₄ alkyl (*e.g.,* isopropyl); Y is CR₆; and R₆ is H. In one embodiment, LGX818 has the following structure:

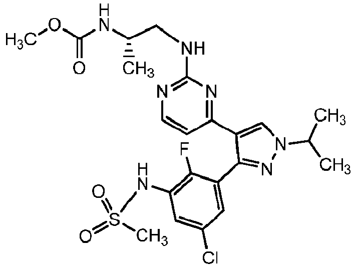

In one embodiment, LGX818 is methyl (S)-(1-((4-(3-(5-chloro-2-fluoro-3-(methylsulfonamido)phenyl)-1-isopropyl-1H-pyrazol-4-yl)pyrimidin-2-yl)amino)propan-2-yl)carbamate or a pharmaceutically acceptable salt thereof.

In one embodiment, the inhibitor of the immune checkpoint molecule (alone or in combination with other immunomodulators) is used in combination with a CAR T cell targeting CD19 to treat a cancer, *e.g.,* a cancer described herein (*e.g.,* a cancer disclosed in Table 1). In one embodiment, the CAR T cell targeting CD19 is disclosed in Table 1, *e.g.,* CTL019, or in a publication recited in Table 1, *e.g.,* WO 2012/079000, *e.g.,* SEQ ID NO: 12 *(e.g.,* full-length CAR) or SEQ ID NO: 14 (*e.g.,* CD19 scFv). In one embodiment, the CAR T cell targeting CD19, *e.g.,* CTL019, has the structure (compound or generic structure) provided in Table 1, or as disclosed in the publication recited in Table 1. In one embodiment, one of Nivolumab, Pembrolizumab or MSB0010718C is used in combination with CTL019 to treat a cancer described in Table 1, *e.g.,* a solid tumor, or a hematological malignancy, *e.g*., a lymphocytic leukemia or a non-Hodgkin lymphoma.

In one embodiment, the CAR T cell targeting CD19 has the USAN designation TISAGENLECLEUCEL-T. CTL019 is made by a gene modification of T cells is mediated by stable insertion via transduction with a self-inactivating, replicationdeficient Lentiviral (LV) vector containing the CTL019 transgene under the control of the EF-1 alpha promoter. CTL019 is a mixture of transgene positive and negative T cells that are delivered to the subject on the basis of percent transgene positive T cells.

In one embodiment, the the inhibitor of the immune checkpoint molecule (alone or in combination with other immunomodulators) is used in combination with a MEK inhibitor to treat a cancer, *e.g.,* a cancer described herein (*e.g.,* a cancer disclosed in Table 1). In one embodiment, the MEK inhibitor is disclosed in Table 1, *e.g.,* MEK162, or in a publication recited in Table 1, *e.g*., WO2003/077914 (*e.g*., Example 18/Compound 29lll or Formula II). In one embodiment, the MEK inhibitor, *e.g.,* MEK162, has the structure (compound or generic structure) provided in Table 1, or as disclosed in the publication recited in Table 1, *e.g.,* WO2003/077914 (*e.g*., Example 18/Compound 29lll or Formula II). In one embodiment, one of Nivolumab, Pembrolizumab or MSB0010718C is used in combination with MEK162 to treat a cancer described in Table 1. In other embodiments, the cancer or disorder treated with the combination is chosen from a melanoma, a colorectal cancer, a non-small cell lung cancer, an ovarian cancer, a breast cancer, a prostate cancer, a pancreatic cancer, a hematological malignancy or a renal cell carcinoma, a multisystem genetic disorder, a digestive/gastrointestinal cancer, a gastric cancer, or a colorectal cancer; or rheumatoid arthritis. In some embodiments, the cancer has, or is identified as having, a KRAS mutation.

In one embodiment, the MEK inhibitor has the following structure: 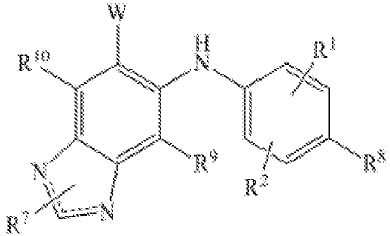 and pharmaceutically accepted salts, prodrugs, and solvates thereof, wherein:
---- is an optional bond, provided that one and only one nitrogen of the ring is double-bonded;
R¹, R², R⁹ and R¹⁰ are independently selected from hydrogen, halogen, cyano, nitro, trifluoromethyl, difluoromethoxy, trifluoromethoxy, azido, -OR³, -C(O)R³, -C(O)OR³, NR⁴C(O)OR⁶, -OC(O)R³, -NR⁴SO₂R⁶, -SO₂NR³R⁴, -NR⁴C(O)R³, -C(O)NR³R⁴,-NR⁵C(O)NR³R⁴, -NR⁵C(NCN)NR³R⁴, -NR³R⁴, and
C₁-C₁₀ alkyl, C₁-C₁₀ alkenyl, C₂-C₁₀ alkynyl, C₃-C₁₀ cycloalkyl, C₃-C₁₀ cycloalkylalkyl,-S(O)ⱼ(C₁-C₆ alkyl), -S(O)j(CR⁴R⁵)ₘ-aryl, aryl, arylalkyl, heteroaryl, heteroarylalkyl, heterocyclyl, heterocyclylalkyl, -O(CR⁴R⁵)ₘ-aryl, -NR⁴(CR⁴R⁵)ₘ-aryl, -O(CR⁴R⁵)ₘ-heteroaryl,-NR⁴(CR⁴R⁵)ₘ-heteroaryl, -O(CR⁴R⁵)ₘ-heterocyclyl and -NR⁴(CR⁴R⁵)ₘ-heterocyclyl, where each alkyl, alkenyl, alkynyl, cycloalkyl, aryl, heteroaryl and heterocyclyl portion is optionally substituted with one to five groups independently selected from oxo, halogen, cyano, nitro, trifluoromethyl, difluoromethoxy, trifluoromethoxy, azido, -NR⁴SO₂R⁶, -SO₂NR³R⁴, -C(O)R³,-C(O)OR³, -OC(O)R³, -NR⁴C(O)OR⁶, -NR⁴C(O)R³, -C(O)NR³R⁴, -NR³R⁴, -NR⁵C(O)NR³R⁴,-NR⁵C(NCN)NR³R⁴, -OR³, aryl, heteroaryl, arylalkyl, heteroarylalkyl, heterocyclyl, and heterocyclylalkyl;
R³ is selected from hydrogen, trifluoromethyl, and
C₁-C₁₀ alkyl, C₂-C₁₀ alkenyl, C₂-C₁₀ alkynyl, C₃-C₁₀ cycloalkyl, C₃-C₁₀ cycloalkylalkyl, aryl, arylalkyl, heteroaryl, heteroarylalkyl, heterocyclyl, and heterocyclylalkyl, where each alkyl, alkenyl, alkynyl, cycloalkyl, aryl, heteroaryl and heterocyclyl portion is optionally substituted with one to five groups independently selected from oxo, halogen, cyano, nitro, trifluoromethyl, difluoromethoxy, trifluoromethoxy, azido, -NR SO₂R, -SO₂NRR, -C(O)R, -C(O)OR,-OC(O)R, -NRC(O)OR"", -NRC(O)R, -C(O)NRR", -SR', -S(O)R"", -SO₂R"", -NRR",-NRC(0)NR"R''', - NRC(NCN)NR"R''', -OR, aryl, heteroaryl, arylalkyl, heteroarylalkyl, heterocyclyl, and heterocyclylalkyl;
R', R" and R'" independently are selected from hydrogen, lower alkyl, lower alkenyl, aryl and arylalkyl;
R"" is selected from lower alkyl, lower alkenyl, aryl and arylalkyl; or
any two of R', R", R'" or R"" can be taken together with the atom to which they are attached to form a 4 to 10 membered carbocyclic, heteroaryl or heterocyclic ring, each of which is optionally substituted with one to three groups independently selected from halogen, cyano, nitro, trifluoromethyl, difluoromethoxy, trifluoromethoxy, azido, aryl, heteroaryl, arylalkyl, heteroarylalkyl, heterocyclyl, and heterocyclylalkyl; or
R³ and R⁴ can be taken together with the atom to which they are attached to form a 4 to 10 membered carbocyclic, heteroaryl or heterocyclic ring, each of which is optionally substituted with one to three groups independently selected from halogen, cyano, nitro, trifluoromethyl, difluoromethoxy, trifluoromethoxy, azido, - NR SO₂R"", -SO₂NR'R", -C(O)R, -C(O)OR ,-OC(O)R, -NRC(O)OR"" , -NRC(O)R", -C(O)NRR", -SO₂R"", -NRR, -NRC(O)NR"R",-NR'C(NCN)NR"R"', -OR, aryl, heteroaryl, arylalkyl, heteroarylalkyl, heterocyclyl, and heterocyclylalkyl; or
R⁴ and R⁵ independently represent hydrogen or C₁-C₆ alkyl; or
R⁴ and R⁵ together with the atom to which they are attached form a 4 to 10 membered carbocyclic, heteroaryl or heterocyclic ring, each of which is optionally substituted with one to three groups independently selected from halogen, cyano, nitro, trifluoromethyl, difluoromethoxy, trifluoromethoxy, azido, -NR SO₂R, -SO₂NR'R", -C(O)R"", -C(O)OR',-OC(O)R, -NR'C(0)0R"", -NR'C(0)R", -C(O)NR'R", -SO₂R"", -NR'R", -NR'C(O)NR"R"',-NRC(NCN)NR"R'"₅-OR, aryl, heteroaryl, arylalkyl, heteroarylalkyl, heterocyclyl, and heterocyclylalkyl;
R⁶ is selected from trifluoromethyl, and
C₁-C₆ alkyl, C₃-C₁₀ cycloalkyl, aryl, arylalkyl, heteroaryl, heteroarylalkyl, heterocyclyl, heterocyclylalkyl, where each alkyl, cycloalkyl, aryl, heteroaryl and heterocyclyl portion is optionally substituted with one to five groups independently selected from oxo, halogen, cyano, nitro, trifluoromethyl, difluoromethoxy, trifluoromethoxy, azido, -NR SO₂R, -SO₂NR'R",-C(O)R , -C(O)OR, -OC(O)R, -NR'C(0)0R"", -NR'C(0)R", -C(O)NRR", -SO₂R"", -NR'R,-NR'C(O)NR"R", -NR'C(NCN)NR"R'", -OR, aryl, heteroaryl, arylalkyl, heteroarylalkyl, heterocyclyl, and heterocyclylalkyl;
R⁷ is selected from hydrogen, and
C₁-C₁₀ alkyl, C₂-C₁₀ alkenyl, C₂-C₁₀ alkynyl, C₃-C₁₀ cycloalkyl, C₃-C₁₀ cycloalkylalkyl, aryl, arylalkyl, heteroaryl, heteroarylalkyl, heterocyclyl, heterocyclylalkyl, where each alkyl, alkenyl, alkynyl, cycloalkyl, aryl, heteroaryl and heterocyclyl portion is optionally substituted with one to five groups independently selected from oxo, halogen, cyano, nitro, trifluoromethyl, difluoromethoxy, trifluoromethoxy, azido, -NR⁴SO₂R⁶, -SO₂NR³R⁴, -C(O)R³, -C(O)OR ³,-OC(O)R³, -NR⁴C(O)OR⁶, -NR⁴C(O)R³, -C(O)NR³R⁴, -SO₂R⁶, -NR³R⁴, -NR⁵C(O)NR³R⁴,-NR⁵C(NCN)NR³R⁴, -OR³, aryl, heteroaryl, arylalkyl, heteroarylalkyl, heterocyclyl, and heterocyclylalkyl;
W is selected from heteroaryl, heterocyclyl, -C(O)OR³, -C(O)NR³R⁴, -C(O)NR⁴OR³,-C(O)R⁴OR³, -C(O)(C₃-C₁₀ cycloalkyl), -C(O)(C₁-C₁₀ alkyl), -C(O)(aryl), -C(O)(heteroaryl) and-C(O)(heterocyclyl), each of which is optionally substituted with 1-5 groups independently selected from -NR³R⁴, -OR³, -R², and C₁- C₁₀ alkyl, C₂-C₁₀ alkenyl, and C₂-C₁₀ alkynyl, each of which is optionally substituted with 1 or 2 groups independently selected from-NR³R⁴ and-OR³;
R⁸ is selected from hydrogen, -SCF₃, -Cl, -Br, -F, cyano, nitro, trifluoromethyl, difluoromethoxy, trifluoromethoxy, azido, -OR³, -C(O)R³, -C(O)OR³,-NR⁴C(O)OR⁶, -OC(O)R³, -NR⁴SO₂R⁶, -SO₂NR³R⁴, -NR⁴C(O)R³, -C(O)NR³R⁴,-NR⁵C(O)NR³R⁴, -NR³R⁴, and
C₁-C₁₀ alkyl, C₂-C₁₀ alkenyl, C₂-C₁₀ alkynyl, C₃-C₁₀ cycloalkyl, C₃-C₁₀ cycloalkylalkyl,-S(O)ⱼ(C₁-C₆ alkyl), -S(O)ⱼ(CR⁴R⁵)ₘ-aryl, aryl, arylalkyl, heteroaryl, heteroarylalkyl, heterocyclyl, heterocyclylalkyl, -O(CR⁴R⁵)ₘ- aryl, -NR⁴(CR⁴R⁵)ₘ-aryl, -O(CR⁴R⁵)ₘ-heteroaryl,-NR⁴(CR⁴R⁵)ₘ- heteroaryl, -O(CR⁴R⁵)ₘ-heterocyclyl and -NR⁴(CR⁴R⁵)ₘ-heterocyclyl, where each alkyl, alkenyl, alkynyl, cycloalkyl, aryl, heteroaryl and heterocyclyl portion is optionally substituted with one to five groups independently selected from oxo, halogen, cyano, nitro, trifluoromethyl, difluoromethoxy, trifluoromethoxy, azido, -NR⁴SO₂R⁶, -SO₂NR³R⁴, -C(O)R³,-C(O)OR³, -OC(O)R³, -NR⁴C(O)OR⁶, -NR⁴C(O)R³, -C(O)NR³R⁴, -NR³R⁴, -NR⁵C(O)NR³R⁴,-NR⁵C(NCN)NR³R⁴, -OR³, aryl, heteroaryl, arylalkyl, heteroarylalkyl, heterocyclyl, and heterocyclylalkyl;
m is 0, 1, 2, 3, 4 or 5;
and j is 1 or 2.

In one embodiment, R⁷ is C_{\}- C₁₀ alkyl, C₃-C₇ cycloalkyl or C₃-C₇ cycloalkylalkyl, each of which can be optionally substituted with 1 - 3 groups independently selected from oxo, halogen, cyano, nitro, trifluoromethyl, difluoromethoxy, trifluoromethoxy, azido, -NR⁴SO₂R⁶,-SO₂NR³R⁴, -C(O)R³, -C(O)OR³, -OC(O)R³, -SO₂R³, -NR⁴C(O)OR⁶, -NR⁴C(O)R³, -C(O)NR³R⁴, - NR³R⁴, -NR⁵C(O)NR³R⁴, -NR⁵C(NCN)NR³R⁴, -OR³, aryl, heteroaryl, arylalkyl, heteroarylalkyl, heterocyclyl, and heterocyclylalkyl.

In one embodiment, R¹ is halogen; R² is hydrogen; R³ is C₁- C₁₀ alkyl substituted with OR' and R' is hydrogen; R⁴ is hydrogen; R⁷ is C₁-C₁₀ alkyl; R⁸ is bromo; R⁹ is halogen; R¹⁰ is hydrogen; and W is -C(O)NR⁴OR³.

In one embodiment, MEK162 has the following structure:

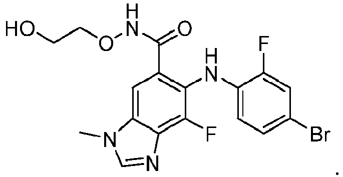

In one embodiment, MEK162 is 5-((4-bromo-2-fluorophenyl)amino)-4-fluoro-N-(2-hydroxyethoxy)-1-methyl-1H-benzo[d]imidazole-6-carboxamide or a pharmaceutically acceptable salt thereof.

In one embodiment, the the inhibitor of the immune checkpoint molecule (alone or in combination with other immunomodulators) is used in combination with a BCR-ABL inhibitor to treat a cancer, *e.g.,* a cancer described herein (*e.g.,* a cancer disclosed in Table 1). In one embodiment, the BCR-ABL inhibitor is disclosed in Table 1, *e.g*., AMN-107, or in a publication recited in Table 1, *e.g.,* in WO 2004/005281 (*e.g.,* in Example 92 or Formula (I) in claim 1) or US 7,169,791 (*e.g*., in claim 8). In one embodiment, AMN-107 has the structure (compound or generic structure) provided in Table 1, or as disclosed in the publication recited in Table 1, *e.g.,* in WO 2004/005281 (*e.g.,* in Example 92 or Formula (I) in claim 1) or US 7,169,791 (*e.g.,* in claim 8). In one embodiment, one of Nivolumab, Pembrolizumab or MSB0010718C is used in combination with AMN-107 to treat a cancer or disorder described in Table 1, *e.g.,* a solid tumor, *e.g.,* a neurologic cancer, a melanoma, a digestive/gastrointestinal cancer, a colorectal cancer, a head and neck cancer; or a hematological malignancy, *e.g*., chronic myelogenous leukemia (CML), a lymphocytic leukemia, a myeloid leukemia; Parkinson's disease; or pulmonary hypertension.

In one embodiment, the BCR-ABL inhibitor has the following structure: 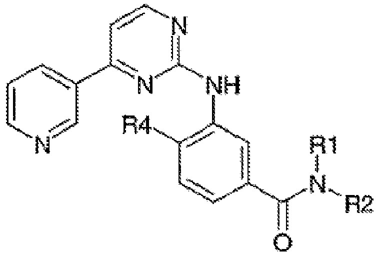 wherein
R₁ represents hydrogen, lower alkyl, lower alkoxy-lower alkyl, acyloxy-lower alkyl, carboxy- lower alkyl, lower alkoxycarbonyl-lower alkyl, or phenyl-lower alkyl;
R₂ represents hydrogen, lower alkyl, optionally substituted by one or more identical or different radicals R₃, cycloalkyl, benzcycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; and
R₃ represents hydroxy, lower alkoxy, acyloxy, carboxy, lower alkoxycarbonyl, carbamoyl, N- mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; or wherein
R₁ and R₂ together represent alkylene with four, five or six carbon atoms optionally mono- or disubstituted by lower alkyl, cycloalkyl, heterocyclyl, phenyl, hydroxy, lower alkoxy, amino, mono- or disubstituted amino, oxo, pyridyl, pyrazinyl or pyrimidinyl; benzalkylene with four or five carbon atoms; oxaalkylene with one oxygen and three or four carbon atoms; or azaalkylene with one nitrogen and three or four carbon atoms wherein nitrogen is unsubstituted or substituted by lower alkyl, phenyl-lower alkyl, lower alkoxycarbonyl-lower alkyl, carboxy-lower alkyl, carbamoyl-lower alkyl, N-mono- or N,N-disubstituted carbamoyl- lower alkyl, cycloalkyl, lower alkoxycarbonyl, carboxy, phenyl, substituted phenyl, pyridinyl, pyrimidinyl, or pyrazinyl;
represents hydrogen, lower alkyl, or halogen;
and a N-oxide or a pharmaceutically acceptable salt of such a compound.

In one embodiment, R₁ is hydrogen, R₂ is phenyl substituted with CF₃ and 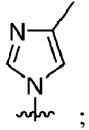 and R4 is CH₃.

In one embodiment, AMN-107 has the following structure:

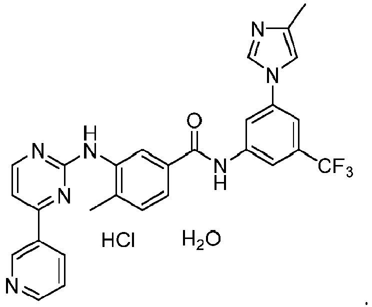

In one embodiment, AMN-107 is 4-methyl-3-[[4-(3-pyridinyl)-2-pyrimidinyl]amino]-N-[5-(4-methyl-1H-imidazol1yl)-3-(trifluoromethyl)phenyl]benzamide or an N-oxide or pharmaceutically acceptable salt thereof.

### LCL161 and Immunomodulators

LCL161, also known as SMAC mimetic LCL161 is an orally bioavailable second mitochondrial-derived activator of caspases (SMAC) mimetic and inhibitor of IAP (Inhibitor of Apoptosis Protein) family of proteins, with antineoplastic activity. SMAC mimetic LCL161 binds to IAPs, such as X chromosome-linked IAP (XIAP) and cellular IAPs 1 and 2. Since IAPs shield cancer cells from the apoptosis process, this agent can be used to restore and promote the induction of apoptosis through apoptotic signaling pathways in cancer cells. IAPs are overexpressed by many cancer cell types and suppress apoptosis by binding and inhibiting active caspases-3, -7 and -9, which play essential roles in apoptosis (programmed cell death), necrosis and inflammation.

In one embodiment, LCL161 has the structure provided in Table 1, or as disclosed in the publication recited in Table 1, *e.g*., International Patent Publication No. WO2008/016893 (*e.g.,* Formula (I), Example 1, and Compound A), European Patent No. 2051990, and U.S. Patent No. 8,546,336.

In one embodiment, LCL161 has the following structure:

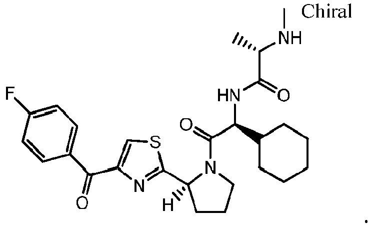

In one embodiment, LCL161 is (S)-N-((S)-1-cyclohexyl-2-((S)-2-(4-(4-fluorobenzoyl)thiazol-2-yl)pyrrolidin-l-yl)-2-oxoethyl)-2-(methylamino)propanamide.

In one embodiment, an immunomodulatory, *e.g*., an inhibitor of the immune checkpoint molecule (*e.g.,* a PD-1 inhibitor, *e.g.,* Nivolumab or Pembrolizumab, a PD-L1 inhibitor, *e.g.,* MSB0010718C, or a TIM-3 inhibitor, *e.g.,* an anti-TIM-3 antibody molecule) is used in combination with LCL161 to treat a cancer or disorder described in Table 1, *e.g.,* a solid tumor, *e.g.,* a breast cancer or a pancreatic cancer; or a hematological malignancy, *e.g.,* multiple myeloma or a hematopoeisis disorder.

In one embodiment, the inhibitor of the immune checkpoint molecule (*e.g*., an anti-PD-1 antibody molecule or an anti-TIM-3 antibody molecule) is administered intravenously. In one embodiment, in a combination therapy, LCL161 is administered orally. In one embodiment, the inhibitor of the immune checkpoint molecule (*e.g.,* the anti-PD-1 antibody molecule or anti-TIM-3 antibody molecule) is administered, *e.g*., intravenously, at least one, two, three, four, five, six, or seven days, *e.g.,* three days, after LCL161 is administered, *e.g.,* orally. In one embodiment, the inhibitor of the immune checkpoint molecule (*e.g.,* the anti-PD-1 antibody molecule or anti-TIM-3 antibody molecule) is administered, *e.g*., intravenously, at least one, two, three, four, five, six, or seven days, *e.g.,* three days, before LCL161 is administered, *e.g.,* orally. In yet another embodiment, the inhibitor of the immune checkpoint molecule (*e.g*., the anti-PD-1 antibody molecule or anti-TIM-3 antibody molecule) is administered, *e.g*., intravenously, on the same day, as LCL161 is administered, *e.g*., orally.

In one embodiment, the administration of the inhibitor of the immune checkpoint molecule (*e.g.,* the anti-PD-1 antibody molecule or anti-TIM-3 antibody molecule) and LCL161 results in a synergistic effect. In certain embodiments, in a combination therapy, the concentration LCL161 that is required to achieve inhibition, *e.g*., growth inhibition, is lower than the therapeutic dose of LCL161 as a monotherapy, *e.g*., 10-20%, 20-30%, 30-40%, 40-50%, 50-60%, 60-70%, 70-80%, or 80-90% lower. In other embodiments, in a combination therapy, the concentration of the inhibitor of the immune checkpoint molecule (*e.g.,* the anti-PD-1 antibody molecule or anti-TIM-3 antibody molecule) that is required to achieve inhibition, *e.g*., growth inhibition, is lower than the therapeutic dose of the inhibitor of the immune checkpoint molecule (*e.g.,* the anti-PD-1 antibody molecule or anti-TIM-3 antibody molecule) as a monotherapy, *e.g.,* 10-20%, 20-30%, 30-40%, 40-50%, 50-60%, 60-70%, 70-80%, or 80-90% lower. In one embodiment, administration of LCL161, alone or in combination with an anti-PD-1 antibody molecule, increases the expression of an immune-active cytokine, *e.g*., IFN-gamma, in the cancer or the subject. In another embodiment, administration of LCL161, alone or in combination with an anti-PD-1 antibody molecule, reduces the expression of an immune-suppressive cytokine, *e.g.,* IL-10, in the cancer or the subject.

In an embodiment, the LCL161 is administered at a dose (*e.g.,* oral dose) of about 10-3000 mg, *e.g.,* about 20-2400 mg, about 50-1800 mg, about 100-1500 mg, about 200-1200 mg, about 300-900 mg, *e.g.,* about 600 mg, about 900 mg, about 1200 mg, about 1500 mg, about 1800 mg, about 2100 mg, or about 2400 mg. In an embodiment, LCL161 is administered once a week or once every two weeks.

### LDK378 and Nivolumab

LDK378 (ceritinib) is an Anaplastic Lymphoma Kinase (ALK) inhibitor. Its chemical formula is 5-chloro-*N*²-(2-isopropoxy-5-methyl-4-(piperidin-4-yl)phenyl)-*N*⁴-[2-(propane-2-sulfonyl)-phenyl]-pyrimidine-2,4-diamine. A process for preparing LDK378 was disclosed in WO2008/073687. The compound has been approved by the US FDA as ZYKADIA® for the treatment of patients with Anaplastic Lymphoma Kinase (ALK)-positive metastatic non-small cell lung cancer (NSCLC), who have progressed on or are intolerant to crizotinib. The currently approved daily dose for use of LDK378 (alone) in NSCLC is 750 mg orally on an empty stomach (*i.e.,* is not to be administered within 2 hours of a meal).

In a clinical study, LDK378 demonstrated a high rate of rapid and durable responses in 246 ALK-positive NSCLC patients treated in the 750 mg dose group (RD). In these patients the overall response rate (ORR) was 58.5%. Among the 144 ALK-positive NSCLC patients with a confirmed complete response (CR) or partial response (PR), 86.1% of those patients achieved a response within 12 weeks, with a median time to response of 6.1 weeks. The estimated median duration of response (DOR) based on investigator assessment was long at 9.69 months. The median progression-free survival (PFS) was 8.21 months with 53.3% of the patients censored. Importantly, ceritinib showed this level of high anti-cancer activity regardless of prior ALK inhibitor status (*i.e.,* whether or not the patient received previous treatment with an ALK inhibitor). A high ORR of 54.6% and 66.3% was observed in patients treated with a prior ALK inhibitor and in ALK inhibitor-naive patients, respectively.

However, metastatic ALK-positive NSCLC remains a difficult disease to treat. Harnessing the immune system to treat patients with NSCLC represents a novel and new treatment approach, and nivolumab can be safely combined with LDK378. Combination therapy involving targeted agent LDK378 and immunotherapy (Nivolumab) can improve progression-free survival and ultimately overall survival in NSCLC patients.

In one aspect, the present disclosure relates to a pharmaceutical combination, especially a pharmaceutical combination product, comprising the combination of an immunomodulator and an agent disclosed herein.

In accordance with the present disclosure the compounds in the pharmaceutical combination, components (i) LDK378, or a pharmaceutically acceptable salt thereof, and (ii) Nivolumab, or a pharmaceutically acceptable salt thereof can be administered separately or together.

The pharmaceutical combination, according to the present disclosure, for use as a medicine, wherein LDK378 and the Nivolumab can be administered independently at the same time or separately within time intervals, wherein time intervals allow that the combination partners are jointly active.

The term "pharmaceutical combination" as used herein refers to a product obtained from mixing or combining in a non-fixed combination the active ingredients, *e.g*. (i) LDK378, or a pharmaceutically acceptable salt thereof, and (ii) Nivolumab or a pharmaceutically acceptable salt thereof separately or together.

The term "non-fixed combination" means that the active ingredients, *e.g*. LDK378 and Nivolumab, are both administered separately or together, independently at the same time or separately within time intervals, wherein such administration provides therapeutically effective levels of the active ingredient in the subject in need. The latter also applies to cocktail therapy, *e.g.* the administration of three or more active ingredients. This term defines especially a "kit of parts" in the sense that the combination partners (i) LDK378 and (ii) Nivolumab (and if present further one or more co-agents) as defined herein can be dosed independently of each other.

The term "jointly therapeutically effective" means that the compounds show synergistic interaction when administered separately or together, independently at the same time or separately within time intervals, to treat a subject in need, such as a warm-blooded animal in particular a human.

It was shown that the combination of the present disclosure possesses beneficial therapeutic properties, *e.g.* synergistic interaction, strong *in-vivo* and *in-vitro* antitumor response, which can be used as a medicine. Its characteristics render it particularly useful for the treatment of cancer.

Suitable cancers that can be treated with the combination of the present disclosure include but are not limited to anaplastic large cell lymphoma (ALCL), neuroblastoma, lung cancer, non-small cell lung cancer (NSCLC). In a preferred embodiment, the cancer is NSCLC.

The combination according to the present disclosure can besides or in addition be administered especially for cancer therapy in combination with chemotherapy, radiotherapy, immunotherapy, surgical intervention, or in combination of these. Long-term therapy is equally possible as is adjuvant therapy in the context of other treatment strategies, as described above. Other possible treatments are therapy to maintain the patient's status after tumor regression, or even chemo-preventive therapy, for example in patients at risk.

The combination of LDK378 and Nivolumab can be used to manufacture a medicament for an ALK mediated disease as described above. Likewise the combination can be used in a method for the treatment of an ALK, as described above, said method comprising administering an effective amount of a combination of (i) LDK378, or a pharmaceutically acceptable salt thereof, and (ii) Nivolumab or a pharmaceutically acceptable salt thereof separately or together, to a subject in need thereof, according to the present disclosure.

For example, the term "jointly (therapeutically) active" may mean that the compounds may be given separately or sequentially (in a chronically staggered manner, especially a sequence specific manner) in such time intervals that they preferably, in the warm-blooded animal, especially human, to be treated, and still show a (preferably synergistic) interaction (joint therapeutic effect). A joint therapeutic effect can, *inter alia,* be determined by following the blood levels, showing that both compounds are present in the blood of the human to be treated at least during certain time intervals, but this is not to exclude the case where the compounds are jointly active although they are not present in blood simultaneously.

The present disclosure also describes the method for the treatment of an ALK mediated disease, wherein the combination of (i) LDK378, or a pharmaceutically acceptable salt thereof, and (ii) Nivolumab or a pharmaceutically acceptable salt thereof separately or together.

The present disclosure relates to a pharmaceutical composition comprising effective amounts of (i) LDK378, or a pharmaceutically acceptable salt thereof, and (ii) Nivolumab, or a pharmaceutically acceptable salt thereof.

The present disclosure also describes the pharmaceutical combination according to the present disclosure in the form of a "kit of parts" for the combined administration. The combination can refer to either a fixed combination in one dosage unit form, or a kit of parts for the combined administration where (i) LDK378, or a pharmaceutically acceptable salt thereof, and (ii) Nivolumab, or a pharmaceutically acceptable salt thereof, may be administered independently at the same time or separately within time intervals, especially where these time intervals allow that the combination partners show a cooperative (= joint) effect. The independent formulations or the parts of the formulation, product, or composition, can then, *e.g.* be administered simultaneously or chronologically staggered, that is at different time points and with equal or different time intervals for any part of the kit of parts. In the combination therapies of the disclosure, the compounds useful according to the disclosure may be manufactured and/or formulated by the same or different manufacturers. Moreover, the combination partners may be brought together into a combination therapy: (i) prior to release of the combination product to physicians (*e.g*. in the case of a kit comprising LDK378 and the Nivolumab); (ii) by the physician themselves (or under the guidance of a physician) shortly before administration; (iii) in the patient themselves, *e.g*. during sequential administration of the compound of the disclosure and the other therapeutic agent. In one embodiment the effect of the combination is synergistic.

The therapeutically effective dosage of the combination of the disclosure, or pharmaceutical composition, is dependent on the species of the subject, the body weight, age and individual condition, the disorder or disease or the severity thereof being treated, and can be determined by standard clinical techniques. In addition, *in vitro* or *in vivo* assays can optionally be employed to help identify optimal dosage ranges. The precise dose to be employed can also depend on the route of administration, and the seriousness of the condition being treated and can be decided according to the judgment of the practitioner and each subject's circumstances in view of, *e.g.,* published clinical studies. In general, satisfactory results are indicated to be obtained systemically at daily dosages of from 150 mg to 750 mg of LDK378 orally. In most cases, the daily dose for LDK378 can be between 300 mg and 750 mg.

When administered in combination with Nivolumab, LDK378 can be administered at 450 mg with 3 mg/kg nivolumab, 600 mg LDK378 with 3 mg/kg Nivolumab, or 300 mg LDK378 with 3 mg/kg nivolumab. The most preferred dose of both compounds for combination therapy is 600 mg of LDK378 with 3 mg/kg Nivolumab. Particularly 600 mg LDK378 with 3 mg/kg Nivolumab is the most preferred dosing regimen for treating ALK-positive (*e.g*., EML4-ALK) NSCLC. Nivolumab can be administered as the fixed dose infusion every two weeks. Ceritinib is to be taken together with a low fat meal. It is acceptable if ceritinib is administered within 30 minutes after consuming a low fat meal. A patient should refrain from eating for at least an hour after intake of ceritinib and the low fat meal. It is expected that administration of ceritinib with daily meal intake can reduce the incidence and/or severity of gastrointestinal events. It is estimated that the steady state exposure of ceritinib at 450 mg and 600 mg with daily low-fat meal intake is within 20% relative to that of ceritinib at the recommended phase II dose of 750 mg administered fasted, as predicted by model-based clinical trial simulation, using a population pharmacokinetic model established for ALK-positive cancer patients in one clinical study in conjunction with absorption parameters estimated from another clinical study.

The "low-fat meal" denotes herein a meal that contains approximately 1.5 to 15 grams of fat and approximately 100 to 500 total calories.

Without being bound by theory, ceritinib does not have a mechanism of action that would be expected to antagonize an immune response. Furthermore, immune-related adverse events have not been frequently reported in ceritinib trials. Potential overlapping toxicities between ceritinib and Nivolumab include diarrhea, nausea, AST and ALT elevations, pneumonitis, and hyperglycemia. The mechanisms of these toxicities are not expected to be similar, given the mechanisms of action of the two compounds and thus the safety profile can be managed.

Another aspect of the disclosure is LDK378 for use as a medicine, wherein LDK378, or a pharmaceutically acceptable salt thereof, is to be administered in combination with Nivolumab, or a pharmaceutically acceptable salt thereof, for the treatment of an ALK mediated disease, *e.g*. cancer.

The term "ALK mediated disease" refers to a disease in which activity of the kinase leads to abnormal activity of the regulatory pathways including overexpression, mutation or relative lack of activity of other regulatory pathways in the cell that result in excessive cell proliferation, *e.g.* cancer. In one embodiment, the ALK mediated disease can be non-small cell lung cancer (NSCLC) that is driven by the echinoderm microtubule-associated protein-like 4 (EML4) - anaplastic lymphoma kinase (ALK) translocation. ALK is a receptor tyrosine kinase of the insulin receptor superfamily that plays a role in neural development and function. ALK is translocated, mutated, and/or amplified in several tumor types, and thus ALK mediated disease include, in addition to NSCLC, neuroblastoma, and anaplastic large cell lymphoma (ALCL). Alterations in ALK play a key role in the pathogenesis of these tumors. Other fusion partners of ALK besides EML4 that can be relevant in an ALK mediated disease are KIF5B, TFG, KLC1 and PTPN3, but are expected to be less common than EML4. Preclinical experiments have shown that the various ALK fusion partners mediate ligand-independent dimerization/oligomerization of ALK resulting in constitutive kinase activity and potent oncogenic activity both *in vitro* and *in vivo* and thus once translocated, ALK is driving, *i.e.,* mediating the disease.

Items that describe further preferred embodiments alone or in combination, are listed below:
1. A pharmaceutical combination comprising (i) LDK378, or a pharmaceutically acceptable salt thereof, and (ii) nivolumab, or a pharmaceutically acceptable salt thereof.
2. The pharmaceutical combination according to item 1 comprising components (i) and (ii) separately or together.
3. The pharmaceutical combination according to items 1 or 2 for use as a medicine, wherein LDK378 and the Nivolumab are administered independently at the same time or separately within time intervals.
4. The pharmaceutical combination according to item 3, wherein time intervals allow that the combination partners are jointly active.
5. The pharmaceutical combination according to any of items 1 to 4 comprising a quantity which is jointly therapeutically effective for the treatment of an ALK mediated disease.
6. The pharmaceutical combination according to item 5, wherein the ALK mediated disease is cancer.
7. The pharmaceutical combination according to item 6, wherein the ALK mediated disease is NSCLC or lymphoma,
8. The pharmaceutical combination according to item 6, wherein the ALK mediated disease is NSCLC.
9. The pharmaceutical combination according to any of the items 1 to 8, for use as a medicine.
10. The pharmaceutical combination according to any of the items 1 to 8, for use in the treatment of cancer.
11. The pharmaceutical combination according to item 10, wherein the cancer is a non-small cell lung cancer.
12. Use of LDK378 in combination with Nivolumab for the manufacture of a medicament for an ALK mediated disease.
13. The use of LDK378 in combination with Nivolumab for the manufacture of a medicament, according to item 12, wherein the disease is cancer.
14. The use of LDK378 in combination with Nivolumab for the manufacture of a medicament according to item 13, wherein the cancer is non-small cell lung cancer.
15. A pharmaceutical composition comprising LDK378 or a pharmaceutically acceptable salt thereof and Nivolumab or a pharmaceutically acceptable salt thereof for simultaneous or separate administration for the treatment of cancer.
16. The pharmaceutical composition according to item 15, wherein the cancer is a non-small cell lung cancer.
17. The pharmaceutical composition according to items 22 or 23, wherein the composition comprises effective amounts of LDK378 and nivolumab.
18. The pharmaceutical composition according to any one of items 15 to 18, wherein the composition further comprises a pharmaceutical acceptable carrier.
19. LDK378 for use as a medicine, wherein LDK378, or a pharmaceutically acceptable salt thereof, is to be administered in combination with Nivolumab, or a pharmaceutically acceptable salt thereof.
20. LDK378 for use as a medicine according to item 19, for the treatment of cancer.
21. LDK378 for use as a medicine according to item 20, wherein the cancer is a non-small cell lung cancer.
22. The pharmaceutical combination according to any one of items 1 to 11 in the form of a kit of parts for the combined administration.
23. The pharmaceutical combination according to item 22, wherein LDK378, or a pharmaceutically acceptable salt thereof, and the Nivolumab, or a pharmaceutically acceptable salt thereof, are administered jointly or independently at the same time or separately within time intervals.
24. A method for treating cancer in a subject in need thereof comprising administering to said subject a therapeutically effective amount of i) LDK378, or a pharmaceutically acceptable salt thereof, and (ii) nivolumab, or a pharmaceutically acceptable salt thereof.
25. The pharmaceutical combination according to any one of items 3 to 11, 22 or 23, use according to any one of items 12 to 14, a method for the treating cancer according to item 24, the pharmaceutical composition according to any one of items 15 to 18, or LDK378 for use as a medicine according to any one of items 19 to 21, wherein LDK378 and Nivolumab are administered to an ALK-naïve patient.
26. The pharmaceutical combination according to any one of items 3 to 11, 22 or 23, use according to any one of items 12 to 14, a method for the treating cancer according to item 24, the pharmaceutical composition according to any one of items 15 to 18, or LDK378 for use as a medicine according to any one of items 19 to 21, wherein LDK378 and Nivolumab are administered to a patient that has been pretreated with an ALK inhibitor.
27. The pharmaceutical combination according to any one of items 3 to 11, 22 or 23, use according to any one of items 12 to 14, a method for the treating cancer according to item 24, the pharmaceutical composition according to any one of items 15 to 18, or LDK378 for use as a medicine according to any one of items 19 to 21, wherein LDK378 and Nivolumab are administered to a patient that has been pretreated with LDK378.
28. The pharmaceutical combination according to any one of items 3 to 11, 22, 23 or 25 to 27, use according to any one of items 12 to 14 or 25 to 27, a method for the treating cancer according to any one of items 24 to 27, the pharmaceutical composition according to any one of items 15 to 18 or 25 to 27, or LDK378 for use as a medicine according to any one of items 19 to 21 or 25 to 27, wherein the cancer comprises ALK translocation or rearrangement.
29. The pharmaceutical combination according to any one of items 3 to 11, 22, 23 or 25 to 27, use according to any one of items 12 to 14 or 25 to 27, a method for the treating cancer according to any one of items 24 to 27, the pharmaceutical composition according to any one of items 15 to 18 or 25 to 27, or LDK378 for use as a medicine according to any one of items 19 to 21 or 25 to 27, wherein the cancer comprises EML4-ALK fusion.
30. The pharmaceutical combination according to any one of items 3 to 11, 22, 23 or 25 to 27, use according to any one of items 12 to 14 or 25 to 27, a method for the treating cancer according to any one of items 24 to 27, the pharmaceutical composition according to any one of items 15 to 18 or 25 to 27, or LDK378 for use as a medicine according to any one of items 19 to 21 or 25 to 27, wherein the cancer comprises ALK-ROS1 fusion.
31. The pharmaceutical combination according to any one of items 1 to 11, 22, 23 or 25 to 30, use according to any one of items 12 to 14 or 25 to 30, a method for the treating cancer according to any one of items 24 to 30, the pharmaceutical composition according to any one of items 15 to 18 or 25 to 30, or LDK378 for use as a medicine according to any one of items 19 to 21 or 25 to 30, wherein ceritinib dose is 450 mg and nivolumab dose is 3 mg/kg.
32. The pharmaceutical combination according to any one of items 1 to 11, 22, 23 or 25 to 30, use according to any one of items 12 to 14 or 25 to 30, a method for the treating cancer according to any one of items 24 to 30, the pharmaceutical composition according to any one of items 15 to 18 or 25 to 30, or LDK378 for use as a medicine according to any one of items 19 to 21 or 25 to 30, wherein ceritinib dose is 600 mg and nivolumab dose is 3 mg/kg.
33. The pharmaceutical combination according to any one of items 1 to 11, 22, 23 or 25 to 32, use according to any one of items 12 to 14 or 25 to 32, a method for the treating cancer according to any one of items 24 to 32, the pharmaceutical composition according to any one of items 15 to 18 or 25 to 32, or LDK378 for use as a medicine according to any one of items 19 to 21 or 25 to 32, wherein ceritinib is administered with a low fat meal.

### EGF816 and Nivolumab

Lung cancer is the most common cancer worldwide and the sub-type non-small cell lung cancer (NSCLC) accounts for approximately 85% of lung cancer cases. In Western nations, 10-15% NSCLC patients develop epidermal growth factor receptor (EGFR) mutations in their tumors and the mutation rate is even higher in Asian nations where rates have been reported to be as high as 40%. L858R and exon 19 deletion (Ex19del) activating EGFR oncogenic mutations predominate in NSCLC patients and account for 38% and 46% of EGFR NSCLC mutations respectively. EGFR Exon 20 insertion mutations (Ex20ins) are also relatively frequent, accounting for 9% of all EGFR mutations in NSCLC patients.

Patients with EGFR mutations are initially treated with reversible EGFR Tyrosine Kinase Inhibitors (TKIs), such as erlotinib and gefitinib, as a first line therapy. However, approximately half of these patients will develop acquired resistance to TKI inhibitors via a secondary "gatekeeper" T790M mutation within 10 to 14 months of treatment.

Second-generation EGFR TKIs (such as afatinib and dacomitinib) have been developed to try to overcome the mechanism of acquired resistance. These agents are irreversible inhibitors that covalently bind to cysteine 797 at the EGFR ATP binding site with potent activity on both activating (L858R, ex19del) and acquired (T790M) EGFR mutations in pre-clinical models. However, their clinical efficacy has proven to be limited, possibly in part due to severe adverse effects caused by concomitant inhibition of wild-type (WT) EGFR.

To overcome the previous issues with the earlier generations of inhibitors, third-generation EGFR TKIs have been developed which are WT EGFR sparing but also have relative equal potency for activating EGFR (L858R and ex19del) and acquired (T790M) mutations. Third generation EFGR TKIs, such as AZD9291 (mereletinib) and CO-1686 (rociletinib), are beginning to enter clinical development and are showing significant initial promise *(e.g.,* see "AZD9291 in EGFR Inhibitor-Resistant Non-Small-Cell Lung Cancer", Hanne et al., N Engl J Med, 2015; 372; 1689-99 and "Rociletinib in EGFR-Mutated Non-Small-Cell Lung Cancer", Sequist et al, J Med, 2015; 372; 1700-9). See also "ASP8273, a novel mutant-selective irreversible EGFR inhibitor, inhibits growth of non-small cell lung cancer (NSCLC) cells with EGFR activating and T790M resistance mutations", Sakagami et al., AACR; Cancer Res 2014; 74; 1728.

Treatment with EGFR inhibitors has however, not been shown to definitively translate into prolonged overall survival and it is unlikely that even third generation inhibitors alone will suffice. Hence there is still a need for additional treatment options for patients with cancer and, in particular, solid tumors. There is also a need for additional treatment options for patients with lung cancer, such as NSCLC. One such method of boosting effectiveness of EGFR inhibitors *in vivo* is by dually targeting other proteins implicated in disease progression of NSCLC patients

The PD-1 pathway was described as contributing to immune escape in mouse models of EGFR driven lung tumors (Akbay et al., Cancer Discov. 2013). However, a non-significant trend toward increased levels of PD-L1 in EGFR-mutant patient-derived NSCLC cell lines was also reported. Thus, it is still unclear whether targeting PD-1/PD-L1 interaction as well as mutated-EGFR in cancer patients, especially NSCLC patients, would be safe or clinically important.

The present invention relates to the surprising finding that a combination treatment comprising the selective mutated-EGFR inhibitor EGF816 and the anti-PD-1 antagonist Nivolumab are safe and tolerated when administered as a combination therapy to treat patients with NSCLC that have mutated-EGFR.

EGF816 is an EGFR inhibitor. EGF816 is also known as (R,E)-N-(7-chloro-1-(1-(4-(dimethylamino)but-2-enoyl)azepan-3-yl)-1Hbenzo[d]imidazol-2-yl)-2-methylisonicotinamide (EGF816), or a pharmaceutically acceptable salt thereof. A particularly useful salt is the mesylate salt thereof. WO2013/l84757, the contents of which are hereby incorporated by reference, describes EGF816, its method of preparation and pharmaceutical compositions comprising EGF816.

EGF816 has the following structure:

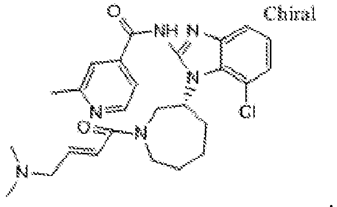

EGF816 is a targeted covalent irreversible EGFR inhibitor that selectively inhibits activating and acquired resistance mutants (L858R, ex19del and T790M), while sparing WT EGFR. *(see* Jia et al., Cancer Res October 1, 2014 74; 1734). EGF816 has shown significant efficacy in EGFR mutant (L858R, ex19del and T790M) cancer models (*in vitro* and *in vivo*) with no indication of WT EGFR inhibition at clinically relevant efficacious concentrations.

In one aspect, the disclosure relates to a pharmaceutical combination, comprising (a) a compound of formula I: 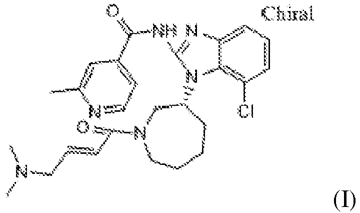 (R,E)-N-(7-chloro-1-(1-(4-(dimethylamino)but-2-enoyl)azepan-3-yl)-1H-benzo[d]imidazol-2-yl)-2-methylisonicotinamide (EGF816), or a pharmaceutically acceptable salt thereof, and (b) Nivolumab.

In one aspect, the disclosure provides a combination for use in a method of treating a cancer, especially an EGFR mutated cancer wherein:
(i) the combined administration has clinical efficacy, *e.g.,* as measured by determining time to disease progression;
(ii) the combined administration shows sustained clinical benefit; or
(iii) increases progression free survival.
or a combination of any of the above benefits.

The progression of cancer may be monitored by methods known to those in the art. For example, the progression may be monitored by way of visual inspection of the cancer, such as, by means of X-ray, CT scan or MRI or by tumor biomarker detection. For example, an increased growth of the cancer indicates progression of the cancer. Progression of cancer such as NSCLC or tumors may be indicated by detection of new tumors or detection of metastasis or cessation of tumor shrinkage. Tumor evaluations can be made based on RECIST criteria (Therasse et al. 2000), New Guidelines to Evaluate the Response to Treatment in Solid Tumors, Journal of National Cancer Institute, Vol. 92; 205-16 and revised RECIST guidelines (version 1.1) (Eisenhauer et al. 2009) European Journal of Cancer; 45:228-247.

Tumor progression may be determined by comparison of tumor status between time points after treatment has commenced or by comparison of tumor status between a time point after treatment has commenced to a time point prior to initiation of the relevant treatment..

In some embodiments, the lymphoma (*e.g*., an anaplastic large-cell lymphoma or non-Hodgkin lymphoma) has, or is identified as having, an ALK translocation, *e.g*., an EML4-ALK fusion.

In some embodiments, the combination is for use in the treatment of NSCLC.

In some embodiments, the combination is for use in the treatment of NSCLC, wherein the NSCLC is characterized by one or more of: aberrant activation, or amplification, or mutations of epidermal growth factor receptor.

In some embodiments, the combination is for use in the treatment of NSCLC, wherein the NSCLC is characterized by harboring an EGFR exon 20 insertion, an EGFR exon 19 deletion, EGFR L858R mutation, EGFR T790M, or any combination thereof.

In some embodiments, the combination is for use in the treatment of NSCLC, wherein the NSCLC is characterized by harboring L858R and T790M mutations of EGFR.

In some embodiments, the combination is for use in the treatment of NSCLC, wherein the NSCLC is characterized by harboring an EGFR exon 20 insertion and T790M mutations of EGFR.

In some embodiments, the combination is for use in the treatment of NSCLC, wherein the NSCLC is characterized by harboring an EGFR exon 19 deletion and T790M mutations of EGFR.

In some embodiments, the combination is for use in the treatment of NSCLC, wherein the NSCLC is characterized by harboring EGFR mutation selected from the group consisting of an exon 20 insertion, an exon 19 deletion, L858R mutation, T790M mutation, and any combination thereof.

In another embodiment, the cancer is an inflammatory myofibroblastic tumor (IMT). In certain embodiments, the inflammatory myofibroblastic tumor has, or is identified as having, an ALK rearrangement or translocation, *e.g.,* an ALK fusion, *e.g.,* an EML4-ALK fusion.

In yet another embodiment, the cancer is a neuroblastoma.

In certain embodiments, the neuroblastoma has, or is identified as having, an ALK rearrangement or translocation, *e.g.,* an ALK fusion, *e.g.,* an EML4-ALK fusion. Methods and compositions disclosed herein are useful for treating metastatic lesions associated with the aforementioned cancers.

EGF816 may be administered at a dose of 75, 100, 150, 225, 150, 200, 225, 300 or 350 mg. These doses may be administered once daily. *E.g.* EGF816 may be administered at a dose of 100 or 150 mg once daily.

Nivolumab may be administered in an amount from about 1 mg/kg to 5 mg/kg, *e.g.,* 3 mg/kg, and may be administered over a period of 60 minutes, ca. once a week to once every 2, 3 or 4 weeks.

In one embodiment, the combination of EGF816 and Nivolumab is administered as a combination therapy wherein the administration protocol is:
(i) 150 mg (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof orally administered daily; and
(ii) 3 mg/kg Nivolumab is administered intravenously over a period of 60 minutes at least one hour after administration of (i), every 2 weeks.

In some embodiments, the administration protocol is repeated for the duration of a 28 day cycle.

The term "pharmaceutical combination" as used herein means a product that results from the mixing or combining of more than one active ingredient and includes both fixed and non-fixed combinations of the active ingredients. The term "fixed combination" means that the active ingredients, *e.g*., a compound of formula (I) and one or more combination partners, are both administered to a patient simultaneously in the form of a single entity or dosage. The term "non-fixed combination" means that the active ingredients, *e.g*., a compound of the present invention and one or more combination partners, are both administered to a patient as separate entities either simultaneously, concurrently or sequentially with no specific time limits, wherein such administration provides therapeutically effective levels of the two compounds in the body of the patient. The latter also applies to cocktail therapy, *e.g.,* the administration of three or more active ingredients.

The present disclosure provides the following aspects, advantageous features and specific embodiments, respectively alone or in combination, as listed in the following Enumerated Embodiments.

### Enumerated Embodiments:

### 1. A pharmaceutical combination comprising:

(a) a compound of formula I 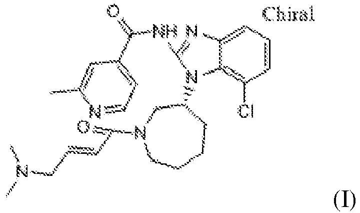 (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof,
and (b) Nivolumab.
   1. The pharmaceutical combination according to Enumerated Embodiment 1, wherein (R,E)-N-(7-chloro-1-(1-(4-(dimethylamino)but-2-enoyl)azepan-3-yl)-1H-benzo[d]imidazol-2-yl)-2-methylisonicotinamide is in mesylate form or hydrochloride salt form.
   2. A pharmaceutical composition comprising a combination according to Enumerated Embodiment 1 or Enumerated Embodiment 2 and at least one pharmaceutically acceptable carrier.
   3. A kit comprising the pharmaceutical combinations according to any one of Enumerated Embodiments 1 to 3 and information about using the constituents of the pharmaceutical combination simultaneously, separately or sequentially, and/or to instruct or administer the constituents of the pharmaceutical combinations according to any one of Enumerated Embodiments 1 to 3, simultaneously, separately or sequentially.
   4. A method of treating or preventing cancer in a subject in need thereof, comprising sequential, simultaneous or separate administration of (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof and Nivolumab according to any one of Enumerated Embodiments 1 to 3 in a jointly therapeutically effective amount to treat or prevent said cancer.
   5. The pharmaceutical combination according to any one of Enumerated Embodiments 1 to 3 in the form of a kit for combined administration comprising (a) one or more dosage units of (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof, and (b) one or more dosage units of Nivolumab.
   6. The pharmaceutical combination according to any one of Enumerated Embodiments 1 to 3 or kit according to Enumerated Embodiment 4 or Enumerated Embodiment 6, for use in the treatment of cancer, wherein (R,E)-N-(7-chloro-1-(1-(4-(dimethylamino)but-2-enoyl)azepan-3-yl)-1H-benzo[d]imidazol-2-yl)-2-methylisonicotinamide and Nivolumab are administered simultaneously or sequentially or separately.
   7. The pharmaceutical combination according any one of Enumerated Embodiments 1 to 3 or kit according to Enumerated Embodiment 4 or Enumerated Embodiment 6, for use according to Enumerated Embodiment 7, wherein the cancer is non-small cell lung cancer.
   8. The pharmaceutical combination according to any one of Enumerated Embodiments 1 to 3 or kit according to Enumerated Embodiment 4 or Enumerated Embodiment 6, for use according to any one of Enumerated Embodiments 7 or Enumerated Embodiment 8, wherein the non-small cell lung cancer is characterized by aberrant activation, or amplification, or mutations of epidermal growth factor receptor (EGFR).
   9. The pharmaceutical combination according to any one of Enumerated Embodiments 1 to 3 or kit according to Enumerated Embodiment 4 or Enumerated Embodiment 6, for use according to any one of Enumerated Embodiments 7 to 9, wherein the non-small cell lung cancer is characterized by harbouring an EGFR exon 20 insertion, an EGFR exon 19 deletion, EGFR L858R mutation, EGFR T790M, or any combination thereof.
   10. The pharmaceutical combination according to any one of Enumerated Embodiments 1 to 3 or kit according to Enumerated Embodiment 4 or Enumerated Embodiment 6, for use according to any one of Enumerated Embodiments 7 to 9, wherein the non-small cell lung cancer is characterized by harbouring L858R and T790M mutations of EGFR.
   11. The pharmaceutical combination according to any one of Enumerated Embodiments 1 to 3 or kit according to Enumerated Embodiment 4 or Enumerated Embodiment 6, for use according to any one of Enumerated Embodiments 7 to 9, wherein the non-small cell lung cancer is characterized by harbouring exon 20 insertion and T790M mutations of EGFR.
   12. The pharmaceutical combination according to any one of Enumerated Embodiments 1 to 3 or kit according to Enumerated Embodiment 4 or Enumerated Embodiment 6, for use according to any one of Enumerated Embodiments 7 to 9, wherein the non-small cell lung cancer is characterized by harbouring exon 19 deletion and T790M mutations of EGFR.
   13. The pharmaceutical combination according to any one of Enumerated Embodiments 1 to 3 or kit according to Enumerated Embodiment 4 or Enumerated Embodiment 6, for use according to any one of Enumerated Embodiments 7 to 9, wherein the non-small cell lung cancer is characterized by harbouring EGFR mutation selected from the group consisting of an exon 20 insertion, an exon 19 deletion, L858R mutation, T790M mutation, and any combination thereof.
   14. A pharmaceutical combination according to any one of Enumerated Embodiments 1 to 3 or kit according to Enumerated Embodiment 4 or Enumerated Embodiment 6 for use according to any one of Enumerated Embodiments 7 to 14, wherein the combination is administered within a specified period and wherein the combination is administered for a duration of time.
   15. A pharmaceutical combination according to any one of Enumerated Embodiments 1 to 3 or kit according to Enumerated Embodiment 4 or Enumerated Embodiment 6 for use according to any one of Enumerated Embodiments 7 to 14, wherein the combination is administered according to Enumerated Embodiment 15 and the amount of (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof is from about 50 to 500 mg, preferably of 75, 100, 150, 225, 150, 200, 225, 300 or 350 mg, more preferably 150 mg; every other day, daily, twice or three times a day.
   16. A pharmaceutical combination according to any one of Enumerated Embodiments 1 to 3 or kit according to Enumerated Embodiment 4 or Enumerated Embodiment 6 for use according to any one of Enumerated Embodiments 7 to 14, wherein the combination is administered according to Enumerated Embodiment 15 and the amount of (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof is from about 50 to about 225 mg, preferably from about 100 to about 150 mg, more preferably 150 mg, administered daily.
   17. A pharmaceutical combination according to any one of Enumerated Embodiments 1 to 3 or kit according to Enumerated Embodiment 4 or Enumerated Embodiment 6 for use according to any one of Enumerated Embodiments 7 to 14, wherein the combination is administered according to Enumerated Embodiment 15 and the amount of Nivolumab is an amount from about 1 mg/kg to about 5 mg/kg, preferably 3 mg/kg and is administered parenterally over a period of 60 minutes, ca. once a week to once every 2, 3 or 4 weeks.
   18. A pharmaceutical combination according to any one of Enumerated Embodiments 1 to 3 or kit according to Enumerated Embodiment 6 for use according to any one of Enumerated Embodiments 7 to 14, wherein the combination is administered according to Enumerated Embodiment 15 and (i) the amount of (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof is in an amount from about 50 to about 500 mg, preferably about 75, 100, 150, 225, 150, 200, 225, 300 or 350 mg, more preferably 150 mg, and is administered daily (ii) the amount of Nivolumab is an amount from about 1 mg/kg to about 5 mg/kg, preferably 3 mg/kg and is administered parenterally over a period of 60 minutes, no less than 12 days between each treatment and is administered every 2 weeks.
   19. A pharmaceutical combination according to any one of Enumerated Embodiments 15 to 20 wherein Nivolumab is administered at least one hour after administration of (R,E)-N-(7-chloro-1-(1-(4-(dimethylamino)but-2-enoyl)azepan-3-yl)-1H-benzo[d]imidazol-2-yl)-2-methylisonicotinamide.
   20. A pharmaceutical combination according to any one of Enumerated Embodiments 1 to 3 or kit according to Enumerated Embodiment 4 or Enumerated Embodiment 6 for use according to any one of Enumerated Embodiments 7 to 14, comprising (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof and Nivolumab, wherein the administration protocol comprises:
      (i) 150 mg (R,E)-N-(7-chloro-1-(1-(4-(dimethylamino)but-2-enoyl)azepan-3-yl)-1H-benzo[d]imidazol-2-yl)-2-methylisonicotinamide, or a pharmaceutically acceptable salt thereof orally administered daily; and
      (ii) 3 mg/kg Nivolumab is administered intravenously over a period of 60 minutes at least one hour after administration of (i), every 2 weeks.
   21. An administration protocol according to Enumerated Embodiment 21, wherein the protocol is repeated for the duration of one or more 28- day cycle.

### Pharmaceutical Compositions and Kits

In another aspect, the present invention provides compositions, *e.g*., pharmaceutically acceptable compositions, which include an antibody molecule described herein, formulated together with a pharmaceutically acceptable carrier. The term "pharmaceutically acceptable" refers to those compounds, materials, compositions, and/or dosage forms which are suitable for use in contact with the tissues of human beings and animals without excessive toxicity, irritation, allergic response, or other problem or complication, commensurate with a reasonable benefit/risk ratio.

As used herein, "pharmaceutically acceptable carrier" includes any and all solvents, dispersion media, isotonic and absorption delaying agents, and the like that are physiologically compatible. The carrier can be suitable for intravenous, intramuscular, subcutaneous, parenteral, rectal, spinal or epidermal administration (*e.g.* by injection or infusion).

Pharmaceutically acceptable salts can be formed, for example, as acid addition salts, preferably with organic or inorganic acids. Suitable inorganic acids are, for example, halogen acids, such as hydrochloric acid. Suitable organic acids are, *e.g.,* carboxylic acids or sulfonic acids, such as fumaric acid or methanesulfonic acid. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred. In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient. The salts of compounds described herein are preferably pharmaceutically acceptable salts; suitable counter-ions forming pharmaceutically acceptable salts are known in the field.

The compositions of this invention may be in a variety of forms. These include, for example, liquid, semi-solid and solid dosage forms, such as liquid solutions (*e.g*., injectable and infusible solutions), dispersions or suspensions, liposomes and suppositories. The preferred form depends on the intended mode of administration and therapeutic application. Typical preferred compositions are in the form of injectable or infusible solutions. The preferred mode of administration is parenteral (*e.g*., intravenous, subcutaneous, intraperitoneal, intramuscular). In a preferred embodiment, the antibody is administered by intravenous infusion or injection. In another preferred embodiment, the antibody is administered by intramuscular or subcutaneous injection.

The pharmaceutical composition can be prepared with a pharmaceutically acceptable carrier, which can be for example any suitable pharmaceutical excipient. The carrier includes any and all binders, fillers, solvents, dispersion media, coatings, surfactants, antioxidants, preservatives (*e.g*., antibacterial agents, antifungal agents), isotonic agents, absorption delaying agents, salts, drug stabilizers, disintegration agents, lubricants, sweetening agents, flavoring agents, dyes, and the like and combinations thereof, as would be known to those skilled in the art (*see,* for example, Remington's Pharmaceutical Sciences, 18th Ed. Mack Printing Company, 1990, pp. 1289- 1329; Remington: The Science and Practice of Pharmacy, 21st Ed. Pharmaceutical Press 2011; and subsequent versions thereof). Except insofar as any conventional carrier is incompatible with the active ingredient, its use in the therapeutic or pharmaceutical compositions is contemplated. Other disclosure herein relating to the pharmaceutical composition can also be followed.

In accordance with the present disclosure, the combination partners can be administered independently at the same time or separately within time intervals in separate unit dosage forms. The two therapeutic partners may be prepared in a manner known per se and are suitable for enteral, such as oral or rectal, topical and parenteral administration to subject in need thereof, including warm-blooded animal, in particular a human being. Suitable pharmaceutical compositions contain, *e.g*., from about 0.1% to about 99.9% of active ingredient.

The pharmaceutical composition can be processed to prepare a final dosage form - a tablet or a capsule. This can be achieved by compressing the final blend of the combination, optionally together with one or more excipients. The compression can be achieved for example with a rotary tablet press. Tablet of different shapes can be prepared (round, ovaloid, or other suitable shape). The tablet can be coated or uncoated by known techniques to delay disintegration and absorption in the gastrointestinal tract and thereby provide a sustained action over a longer period. If not indicated otherwise, these are prepared in a manner known per se, *e.g.* by means of mixing, granulating, sugar-coating processes. Formulation for oral use can be presented as hard gelatin capsules wherein the active ingredient is mixed with an inert solid diluent, for example, calcium carbonate, calcium phosphate or cellulose-based excipient, or as soft gelatin capsules wherein the active ingredient is mixed with water or an oil medium, for example, olive oil, liquid paraffin or peanut oil.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Therapeutic compositions typically should be sterile and stable under the conditions of manufacture and storage. The composition can be formulated as a solution, microemulsion, dispersion, liposome, or other ordered structure suitable to high antibody concentration. Sterile injectable solutions can be prepared by incorporating the active compound (*i.e.,* antibody or antibody portion) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The antibody molecules can be administered by a variety of methods known in the art, although for many therapeutic applications, the preferred route/mode of administration is intravenous injection or infusion. For example, the antibody molecules can be administered by intravenous infusion at a rate of less than 10 mg/min; preferably less than or equal to 5 mg/min to reach a dose of about 1 to 100 mg/m², preferably about 5 to 50 mg/m², about 7 to 25 mg/m² and more preferably, about 10 mg/m². As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J. R. Robinson, ed., Marcel Dekker, Inc., New York, 1978.

In certain embodiments, an antibody molecule can be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation. Therapeutic compositions can also be administered with medical devices known in the art.

Dosage regimens are adjusted to provide the optimum desired response (*e.g*., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

The term "effective amount" refers to the amount of the subject compound that can engender a biological or medical response in a cell, tissue, organ, system, animal or human that is being sought by the researcher, veterinarian, medical doctor or other clinician. The effective dosage of each combination partner agents employed in the combinations disclosed herein may vary depending on the particular compound or pharmaceutical composition employed, the mode of administration, the condition being treated, the severity the condition being treated. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the combination's drugs availability to target sites. This involves a consideration of the distribution, equilibrium and elimination of a drug.

A "therapeutically effective amount" refers to an amount effective, at dosages and for periods of time necessary, to achieve the desired therapeutic result. A therapeutically effective amount of the modified antibody or antibody fragment may vary according to factors such as the disease state, age, sex, and weight of the individual, and the ability of the antibody or antibody portion to elicit a desired response in the individual. A therapeutically effective amount is also one in which any toxic or detrimental effects of the modified antibody or antibody fragment is outweighed by the therapeutically beneficial effects. A "therapeutically effective dosage" preferably inhibits a measurable parameter, *e.g.,* tumor growth rate by at least about 20%, more preferably by at least about 40%, even more preferably by at least about 60%, and still more preferably by at least about 80% relative to untreated subjects. The ability of a compound to inhibit a measurable parameter, *e.g*., cancer, can be evaluated in an animal model system predictive of efficacy in human tumors. Alternatively, this property of a composition can be evaluated by examining the ability of the compound to inhibit, such inhibition *in vitro* by assays known to the skilled practitioner.

A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Methods of administering the antibody molecules are known in the art and are described below. Suitable dosages of the molecules used will depend on the age and weight of the subject and the particular drug used. Dosages and therapeutic regimens of the anti-PD-1 antibody molecule can be determined by a skilled artisan. In certain embodiments, the anti-PD-1 antibody molecule is administered by injection (*e.g*., subcutaneously or intravenously) at a dose of about 1 to 30 mg/kg, *e.g.,* about 5 to 25 mg/kg, about 10 to 20 mg/kg, about 1 to 5 mg/kg, or about 3 mg/kg. The dosing schedule can vary from *e.g.,* once a week to once every 2, 3, or 4 weeks. In one embodiment, the anti-PD-1 antibody molecule is administered at a dose from about 10 to 20 mg/kg every other week.

An exemplary, non-limiting range for a therapeutically or prophylactically effective amount of an antibody molecule is 0.1-30 mg/kg, more preferably 1-25 mg/kg. Dosages and therapeutic regimens of the anti-PD-1 antibody molecule can be determined by a skilled artisan. In certain embodiments, the anti-PD-1 antibody molecule is administered by injection (*e.g*., subcutaneously or intravenously) at a dose of about 1 to 30 mg/kg, *e.g.,* about 5 to 25 mg/kg, about 10 to 20 mg/kg, about 1 to 5 mg/kg, 1 to 10 mg/kg, 5 to 15 mg/kg, 10 to 20 mg/kg, 15 to 25 mg/kg, or about 3 mg/kg. The dosing schedule can vary from *e.g.,* once a week to once every 2, 3, or 4 weeks. In one embodiment, the anti-PD-1 antibody molecule is administered at a dose from about 10 to 20 mg/kg every other week. The antibody molecule can be administered by intravenous infusion at a rate of less than 10 mg/min, preferably less than or equal to 5 mg/min to reach a dose of about 1 to 100 mg/m², preferably about 5 to 50 mg/m², about 7 to 25 mg/m², and more preferably, about 10 mg/m². It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

The antibody molecules can be used by themselves or conjugated to a second agent, *e.g.,* a cytotoxic drug, radioisotope, or a protein, *e.g.,* a protein toxin or a viral protein. This method includes: administering the antibody molecule, alone or conjugated to a cytotoxic drug, to a subject requiring such treatment. The antibody molecules can be used to deliver a variety of therapeutic agents, *e.g.,* a cytotoxic moiety, *e.g.,* a therapeutic drug, a radioisotope, molecules of plant, fungal, or bacterial origin, or biological proteins (*e.g.,* protein toxins) or particles (*e.g.,* a recombinant viral particles, *e.g*.; via a viral coat protein), or mixtures thereof.

Also within the scope of the invention is a kit that includes a combination therapy described herein. The kit can include one or more other elements including: instructions for use; other reagents, *e.g.,* a label, a therapeutic agent, or an agent useful for chelating, or otherwise coupling, an antibody to a label or therapeutic agent, or a radioprotective composition; devices or other materials for preparing the antibody for administration; pharmaceutically acceptable carriers; and devices or other materials for administration to a subject.

### Uses of Combination Therapies

The combination therapies disclosed herein have *in vitro and in vivo* therapeutic and prophylactic utilities. For example, these molecules can be administered to cells in culture, *in vitro* or *ex vivo,* or to a subject, *e.g.,* a human subject, to treat, prevent, and/or diagnose a variety of disorders, such as cancers.

As used herein, the terms "treat", "treatment" and "treating" refer to the reduction or amelioration of the progression, severity and/or duration of a disorder, *e.g.,* a proliferative disorder (*e.g.,* a cancer), or the amelioration of one or more symptoms (preferably, one or more discernible symptoms) of a disorder, *e.g*., a proliferative disorder, resulting from the administration of one or more therapies (*e.g*., one or more therapeutic agents such as the combination therapies disclosed herein). In specific embodiments, the terms "treat", "treatment" and "treating" refer to the amelioration of at least one measurable physical parameter of a proliferative disorder (*e.g.,* a cancer), such as growth of a tumor, not necessarily discernible by the subject, *e.g*., a patient. In other embodiments the terms "treat", "treatment" and "treating" refer to the inhibition of the progression of a proliferative disorder, either physically by, *e.g.,* stabilization of a discernible symptom, physiologically by, *e.g*., stabilization of a physical parameter, or both. In other embodiments the terms "treat", "treatment" and "treating" refer to the reduction or stabilization of tumor size or cancerous cell count.

In some embodiments, ameliorating the disorder includes one or more of: slowing or arresting or reducing the development of the disease or at least one of the clinical symptoms thereof), to preventing or delaying the onset or development or progression of the disease or disorder. In addition those terms refers to alleviating or ameliorating at least one physical parameter including those which may not be discernible by the patient and also to modulating the disease or disorder, either physically (*e.g.* stabilization of a discernible symptom), physiologically (*e.g.* stabilization of a physical parameter), or both.

The term "treatment" comprises, for example, the therapeutic administration of one or more combination therapies disclosed herein to a subject, *e.g*., warm-blooded animal, in particular a human being, in need of such treatment. In embodiment, the treatment aims to cure the disease or to have an effect on disease regression or on the delay of progression of a disease.

As used herein, the term "subject" is intended to include human and non-human animals. In one embodiment, the subject is a human subject, *e.g.,* a human patient having a disorder or condition characterized by abnormal cell proliferation and/or immune functioning. The term "non-human animals" includes mammals and non-mammals, such as non-human primates. In one embodiment, the subject is a human. In one embodiment, the subject is a human patient in need of enhancement of an immune response. The term "subject in need" refers to a warm-blooded animal, in particular a human being that would benefit biologically, medically or in quality of life from the treatment. In one embodiment, the subject is immunocompromised, *e.g*., the subject is undergoing, or has undergone a chemotherapeutic or radiation therapy. Alternatively, or in combination, the subject is, or is at risk of being, immunocompromised as a result of an infection. The methods and compositions described herein are suitable for treating human patients having a disorder that can be treated by augmenting the T-cell mediated immune response. For example, the methods and compositions described herein can enhance a number of immune activities. In one embodiment, the subject has increased number or activity of tumour-infiltrating T lymphocytes (TILs). In another embodiment, the subject has increased expression or activity of interferon-gamma (IFN-γ). In yet another embodiment, the subject has decreased PD-L1 expression or activity.

Accordingly, in one aspect, the invention provides a method of modifying an immune response in a subject comprising administering to the subject the antibody molecule described herein, such that the immune response in the subject is modified. In one embodiment, the immune response is enhanced, stimulated or up-regulated. In one embodiment, the antibody molecules enhance an immune response in a subject by blockade of a checkpoint inhibitor (*e.g*., PD-1, PD-L1, LAG-3 or TIM-3).

### Cancer

Blockade of checkpoint inhibitors, *e.g*., PD-1, can enhance an immune response to cancerous cells in a subject. The ligand for PD-1, PD-L1, is not expressed in normal human cells, but is abundant in a variety of human cancers (Dong et al. (2002) Nat Med 8:787-9). The interaction between PD-1 and PD-L1 can result in a decrease in tumor infiltrating lymphocytes, a decrease in T-cell receptor mediated proliferation, and/or immune evasion by the cancerous cells (Dong et al. (2003) J Mol Med 81:281-7; Blank et al. (2005) Cancer Immunol. Immunother. 54:307-314; Konishi et al. (2004) Clin. Cancer Res. 10:5094-100).

In one aspect, the invention relates to treatment of a subject *in vivo* using an immunomodulatory, *e.g*., anti-PD-1 or anti-PD-Ll antibody molecule, alone or in combination with a second agent described herein, such that growth of cancerous tumors is inhibited or reduced. An immunomodulator may be used alone to inhibit the growth of cancerous tumors. Alternatively, an anti-PD-1 or anti-PD-Ll antibody may be used in combination with one or more of: an agent disclosed in Table 1, a standard of care treatment (*e.g*., for cancers), another antibody or antigen-binding fragment thereof, another immunomodulator (*e.g*., an activator of a costimulatory molecule or an inhibitor of an inhibitory molecule); a vaccine, *e.g.,* a therapeutic cancer vaccine; or other forms of cellular immunotherapy, as described below.

Accordingly, in one embodiment, the invention provides a method of inhibiting growth of tumor cells in a subject, comprising administering to the subject a therapeutically effective amount of a combination therapy disclosed herein. In one embodiment, the methods are suitable for the treatment of cancer *in vivo.* When antibodies to PD-1 are administered in combination with one or more agents, the combination can be administered in either order or simultaneously.

In another aspect, a method of treating a subject, *e.g*., reducing or ameliorating, a proliferative condition or disorder (*e.g.,* a cancer), *e.g.,* solid tumor, a soft tissue tumor, or a metastatic lesion, in a subject is provided. The method includes administering to the subject one or more immunomodulators, *e.g*., anti-PD-1 or PD-L1 antibody molecules described herein, alone or in combination with other agents or therapeutic modalities (*e.g*., one or more agents from Table 1).

As used herein, the term "cancer" is meant to include all types of cancerous growths or oncogenic processes, metastatic tissues or malignantly transformed cells, tissues, or organs, irrespective of histopathologic type or stage of invasiveness. Examples of cancerous disorders include, but are not limited to, solid tumors, soft tissue tumors, and metastatic lesions. Examples of solid tumors include malignancies, *e.g*., sarcomas, adenocarcinomas, and carcinomas, of the various organ systems, such as those affecting liver, lung, breast, lymphoid, gastrointestinal (*e.g*., colon), genitourinary tract (*e.g.,* renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, *e.g*., an advanced stage melanoma. Metastatic lesions of the aforementioned cancers can also be treated or prevented using the methods and compositions of the invention.

Exemplary cancers whose growth can be inhibited using the antibodies molecules disclosed herein include cancers typically responsive to immunotherapy. Non-limiting examples of preferred cancers for treatment include melanoma (*e.g.,* metastatic malignant melanoma), renal cancer (*e.g.,* clear cell carcinoma), prostate cancer (*e.g.,* hormone refractory prostate adenocarcinoma), breast cancer, colon cancer and lung cancer (*e.g*., non-small cell lung cancer). Additionally, refractory or recurrent malignancies can be treated using the antibody molecules described herein.

Examples of other cancers that can be treated include bone cancer, pancreatic cancer, skin cancer, cancer of the head or neck, cutaneous or intraocular malignant melanoma, uterine cancer, ovarian cancer, rectal cancer, anal cancer, gastro-esophageal, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers.

Treatment of metastatic cancers, *e.g.,* metastatic cancers that express PD-L1 (Iwai et al. (2005) Int. Immunol. 17:133-144) can be effected using the antibody molecules described herein. In one embodiment, the cancer expresses an elevated level of PD-L1, IFNγ and /or CD8.

Hematological cancer conditions are the types of cancer such as leukemia and malignant lymphoproliferative conditions that affect blood, bone marrow and the lymphatic system. Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

In other embodiments, the cancer is a hematological malignancy or cancer including but is not limited to a leukemia or a lymphoma. For example, the combination therapy can be used to treat cancers and malignancies including, but not limited to, *e.g.,* acute leukemias including but not limited to, *e.g.,* B-cell acute lymphoid leukemia ("BALL"), T-cell acute lymphoid leukemia ("TALL"), acute lymphoid leukemia (ALL); one or more chronic leukemias including but not limited to, *e.g.,* chronic myelogenous leukemia (CML), chronic lymphocytic leukemia (CLL); additional hematologic cancers or hematologic conditions including, but not limited to, *e.g.,* B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. In some embodiments, the lymphoma (*e.g*., an anaplastic large-cell lymphoma or non-Hodgkin lymphoma) has, or is identified as having, an ALK translocation, *e.g*., an EML4-ALK fusion.

In one embodiment, the cancer is chosen from a lung cancer (*e.g.,* a non-small cell lung cancer (NSCLC) (*e.g.,* a NSCLC with squamous and/or non-squamous histology)), a melanoma (*e.g.,* an advanced melanoma), a renal cancer (*e.g.,* a renal cell carcinoma, *e.g.,* clear cell renal cell carcinoma), a liver cancer, a myeloma (*e.g*., a multiple myeloma), a prostate cancer, a breast cancer (*e.g.,* a breast cancer that does not express one, two or all of estrogen receptor, progesterone receptor, or Her2/neu, *e.g*., a triple negative breast cancer), a colorectal cancer, a pancreatic cancer, a head and neck cancer (*e.g*., head and neck squamous cell carcinoma (HNSCC), anal cancer, gastro-esophageal cancer, thyroid cancer, cervical cancer, a lymphoproliferative disease (*e.g.,* a post-transplant lymphoproliferative disease) or a hematological cancer, T-cell lymphoma, a non-Hogdkin lymphoma, or a leukemia (*e.g*., a myeloid leukemia).

In another embodiment, the cancer is chosen form a carcinoma (*e.g*., advanced or metastatic carcinoma), melanoma or a lung carcinoma, *e.g.,* a non-small cell lung carcinoma.

In one embodiment, the cancer is a lung cancer, *e.g.,* a non-small cell lung cancer (NSCLC). In certain embodiments, the lung cancer, *e.g.,* the non-small cell lung cancer, has, or is identified as having, an ALK rearrangement or translocation, *e.g.,* an ALK fusion, *e.g.,* an EML4-ALK fusion.

In another embodiment, the cancer is an inflammatory myofibroblastic tumor (IMT). In certain embodiments, the inflammatory myofibroblastic tumor has, or is identified as having, an ALK rearrangement or translocation, *e.g.,* an ALK fusion, *e.g.,* an EML4-ALK fusion.

In other embodiments, the cancer is NSCLC wherein the NSCLC is characterized by one or more of: aberrant activation, amplification, or a mutation of epidermal growth factor receptor (EGFR). In certain embodiments the cancer is NSCLC wherein the NSCLC is characterized by harbouring an EGFR exon 20 insertion, an EGFR exon 19 deletion, EGFR L858R mutation, EGFR T790M, or any combination thereof. In some embodiments, the NSCLC is characterized by harboring L858R and T790M mutations of EGFR. In some embodiments, the NSCLC is characterized by harboring an EGFR exon 20 insertion and T790M mutations of EGFR. In some embodiments, the NSCLC is characterized by harboring an EGFR exon 19 deletion and T790M mutations of EGFR. In some embodiments, the NSCLC is characterized by harboring EGFR mutation selected from the group consisting of an exon 20 insertion, an exon 19 deletion, L858R mutation, T790M mutation, and any combination thereof.

In yet another embodiment, the cancer is a neuroblastoma.

In certain embodiments, the neuroblastoma has, or is identified as having, an ALK rearrangement or translocation, *e.g.,* an ALK fusion, *e.g.,* an EML4-ALK fusion. Methods and compositions disclosed herein are useful for treating metastatic lesions associated with the aforementioned cancers.

In another embodiment, the cancer is a hepatocarcinoma, *e.g*., an advanced hepatocarcinoma, with or without a viral infection, *e.g.,* a chronic viral hepatitis.

In another embodiment, the cancer is a prostate cancer, *e.g.,* an advanced prostate cancer.

In yet another embodiment, the cancer is a myeloma, *e.g*., multiple myeloma.

In yet another embodiment, the cancer is a renal cancer, *e.g.,* a renal cell carcinoma (RCC) (*e.g.,* a metastatic RCC or clear cell renal cell carcinoma).

In one embodiment, the cancer is a melanoma, *e.g*., an advanced melanoma. In one embodiment, the cancer is an advanced or unresectable melanoma that does not respond to other therapies. In other embodiments, the cancer is a melanoma with a BRAF mutation (*e.g.,* a BRAF V600 mutation). In yet other embodiments, the anti-PD-1 or PD-L1 antibody molecule is administered after treatment with an anti-CTLA-4 antibody (*e.g*., ipilimumab) with or without a BRAF inhibitor (*e.g*., vemurafenib or dabrafenib).

In another embodiment, the cancer is an inflammatory myofibroblastic tumor (IMT). In certain embodiments, the inflammatory myofibroblastic tumor has, or is identified as having, an ALK rearrangement or translocation, *e.g.,* an ALK fusion, *e.g.,* an EML4-ALK fusion.

Methods and compositions disclosed herein are useful for treating metastatic lesions associated with the aforementioned cancers.

### Additional Combination Therapies

The combination therapy disclosed herein can be further co-formulated with, and/or co-administered with, one or more additional therapeutic agents, *e.g.,* one or more anti-cancer agents, cytotoxic or cytostatic agents, hormone treatment, vaccines, and/or other immunotherapies. In other embodiments, the antibody molecules are administered in combination with other therapeutic treatment modalities, including surgery, radiation, cryosurgery, and/or thermotherapy. Such combination therapies may advantageously utilize lower dosages of the administered therapeutic agents, thus avoiding possible toxicities or complications associated with the various monotherapies.

For example, the combination therapies disclosed herein can also be combined with a standard cancer treatment. For example, PD-1 blockade may be effectively combined with chemotherapeutic regimes. In these instances, it may be possible to reduce the dose of chemotherapeutic reagent administered (Mokyr, M. et al. (1998) Cancer Research 58: 5301-5304). In certain embodiments, the methods and compositions described herein are administered in combination with one or more of other antibody molecules, chemotherapy, other anti-cancer therapy (*e.g.,* targeted anti-cancer therapies, or oncolytic drugs), cytotoxic agents, immune-based therapies (*e.g*., cytokines), surgical and/or radiation procedures. Exemplary cytotoxic agents that can be administered in combination with include antimicrotubule agents, topoisomerase inhibitors, anti-metabolites, mitotic inhibitors, alkylating agents, anthracyclines, vinca alkaloids, intercalating agents, agents capable of interfering with a signal transduction pathway, agents that promote apoptosis, proteosome inhibitors, and radiation (*e.g*., local or whole body irradiation).

Exemplary combinations of with the standard of care for cancer, include at least the following.

In certain embodiments, the combination therapy, is used in combination with a standard of cancer care chemotherapeutic agent including, but not limited to, anastrozole (Arimidex®), bicalutamide (Casodex®), bleomycin sulfate (Blenoxane®), busulfan (Myleran®), busulfan injection (Busulfex®), capecitabine (Xeloda®), N4-pentoxycarbonyl-5-deoxy-5-fluorocytidine, carboplatin (Paraplatin®), carmustine (BiCNU®), chlorambucil (Leukeran®), cisplatin (Platinol®), cladribine (Leustatin®), cyclophosphamide (Cytoxan® or Neosar®), cytarabine, cytosine arabinoside (Cytosar-U®), cytarabine liposome injection (DepoCyt®), dacarbazine (DTIC-Dome®), dactinomycin (Actinomycin D, Cosmegan), daunorubicin hydrochloride (Cerubidine®), daunorubicin citrate liposome injection (DaunoXome®), dexamethasone, docetaxel (Taxotere®), doxorubicin hydrochloride (Adriamycin®, Rubex®), etoposide (Vepesid®), fludarabine phosphate (Fludara®), 5-fluorouracil (Adrucil®, Efudex®), flutamide (Eulexin®), tezacitibine, Gemcitabine (difluorodeoxycitidine), hydroxyurea (Hydrea®), Idarubicin (Idamycin®), ifosfamide (IFEX®), irinotecan (Camptosar®), L-asparaginase (ELSPAR®), leucovorin calcium, melphalan (Alkeran®), 6-mercaptopurine (Purinethol®), methotrexate (Folex®), mitoxantrone (Novantrone®), mylotarg, paclitaxel (Taxol®), nab-paclitaxel (Abraxane®), phoenix (Yttrium90/MX-DTPA), pentostatin, polifeprosan 20 with carmustine implant (Gliadel®), tamoxifen citrate (Nolvadex®), teniposide (Vumon®), 6-thioguanine, thiotepa, tirapazamine (Tirazone®), topotecan hydrochloride for injection (Hycamptin®), vinblastine (Velban®), vincristine (Oncovin®), and vinorelbine (Navelbine®).

Exemplary alkylating agents include, without limitation, nitrogen mustards, ethylenimine derivatives, alkyl sulfonates, nitrosoureas and triazenes): uracil mustard (Aminouracil Mustard®, Chlorethaminacil®, Demethyldopan®, Desmethyldopan®, Haemanthamine®, Nordopan®, Uracil nitrogen mustard®, Uracillost®, Uracilmostaza®, Uramustin®, Uramustine®), chlormethine (Mustargen®), cyclophosphamide (Cytoxan®, Neosar®, Clafen®, Endoxan®, Procytox®, Revimmune™), ifosfamide (Mitoxana®), melphalan (Alkeran®), Chlorambucil (Leukeran®), pipobroman (Amedel®, Vercyte®), triethylenemelamine (Hemel®, Hexalen®, Hexastat®), triethylenethiophosphoramine, Temozolomide (Temodar®), thiotepa (Thioplex®), busulfan (Busilvex®, Myleran®), carmustine (BiCNU®), lomustine (CeeNU®), streptozocin (Zanosar®), and Dacarbazine (DTIC-Dome®). Additional exemplary alkylating agents include, without limitation, Oxaliplatin (Eloxatin®); Temozolomide (Temodar® and Temodal®); Dactinomycin (also known as actinomycin-D, Cosmegen®); Melphalan (also known as L-PAM, L-sarcolysin, and phenylalanine mustard, Alkeran®); Altretamine (also known as hexamethylmelamine (HMM), Hexalen®); Carmustine (BiCNU®); Bendamustine (Treanda®); Busulfan (Busulfex® and Myleran®); Carboplatin (Paraplatin®); Lomustine (also known as CCNU, CeeNU®); Cisplatin (also known as CDDP, Platinol® and Platinol®-AQ); Chlorambucil (Leukeran®); Cyclophosphamide (Cytoxan® and Neosar®); Dacarbazine (also known as DTIC, DIC and imidazole carboxamide, DTIC-Dome®); Altretamine (also known as hexamethylmelamine (HMM), Hexalen®); Ifosfamide (Ifex®); Prednumustine; Procarbazine (Matulane®); Mechlorethamine (also known as nitrogen mustard, mustine and mechloroethamine hydrochloride, Mustargen®); Streptozocin (Zanosar®); Thiotepa (also known as thiophosphoamide, TESPA and TSPA, Thioplex®); Cyclophosphamide (Endoxan®, Cytoxan®, Neosar®, Procytox®, Revimmune®); and Bendamustine HCl (Treanda®).

Exemplary anthracyclines include, *e.g*., doxorubicin (Adriamycin® and Rubex®); bleomycin (lenoxane®); daunorubicin (dauorubicin hydrochloride, daunomycin, and rubidomycin hydrochloride, Cerubidine®); daunorubicin liposomal (daunorubicin citrate liposome, DaunoXome®); mitoxantrone (DHAD, Novantrone®); epirubicin (Ellence™); idarubicin (Idamycin®, Idamycin PFS®); mitomycin C (Mutamycin®); geldanamycin; herbimycin; ravidomycin; and desacetylravidomycin.

Exemplary vinca alkaloids that can be used in combination with a combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), include, but are not limited to, vinorelbine tartrate (Navelbine®), Vincristine (Oncovin®), and Vindesine (Eldisine®)); vinblastine (also known as vinblastine sulfate, vincaleukoblastine and VLB, Alkaban-AQ® and Velban®); and vinorelbine (Navelbine®).

Exemplary proteosome inhibitors that can be used in combination with combination therapy disclosed herein (*e.g.,* an anti-PD-1 or PD-L1 antibody molecule, alone or in combination with another immunomodulator (*e.g.,* an anti-LAG-3, or anti-TIM-3 antibody molecule) and a compound of Table 1), include, but are not limited to, bortezomib (Velcade®); carfilzomib (PX-171-007, (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)-pentanamide); marizomib (NPI-0052); ixazomib citrate (MLN-9708); delanzomib (CEP-18770); O-Methyl-*N*-[(2-methyl-5-thiazolyl)carbonyl]-L-seryl-O-methyl-*N-*[(1*S*)-2-[(2*R*)-2-methyl-2-oxiranyl]-2-oxo-1-(phenylmethyl)ethyl]-L-serinamide (ONX-0912); danoprevir (RG7227, CAS 850876-88-9); ixazomib (MLN2238, CAS 1072833-77-2); and (S)-N-[(phenylmethoxy)carbonyl]-L-leucyl-N-(1-formyl-3-methylbutyl)-L-Leucinamide (MG-132, CAS 133407-82-6).

In some embodiments, the combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), in combination with a tyrosine kinase inhibitor (*e.g*., a receptor tyrosine kinase (RTK) inhibitor). Exemplary tyrosine kinase inhibitor include, but are not limited to, an epidermal growth factor (EGF) pathway inhibitor (*e.g*., an epidermal growth factor receptor (EGFR) inhibitor), a vascular endothelial growth factor (VEGF) pathway inhibitor (*e.g*., a vascular endothelial growth factor receptor (VEGFR) inhibitor (*e.g*., a VEGFR-1 inhibitor, a VEGFR-2 inhibitor, a VEGFR-3 inhibitor)), a platelet derived growth factor (PDGF) pathway inhibitor (*e.g*., a platelet derived growth factor receptor (PDGFR) inhibitor (*e.g.,* a PDGFR-β inhibitor)), a RAF-1 inhibitor, a KIT inhibitor and a RET inhibitor. In some embodiments, the anti-cancer agent used in combination with the hedgehog inhibitor is selected from the group consisting of: axitinib (AG013736), bosutinib (SKI-606), cediranib (RECENTIN™, AZD2171), dasatinib (SPRYCEL®, BMS-354825), erlotinib (TARCEVA®), gefitinib (IRESSA®), imatinib (Gleevec®, CGP57148B, STI-571), lapatinib (TYKERB®, TYVERB®), lestaurtinib (CEP-701), neratinib (HKI-272), nilotinib (TASIGNA®), semaxanib (semaxinib, SU5416), sunitinib (SUTENT®, SU11248), toceranib (PALLADIA®), vandetanib (ZACTIMA®, ZD6474), vatalanib (PTK787, PTK/ZK), trastuzumab (HERCEPTIN®), bevacizumab (AVASTIN®), rituximab (RITUXAN®), cetuximab (ERBITUX®), panitumumab (VECTIBIX®), ranibizumab (Lucentis®), nilotinib (TASIGNA®), sorafenib (NEXAVAR®), alemtuzumab (CAMPATH®), gemtuzumab ozogamicin (MYLOTARG®), ENMD-2076, PCI-32765, AC220, dovitinib lactate (TKI258, CHIR-258), BIBW 2992 (TOVOK™), SGX523, PF-04217903, PF-02341066, PF-299804, BMS-777607, ABT-869, MP470, BIBF 1120 (VARGATEF®), AP24534, JNJ-26483327, MGCD265, DCC-2036, BMS-690154, CEP-11981, tivozanib (AV-951), OSI-930, MM-121, XL-184, XL-647, XL228, AEE788, AG-490, AST-6, BMS-599626, CUDC-101, PD153035, pelitinib (EKB-569), vandetanib (zactima), WZ3146, WZ4002, WZ8040, ABT-869 (linifanib), AEE788, AP24534 (ponatinib), AV-951(tivozanib), axitinib, BAY 73-4506 (regorafenib), brivanib alaninate (BMS-582664), brivanib (BMS-540215), cediranib (AZD2171), CHIR-258 (dovitinib), CP 673451, CYC116, E7080, Ki8751, masitinib (AB1010), MGCD-265, motesanib diphosphate (AMG-706), MP-470, OSI-930, Pazopanib Hydrochloride, PD173074, Sorafenib Tosylate(Bay 43-9006), SU 5402, TSU-68(SU6668), vatalanib, XL880 (GSK1363089, EXEL-2880). Further examples of hedgehog inhibitors include, but are not limited to,vismodegib (2-chloro-N-[4-chloro-3-(2-pyridinyl)phenyl]-4-(methylsulfonyl)- benzamide, GDC-0449, described in PCT Publication No. WO 06/028958); 1-(4-Chloro-3-(trifluoromethyl)phenyl)-3-((3-(4-fluorophenyl)-3,4-dihydro-4-oxo-2-quinazolinyl)methyl)-urea (CAS 330796-24-2); N-[(2S,3R,3'R,3aS,4'aR,6S,6'aR,6'bS,7aR,12'aS,12'bS)-2',3',3a,4,4',4'a,5,5',6,6',6'a,6'b,7,7',7a, 8',10',12',12'a,12'b-Eicosahydro-3,6,11',12'b-tetramethylspiro[furo[3,2-b]pyridine-2(3H),9'(1'H)-naphth[2,1-a]azulen]-3'-yl]-methanesulfonamide (IPI926, CAS 1037210-93-7); and 4-Fluoro-N-methyl-N-[1-[4-(1-methyl-1H-pyrazol-5-yl)-1-phthalazinyl]-4-piperidinyl]-2-(trifluoromethyl)-benzamide (LY2940680, CAS 1258861-20-9); and Erismodegib (LDE225). Selected tyrosine kinase inhibitors are chosen from sunitinib, erlotinib, gefitinib;, or sorafenib erlotinib hydrochloride (Tarceva®); linifanib (N-[4-(3-amino-1H-indazol-4-yl)phenyl]-N'-(2-fluoro-5-methylphenyl)urea, also known as ABT 869, available from Genentech); sunitinib malate (Sutent®); bosutinib (4-[(2,4-dichloro-5-methoxyphenyl)amino]-6-methoxy-7-[3-(4-methylpiperazin-1-yl)propoxy]quinoline-3-carbonitrile, also known as SKI-606, described in US Patent No. 6,780,996); dasatinib (Sprycel®); pazopanib (Votrient®); sorafenib (Nexavar®); zactima (ZD6474); and imatinib or imatinib mesylate (Gilvec® and Gleevec®).

In certain embodiments, the combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), in combination with a Vascular Endothelial Growth Factor (VEGF) receptor inhibitors, including but not limited to, Bevacizumab (Avastin®), axitinib (Inlyta®); Brivanib alaninate (BMS-582664, (*S*)-((*R*)-1-(4-(4-Fluoro-2-methyl-1*H*-indol-5-yloxy)-5-methylpyrrolo[2,1-*f*][1,2,4]triazin-6-yloxy)propan-2-yl)2-aminopropanoate); Sorafenib (Nexavar®); Pazopanib (Votrient®); Sunitinib malate (Sutent®); Cediranib (AZD2171, CAS 288383-20-1); Vargatef (BIBF1120, CAS 928326-83-4); Foretinib (GSK1363089); Telatinib (BAY57-9352, CAS 332012-40-5); Apatinib (YN968D1, CAS 811803-05-1); Imatinib (Gleevec®); Ponatinib (AP24534, CAS 943319-70-8); Tivozanib (AV951, CAS 475108-18-0); Regorafenib (BAY73-4506, CAS 755037-03-7); Vatalanib dihydrochloride (PTK787, CAS 212141-51-0); Brivanib (BMS-540215, CAS 649735-46-6); Vandetanib (Caprelsa® or AZD6474); Motesanib diphosphate (AMG706, CAS 857876-30-3, N-(2,3-dihydro-3,3-dimethyl-1H-indol-6-yl)-2-[(4-pyridinylmethyl)amino]-3-pyridinecarboxamide, described in PCT Publication No. WO 02/066470); Dovitinib dilactic acid (TKI258, CAS 852433-84-2); Linfanib (ABT869, CAS 796967-16-3); Cabozantinib (XL184, CAS 849217-68-1); Lestaurtinib (CAS 111358-88-4); N-[5-[[[5-(1,1-Dimethylethyl)-2-oxazolyl]methyl]thio]-2-thiazolyl]-4-piperidinecarboxamide (BMS38703, CAS 345627-80-7); (3R,4R)-4-Amino-1-((4-((3-methoxyphenyl)amino)pyrrolo[2,1-f][1,2,4]triazin-5-yl)methyl)piperidin-3-ol (BMS690514); *N*-(3,4-Dichloro-2-fluorophenyl)-6-methoxy-7-[[(3aα,5β,6aα)-octahydro-2-methylcyclopenta[*c*]pyrrol-5-yl]methoxy]-4-quinazolinamine (XL647, CAS 781613-23-8); 4-Methyl-3-[[1-methyl-6-(3-pyridinyl)-1*H*-pyrazolo[3,4-*d*]pyrimidin-4-yl]amino]-*N*-[3-(trifluoromethyl)phenyl]-benzamide (BHG712, CAS 940310-85-0); and Aflibercept (Eylea®).

Exemplary anti-VEGF antibodies include, but are not limited to, a monoclonal antibody that binds to the same epitope as the monoclonal anti-VEGF antibody A4.6.1 produced by hybridoma ATCC HB 10709; a recombinant humanized anti-VEGF monoclonal antibody generated according to Presta et al. (1997) Cancer Res. 57:4593-4599. In one embodiment, the anti-VEGF antibody is Bevacizumab (BV), also known as rhuMAb VEGF or AVASTIN®. It comprises mutated human IgGl framework regions and antigen-binding complementarity-determining regions from the murine anti-hVEGF monoclonal antibody A.4.6.1 that blocks binding of human VEGF to its receptors. Bevacizumab and other humanized anti-VEGF antibodies are further described in U.S. Pat. No. 6,884,879 issued Feb. 26, 2005. Additional antibodies include the G6 or B20 series antibodies (*e.g*., G6-31, B20-4.1), as described in PCT Publication No. WO2005/012359, PCT Publication No. WO2005/044853, the contents of these patent applications are expressly incorporated herein by reference. For additional antibodies see U.S. Pat. Nos. 7,060,269, 6,582,959, 6,703,020, 6,054,297, WO98/45332, WO 96/30046, WO94/10202, EP 0666868B1, U.S. Patent Application Publication Nos. 2006009360, 20050186208, 20030206899, 20030190317, 20030203409, and 20050112126; and Popkov et al, Journal of Immunological Methods 288: 149-164 (2004). Other antibodies include those that bind to a functional epitope on human VEGF comprising of residues F17, Ml 8, D19, Y21, Y25, Q89, 191 , Kl 01, El 03, and C104 or, alternatively, comprising residues F17, Y21, Q22, Y25, D63, 183 and Q89.

In some embodiments, the combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), in combination with a PI3K inhibitor. In one embodiment, the PI3K inhibitor is an inhibitor of delta and gamma isoforms of PI3K. Exemplary PI3K inhibitors that can be used in combination are described in, *e.g.,* WO 2010/036380, WO 2010/006086, WO 09/114870, WO 05/113556, GSK 2126458, GDC-0980, GDC-0941, Sanofi XL147, XL756, XL147, PF-46915032, BKM 120, CAL-101, CAL 263, SF1126, PX-886, and a dual PI3K inhibitor (*e.g*., Novartis BEZ235). Further examples of PI3K inhibitors include, but are not limited to, 4-[2-(1H-Indazol-4-yl)-6-[[4-(methylsulfonyl)piperazin-1-yl]methyl]thieno[3,2-d]pyrimidin-4-yl]morpholine (also known as GDC 0941, described in PCT Publication Nos. WO 09/036082 and WO 09/055730); 2-Methyl-2-[4-[3-methyl-2-oxo-8-(quinolin-3-yl)-2,3-dihydroimidazo[4,5-c]quinolin-1-yl]phenyl]propionitrile (also known as BEZ235 or NVP-BEZ 235, described in PCT Publication No. WO 06/122806); 4-(trifluoromethyl)-5-(2,6-dimorpholinopyrimidin-4-yl)pyridin-2-amine (also known as BKM120 or NVP-BKM120, described in PCT Publication No. WO2007/084786); Tozasertib (VX680 or MK-0457, CAS 639089-54-6); (5Z)-5-[[4-(4-Pyridinyl)-6-quinolinyl]methylene]-2,4-thiazolidinedione (GSK1059615, CAS 958852-01-2); (1E,4S,4aR,5R,6aS,9aR)-5-(Acetyloxy)-1-[(di-2-propenylamino)methylene]-4,4a,5,6,6a,8,9,9a-octahydro-11-hydroxy-4-(methoxymethyl)-4a,6a-dimethyl-cyclopenta[5,6]naphtho[1,2-c]pyran-2,7,10(1H)-trione (PX866, CAS 502632-66-8); 8-Phenyl-2-(morpholin-4-yl)-chromen-4-one (LY294002, CAS 154447-36-6); 2-Amino-8-ethyl-4-methyl-6-(1H-pyrazol-5-yl)pyrido[2,3-d]pyrimidin-7(8H)-one (SAR 245409 or XL 765); 1,3-Dihydro-8-(6-methoxy-3-pyridinyl)-3-methyl-1-[4-(1-piperazinyl)-3-(trifluoromethyl)phenyl]-2H-imidazo[4,5-c]quinolin-2-one,, (2Z)-2-butenedioate (1:1) (BGT 226); 5-Fluoro-3-phenyl-2-[(1S)-1-(9H-purin-6-ylamino)ethyl]-4(3H)-quinazolinone (CAL101); 2-Amino-N-[3-[N-[3-[(2-chloro-5-methoxyphenyl)amino]quinoxalin-2-yl]sulfamoyl]phenyl]-2-methylpropanamide (SAR 245408 or XL 147); and (S)-Pyrrolidine-1,2-dicarboxylic acid 2-amide 1-({4-methyl-5-[2-(2,2,2-tnfluoro-1,1-dimethyl-ethyl)-pyridin-4-yl]-thiazol-2-yl}-amide) (BYL719).

In some embodiments, the combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), in combination with a mTOR inhibitor, *e.g*., one or more mTOR inhibitors chosen from one or more of rapamycin, temsirolimus (TORISEL®), AZD8055, BEZ235, BGT226, XL765, PF-4691502, GDC0980, SF1126, OSI-027, GSK1059615, KU-0063794, WYE-354, Palomid 529 (P529), PF-04691502, or PKI-587. ridaforolimus (formally known as deferolimus, (1*R*,2*R*,4*S*)-4-[(2*R*)-2 [(1*R*,9*S*,12*S*,15*R*,16*E*,18*R*,19*R*,21*R*,23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); everolimus (Afinitor® or RAD001); rapamycin (AY22989, Sirolimus®); simapimod (CAS 164301-51-3); emsirolimus, (5-{2,4-Bis[(3*S*)-3-methylmorpholin-4-yl]pyrido[2,3-*d*]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502, CAS 1013101-36-4); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126, CAS 936487-67-1), (1r,4r)-4-(4-amino-5-(7-methoxy-1H-indol-2-yl)imidazo[1,5-f][1,2,4]triazin-7-yl)cyclohexanecarboxylic acid (OSI-027); and XL765.

In some embodiments, the combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), in combination with a BRAF inhibitor, *e.g*., GSK2118436, RG7204, PLX4032, GDC-0879, PLX4720, and sorafenib tosylate (Bay 43-9006). In further embodiments, a BRAF inhibitor includes, but is not limited to, regorafenib (BAY73-4506, CAS 755037-03-7); tuvizanib (AV951, CAS 475108-18-0); vemurafenib (Zelboraf®, PLX-4032, CAS 918504-65-1); encorafenib (also known as LGX818); 1-Methyl-5-[[2-[5-(trifluoromethyl)-1H-imidazol-2-yl]-4-pyridinyl]oxy]-N-[4-(trifluoromethyl)phenyl-1H-benzimidazol-2-amine (RAF265, CAS 927880-90-8); 5-[1-(2-Hydroxyethyl)-3-(pyridin-4-yl)-1H-pyrazol-4-yl]-2,3-dihydroinden-1-one oxime (GDC-0879, CAS 905281-76-7); 5-[2-[4-[2-(Dimethylamino)ethoxy]phenyl]-5-(4-pyridinyl)-1H-imidazol-4-yl]-2,3-dihydro-1H-Inden-1-one oxime (GSK2118436 or SB590885); (+/-)-Methyl (5-(2-(5-chloro-2-methylphenyl)-1-hydroxy-3-oxo-2,3-dihydro-1H-isoindol-1-yl)-1H-benzimidazol-2-yl)carbamate (also known as XL-281 and BMS908662) and N-(3-(5-chloro-1H-pyrrolo[2,3-b]pyridine-3-carbonyl)-2,4-difluorophenyl)propane-1-sulfonamide (also known as PLX4720).

In some embodiments, the combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), in combination with a MEK inhibitor. In some embodiments, the combination of the anti-PD-1 antibody and the MEK inhibitor is used to treat a cancer (*e.g.,* a cancer described herein). In some embodiments, the cancer treated with the combination is chosen from a melanoma, a colorectal cancer, a non-small cell lung cancer, an ovarian cancer, a breast cancer, a prostate cancer, a pancreatic cancer, a hematological malignancy or a renal cell carcinoma. In certain embodiments, the cancer includes a BRAF mutation (*e.g*., a BRAF V600E mutation), a BRAF wildtype, a KRAS wildtype or an activating KRAS mutation. The cancer may be at an early, intermediate or late stage. Any MEK inhibitor can be used in combination including, but not limited to, selumetinib (5-[(4-bromo-2-chlorophenyl)amino]-4-fluoro-N-(2-hydroxyethoxy)-1-methyl-1H-benzimidazole-6-carboxamide, also known as AZD6244 or ARRY 142886, described in PCT Publication No. WO2003077914);ARRY-142886 trametinib dimethyl sulfoxide (GSK-1120212, CAS 1204531-25-80);, G02442104 (also known as GSK1120212), RDEA436, ; N-[3,4-Difluoro-2-[(2-fluoro-4-iodophenyl)amino]-6-methoxyphenyl]-1-[(2R)-2,3-dihydroxypropyl]-cyclopropanesulfonamide (also known as RDEA119 or BAY869766, described in PCT Publication No. WO2007014011); RDEA119/BAY 869766, AS703026, ; G00039805 (also known as AZD-6244 or selumetinib), BIX 02188, ; BIX 02189, ; 2-[(2-Chloro-4-iodophenyl)amino]-N-(cyclopropylmethoxy)-3,4-difluoro-benzamide (also known as CI-1040 or PD184352, described in PCT Publication No. WO2000035436);CI-1040 (PD-184352), N-[(2R)-2,3-Dihydroxypropoxy]-3,4-difluoro-2-[(2-fluoro-4-iodophenyl)amino]- benzamide (also known as PD0325901 and described in PCT Publication No. WO2002006213); PD03259012'-amino-3'-methoxyflavone (also known as PD98059 available from Biaffin GmbH & Co., KG, Germany);, PD98059, 2,3-bis[amino[(2-aminophenyl)thio]methylene]-butanedinitrile (also known as U0126 and described in US Patent No. 2,779,780);U0126, XL-518 (also known as GDC-0973, Cas No. 1029872-29-4, available from ACC Corp.);GDC-0973 (Methanone, [3,4-difluoro-2-[(2-fluoro-4-iodophenyl)amino]phenyl][3-hydroxy-3-(25)-2-piperidinyl-1-azetidinyl]-), G-38963, ; and G02443714 (also known as AS703206), or a pharmaceutically acceptable salt or solvate thereof..Additional examples of MEK inhibitors are disclosed in WO 2013/019906, WO 03/077914, WO 2005/121142, WO 2007/04415, WO 2008/024725 and WO 2009/085983, the contents of which are incorporated herein by reference. Further examples of MEK inhibitors include, but are not limited to, benimetinib (6-(4-bromo-2-fluorophenylamino)-7-fluoro-3-methyl-3H-benzoimidazole-5-carboxylic acid (2-hydroxyethyoxy)-amide, also known as MEK162, CAS 1073666-70-2, described in PCT Publication No. WO2003077914); 2,3-Bis[amino[(2-aminophenyl)thio]methylene]-butanedinitrile (also known as U0126 and described in US Patent No. 2,779,780); (3S,4R,5Z,8S,9S,11E)-14-(Ethylamino)-8,9,16-trihydroxy-3,4-dimethyl-3,4,9, 19-tetrahydro-1H-2-benzoxacyclotetradecine-1,7(8H)-dione] (also known as E6201, described in PCT Publication No. WO2003076424); vemurafenib (PLX-4032, CAS 918504-65-1); (R)-3-(2,3-Dihydroxypropyl)-6-fluoro-5-(2-fluoro-4-iodophenylamino)-8-methylpyrido[2,3-d]pyrimidine-4,7(3H,8H)-dione (TAK-733, CAS 1035555-63-5); pimasertib (AS-703026, CAS 1204531-26-9); 2-(2-Fluoro-4-iodophenylamino)-N-(2-hydroxyethoxy)-1,5-dimethyl-6-oxo-1,6-dihydropyridine-3-carboxamide (AZD 8330); and 3,4-Difluoro-2-[(2-fluoro-4-iodophenyl)amino]-N-(2-hydroxyethoxy)-5-[(3-oxo-[1,2]oxazinan-2-yl)methyl]benzamide (CH 4987655 or Ro 4987655).

In some embodiments, the combination therapy disclosed herein (*e.g.,* an anti-PD-1 or PD-L1 antibody molecule, alone or in combination with another immunomodulator (*e.g*., an anti-LAG-3, or anti-TIM-3 antibody molecule) and a compound of Table 1), in combination with a JAK2 inhibitor, *e.g*., CEP-701, INCB18424, CP-690550 (tasocitinib). Exemplary JAK inhibitors include, but are not limited to, ruxolitinib (Jakafi®); tofacitinib (CP690550); axitinib (AG013736, CAS 319460-85-0); 5-Chloro-N2-[(1S)-1-(5-fluoro-2-pyrimidinyl)ethyl]-N4-(5-methyl-1H-pyrazol-3-y)-l2,4-pyrimidinediamine (AZD1480, CAS 935666-88-9); (9E)-15-[2-(1-Pyrrolidinyl)ethoxy]- 7,12,26-trioxa-19,21,24-triazatetracyclo[18.3.1.12,5.114,18]-hexacosa-1(24),2,4,9,14,16,18(25),20,22-nonaene (SB-1578, CAS 937273-04-6); momelotinib (CYT 387); baricitinib (INCB-028050 or LY-3009104); pacritinib (SB1518); (16E)-14-Methyl-20-oxa-5,7,14,27-tetraazatetracyclo[19.3.1.12,6.18,12]heptacosa-1(25),2,4,6(27),8,10,12(26),16,21,23-decaene (SB 1317); gandotinib (LY 2784544); and N,N-cicyclopropyl-4-[(1,5-dimethyl-1H-pyrazol-3-yl)amino]-6-ethyl-1,6-dihydro-1-methyl- imidazo[4,5-d]pyrrolo[2,3-b]pyridine-7-carboxamide (BMS 911543).

In some embodiments, the combination therapies disclosed herein include paclitaxel or a paclitaxel agent, *e.g.,* TAXOL®, protein-bound paclitaxel (*e.g.,* ABRAXANE®). Exemplary paclitaxel agents include, but are not limited to, nanoparticle albumin-bound paclitaxel (ABRAXANE, marketed by Abraxis Bioscience), docosahexaenoic acid bound-paclitaxel (DHA-paclitaxel, Taxoprexin, marketed by Protarga), polyglutamate bound-paclitaxel (PG-paclitaxel, paclitaxel poliglumex, CT-2103, XYOTAX, marketed by Cell Therapeutic), the tumor-activated prodrug (TAP), ANG105 (Angiopep-2 bound to three molecules of paclitaxel, marketed by ImmunoGen), paclitaxel-EC-1 (paclitaxel bound to the erbB2-recognizing peptide EC-1; *see* Li et al., Biopolymers (2007) 87:225-230), and glucose-conjugated paclitaxel (*e.g.,* 2'-paclitaxel methyl 2-glucopyranosyl succinate, *see* Liu et al., Bioorganic & Medicinal Chemistry Letters (2007) 17:617-620).

In certain embodiments, the anti-PD-1 or PD-L1 antibody molecule, alone or in combination with another immunomodulator (*e.g*., an anti-LAG-3 or anti-TIM-3 antibody molecule), is administered in combination with an antibody against a Killer-cell Immunoglobulin-like Receptors (also referred to herein as an "anti-KIR antibody"). In certain embodiments, the combination of anti-PD-1 antibody molecule and anti-KIR antibody described herein is used to treat a cancer, *e.g.,* a cancer as described herein (*e.g.,* a solid tumor, *e.g.,* an advanced solid tumor).

In one embodiment, the anti-PD-1 or PD-L1 antibody molecule, alone or in combination with another immunomodulator (*e.g*., an anti-LAG-3 or anti-TIM-3 antibody molecule), is administered in combination with a cellular immunotherapy (*e.g.,* Provenge (*e.g.,* Sipuleucel)), and optionally in combination with cyclophosphamide. In certain embodiments, the combination of anti-PD-1 antibody molecule, Provenge and/or cyclophosphamide is used to treat a cancer, *e.g.,* a cancer as described herein (*e.g.,* a prostate cancer, *e.g.,* an advanced prostate cancer).

In another embodiment, the anti-PD-1 or PD-L1 antibody molecule, alone or in combination with another immunomodulator (*e.g*., an anti-LAG-3 or anti-TIM-3 antibody molecule), is administered in combination with a vaccine, *e.g.,* a dendritic cell renal carcinoma (DC-RCC) vaccine. In certain embodiments, the combination of anti-PD-1 antibody molecule and the DC-RCC vaccine is used to treat a cancer, *e.g.,* a cancer as described herein (*e.g.,* a renal carcinoma, *e.g*., metastatic renal cell carcinoma (RCC) or clear cell renal cell carcinoma (CCRCC)).

In yet another embodiment, the anti-PD-1 or PD-L1 antibody molecule, alone or in combination with another immunomodulator (*e.g*., an anti-LAG-3 or anti-TIM-3 antibody molecule), is administered in combination with chemotherapy, and/or immunotherapy. For example, the anti-PD-1 or PD-L1 antibody molecule can be used to treat a myeloma, alone or in combination with one or more of: chemotherapy or other anti-cancer agents (*e.g*., thalidomide analogs, *e.g*., lenalidomide), an anti-TIM-3 antibody, tumor antigen-pulsed dendritic cells, fusions (*e.g*., electrofusions) of tumor cells and dendritic cells, or vaccination with immunoglobulin idiotype produced by malignant plasma cells. In one embodiment, the anti-PD-1 or PD-L1 antibody molecule is used in combination with an anti-TIM-3 antibody to treat a myeloma, *e.g.,* a multiple myeloma.

In one embodiment, the anti-PD-1 or PD-L1 antibody molecule, alone or in combination with another immunomodulator (*e.g*., an anti-LAG-3 or anti-TIM-3 antibody molecule), is used in combination with chemotherapy to treat a lung cancer, *e.g*., non-small cell lung cancer. In one embodiment, the anti-PD-1 or PD-L1 antibody molecule is used with platinum doublet therapy to treat lung cancer.

In yet another embodiment, the anti-PD-1 or PD-L1 antibody molecule, alone or in combination with another immunomodulator (*e.g*., an anti-LAG-3 or anti-TIM-3 antibody molecule), is used to treat a renal cancer, *e.g.,* renal cell carcinoma (RCC) (*e.g.,* clear cell renal cell carcinoma (CCRCC) or metastatic RCC. The anti-PD-1 or PD-L1 antibody molecule can be administered in combination with one or more of: an immune-based strategy (*e.g*., interleukin-2 or interferon-α), a targeted agent (*e.g.,* a VEGF inhibitor such as a monoclonal antibody to VEGF); a VEGF tyrosine kinase inhibitor such as sunitinib, sorafenib, axitinib and pazopanib; an RNAi inhibitor), or an inhibitor of a downstream mediator of VEGF signaling, *e.g*., an inhibitor of the mammalian target of rapamycin (mTOR), *e.g*., everolimus and temsirolimus.

An example of suitable therapeutics for use in combination for treatment of pancreatic cancer includes, but is not limited to, a chemotherapeutic agent, *e.g*., paclitaxel or a paclitaxel agent (*e.g.,* a paclitaxel formulation such as TAXOL, an albumin-stabilized nanoparticle paclitaxel formulation (*e.g*., ABRAXANE) or a liposomal paclitaxel formulation); gemcitabine (*e.g.,* gemcitabine alone or in combination with AXP107-11); other chemotherapeutic agents such as oxaliplatin, 5-fluorouracil, capecitabine, rubitecan, epirubicin hydrochloride, NC-6004, cisplatin, docetaxel (*e.g*., TAXOTERE), mitomycin C, ifosfamide; interferon; tyrosine kinase inhibitor (*e.g.,* EGFR inhibitor (*e.g.,* erlotinib, panitumumab, cetuximab, nimotuzumab); HER2/neu receptor inhibitor (*e.g.,* trastuzumab); dual kinase inhibitor (*e.g.,* bosutinib, saracatinib, lapatinib, vandetanib); multikinase inhibitor (*e.g*., sorafenib, sunitinib, XL184, pazopanib); VEGF inhibitor (*e.g.,* bevacizumab, AV-951, brivanib); radioimmunotherapy (*e.g.,* XR303); cancer vaccine (*e.g.,* GVAX, survivin peptide); COX-2 inhibitor (*e.g.,* celecoxib); IGF-1 receptor inhibitor (*e.g.,* AMG 479, MK-0646); mTOR inhibitor (*e.g.,* everolimus, temsirolimus); IL-6 inhibitor (*e.g.,* CNTO 328); cyclin-dependent kinase inhibitor (*e.g.,* P276-00, UCN-01); Altered Energy Metabolism-Directed (AEMD) compound (*e.g*., CPI-613); HDAC inhibitor (*e.g.,* vorinostat); TRAIL receptor 2 (TR-2) agonist (*e.g.,* conatumumab); MEK inhibitor (*e.g.,* AS703026, selumetinib, GSK1120212); Raf/MEK dual kinase inhibitor *(e.g.,* RO5126766); Notch signaling inhibitor (*e.g*., MK0752); monoclonal antibody-antibody fusion protein (*e.g.,* L19IL2); curcumin; HSP90 inhibitor (*e.g.,* tanespimycin, STA-9090); rIL-2;, denileukin diftitox; topoisomerase 1 inhibitor (*e.g.,* irinotecan, PEP02); statin (*e.g.,* simvastatin); Factor VIIa inhibitor (*e.g.,* PCI-27483); AKT inhibitor (*e.g.,* RX-0201); hypoxia-activated prodrug (*e.g.,* TH-302); metformin hydrochloride, gamma-secretase inhibitor (*e.g.,* RO4929097); ribonucleotide reductase inhibitor (*e.g.,* 3-AP); immunotoxin (*e.g.,* HuC242-DM4); PARP inhibitor (*e.g.,* KU-0059436, veliparib); CTLA-4 inhbitor (*e.g.,* CP-675,206, ipilimumab); AdV-tk therapy; proteasome inhibitor (*e.g.,* bortezomib (Velcade), NPI-0052); thiazolidinedione (*e.g.,* pioglitazone); NPC-1C; Aurora kinase inhibitor (*e.g.,* R763/AS703569), CTGF inhibitor (*e.g.,* FG-3019); siG12D LODER; and radiation therapy (*e.g*., tomotherapy, stereotactic radiation, proton therapy), surgery, and a combination thereof. In certain embodiments, a combination of paclitaxel or a paclitaxel agent, and gemcitabine can be used with the anti-PD-1 antibody molecules described herein.

An example of suitable therapeutics for use in combination for treatment of small cell lung cancer includes, but is not limited to, a chemotherapeutic agent, *e.g.,* etoposide, carboplatin, cisplatin, irinotecan, topotecan, gemcitabine, liposomal SN-38, bendamustine, temozolomide, belotecan, NK012, FR901228, flavopiridol); tyrosine kinase inhibitor (*e.g.,* EGFR inhibitor (*e.g.,* erlotinib, gefitinib, cetuximab, panitumumab); multikinase inhibitor (*e.g*., sorafenib, sunitinib); VEGF inhibitor (*e.g.,* bevacizumab, vandetanib); cancer vaccine (*e.g.,* GVAX); Bcl-2 inhibitor (*e.g.,* oblimersen sodium, ABT-263); proteasome inhibitor (*e.g.,* bortezomib (Velcade), NPI-0052), paclitaxel or a paclitaxel agent; docetaxel; IGF-1 receptor inhibitor (*e.g*., AMG 479); HGF/SF inhibitor (*e.g.,* AMG 102, MK-0646); chloroquine; Aurora kinase inhibitor (*e.g.,* MLN8237); radioimmunotherapy (*e.g.,* TF2); HSP90 inhibitor (*e.g.,* tanespimycin, STA-9090); mTOR inhibitor (*e.g.,* everolimus); Ep-CAM-/CD3-bispecific antibody (*e.g.,* MT110); CK-2 inhibitor (*e.g.,* CX-4945); HDAC inhibitor (*e.g.,* belinostat); SMO antagonist (*e.g.,* BMS 833923); peptide cancer vaccine, and radiation therapy (*e.g.,* intensity-modulated radiation therapy (IMRT), hypofractionated radiotherapy, hypoxia-guided radiotherapy), surgery, and combinations thereof.

An example of suitable therapeutics for use in combination for treatment of non-small cell lung cancer includes, but is not limited to, a chemotherapeutic agent, *e.g*., vinorelbine, cisplatin, docetaxel, pemetrexed disodium, etoposide, gemcitabine, carboplatin, liposomal SN-38, TLK286, temozolomide, topotecan, pemetrexed disodium, azacitidine, irinotecan, tegafur-gimeracil-oteracil potassium, sapacitabine); tyrosine kinase inhibitor (*e.g.,* EGFR inhibitor (*e.g.,* erlotinib, gefitinib, cetuximab, panitumumab, necitumumab, PF-00299804, nimotuzumab, RO5083945), MET inhibitor (*e.g.,* PF-02341066, ARQ 197), PI3K kinase inhibitor (*e.g.,* XL147, GDC-0941), Raf/MEK dual kinase inhibitor (*e.g*., RO5126766), PI3K/mTOR dual kinase inhibitor (*e.g.,* XL765), SRC inhibitor (*e.g.,* dasatinib), dual inhibitor (*e.g.,* BIBW 2992, GSK1363089, ZD6474, AZD0530, AG-013736, lapatinib, MEHD7945A, linifanib), multikinase inhibitor (*e.g*., sorafenib, sunitinib, pazopanib, AMG 706, XL184, MGCD265, BMS-690514, R935788), VEGF inhibitor (*e.g*., endostar, endostatin, bevacizumab, cediranib, BIBF 1120, axitinib, tivozanib, AZD2171), cancer vaccine (*e.g*., BLP25 liposome vaccine , GVAX, recombinant DNA and adenovirus expressing L523S protein), Bcl-2 inhibitor (*e.g*., oblimersen sodium), proteasome inhibitor (*e.g*., bortezomib, carfilzomib, NPI-0052, MLN9708), paclitaxel or a paclitaxel agent, docetaxel, IGF-1 receptor inhibitor (*e.g*., cixutumumab, MK-0646, OSI 906, CP-751,871, BIIB022), hydroxychloroquine, HSP90 inhibitor (*e.g*., tanespimycin, STA-9090, AUY922, XL888), mTOR inhibitor (*e.g*., everolimus, temsirolimus, ridaforolimus), Ep-CAM-/CD3-bispecific antibody (*e.g.,* MT110), CK-2 inhibitor (*e.g.,* CX-4945), HDAC inhibitor (*e.g.,* MS 275, LBH589, vorinostat, valproic acid, FR901228), DHFR inhibitor (*e.g.,* pralatrexate), retinoid (*e.g.,* bexarotene, tretinoin), antibody-drug conjugate (*e.g.,* SGN-15), bisphosphonate (*e.g.,* zoledronic acid), cancer vaccine (*e.g.,* belagenpumatucel-L), low molecular weight heparin (LMWH) (*e.g*., tinzaparin, enoxaparin), GSK1572932A, melatonin, talactoferrin, dimesna, topoisomerase inhibitor (*e.g*., amrubicin, etoposide, karenitecin), nelfinavir, cilengitide, ErbB3 inhibitor (*e.g.,* MM-121, U3-1287), survivin inhibitor (*e.g.,* YM155, LY2181308), eribulin mesylate, COX-2 inhibitor (*e.g*., celecoxib), pegfilgrastim, Polo-like kinase 1 inhibitor (*e.g.,* BI 6727), TRAIL receptor 2 (TR-2) agonist (*e.g.,* CS-1008), CNGRC peptide-TNF alpha conjugate, dichloroacetate (DCA), HGF inhibitor (*e.g*., SCH 900105), SAR240550, PPAR-gamma agonist (*e.g.,* CS-7017), gamma-secretase inhibitor (*e.g.,* RO4929097), epigenetic therapy (*e.g.,* 5-azacitidine), nitroglycerin, MEK inhibitor (*e.g.,* AZD6244), cyclin-dependent kinase inhibitor (*e.g.,* UCN-01), cholesterol-Fusl, antitubulin agent (*e.g.,* E7389), farnesyl-OH-transferase inhibitor (*e.g*., lonafarnib), immunotoxin (*e.g.,* BB-10901, SS1 (dsFv) PE38), fondaparinux, vascular-disrupting agent (*e.g.,* AVE8062), PD-L1 inhibitor (*e.g.,* MDX-1105, MDX-1106), beta-glucan, NGR-hTNF, EMD 521873, MEK inhibitor (*e.g*., GSK1120212), epothilone analog (*e.g.,* ixabepilone), kinesin-spindle inhibitor (*e.g.,* 4SC-205), telomere targeting agent (*e.g.,* KML-001), P70 pathway inhibitor (*e.g.,* LY2584702), AKT inhibitor (*e.g.,* MK-2206), angiogenesis inhibitor (*e.g.,* lenalidomide), Notch signaling inhibitor (*e.g.,* OMP-21M18), radiation therapy, surgery, and combinations thereof.

An example of suitable therapeutics for use in combination for treatment of ovarian cancer includes, but is not limited to, a chemotherapeutic agent (*e.g*., paclitaxel or a paclitaxel agent; docetaxel; carboplatin; gemcitabine; doxorubicin; topotecan; cisplatin; irinotecan, TLK286, ifosfamide, olaparib, oxaliplatin, melphalan, pemetrexed disodium, SJG-136, cyclophosphamide, etoposide, decitabine); ghrelin antagonist (*e.g*., AEZS-130), immunotherapy (*e.g.,* APC8024, oregovomab, OPT-821), tyrosine kinase inhibitor (*e.g.,* EGFR inhibitor (*e.g.,* erlotinib), dual inhibitor (*e.g.,* E7080), multikinase inhibitor (*e.g.,* AZD0530, JI-101, sorafenib, sunitinib, pazopanib), ON 01910.Na), VEGF inhibitor (*e.g*., bevacizumab, BIBF 1120, cediranib, AZD2171), PDGFR inhibitor (*e.g*., IMC-3G3), paclitaxel, topoisomerase inhibitor (*e.g*., karenitecin, Irinotecan), HDAC inhibitor (*e.g*., valproate, vorinostat), folate receptor inhibitor (*e.g*., farletuzumab), angiopoietin inhibitor (*e.g*., AMG 386), epothilone analog (*e.g*., ixabepilone), proteasome inhibitor (*e.g*., carfilzomib), IGF-1 receptor inhibitor (*e.g*., OSI 906, AMG 479), PARP inhibitor (*e.g*., veliparib, AG014699, iniparib, MK-4827), Aurora kinase inhibitor (*e.g*., MLN8237, ENMD-2076), angiogenesis inhibitor (*e.g*., lenalidomide), DHFR inhibitor (*e.g*., pralatrexate), radioimmunotherapeutic agnet (*e.g*., Hu3S193), statin (*e.g.*, lovastatin), topoisomerase 1 inhibitor (*e.g*., NKTR-102), cancer vaccine (*e.g*., p53 synthetic long peptides vaccine, autologous OC-DC vaccine), mTOR inhibitor (*e.g*., temsirolimus, everolimus), BCR/ABL inhibitor (*e.g*., imatinib), ET-A receptor antagonist (*e.g*., ZD4054), TRAIL receptor 2 (TR-2) agonist (*e.g*., CS-1008), HGF/SF inhibitor (*e.g*., AMG 102), EGEN-001, Polo-like kinase 1 inhibitor (*e.g.*, BI 6727), gamma-secretase inhibitor *(*e*.g.*, RO4929097), Wee-1 inhibitor (*e.g.*, MK-1775), antitubulin agent (*e.g.*, vinorelbine, E7389), immunotoxin (*e.g.*, denileukin diftitox), SB-485232, vascular-disrupting agent (*e.g.*, AVE8062), integrin inhibitor (*e.g.*, EMD 525797), kinesin-spindle inhibitor (*e.g.*, 4SC-205), revlimid, HER2 inhibitor (*e.g.*, MGAH22), ErrB3 inhibitor (*e.g*., MM-121), radiation therapy; and combinations thereof.

An example of suitable therapeutics for use in combination to treat a myeloma, alone or in combination with one or more of: chemotherapy or other anti-cancer agents (*e.g*., thalidomide analogs, *e.g.*, lenalidomide), HSCT (Cook, R. (2008) J Manag Care Pharm. 14(7 Suppl):19-25), an anti-TIM-3 antibody (Hallett, WHD et al. (2011) J of American Society for Blood and Marrow Transplantation 17(8):1133-145), tumor antigen-pulsed dendritic cells, fusions (*e.g.*, electrofusions) of tumor cells and dendritic cells, or vaccination with immunoglobulin idiotype produced by malignant plasma cells (reviewed in Yi, Q. (2009) Cancer J. 15(6):502-10).

An example of suitable therapeutics for use in combination to treat a renal cancer, *e.g*., renal cell carcinoma (RCC) or metastatic RCC. The anti-PD-1 antibody molecule can be administered in combination with one or more of: an immune-based strategy (*e.g*., interleukin-2 or interferon-a), a targeted agent (*e.g.*, a VEGF inhibitor such as a monoclonal antibody to VEGF, *e.g.*, bevacizumab (Rini, B.I. et al. (2010) J. Clin. Oncol. 28(13):2137-2143)); a VEGF tyrosine kinase inhibitor such as sunitinib, sorafenib, axitinib and pazopanib (reviewed in Pal. S.K. et al. (2014) Clin. Advances in Hematology & Oncology 12(2):90-99)); an RNAi inhibitor), or an inhibitor of a downstream mediator of VEGF signaling, *e.g*., an inhibitor of the mammalian target of rapamycin (mTOR), *e.g*., everolimus and temsirolimus (Hudes, G. et al. (2007) N. Engl. J. Med. 356(22):2271-2281, Motzer, R.J. et al. (2008) Lancet 372: 449-456).

An example of suitable therapeutics for use in combination for treatment of chronic myelogenous leukemia (AML) according to the invention includes, but is not limited to, a chemotherapeutic (*e.g*., cytarabine, hydroxyurea, clofarabine, melphalan, thiotepa, fludarabine, busulfan, etoposide, cordycepin, pentostatin, capecitabine, azacitidine, cyclophosphamide, cladribine, topotecan), tyrosine kinase inhibitor (*e.g.*, BCR/ABL inhibitor (*e.g.*, imatinib, nilotinib), ON 01910.Na, dual inhibitor (*e.g.*, dasatinib, bosutinib), multikinase inhibitor (*e.g.*, DCC-2036, ponatinib, sorafenib, sunitinib, RGB-286638)), interferon alfa, steroids, apoptotic agent (*e.g.*, omacetaxine mepesuccinat), immunotherapy (*e.g.*, allogeneic CD4+ memory Th1-like T cells/microparticle-bound anti-CD3/anti-CD28, autologous cytokine induced killer cells (CIK), AHN-12), CD52 targeting agent (*e.g*.*,* alemtuzumab), HSP90 inhibitor (*e.g.*, tanespimycin, STA-9090, AUY922, XL888), mTOR inhibitor (*e.g*., everolimus), SMO antagonist (*e.g.*, BMS 833923), ribonucleotide reductase inhibitor (*e.g.*, 3-AP), JAK-2 inhibitor (*e.g.*, INCB018424), Hydroxychloroquine, retinoid (*e.g.*, fenretinide), cyclin-dependent kinase inhibitor (*e.g.*, UCN-01), HDAC inhibitor (*e.g.*, belinostat, vorinostat, JNJ-26481585), PARP inhibitor (*e.g.*, veliparib), MDM2 antagonist (*e.g.*, RO5045337), Aurora B kinase inhibitor (*e.g*., TAK-901), radioimmunotherapy (*e.g*., actinium-225-labeled anti-CD33 antibody HuM195), Hedgehog inhibitor (*e.g.*, PF-04449913), STAT3 inhibitor (*e.g*., OPB-31121), KB004, cancer vaccine (*e.g*., AG858), bone marrow transplantation, stem cell transplantation, radiation therapy, and combinations thereof.

An example of suitable therapeutics for use in combination for treatment of chronic lymphocytic leukemia (CLL) includes, but is not limited to, a chemotherapeutic agent (*e.g*., fludarabine, cyclophosphamide, doxorubicin, vincristine, chlorambucil, bendamustine, chlorambucil, busulfan, gemcitabine, melphalan, pentostatin, mitoxantrone, 5-azacytidine, pemetrexed disodium), tyrosine kinase inhibitor (*e.g.*, EGFR inhibitor (*e.g.*, erlotinib), BTK inhibitor (*e.g.*, PCI-32765), multikinase inhibitor (*e.g.*, MGCD265, RGB-286638), CD-20 targeting agent (*e.g*., rituximab, ofatumumab, RO5072759, LFB-R603), CD52 targeting agent (*e.g.*, alemtuzumab), prednisolone, darbepoetin alfa, lenalidomide, Bcl-2 inhibitor (*e.g.*, ABT-263), immunotherapy (*e.g*., allogeneic CD4+ memory Th1-like T cells/microparticle-bound anti-CD3/anti-CD28, autologous cytokine induced killer cells (CIK)), HDAC inhibitor (*e.g*., vorinostat, valproic acid, LBH589, JNJ-26481585, AR-42), XIAP inhibitor (*e.g*., AEG35156), CD-74 targeting agent (*e.g*., milatuzumab), mTOR inhibitor (*e.g.*, everolimus), AT-101, immunotoxin (*e.g.*, CAT-8015, anti-Tac(Fv)-PE38 (LMB-2)), CD37 targeting agent (*e.g.*, TRU-016), radioimmunotherapy (*e.g*., 131-tositumomab), hydroxychloroquine, perifosine, SRC inhibitor (*e.g.*, dasatinib), thalidomide, PI3K delta inhibitor (*e.g.*, CAL-101), retinoid (*e.g.*, fenretinide), MDM2 antagonist (*e.g.*, RO5045337), plerixafor, Aurora kinase inhibitor (*e.g.*, MLN8237, TAK-901), proteasome inhibitor (*e.g.*, bortezomib), CD-19 targeting agent (*e.g.*, MEDI-551, MOR208), MEK inhibitor (*e.g.*, ABT-348), JAK-2 inhibitor (*e.g.*, INCB018424), hypoxia-activated prodrug (*e.g.*, TH-302), paclitaxel or a paclitaxel agent, HSP90 inhibitor, AKT inhibitor (*e.g.*, MK2206), HMG-CoA inhibitor (*e.g.*, simvastatin), GNKG186, radiation therapy, bone marrow transplantation, stem cell transplantation, and a combination thereof.

An example of suitable therapeutics for use in combination for treatment of acute lymphocytic leukemia (ALL) includes, but is not limited to, a chemotherapeutic agent (*e.g*., prednisolone, dexamethasone, vincristine, asparaginase, daunorubicin, cyclophosphamide, cytarabine, etoposide, thioguanine, mercaptopurine, clofarabine, liposomal annamycin, busulfan, etoposide, capecitabine, decitabine, azacitidine, topotecan, temozolomide), tyrosine kinase inhibitor (*e.g.*, BCR/ABL inhibitor (*e.g.*, imatinib, nilotinib), ON 01910.Na, multikinase inhibitor (*e.g.*, sorafenib)), CD-20 targeting agent (*e.g.*, rituximab), CD52 targeting agent (*e.g.*, alemtuzumab), HSP90 inhibitor (*e.g.*, STA-9090), mTOR inhibitor (*e.g.*, everolimus, rapamycin), JAK-2 inhibitor (*e.g.*, INCB018424), HER2/neu receptor inhibitor (*e.g.*, trastuzumab), proteasome inhibitor (*e.g*., bortezomib), methotrexate, asparaginase, CD-22 targeting agent (*e.g.*, epratuzumab, inotuzumab), immunotherapy (e*.g.*, autologous cytokine induced killer cells (CIK), AHN-12), blinatumomab, cyclin-dependent kinase inhibitor (*e.g.*, UCN-01), CD45 targeting agent (*e.g.*, BC8), MDM2 antagonist (*e.g.*, RO5045337), immunotoxin (*e.g.*, CAT-8015, DT2219ARL), HDAC inhibitor (*e.g.*, JNJ-26481585), JVRS-100, paclitaxel or a paclitaxel agent, STAT3 inhibitor (*e.g.*, OPB-31121), PARP inhibitor (*e.g.*, veliparib), EZN-2285, radiation therapy, steroid, bone marrow transplantation, stem cell transplantation, or a combination thereof.

An example of suitable therapeutics for use in combination for treatment of acute myeloid leukemia (AML) includes, but is not limited to, a chemotherapeutic agent (*e.g.*, cytarabine, daunorubicin, idarubicin, clofarabine, decitabine, vosaroxin, azacitidine, clofarabine, ribavirin, CPX-351, treosulfan, elacytarabine, azacitidine), tyrosine kinase inhibitor (*e.g*., BCR/ABL inhibitor (*e.g.*, imatinib, nilotinib), ON 01910.Na, multikinase inhibitor (*e.g.*, midostaurin, SU 11248, quizartinib, sorafinib)), immunotoxin (*e.g*., gemtuzumab ozogamicin), DT388IL3 fusion protein, HDAC inhibitor (*e.g*., vorinostat, LBH589), plerixafor, mTOR inhibitor (*e.g.*, everolimus), SRC inhibitor (*e.g.*, dasatinib), HSP90 inhbitor (*e.g.*, STA-9090), retinoid (*e.g.*, bexarotene, Aurora kinase inhibitor (*e.g.*, BI 811283), JAK-2 inhibitor (*e.g.*, INCB018424), Polo-like kinase inhibitor (*e.g.*, BI 6727), cenersen, CD45 targeting agent (*e.g*., BC8), cyclin-dependent kinase inhibitor (*e.g.*, UCN-01), MDM2 antagonist (*e.g.*, RO5045337), mTOR inhibitor (*e.g*., everolimus), LY573636-sodium, ZRx-101, MLN4924, lenalidomide, immunotherapy (*e.g*., AHN-12), histamine dihydrochloride, radiation therapy, bone marrow transplantation, stem cell transplantation, and a combination thereof.

An example of suitable therapeutics for use in combination for treatment of multiple myeloma (MM) includes, but is not limited to, a chemotherapeutic agent (*e.g*., melphalan, amifostine, cyclophosphamide, doxorubicin, clofarabine, bendamustine, fludarabine, adriamycin, SyB L-0501), thalidomide, lenalidomide, dexamethasone, prednisone, pomalidomide, proteasome inhibitor (*e.g.*, bortezomib, carfilzomib, MLN9708), cancer vaccine (*e.g.*, GVAX), CD-40 targeting agent (*e.g.*, SGN-40, CHIR-12.12), perifosine, zoledronic acid, Immunotherapy (*e.g.*, MAGE-A3, NY-ESO-1 , HuMax-CD38), HDAC inhibitor (*e.g.*, vorinostat, LBH589, AR-42), aplidin, cycline-dependent kinase inhibitor (*e.g*., PD-0332991, dinaciclib), arsenic trioxide, CB3304, HSP90 inhibitor (*e.g.*, KW-2478), tyrosine kinase inhibitor (*e.g.*, EGFR inhibitor (*e.g.,* cetuximab), multikinase inhibitor (*e.g.*, AT9283)), VEGF inhibitor (*e.g.*, bevacizumab), plerixafor, MEK inhibitor (*e.g.*, AZD6244), IPH2101, atorvastatin, immunotoxin (*e.g.*, BB-10901), NPI-0052, radioimmunotherapeutic (*e.g*., yttrium Y 90 ibritumomab tiuxetan), STAT3 inhibitor (*e.g.*, OPB-31121), MLN4924, Aurora kinase inhibitor (*e.g.*, ENMD-2076), IMGN901, ACE-041, CK-2 inhibitor (*e.g*., CX-4945), radiation therapy, bone marrow transplantation, stem cell transplantation, and a combination thereof.

An example of suitable therapeutics for use in combination for treatment of prostate cancer includes, but is not limited to, a chemotherapeutic agent (*e.g*., docetaxel, carboplatin, fludarabine), abiraterone, hormonal therapy (*e.g*., flutamide, bicalutamide, nilutamide, cyproterone acetate, ketoconazole, aminoglutethimide, abarelix, degarelix, leuprolide, goserelin, triptorelin, buserelin), tyrosine kinase inhibitor (*e.g.*, dual kinase inhibitor (*e.g.*, lapatanib), multikinase inhibitor (*e.g.*, sorafenib, sunitinib)), VEGF inhibitor (*e.g.*, bevacizumab), TAK-700, cancer vaccine (*e.g.*, BPX-101, PEP223), lenalidomide, TOK-001, IGF-1 receptor inhibitor *(e.g.,* cixutumumab), TRC105, Aurora A kinase inhibitor (*e.g*., MLN8237), proteasome inhibitor (*e.g.*, bortezomib), OGX-011, radioimmunotherapy (*e.g.*, HuJ591-GS), HDAC inhibitor (*e.g*., valproic acid, SB939, LBH589), hydroxychloroquine, mTOR inhibitor (*e.g*., everolimus), dovitinib lactate, diindolylmethane, efavirenz, OGX-427, genistein, IMC-3G3, bafetinib, CP-675,206, radiation therapy, surgery, or a combination thereof.

The combination therapies can be administered in combination with one or more of the existing modalities for treating cancers, including, but not limited to: surgery; radiation therapy (*e.g*., external-beam therapy which involves three dimensional, conformal radiation therapy where the field of radiation is designed, local radiation (*e.g*., radition directed to a preselected target or organ), or focused radiation). Focused radiation can be selected from the group consisting of stereotactic radiosurgery, fractionated stereotactic radiosurgery, and intensity-modulated radiation therapy. The focused radiation can have a radiation source selected from the group consisting of a particle beam (proton), cobalt-60 (photon), and a linear accelerator (x-ray), *e.g.*, as decribed in WO 2012/177624.

Radiation therapy can be administered through one of several methods, or a combination of methods, including without limitation external-beam therapy, internal radiation therapy, implant radiation, stereotactic radiosurgery, systemic radiation therapy, radiotherapy and permanent or temporary interstitial brachytherapy. The term "brachytherapy," refers to radiation therapy delivered by a spatially confined radioactive material inserted into the body at or near a tumor or other proliferative tissue disease site. The term is intended without limitation to include exposure to radioactive isotopes (*e.g*. At-211, 1-131, 1-125, Y-90, Re-186, Re-188, Sm-153, Bi-212, P-32, and radioactive isotopes of Lu). Suitable radiation sources for use as a cell conditioner of the present invention include both solids and liquids. By way of non-limiting example, the radiation source can be a radionuclide, such as 1-125, 1-131, Yb-169, Ir-192 as a solid source, 1-125 as a solid source, or other radionuclides that emit photons, beta particles, gamma radiation, or other therapeutic rays. The radioactive material can also be a fluid made from any solution of radionuclide(s), *e.g.*, a solution of 1-125 or 1-131, or a radioactive fluid can be produced using a slurry of a suitable fluid containing small particles of solid radionuclides, such as Au-198, Y-90. Moreover, the radionuclide(s) can be embodied in a gel or radioactive micro spheres.

### Nucleic Acids

The invention also features nucleic acids comprising nucleotide sequences that encode heavy and light chain variable regions and CDRs or hypervariable loops of the antibody molecules, as described herein. The nucleic acid can comprise a nucleotide sequence as set forth herein, or a sequence substantially identical thereto (*e.g.*, a sequence at least about 85%, 90%, 95%, 99% or more identical thereto, or which differs by no more than 3, 6, 15, 30, or 45 nucleotides from the sequences shown in the tables herein.

### Vectors

Further provided herein are vectors comprising nucleotide sequences encoding an antibody molecule described herein. In one embodiment, the vectors comprise nucleotides encoding an antibody molecule described herein. In one embodiment, the vectors comprise the nucleotide sequences described herein. The vectors include, but are not limited to, a virus, plasmid, cosmid, lambda phage or a yeast artificial chromosome (YAC).

Numerous vector systems can be employed. For example, one class of vectors utilizes DNA elements which are derived from animal viruses such as, for example, bovine papilloma virus, polyoma virus, adenovirus, vaccinia virus, baculovirus, retroviruses (Rous Sarcoma Virus, MMTV or MOMLV) or SV40 virus. Another class of vectors utilizes RNA elements derived from RNA viruses such as Semliki Forest virus, Eastern Equine Encephalitis virus and Flaviviruses.

Additionally, cells which have stably integrated the DNA into their chromosomes may be selected by introducing one or more markers which allow for the selection of transfected host cells. The marker may provide, for example, prototropy to an auxotrophic host, biocide resistance (*e.g*., antibiotics), or resistance to heavy metals such as copper, or the like. The selectable marker gene can be either directly linked to the DNA sequences to be expressed, or introduced into the same cell by cotransformation. Additional elements may also be needed for optimal synthesis of mRNA. These elements may include splice signals, as well as transcriptional promoters, enhancers, and termination signals.

Once the expression vector or DNA sequence containing the constructs has been prepared for expression, the expression vectors may be transfected or introduced into an appropriate host cell. Various techniques may be employed to achieve this, such as, for example, protoplast fusion, calcium phosphate precipitation, electroporation, retroviral transduction, viral transfection, gene gun, lipid based transfection or other conventional techniques. In the case of protoplast fusion, the cells are grown in media and screened for the appropriate activity.

Methods and conditions for culturing the resulting transfected cells and for recovering the antibody molecule produced are known to those skilled in the art, and may be varied or optimized depending upon the specific expression vector and mammalian host cell employed, based upon the present description.

### Cells

The invention also provides host cells comprising a nucleic acid encoding an antibody molecule as described herein.

In one embodiment, the host cells are genetically engineered to comprise nucleic acids encoding the antibody molecule.

In one embodiment, the host cells are genetically engineered by using an expression cassette. The phrase "expression cassette," refers to nucleotide sequences, which are capable of affecting expression of a gene in hosts compatible with such sequences. Such cassettes may include a promoter, an open reading frame with or without introns, and a termination signal. Additional factors necessary or helpful in effecting expression may also be used, such as, for example, an inducible promoter.

The invention also provides host cells comprising the vectors described herein.

The cell can be, but is not limited to, a eukaryotic cell, a bacterial cell, an insect cell, or a human cell. Suitable eukaryotic cells include, but are not limited to, Vero cells, HeLa cells, COS cells, CHO cells, HEK293 cells, BHK cells and MDCKII cells. Suitable insect cells include, but are not limited to, Sf9 cells.

The following Examples illustrate the disclosure and provide specific embodiments, however without limiting the scope of the disclosure.

### EXAMPLES

### Example 1: Effects of Targeted Agents on PD-L1 Modulation

This example evaluates the effects of selected therapeutic agents (*e.g*., INC280, MEK162, LGX818 and LDK378) on PD-L1 (CD274) modulation. Selected therapeutic agents were examined by real time PCR and flow cytometry on PD-L1 levels. Significant inhibition of PD-L1 by INC280, INC424, MEK162, LGX818 and LDK378 on tumor cells was observed.

### INC280 downregulation of PD-L1 protein

PD-L1 (CD274) expression was analyzed in cancer cell lines treated with INC280. Cells were obtained from ATCC and cultured *in vitro* following ATCC directions. The cell lines used were previously characterized by the Cancer Cell Line Encyclopedia Project (www.broadinstitute.org/ccle/home).

Cells plated in six-well culture plates were treated with the INC280 at different concentrations (10 nM, 100 nM, and 1000 nM) for 24, 48 and 72 hours. Equal amount of vehicle (DMSO) was used as a control. Cells were washed with PBS and then harvested using a cell scraper.

For each reaction, 0.5-1 x 10⁶ cells were stained with 20µL of anti-human monoclonal PD-L1 - PE antibody, clone M1H1 (BD) for 30-60 minutes at 4°C. Cells were washed twice and data was acquired using a Canto II with FACSDiva software (BD Bioscience). Data analysis was performed using FlowJo software (Tree Star). Mean fluorescence intensity (MFI) was determined by gating on single cells. Unstained cells were used as a gating control.

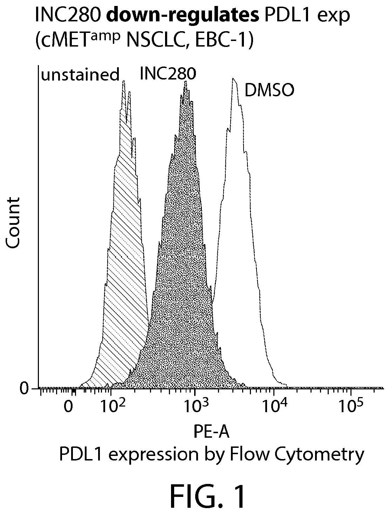

*In vitro* treatment of EBC-1 cells (Non-Small Cell Lung Cancer (NSCLC) with cMET amplification) with INC280 led to significant downregulation of surface expression of PD-L1 as observed by flow cytometry (**Figure 1**). The results presented herein suggest that INC280 functions as a PD-L1/PD-1 inhibitor.

### INC280, MEK162, INC424, LGX818 and LDK 378 downregulate PD-L1 mRNA

TaqMan RT PCR assays were developed to detect changes of expression levels of PD-L1 (CD274) in cell lines and xenograft tumors. mRNA was isolated from frozen cell pellets or tumor fragments using the Qiagen RNeasy Mini kit. Isolated RNA was frozen at -80°C. RNA quality was checked and RNA was quantified using a 2100 Agilent Bioanalyzer following the protocol for the Agilent RNA 6000 Nano Kit. cDNA was prepared using a High Capacity RNA-to cDNA Kit (Applied Biosystems).

Real-time PCR reactions were carried out in 20µl total volume, including 10µl of Universal PCR master mix (Applied Biosystems), 1µl of human PD-L1 (CD274) probe/primer set (Applied Biosystems), and 8 µl of cDNA. Each sample was run in triplicate. The amount of cDNA produced from 25-50 ng of RNA in the reverse transcription reaction was used in each PCR reaction. Due to difference in mRNA levels between PD-L1 and GAPDH, the two real-time PCR reactions were done in separate tubes using same amount of cDNA. The real-time PCR reaction was run on the C1000 Thermal Cycle (BioRad) with the cycle program as follows: a 10 minute incubation at 95°C followed by 40 cycles of 95°C for 15 seconds and 60°C for 1 minute. After the reaction was complete, the PD-L1 average Ct was normalized relative to each Ct value from the GAPDH reference reaction. Each normalized logarithmic value was then converted into a linear value.

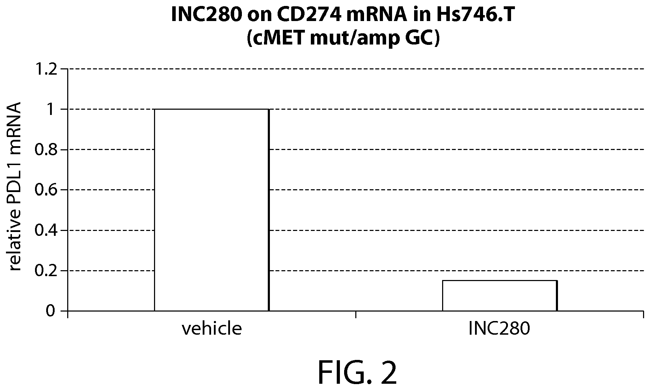

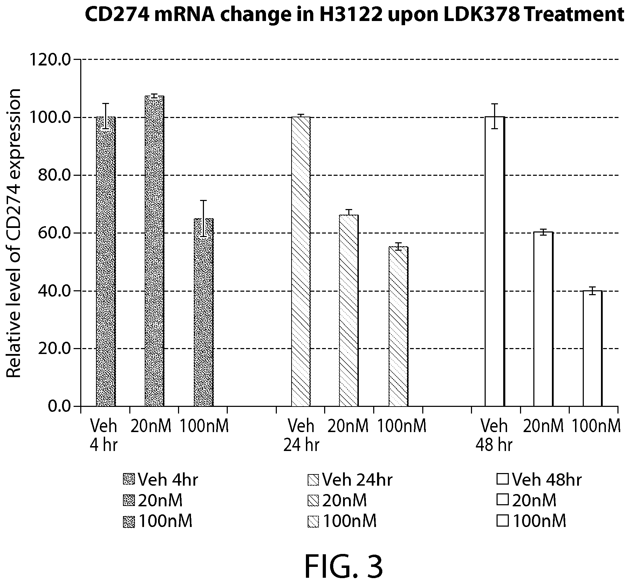

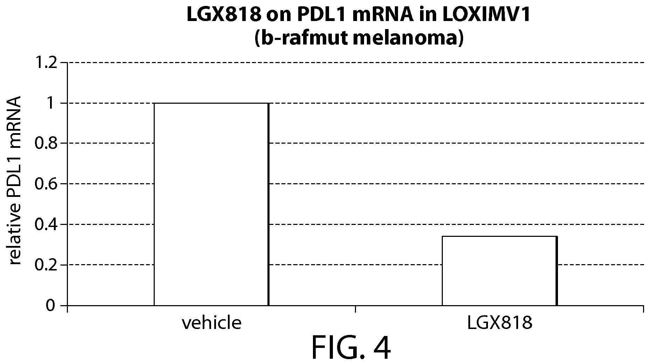

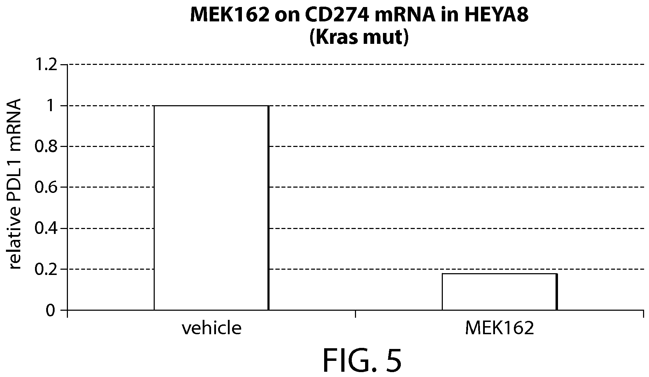

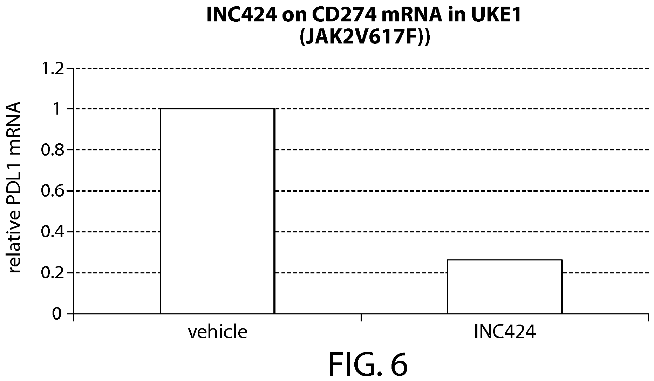

Inhibition of PD-L1 expression (mRNA) by INC280 was observed in a Hs.746.T tumor (gastric cancer cell with cMET amplification & mutation) xenograft (**Figure 2**). Inhibition of PD-L1 mRNA by LDK378 was observed in H3122 (Non-Small Cell Lung Cancer (NSCLC) with ALK translocation) *in vitro* (**Figure 3**). Downregulation of PD-L1 mRNA by LGX818, and MEK162 was observed in tumor xenograft models bearing LOXIMV1 (BRAF mutant melanoma, **Figure 4**) and HEYA8 (KRAF mutant ovarian cancer, **Figure 5**) tumors, respectively. Downregulation of PD-L1 mRNA by INC424 was observed in tumor xenograft models bearing UKE-1 (Myeloproliferative Neoplasm (MPN) line with JAK2V617F mutation, **Figure 6**).

The results presented herein demonstrate a role of INC280, MEK162, INC424, LGX818 and LDK 378 in the regulation of immunecheckpoint molecules on cancer. The observed inhibition of PD-L1 expression by these agents suggests that these targeted agents may have immune-modulatory activities, in addition to their effects on cancer signaling. Thus, the results presented herein suggest that administration of targeted agents with inhibitors of immunecheckpoint inhibitors such as PD-1, PD-L1, LAG-3 and/or TIM-3 will achieve a more potent reversal of the immunecheckpoint-mediated immune suppression.

### Example 2: Effects of LCL161 on Immune Stimulation

This example evaluates the effects of LCL161 on immune stimulation *in vitro.*

Blood was obtained from normal healthy donors. Peripheral blood mononuclear cells (PBMCs) were isolated by centrifuging blood in CPT™ tubes for 18 minutes at 1800 x g. Cells were washed twice with cold PBS, enumerated and then stimulated in 96-well round bottomed tissue culture plates (500,000 cells per well) for 5 days at 37 °C, 5% CO₂. Cells were either left untreated (DMSO control) or treated with different concentrations of LCL161 (1, 50, 100, or 1000 nM). For the cytokine analyses, cells were stimulated with a suboptimal anti-CD3 stimulus (0.005 ug/ml of soluble clone UCHT1). At day 5 post stimulation, supernatants were collected and analyzed for cytokines (Luminex).

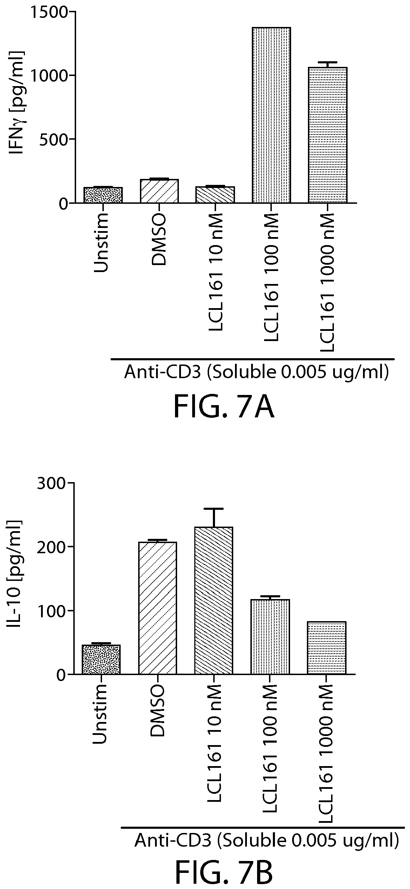

As shown in **Figures 7A-7B****,** LCL161 treatment led to increases in immune-active cytokine, IFN-gamma, *in vitro,* with a corresponding reduction in immune-suppressive cytokine IL-10.

For the proliferation analyses, cells were labeled with 5uM carboxyfluorescein diacetate succinimidyl ester (CFSE) before stimulation, and then treated with 1 ug/ml soluble anti-CD3. Covalently bound CFSE is divided equally between daughter cells, allowing discrimination of successive rounds of cell division and to track proliferating cells (Lyons et al., Curr Protoc Cytom. 2013; Chapter 9: Unit 9.11). PBMCs were stimulated in the presence of increasing concentrations of LCL161 (or DMSO control) for 5 days. At day 5 post stimulation, cells were harvested, stained with anti-CD4, -CD8, -PD-1, or -CD127, followed by FACS analysis. Results for one donor are shown. Similar results were obtained with PBMCs from 4 other donors.

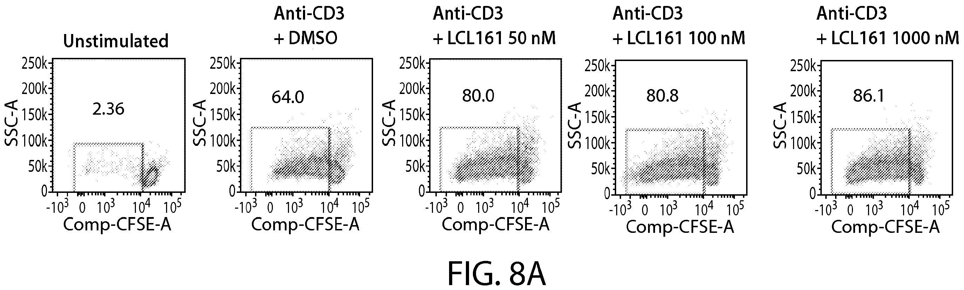

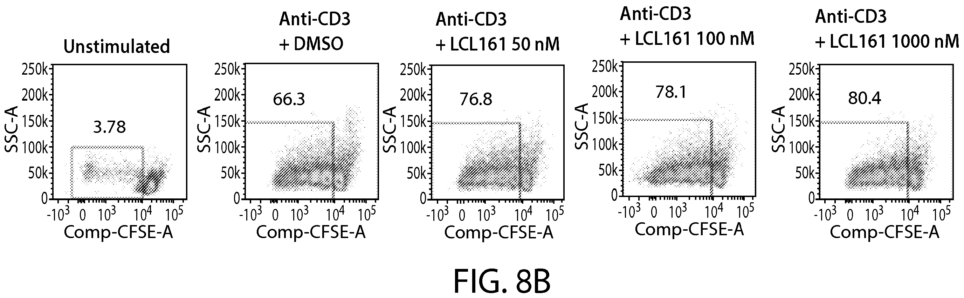

As shown in **Figures 8A-8B****,** LCL161 enhanced proliferation of human CD4+ and CD8+ T cells *in vitro.* These results indicate enhancement of CFSE dilution by LCL161, indicative of increased lymphocyte proliferation under LCL161.

### Example 3: Effects of LCL161 on Immune Checkpoint Modulation

This example evaluates the effects of LCL161 on immune checkpoint modulation *in vitro.*

PBMCs were isolated from healthy donors via BD CPT™ vacutainer tubes. 1 million cells/mL were plated in RPMI + 10% FBS +/- 100ng/mL of anti-CD3 antibody +/- DMSO or 100nM LCL161 for 4 days. Cells were harvested, washed, stained with a panel of metal-conjugated antibodies listed below in Table 2 for analysis by CyTOF mass cytometry. Data were visualized by SPADE using Cytobank webware. SPADE and its use are described, *e.g*., in Peng et al., Nature Biotechnology, 29, 886-891 (2011); Sean et al, Science, 332 (6030): 687-696 (2011).

### A Panel of Metal-Conjugated Antibodies for Analysis by CyTOF Mass Cytometry

| **Metal label** | **Specificity** |
|---|---|
| 141Pr | CD235a/b |
| 142Nd | CD19 |
| 145Nd | CD4 |
| 146Nd | CD8a |
| 147Sm | CD20 |
| 148Nd | CD16 |
| 149Sm | CD66 |
| 151Eu | CD123 |
| 153Eu | TIM-3 |
| 154Sm | CD45 |
| 156Gd | PD-L1 |
| 159Tb | CD11c |
| 160Gd | CD14 |
| 165Ho | LAG-3 |
| 167Er | CD27 |
| 169Tm | CD45RA |
| 170Er | CD3 |
| 172Yb | CD38 |
| 174Yb | HLA-DR |
| 175Lu | PD-1 |
| 191Ir | DNA1 |
| 193Ir | DNA2 |
| 195Pt | Live/Dead |

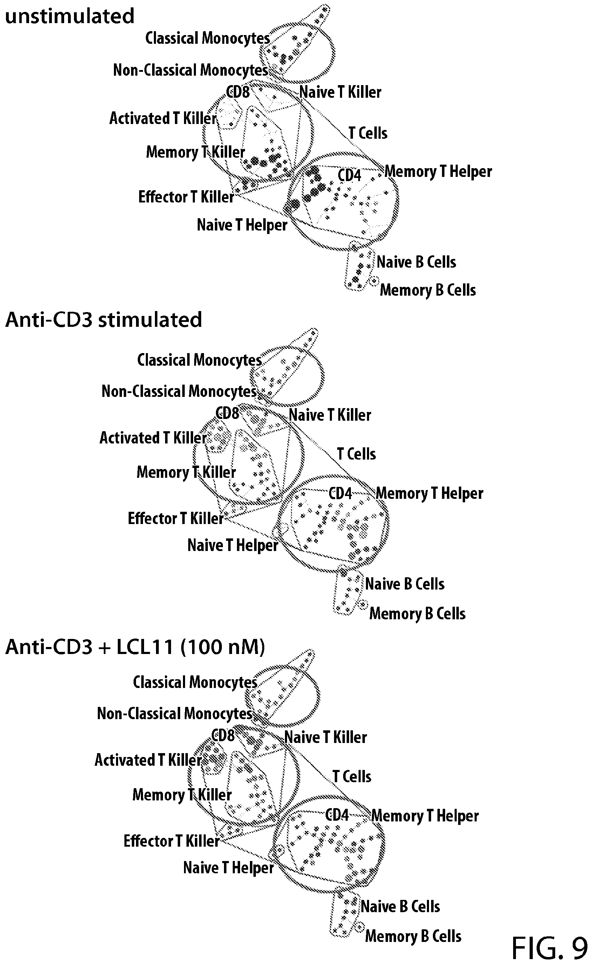

**Figure 9** shows an increase in TIM-3 expression in several nodes of several cell types including: monocytes, naive, memory, and activated T killer cells as well as memory T helper cells. This is direct evidence of LCL161-mediated immunecheckpoint TIM-3 induction. It provides, at least in part, the scientific rationale for combining LCL161 and immune checkpoint modulators, *e.g*., anti-TIM-3 antibody, in cancer therapy.

### Example 4: Effects of LCL161/Anti-PD-1 Combination on Immune Modulation

This example evaluates the effects of LCL161/anti-PD-1 combination on immune checkpoint modulation *in vivo.*

C57Bl/6 mice were implanted with 1x10⁶ MC38 murine colon carcinoma cells/mouse and randomized based on tumor measurements on day 5. The mice received a dose of LCL161 (50mg/kg, po), anti-mouse PD-1 (10mg/kg, i.v.), or both, on the day of randomization. In the control group, mice were dosed with Vehicle (p.o.) and Isotype (mIgG1, 10mg/kg, i.v.). Seven days post-treatment, the animals were euthanized, and the tumors were collected for molecular analysis.

For genomic expression analysis, total RNA was extracted from the aforementioned samples. mRNA expression of mRNA was analyzed on a customized panel of ∼1050 genes on the Nanostring platform (NanoString Technologies).

Gene signatures were derived from mRNA-sequencing data representing 27 separate indications available as part of The Cancer Genome Atlas (TCGA). For each indication, 5,000 genes were clustered into sets with very high correlation across samples. Clustering was performed using the Affinity propagation algorithm (Frey and Dueck (2007) Science 315: 972-976) on the gene-gene Pearson correlation matrix. The 5,000 genes clustered in each indication were comprised of a curated set of 1,000 cell lineage markers and genes involved in immune processes, as well as the 4,000 most variably expressed genes in that indication.

Clusters were annotated to identify co-expressed genes representing specific cell types or immune processes. Annotations included mean log expression level by immune cell type (using the Immgen consortium expression data, immgen.org), mean log expression level by normal tissue type (using GTEx data, www.gtexportal.org), and gene set enrichment using the MSigDB collection (calculated as the Fisher's exact test p-value testing the null hypothesis of random overlap between cluster genes and MSigDB gene sets). Based on these annotations, clusters that were not enriched for genes involved in immune processes were removed.

Gene signatures were generated by pooling clusters from all indications and identifying those with consistent annotations (*e.g*., enriched expression in common cell types or common MSigDB pathway enrichment). Genes from these pooled clusters were then assessed for correlation on an indication-by-indication basis. Only genes whose high level of correlation was preserved across 80% (22/27) of indications or more were included in the final signature.

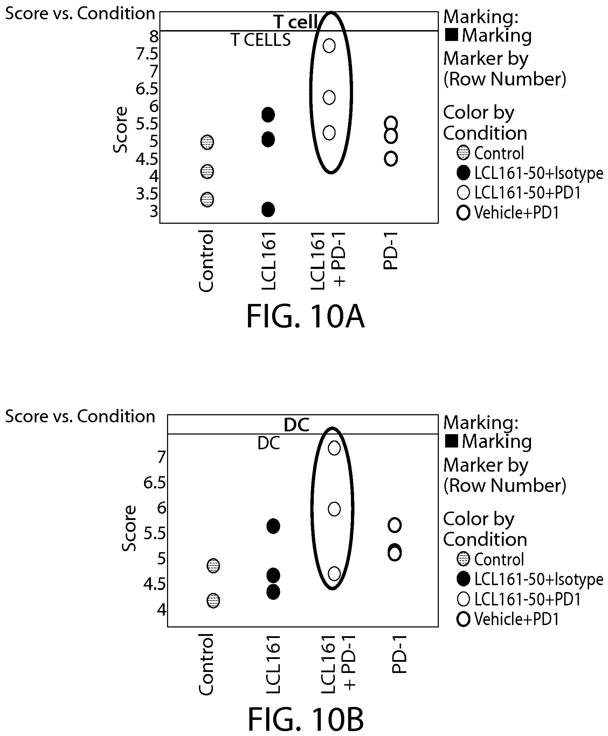

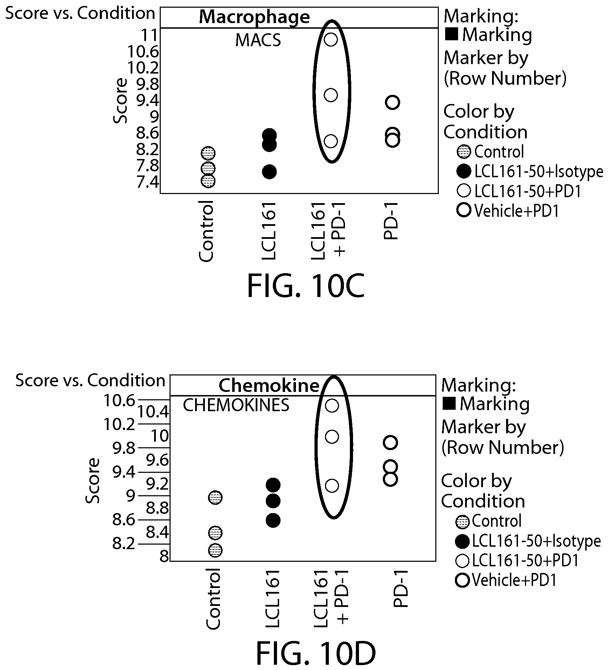

The Nanostring gene signature analysis shows that combination treatment using LCL161 and anti-PD-1 elevated expression signatures related to T cells, dendritic cells, macrophages and chemokine expression (**Figures 10A-10D**). The signature scores with the combination is higher than that of each of the monotherapy. These data indicate that the LCL161/anti-PD-1 combination was immune-stimulatory and the combination of LCL161 with immunecheckpoint therapies would further enhance anti-tumor immunity.

### Example 5: Efficacy of LCL161/Anti-PD-1 Combination

This example evaluates the efficacy of the LCL161/anti-PD-1 combination *in vivo.*

C57Bl/6 mice were implanted with 1x10⁶ MC38 cells/mouse and randomized based on tumor measurements on day 4. Vehicle and LCL161 were given twice a day, every week, by p.o. administration. Isotype and anti-mouse PD-1 were given once per week, by i.v. administration. Two treatment schedules were tested: 1) anti-mouse PD-1 was administred three days after administration of LCL161; or 2) LCL161 and anti-mouse PD-1 were administered concurrently. The design for the studies is summarized below.

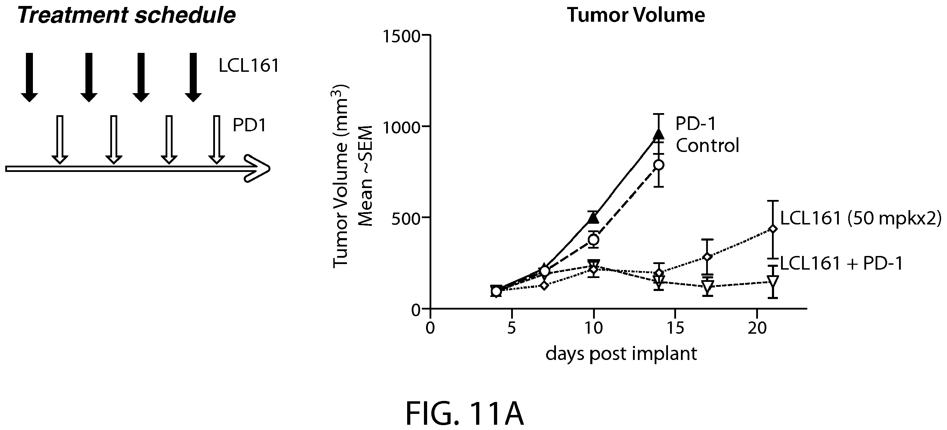

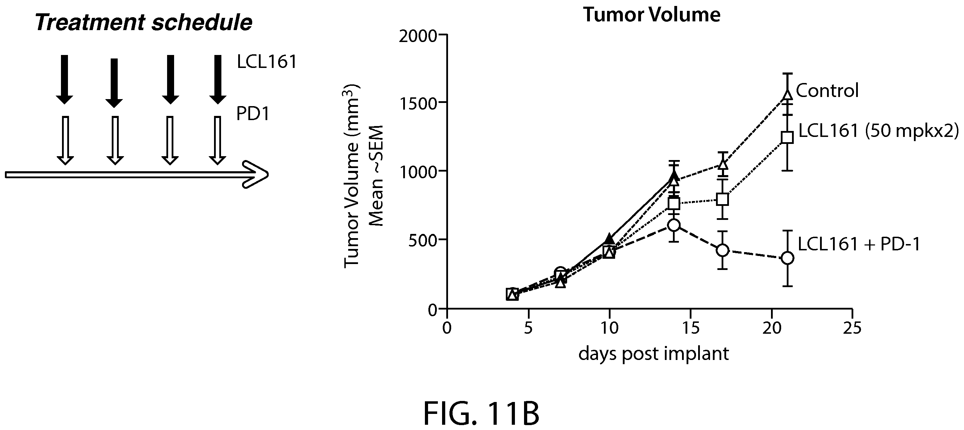

Tumor dimensions and body weights were collected and recorded twice a week. As shown in **Figures 11A-11B****,** the LCL161/anti-PD-1 combination demonstrated anti-tumor efficacy.

| **Group** | **Treatment (Schedule 1)** | **mice/group** |
|---|---|---|
| 1 | Vehicle, bid (1x/week) -starting on day 4 -, po + Isotype mIgG1 (MOPC-21) - 10mpk, 1x/week-starting on day 7-, iv | 9 |
| 2 | LCL161, 50mg/kg, bid (1x/week) -starting on day 4-, po + Isotype mIgG1 (MOPC-21) - 10mpk, 1x/week-starting on day 7-, iv | 9 |
| 3 | Anti-PD-1 (1D2) - 10mpk, 1x/week-starting on day7, iv | 10 |
| 4 | LCL161, 50mg/kg, bid (1x/week) -starting on day 4-, po + Anti-PD-1 (1D2) - 10mpk, 1x/week starting on day 7-, iv | 9 |

| **Group** | **Treatment (Schedule 2)** | **mice/group** |
|---|---|---|
| 1 | Vehicle, bid (1x/week) -starting on day 7-, po + Isotype mIgG1 (MOPC-21) - 10mpk, 1x/week-starting on day 7-, iv | 9 |
| 2 | LCL161, 50mg/kg, bid (1x/week) -starting on day 7-, po + Isotype mIgG1 (MOPC-21) - 10mpk, 1x/week-starting on day 7-, iv | 9 |
| 3 | Anti-PD-1 (1D2) - 10mpk, 1x/week-starting on day 7, iv | 10 |
| 4 | LCL161, 50mg/kg, bid (1x/week) -starting on day 7 -, po + Anti-PD-1 (1D2) - 10mpk, 1x/week-starting on day 7-, iv | 9 |

### Summary:

Thedata on *in vitro* mechanism-of-action supports the immune-stimulatory roles of LCL161. The comprehensive immune profiling by CyTOF indicates the connection between immunecheckpoint (TIM-3) and LCL161. Furthermore, *in vivo* experiments demonstrate synergistic effects with LCL161 and PD-1 in broader immune stimulation and anti-tumor efficacy. Taken together, the data presented in Examples 2-5 demonstrate the combination benefit of LCL161 with immunecheckpoint therapies in cancer.

### Example 6: Dose Escalation and Expansion Study of the LDK (Certinib) and Nivolumab Combination

Efficacy and safety of the ceritinib (LDK378) and nivolumab combination can be assessed in an open-label, multi-center dose escalation and expansion study. In addition to the safety and efficacy, the tolerability and PK/PD of combination of ceritinib and nivolumab for the treatment of patients with metastatic, ALK-positive non-small cell luncer cancer (NSCLC) can also be evaluated. The study can begin with a screening period of up to and including 28 days prior to the first dose of the study drugs to assess eligibility. The treatment period can begin on the first day of the first cycle. The cycles are 28 days long.

Treatment with ceritinib and nivolumab may for example continue until the patient experiences unacceptable toxicity that precludes further treatment and/or disease progression.

In cases of isolated brain progression or other local progression, patients may in addition receive palliative radiotherapy.

The study can include a dose-escalation phase and a dose-expansion phase.

### 1. Dose-escalation phase

The dose-escalation phase of the study can evaluate the maximum tolerated dose (MTD)/ recommended dose for expansion (RDE) of the combination of oral daily ceritinib with a low-fat meal and intravenous nivolumab every 2 weeks (Q2W) based on dose limiting toxicities (DLTs) using a Bayesian Logistic Regression Model (BLRM). For example, 12 patients are enrolled in this phase of the study. The initial dose level of ceritinib can be 450 mg daily and nivolumab is administered at the dose of 3 mg/kg Q2W. The provisional dose levels are as follows:
- [-1 dose cohort] ceritinib 300 mg + nivolumab (3 mg/kg)
- [1st dose cohort] ceritinib 450 mg + nivolumab (3 mg/kg)
- [2nd dose cohort] ceritinib 600 mg + nivolumab (3 mg/kg)

The MTD is the highest drug dosage of both agents not expected to cause DLT in more than 35% of the treated patients in the first 6 weeks of treatment. The final recommended MTD/RDE for combination ceritinib and nivolumab is based on the recommendation from the BLRM, and on an overall assessment of safety taking into consideration tolerability and pharmacokinetic data from subsequent cycles at the tested doses. If the MTD for combination ceritinib and nivolumab is not established after the evaluation of all planned dose levels including the target doses of ceritinib (600 mg with low-fat meal) and nivolumab (3 mg/kg), the RDE is determined after the evaluation of all available safety, PK, and efficacy data.

### 2. Dose-expansion phase

Once the MTD of the combination has been declared and/or the RDE is determined, additional patients are evaluated in the expansion phase of the study at the RDE combination dose. For example, 60 patients are enrolled into the expansion phase of the study. The expansion phase evaluates the safety and preliminary efficacy of the ceritinib and nivolumab combination at the RDE and consists of 2 arms (approximately 30 patients in each arm):
- Arm 1: ALK inhibitor-treated (Prior treatment with any ALK inhibitor except ceritinib is allowed.)
- Arm 2: ALK inhibitor-naive

The data cut-off for the primary clinical study report can occur once all patients in the expansion phase have completed at least 6 cycles (24 weeks) of treatment or have discontinued earlier.

### Example 7: Effects of the LDK378 and Nivolumab Combination in Humans

Eight patients were enrolled to the first dose cohort in the study just as outlined in the Example 6 and below is the data of the only patient with a valid tumor assessment. A partial response was observed with this patient. A second assessment is required to fully confirm the response.

Patient assessed was a 64 year old Caucasian male with diagnosed stage IV NSCLC. Sites of disease included lung, adrenal and abdominal lymph nodes. The patient received one prior chemotherapy regimen of cisplatin and pemetrexed and achieved a partial response. Additional medical conditions include adrenal insufficiency, mitral valve prolapse, hypercholesterolemia, and urolithiasis.

The patient started study treatment with LDK378 450mg QD (oral), administered with a low fat meal, in combination with Nivolumab 3mg/kg every 2 weeks (intravenous). 29 days after the first dose of the study medications (combination of LDK378 + Nivolumab) the patient presented with fever, abdominal pain, nausea, and vomiting. Abdominal ultrasound was negative but computerized tomogram (CT) of the abdomen demonstrated acute pancreatitis. In addition, there were elevations in lipase, amylase, ALT, AST, bilirubin, ALP, and GGT. The patient was hospitalized and treatment with study medication LDK378 was temporarily interrupted. Treatment with intravenous fluids, paracetamol, Contramal (tramadol hydrochloride) and Litican (alizapride) was administered. In the following days the patient's laboratory results improved and the patient's condition improved; there were no more complaints of pain, fever, nausea and vomiting. All supporting medications were stopped and the patient was discharged from the hospital.

At a later evaluation at the clinic there were no complaints (no fever, vomiting, nausea or abdominal pain). After the patient had been discharged from the hospital, the patient did not receive pain medication or anti-emetics. Blood chemistry showed:
Lipase and amylase within normal limits, Gr1: bilirubin, AST, and ALT Gr2: Alkaline Phosphatase Grade 3: GGT
1 day after the evaluation at the clinic LDK378 was restarted at a reduced dose of 300mg daily. Nivolumab treatment was restarted about a week later.

Patient had vomited once without nausea or abdominal pain. He was afebrile although once had a fever of 38 degrees. Patient had no physical complaints.

Lab values when Nivolumab was restarted: AST: 120 U/L, ALT: 139 U/L, Bilirubin total 33 Umol/L, Alkaline Phosphatase 551 U/L. Amylase and Lipase were normal.

LDK378 was discontinued and restarted again at 300 mg dose.

### Tumor assessment:

CT scan at the first tumor assessment demonstrated a 62.9% decrease in overall target lesions in the right adrenal gland and abdominal lymph nodes from the baseline CT scan. There is also a non-target lesion in the Left lower lobe of the lung which was assessed as present.

| **RECIST Target lesion** | | | | |
|---|---|---|---|---|
| Location | Adrenal, lesion #1 | | | |
| | Lesion diameter | | | |
| Baseline | 17 mm | | | |
| Tumor Assessment | 0 mm | | | |
| | | | | |

| Location | Other lymph nodes (abdominal), lesion #2 | | | |
|---|---|---|---|---|
| | Lesion diameter | | | |
| Baseline | 22 mm | | | |
| Tumor Assessment | 7 mm | | | |
| | | | | |

| Location | Other lymph nodes (abdominal), lesion #3 | | | |
|---|---|---|---|---|
| | Lesion diameter | | | |
| Baseline | 23 mm | | | |
| Tumor Assessment | 16 mm | | | |

| **RECIST Non-Target lesion** | | | | |
|---|---|---|---|---|
| Location | Lung, lower lobe | | | |
| | Lesion status | | | |
| Baseline | Present | | | |
| Tumor Assessment | Present | | | |
| | | | | |

| Location | Other lymph nodes (abdominal) | | | |
|---|---|---|---|---|
| | Lesion diameter | | | |
| Evaluation | 22 mm | | | |
| Baseline | 7 mm | | | |

### Example 8: A Phase II, Multicenter, Open-Label Study of EGF816 in Combination with Nivolumab in Adult Patients with EGFR Mutated Non-Small Cell Lung Cancer

Currently approved EGFR TKIs are effective in activated EGFR mutant NSCLC, however nearly all patients develop resistance. Harnessing the immune system to treat patients with non-small cell lung cancer (NSCLC) is a novel and exciting new treatment approach.

Concurrent treatment with an immune checkpoint inhibitor along with a targeted therapy is considered to be safe and is expected to result in durable and sustained responses. Nivolumab is combined with the third generation EGFR TKI, EGF816, in EGFR T790M NSCLC patients who have developed resistance to EGFR TKI treatment. It is expected that the combination of Nivolumab with the EGFR inhibitor, EGF816, provides sustained clinical benefit to NSCLC patients whose tumors have become resistant to EGFR TKI treatment through acquiring T790M mutation by stimulating the host immune system and inhibiting EGFR T790M.

This study is a phase II, multicenter, open-label study of EGF816 in combination with Nivolumab in adult patients with EGFR mutated non-small cell lung cancer, *e.g*., patients with NSCLC progressing on standard of care (*i.e*., erlotinib or gefitinib for EGFR-mutant NSCLC).

An exemplary dose of EGF816 is 150 mg qd on a continuous daily dose for EGF816 (capsule formulation). Different EGF816 doses may also be used. EGF816 is administered prior to Nivolumab. A minimum of 1 hour must pass from the time of EGF816 administration to the administration of EGF816.

The dose and schedule of Nivolumab is 3 mg/kg every 2 weeks. This dose and schedule selection is based on results of safety, efficacy, and exposure-response analyses obtained from studies. This dose and schedule of Nivolumab has been safely combined with other EGFR inhibitors (*e.g*., erlotinib) at registered doses as well as with other standard of care therapies.

This is a phase II, multi-center, open-label study of patients with advanced NSCLC.

Patients are allocated based on their EGFR status *e.g*., EGFR-T790M NSCLC.

Suitable patients may be patients with advanced, recurrent or metastatic/unresectable EGFRT790M NSCLC progressing on standard of care (*i.e*., erlotinib, gefitinib or other approved
EGFR TKI).

EGFR mutation status may be determined by tests available in the art, *e.g.,* QIAGEN therascreen® EGFR test. The therascreen EGFR RGQ PCR Kit is an FDA-approved, qualitative real-time PCR assay for the detection of specific mutations in the EGFR oncogene. Evidence of EGFR mutation can be obtained from existing local data and testing of tumor samples.EGFR mutation status may be determined from any available tumor tissue.

Patients are treated as follows:
EGF816 is administered prior to nivolumab+Nivolumab

A cycle will be defined as 28 days.

At least six patients of each group constitute a safety monitoring cohort for that group.

For each cohort, patients are treated with either Nivolumab 3mg/kg every two weeks and EGF816 at 150mg qd (once daily).

As part of the safety monitoring cohort, steady state PK profile for EGF816 is collected on Cycle 1 day 15; and trough samples for Nivolumab is collected on Cycle 1 Day 15.

The treatment period begins on Cycle 1 Day 1. The study treatment is administered during 28-days cycles. Patients are treated until unacceptable toxicity, progressive disease, treatment discontinuation at the discretion of the investigator, or withdrawal of consent.

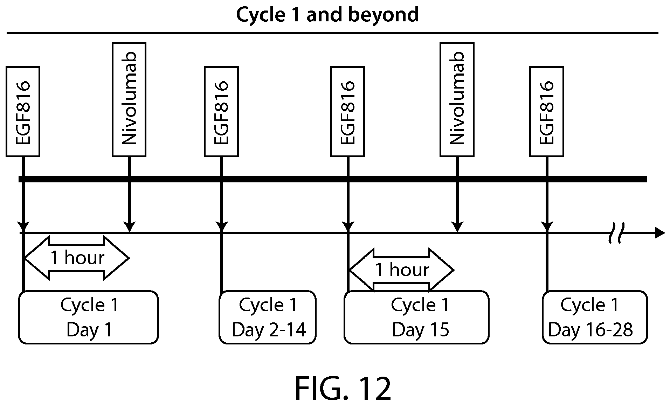

The sequence of drug administration for patients enrolled in the Phase II trial that will be treated with EGF816 and Nivolumab is shown in **Figure 12****.**

**Table 2: Trial objectives and related endpoints**

| **Objective** | **Endpoint** |
|---|---|
| **Primary** | |
| To estimate the clinical activity of Nivolumab in combination with EGF816 | 6 month PFS rate using RECIST version1.1 (6mo PFS rate=6 cycles=168days) |

| **Secondary** | |
|---|---|
| To evaluate the preliminary antitumor activity of EGF816 and Nivolumab | ORR, DCR, other PFS measures, OS |
| To characterize the safety and tolerability of EGF816 and Nivolumab | Safety, incidence and severity of AEs, including changes in hematology and chemistry values, vital signs and ECGs |
| | Tolerability: Dose interruptions, reductions, and dose intensity |
| To evaluate PK of EGF816 and Nivolumab in the combination setting | PK parameters of Nivolumab and EGF816 as Cmax, AUC and Cmin |

| **Exploratory** | |
|---|---|
| Explore correlation of baseline PD-L1 & other immune checkpointmolecules levels in relation to disease progression | Baseline levels of PD-L1 & other immune checkpoint molecules in tumor |

**Table 3: Dose and treatment schedule**

| **Study treatments** | **Pharmaceutical form and route of administration** | **Dose** | **Frequency and/or Regimen** |
|---|---|---|---|
| EGF816 | Capsule for oral use | 150 mg | Daily |
| Nivolumab | Solution for injection | 3mg/kg | Every two weeks |

### INCORPORATION BY REFERENCE

All publications, patents, and Accession numbers mentioned herein are hereby incorporated by reference in their entirety as if each individual publication or patent was specifically and individually indicated to be incorporated by reference.

### EQUIVALENTS

While specific embodiments of the subject invention have been discussed, the above specification is illustrative and not restrictive. Many variations of the invention will become apparent to those skilled in the art upon review of this specification and the claims below. The full scope of the invention should be determined by reference to the claims, along with their full scope of equivalents, and the specification, along with such variations.

### The invention comprises at least the following Numbered Embodiments

### List of Numbered Embodiments:

1. A combination comprising an immunomodulator and a second therapeutic agent for use in treating a cancer in a subject, wherein:
   (i) the immunomodulator is an inhibitor of an immune checkpoint molecule or an activator of a costimulatory molecule, or a combination thereof,
      wherein the inhibitor of an immune checkpoint molecule is chosen from an inhibitor of one or more of PD-1, PD-L1, PD-L2, CTLA-4, TIM-3, LAG-3, CEACAM, VISTA, BTLA, TIGIT, LAIR1, CD160, 2B4 or TGFR beta, and
      wherein the activator of the costimulatory molecule is chosen from an agonist of one or more of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand; and
   (ii) the second therapeutic agent is chosen from one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; or 8) an FGF receptor inhibitor, as provided in Table 1.
2. A combination comprising an immunomodulator and a second therapeutic agent for use in treating a cancer in a subject, wherein:
   (i) the immunomodulator is an inhibitor of an immune checkpoint molecule or an activator of a costimulatory molecule, or a combination thereof,
      wherein the inhibitor of an immune checkpoint molecule is chosen from an inhibitor of one or more of PD-1, PD-L1, PD-L2, CTLA-4, TIM-3, LAG-3, CEACAM, VISTA, BTLA, TIGIT, LAIR1, CD160, 2B4 or TGFR beta, and
      wherein the activator of the costimulatory molecule is chosen from an agonist of one or more of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand; and
   (ii) the second therapeutic agent is chosen from one or more of:
      1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
      2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone);
      3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
      4)N-(4-((1R,3S,5S)-3-amino-5-methylcyclohexyl)pyridin-3-yl)-6-(2,6-difluorophenyl)-5-fluoropicolinamide;
      5) anti-HER3 monoclonal antibody or antigen binding fragment thereof, that comprises a VH of SEQ ID NO: 141 and VL of SEQ ID NO: 140, as described in U.S. 8,735,551;
      6) (E)-N-hydroxy-3-(4-(((2-(2-methyl-1H-indol-3-yl)ethyl)amino)methyl)phenyl)acrylamide;
      7) (3R)-3-cyclopentyl-3-[4-(7H-pyrrolo-[2,3-d]pyrimidin-4-yl)-1H-pyrazol-1-yl]propanenitrile; and/or
      8) 8-(2,6-difluoro-3,5-dimethoxy-phenyl)-quinoxaline-5-carboxylic acid (4-dimethylaminomethyl-1H-imidazol-2-yl)-amide.
3. A method of treating a cancer in a subject, comprising administering to the subject an immunomodulator and a second therapeutic agent, wherein:
   (i) the immunomodulator is an inhibitor of an immune checkpoint molecule or an activator of a costimulatory molecule, or a combination thereof
      wherein the inhibitor of an immune checkpoint molecule is chosen from an inhibitor of one or more of PD-1, PD-L1, PD-L2, CTLA-4, TIM-3, LAG-3, CEACAM, VISTA, BTLA, TIGIT, LAIR1, CD160, 2B4 or TGFR beta, and
      wherein the activator of the costimulatory molecule is chosen from an agonist of one or more of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand; and
   (ii) the second therapeutic agent is chosen from one or more of 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; or 8) an FGF receptor inhibitor, as provided in Table 1,
      thereby treating the cancer.
4. A method of treating a cancer in a subject, comprising administering to the subject an immunomodulator and a second therapeutic agent, wherein:
   (i) the immunomodulator is an inhibitor of an immune checkpoint molecule or an activator of a costimulatory molecule, or a combination thereof
      wherein the inhibitor of an immune checkpoint molecule is chosen from an inhibitor of one or more of PD-1, PD-L1, PD-L2, CTLA-4, TIM-3, LAG-3, CEACAM, VISTA, BTLA, TIGIT, LAIR1, CD160, 2B4 or TGFR beta, and
      wherein the activator of the costimulatory molecule is chosen from an agonist of one or more of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand; and
   (ii) the second therapeutic agent is chosen from one or more of:
      1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
      2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone);
      3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
      4)N-(4-((1R,3S,5S)-3-amino-5-methylcyclohexyl)pyridin-3-yl)-6-(2,6-difluorophenyl)-5-fluoropicolinamide;
      5) anti-HER3 monoclonal antibody or antigen binding fragment thereof, that comprises a VH of SEQ ID NO: 141 and VL of SEQ ID NO: 140, as described in U.S. 8,735,551;
      6) (E)-N-hydroxy-3-(4-(((2-(2-methyl-1H-indol-3-yl)ethyl)amino)methyl)phenyl)acrylamide;
      7) (3R)-3-cyclopentyl-3-[4-(7H-pyrrolo-[2,3-d]pyrimidin-4-yl)-1H-pyrazol-1-yl]propanenitrile; and/or
      8) 8-(2,6-difluoro-3,5-dimethoxy-phenyl)-quinoxaline-5-carboxylic acid (4-dimethylaminomethyl-1H-imidazol-2-yl)-amide,
         thereby treating the cancer.
5. A method of reducing growth, survival, or viability, or all, of a cancer cell, comprising contacting the cell with an immunomodulator and a second therapeutic agent, wherein:
   (i) the immunomodulator is an inhibitor of an immune checkpoint molecule or an activator of a costimulatory molecule, or a combination thereof,
      wherein the inhibitor of an immune checkpoint molecule is chosen from an inhibitor of one or more of PD-1, PD-L1, PD-L2, CTLA-4, TIM-3, LAG-3, CEACAM, VISTA, BTLA, TIGIT, LAIR1, CD160, 2B4 or TGFR beta, and
      wherein the activator of the costimulatory molecule is chosen from an agonist of one or more of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand; and
   (ii) the second therapeutic agent is chosen from one or more of 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; or 8) an FGF receptor inhibitor, as provided in Table 1,
      thereby reducing the growth, survival, or viability of the cancer cell.
6. The use of Numbered Embodiment 1 or 2, or the method of any of Numbered Embodiments 3-5, wherein the inhibitor of the immune checkpoint molecule is chosen from one or more of PD-1, PD-L1, PD-L2, CTLA-4, TIM-3, LAG-3, CEACAM, or any combination thereof.
7. The use of any of Numbered Embodiments 1-2 or 6, or the method of any of Numbered Embodiments 3-6, wherein the agonist of the costimulatory molecule is chosen from an agonist of one or more of OX40, ICOS (CD278), 4-1BB (CD137), GITR, CD30, CD40, BAFFR, HVEM, CD7, NKG2C, SLAMF7, NKp80, CD160, B7-H3 or CD83 ligand.
8. The use of any of Numbered Embodiments 1-2 or 6-7, or the method of any of Numbered Embodiments 3-7, wherein the combination of the immunomodulator and the second therapeutic agent is administered together in a single composition or administered separately in two or more different compositions or dosage forms.
9. The use of any of Numbered Embodiments 1-2 or 6-8, or the method of any of Numbered Embodiments 3-8, wherein the combination of the immunomodulator and the second agent is administered or contacted concurrently with, prior to, or subsequent to, the second agent.
10. The use of any of Numbered Embodiments 1-2 or 6-9, or the method of any of Numbered Embodiments 8-9, wherein the inhibitor of the immune checkpoint molecule is a soluble ligand or an antibody or antigen-binding fragment thereof, that binds to the immune checkpoint molecule.
11. The use or method of Numbered Embodiment 10, wherein the antibody or antigen-binding fragment comprises a constant region from a human IgG1 or IgG4, or an altered form thereof.
12. The use or method of Numbered Embodiment 11, wherein the altered constant region is mutated to increase or decrease one or more of: Fc receptor binding, antibody glycosylation, the number of cysteine residues, effector cell function, or complement function.
13. The use of method of Numbered Embodiment 10, wherein the antibody molecule is a bispecific or multispecific antibody molecule that has a first binding specificity to PD-1 or PD-L1 and a second binding specifity to TIM-3, LAG-3, or PD-L2.
14. The use of any of Numbered Embodiments 1-2 or 6-13, or the method of any of Numbered Embodiments 3-13, wherein the immunomodulator is an anti-PD-1 antibody chosen from Nivolumab, Pembrolizumab or Pidilizumab.
15. The use of any of Numbered Embodiments 1-2 or 6-13, or the method of any of Numbered Embodiments 3-13, wherein the immunomodulator is an anti-PD-Ll antibody chosen from YW243.55.S70, MPDL3280A, MEDI-4736, MSB-0010718C, or MDX-1105.
16. The use of any of Numbered Embodiments 1-2 or 6-13, or the method of any of Numbered Embodiments 3-13, wherein the immunomodulator is an anti-LAG-3 antibody molecule.
17. The use or method of Numbered Embodiment 16, wherein the anti-LAG-3 antibody molecule is BMS-986016.
18. The use of any of Numbered Embodiments 1-2 or 6-13, or the method of any of Numbered Embodiments 3-13, wherein the immunomodulator is an anti-PD-1 antibody comprising the heavy chain amino acid sequence of SEQ ID NO: 2 and the light chain amino acid sequence of SEQ ID NO: 3; or the heavy chain amino acid sequence of SEQ ID NO: 4 and the light chain amino acid sequence of SEQ ID NO: 5.
19. The use of any of Numbered Embodiments 1-2 or 6-13, or the method of any of Numbered Embodiments 3-13, wherein the immunomodulator is the anti-PD-Ll antibody comprising the heavy chain variable amino acid sequence of SEQ ID NO: 6 and the light chain variable amino acid sequence of SEQ ID NO: 7.
20. The use of any of Numbered Embodiments 1-2 or 6-13, or the method of any of Numbered Embodiments 3-13, wherein the immunomodulator is a TIM-3 inhibitor.
21. The use or method of Numbered Embodiment 20, wherein the TIM-3 inhibitor is an antibody molecule to TIM-3.
22. The use of any of Numbered Embodiments 1-2 or 6-21, or the method of any of Numbered Embodiments 3-21, wherein the cancer is a solid tumor, or a soft tissue tumor chosen from a hematological cancer, leukemia, lymphoma, or myeloma, or a metastatic lesion of any of the aforesaid cancers.
23. The use of any of Numbered Embodiments 1-2 or 6-21, or the method of any of Numbered Embodiments 3-21, wherein the cancer is a solid tumor from the lung, breast, ovarian, lymphoid, gastrointestinal (*e.g*., colon), anal, genitals and genitourinary tract (*e.g*., renal, urothelial, bladder cells, prostate), pharynx, CNS (*e.g*., brain, neural or glial cells), head and neck, skin (*e.g*., melanoma), pancreas, colon, rectum, renal-cell carcinoma, liver, lung, non-small cell lung cancer, small intestine or the esophagus.
24. The use of any of Numbered Embodiments 1-2 or 6-21, or the method of any of Numbered Embodiments 3-21, wherein the cancer is a hematological cancer chosen from a Hogdkin lymphoma, a non-Hodgkin lymphoma, a lymphocytic leukemia, or a myeloid leukemia.
25. The use of any of Numbered Embodiments 1-2 or 6-21, or the method of any of Numbered Embodiments 3-21, wherein the cancer is chosen from a cancer disclosed in Table 1.
26. The use of any of Numbered Embodiments 1-2 or 6-25, or the method of any of Numbered Embodiments 3-25, wherein the subject is a human (*e.g*., a patient having, or at risk of having, a cancer).
27. The use of any of Numbered Embodiments 1-2 or 6-26, or the method of any of Numbered Embodiments 3-26, wherein the immunomodulator is an anti-PD-1 antibody molecule administered by injection (*e.g*., subcutaneously or intravenously) at a dose of about 1 to 30 mg/kg, *e.g*., about 5 to 25 mg/kg, about 10 to 20 mg/kg, about 1 to 5 mg/kg, or about 3 mg/kg., *e.g.*, once a week to once every 2, 3, or 4 weeks.
28. The use or method of Numbered Embodiment 27, wherein the anti-PD-1 antibody molecule is administered at a dose from about 1 to 20 mg/kg every other week.
29. The use or method of Numbered Embodiment 26, wherein the anti-PD-1 antibody molecule, *e.g.,* Nivolumab, is administered intravenously at a dose from about 1 mg/kg to 3 mg/kg, *e.g.,* about 1 mg/kg, 2 mg/kg or 3 mg/kg, every two weeks.
30. The use or method of Numbered Embodiment 26, wherein the anti-PD-1 antibody molecule, *e.g.,* Nivolumab, is administered intravenously at a dose of about 2 mg/kg at 3-week intervals.
31. The use of any of Numbered Embodiments 1-2 or 6-30, or the method of any of Numbered Embodiments 3-30, wherein the immunomodulator is Nivolumab, Pembrolizumab, or MSB0010718C used in combination with an IAP inhibitor.
32. The use of any of Numbered Embodiments 1-2 or 6-30, or the method of any of Numbered Embodiments 3-30, wherein the immunomodulator is Nivolumab, Pembrolizumab, or MSB0010718C used in combination with LCL161 to treat a cancer or disorder described in Table 1, *e.g.*, a solid tumor,*e.g*., a breast cancer, a colon cancer, or a pancreatic cancer; or a hematological malignancy, *e.g*., multiple myeloma or a hematopoeisis disorder, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide.
33. The use or method of Numbered Embodiment 32, wherein LCL161 is administered at an oral dose of about 10-3000 mg, *e.g*., about 20-2400 mg, about 50-1800 mg, about 100-1500 mg, about 200-1200 mg, about 300-900 mg, *e.g*., about 600 mg, about 900 mg, about 1200 mg, about 1500 mg, about 1800 mg, about 2100 mg, or about 2400 mg. In an embodiment, LCL161 is administered once a week or once every two weeks.
34. The use of any of Numbered Embodiments 1-2 or 6-30, or the method of any of Numbered Embodiments 3-30, wherein the immunomodulator is Nivolumab, Pembrolizumab, or MSB0010718C used in combination with a TOR kinase inhibitor.
35. The use of any of Numbered Embodiments 1-2 or 6-30, or the method of any of Numbered Embodiments 3-30, wherein the immunomodulator is Nivolumab, Pembrolizumab, or MSB0010718C used in combination with Rad-001 to treat a cancer or disorder described in Table 1, *e.g.*, a solid tumor, *e.g*., a sarcoma, a lung cancer (*e.g.*, a non-small cell lung cancer (NSCLC) (*e.g.*, a NSCLC with squamous and/or non-squamous histology)), a melanoma (*e.g.*, an advanced melanoma), a digestive/gastrointestinal cancer, a gastric cancer, a neurologic cancer, a prostate cancer, a bladder cancer, a breast cancer; or a hematological malignancy, *e.g*., a lymphoma or leukemia, wherein Rad-001 is ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone).
36. The use of any of Numbered Embodiments 1-2 or 6-30, or the method of any of Numbered Embodiments 3-30, wherein the immunomodulator is Nivolumab, Pembrolizumab, or MSB0010718C used in combination with an HDM2 ligase inhibitor.
37. The use of any of Numbered Embodiments 1-2 or 6-30, or the method of any of Numbered Embodiments 3-30, wherein the immunomodulator is Nivolumab, Pembrolizumab, or MSB0010718C used in combination with CGM097 to treat a cancer or disorder described in Table 1, *e.g.*, a solid tumor, wherein CGM097 is (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one.
38. The use of any of Numbered Embodiments 1-2 or 6-30, or the method of any of Numbered Embodiments 3-30, wherein the immunomodulator is Nivolumab, Pembrolizumab, or MSB0010718C used in combination with a PIM kinase inhibitor.
39. The use of any of Numbered Embodiments 1-2 or 6-30, or the method of any of Numbered Embodiments 3-30, wherein the immunomodulator is Nivolumab, Pembrolizumab, or MSB0010718C used in combination with LGH447 to treat a cancer or disorder described in Table 1, *e.g*., hematological malignancy, *e.g*., multiple myeloma, myelodysplastic syndrome, myeloid leukemia, or non-Hodgkin lymphoma, wherein LGH447 is N-(4-((lR,3S,5S)-3-amino-5-methylcyclohexyl)pyridine-3-yl)-6-(2,6-difluorophenyl)-3-fluoropicolinamide.
40. The use of any of Numbered Embodiments 1-2 or 6-30, or the method of any of Numbered Embodiments 3-30, wherein the immunomodulator is Nivolumab, Pembrolizumab, or MSB0010718C used in combination with a HER3 kinase inhibitor.
41. The use of any of Numbered Embodiments 1-2 or 6-30, or the method of any of Numbered Embodiments 3-30, wherein the immunomodulator is Nivolumab, Pembrolizumab, or MSB0010718C used in combination with a LJM716 to treat a cancer or disorder described in Table 1, *e.g.*, a solid tumor, *e.g.* a gastric cancer, an esophageal cancer, a breast cancer, a head and neck cancer, a stomach cancer, or a digestive/gastrointestinal cancer therapy, wherein LJM716 is an anti-HER3 monoclonal antibody or antigen binding fragment thereof, that comprises a VH of SEQ ID NO: 141 and VL of SEQ ID NO: 140, as described in U.S. 8,735,551.
42. The use of any of Numbered Embodiments 1-2 or 6-30, or the method of any of Numbered Embodiments 3-30, wherein the immunomodulator is Nivolumab, Pembrolizumab, or MSB0010718C used in combination with an HDAC inhibitor.
43. The use of any of Numbered Embodiments 1-2 or 6-30, or the method of any of Numbered Embodiments 3-30, wherein the immunomodulator is Nivolumab, Pembrolizumab, or MSB0010718C used in combination with LBH589 to treat a cancer or disorder described in Table 1, *e.g*., a solid tumor,*e.g*., a bone cancer, a small cell lung cancer, a respiratory/thoracic cancer a prostate cancer, a non-small cell lung cancer (NSCLC), a nerologic cancer, a gastric cancer, a melanoma, a breast cancer, a pancreatic cancer, a colorectal cancer, a renal cancer, or a head and neck cancer, or a liver cancer; or a hematological malignancy, *e.g*., multiple myeloma, a hematopoeisis disorder, myelodysplastic syndrome, lymphoma (*e.g*., non-Hodgkin lymphoma), or leukemia (*e.g*., myeloid leukemia), wherein LBH589 is (E)-N-hydroxy-3-(4-(((2-(2-methyl-1H-indol-3-yl)ethyl)amino)methyl)phenyl)acrylamide.
44. The use of any of Numbered Embodiments 1-2 or 6-30, or the method of any of Numbered Embodiments 23-30, wherein the immunomodulator is Nivolumab, Pembrolizumab, or MSB0010718C used in combination with a Janus kinase inhibitor.
45. The use of any of Numbered Embodiments 1-2 or 6-30, or the method of any of Numbered Embodiments 3-30, wherein the immunomodulator is Nivolumab, Pembrolizumab, or MSB0010718C used in combination with INC424 to treat a cancer or disorder described in Table 1, *e.g.*, a solid tumor,*e.g.*, a prostate cancer, a lung cancer, a breast cancer, a pancreatic cancer, a colorectal cancer; or a hematological malignancy, *e.g*., multiple myeloma, lymphoma (*e.g.*, non-Hodgkin lymphoma), or leukemia (*e.g*., myeloid leukemia, lymphocytic leukemia), wherein INC424 is (3R)-3-cyclopentyl-3-[4-(7H-pyrrolo-[2,3-d]pyrimidin-4-yl)-1H-pyrazol-1-yl]propanenitrile.
46. The use or method of Numbered Embodiment 45, wherein the cancer has, or is identified as having, a JAK mutation, *e.g*., a JAK2 V617F mutation.
47. The use of any of Numbered Embodiments 1-2 or 6-30, or the method of any of Numbered Embodiments 3-30, wherein the immunomodulator is Nivolumab, Pembrolizumab, or MSB0010718C used in combination with an FGF receptor inhibitor.
48. The use of any of Numbered Embodiments 1-2 or 6-30, or the method of any of Numbered Embodiments 3-30, wherein the immunomodulator is Nivolumab, Pembrolizumab, or MSB0010718C used in combination with BUW078 to treat a cancer described in Table 1, *e.g.,* a solid tumor, *e.g.,* a digestive/gastrointestinal cancer; or a hematological cancer, wherein BUW078 is 8-(2,6-difluoro-3,5-dimethoxy-phenyl)-quinoxaline-5-carboxylic acid (4-dimethylaminomethyl-1H-imidazol-2-yl)-amide.
49. A combination comprising an anti-PD-1 antibody or an anti-TIM-3 antibody and a second therapeutic agent for use in treating a cancer in a subject, wherein:
   (i) the PD-1 inhibitor is chosen from Nivolumab, Pembrolizumab, or Pidilizumab; and
   (ii) the second therapeutic agent is chosen from one or more of:
      1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
      2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone);
      3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
      4) N-(4-((1R,3S,5S)-3-amino-5-methylcyclohexyl)pyridin-3-yl)-6-(2,6-difluorophenyl)-5-fluoropicolinamide;
      5) anti-HER3 monoclonal antibody or antigen binding fragment thereof, that comprises a VH of SEQ ID NO: 141 and VL of SEQ ID NO: 140, as described in U.S. 8,735,551;
      6) (E)-N-hydroxy-3-(4-(((2-(2-methyl-1H-indol-3-yl)ethyl)amino)methyl)phenyl)acrylamide;
      7) (3R)-3-cyclopentyl-3-[4-(7H-pyrrolo-[2,3-d]pyrimidin-4-yl)-1H-pyrazol-1-yl]propanenitrile; and/or
      8) 8-(2,6-difluoro-3,5-dimethoxy-phenyl)-quinoxaline-5-carboxylic acid (4-dimethylaminomethyl-1H-imidazol-2-yl)-amide.
50. A method of treating a cancer in a subject, comprising administering to the subject an anti-PD-1 antibody or an anti-TIM-3 antibody and a second therapeutic agent, wherein:
   (i) the PD-1 inhibitor is chosen from Nivolumab, Pembrolizumab, or Pidilizumab; and
   (ii) the second therapeutic agent is chosen from one or more of:
      1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
      2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone);
      3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
      4) N-(4-((1R,3S,5S)-3-amino-5-methylcyclohexyl)pyridin-3-yl)-6-(2,6-difluorophenyl)-5-fluoropicolinamide;
      5) anti-HER3 monoclonal antibody or antigen binding fragment thereof, that comprises a VH of SEQ ID NO: 141 and VL of SEQ ID NO: 140, as described in U.S. 8,735,551;
      6) (E)-N-hydroxy-3-(4-(((2-(2-methyl-1H-indol-3-yl)ethyl)amino)methyl)phenyl)acrylamide;
      7) (3R)-3-cyclopentyl-3-[4-(7H-pyrrolo-[2,3-d]pyrimidin-4-yl)-1H-pyrazol-1-yl]propanenitrile; and/or
      8) 8-(2,6-difluoro-3,5-dimethoxy-phenyl)-quinoxaline-5-carboxylic acid (4-dimethylaminomethyl-1H-imidazol-2-yl)-amide,
         thereby treating the cancer.
51. A composition (*e.g*., one or more compositions or dosage forms), comprising an immunomodulator (*e.g*., one or more of: an activator of a costimulatory molecule or an inhibitor of an immune checkpoint molecule) and a second therapeutic agent, *e.g.*, a second therapeutic agent chosen from one or more of 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; or 8) an FGF receptor inhibitor, as provided in Table 1.

## Claims

1. A combination comprising an immunomodulator and a second therapeutic agent for use in treating a cancer in a subject, wherein:
(i) the immunomodulator is an inhibitor of an immune checkpoint molecule or an activator of a costimulatory molecule, or a combination thereof,
wherein the inhibitor of an immune checkpoint molecule is chosen from an inhibitor of one or more of PD-1, PD-L1, PD-L2, CTLA-4, TIM-3, LAG-3, CEACAM, VISTA, BTLA, TIGIT, LAIR1, CD160, 2B4 or TGFR beta, and
wherein the activator of the costimulatory molecule is chosen from an agonist of one or more of OX40, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand; and
(ii) the second therapeutic agent is chosen from one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; or 8) an FGF receptor inhibitor, as provided in Table 1.

2. The combination for use according to claim 1, wherein:
the second therapeutic agent is chosen from one or more of:
1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone);
3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
4) N-(4-((1R,3S,5S)-3-amino-5-methylcyclohexyl)pyridin-3-yl)-6-(2,6-difluorophenyl)-5-fluoropicolinamide;
5) anti-HER3 monoclonal antibody or antigen binding fragment thereof, that comprises a VH of SEQ ID NO: 141 and VL of SEQ ID NO: 140, as described in U.S. 8,735,551;
6) (E)-N-hydroxy-3-(4-(((2-(2-methyl-1H-indol-3-yl)ethyl)amino)methyl)phenyl)acrylamide;
7) (3R)-3-cyclopentyl-3-[4-(7H-pyrrolo-[2,3-d]pyrimidin-4-yl)-1H-pyrazol-1-yl]propanenitrile; and/or
8) 8-(2,6-difluoro-3,5-dimethoxy-phenyl)-quinoxaline-5-carboxylic acid (4-dimethylaminomethyl-1H-imidazol-2-yl)-amide.

3. The combination for use according to claim 1 or claim 2, wherein:
the cancer is treated by reducing growth, survival, or viability, or all, of a cancer cell; and/or
wherein the inhibitor of the immune checkpoint molecule is chosen from one or more of PD-1, PD-L1, PD-L2, CTLA-4, TIM-3, LAG-3, CEACAM, or any combination thereof; and/or
wherein the agonist of the costimulatory molecule is chosen from an agonist of one or more of OX40, ICOS (CD278), 4-1BB (CD137), GITR, CD30, CD40, BAFFR, HVEM, CD7, NKG2C, SLAMF7, NKp80, CD160, B7-H3 or CD83 ligand; and/or wherein the combination of the immunomodulator and the second therapeutic agent is administered together in a single composition or administered separately in two or more different compositions or dosage forms; and/or
wherein the combination of the immunomodulator and the second agent is administered or contacted concurrently with, prior to, or subsequent to, the second agent; and/or
wherein the inhibitor of the immune checkpoint molecule is a soluble ligand or an antibody or antigen-binding fragment thereof, that binds to the immune checkpoint molecule, optionally (i) wherein the antibody or antigen-binding fragment comprises a constant region from a human IgG1 or IgG4, or an altered form thereof, wherein the altered constant region is optionally mutated to increase or decrease one or more of: Fc receptor binding, antibody glycosylation, the number of cysteine residues, effector cell function, or complement function, or (ii) wherein the antibody molecule is a bispecific or multispecific antibody molecule that has a first binding specificity to PD-1 or PD-L1 and a second binding specificity to TIM-3, LAG-3, or PD-L2.

4. The combination for use of any of claims 1-3, wherein:
the immunomodulator is an anti-PD-1 antibody chosen from Nivolumab, Pembrolizumab or Pidilizumab;
the immunomodulator is an anti-PD-L1 antibody chosen from YW243.55.S70, MPDL3280A, MEDI-4736, MSB-0010718C, or MDX-1105;
the immunomodulator is an anti-LAG-3 antibody molecule, optionally wherein the anti-LAG-3 antibody molecule is BMS-986016.
the immunomodulator is an anti-PD-1 antibody comprising the heavy chain amino acid sequence of SEQ ID NO: 2 and the light chain amino acid sequence of SEQ ID NO: 3; or the heavy chain amino acid sequence of SEQ ID NO: 4 and the light chain amino acid sequence of SEQ ID NO: 5;
the immunomodulator is the anti-PD-L1 antibody comprising the heavy chain variable amino acid sequence of SEQ ID NO: 6 and the light chain variable amino acid sequence of SEQ ID NO: 7; or
the immunomodulator is a TIM-3 inhibitor, optionally wherein the TIM-3 inhibitor is an antibody molecule to TIM-3.

5. The combination for use of any preceding claim, wherein:
the cancer is a solid tumor, or a soft tissue tumor chosen from a hematological cancer, leukemia, lymphoma, or myeloma, or a metastatic lesion of any of the aforesaid cancers; or
the cancer is a solid tumor from the lung, breast, ovarian, lymphoid, gastrointestinal (*e.g.*, colon), anal, genitals and genitourinary tract (*e.g.*, renal, urothelial, bladder cells, prostate), pharynx, CNS (*e.g.*, brain, neural or glial cells), head and neck, skin (*e.g.*, melanoma), pancreas, colon, rectum, renal-cell carcinoma, liver, lung, non-small cell lung cancer, small intestine or the esophagus; or
the cancer is a hematological cancer chosen from a Hogdkin lymphoma, a non-Hodgkin lymphoma, a lymphocytic leukemia, or a myeloid leukemia; or
wherein the cancer is chosen from a cancer disclosed in Table 1.

6. The combination for use of any preceding claim, wherein:
(i) the subject is a human (*e.g.*, a patient having, or at risk of having, a cancer); and/or
(ii) wherein the immunomodulator is an anti-PD-1 antibody molecule administered by injection (*e.g.*, subcutaneously or intravenously) at a dose of about 1 to 30 mg/kg, *e.g.*, about 5 to 25 mg/kg, about 10 to 20 mg/kg, about 1 to 5 mg/kg, or about 3 mg/kg., *e.g.*, once a week to once every 2, 3, or 4 weeks; optionally wherein the anti-PD-1 antibody molecule is administered at a dose from about 1 to 20 mg/kg every other week.

7. The composition for use or method of claim 6(i), wherein:
the anti-PD-1 antibody molecule, *e.g.,* Nivolumab, is administered intravenously at a dose from about 1 mg/kg to 3 mg/kg, *e.g.,* about 1 mg/kg, 2 mg/kg or 3 mg/kg, every two weeks; or
the anti-PD-1 antibody molecule, *e.g.,* Nivolumab, is administered intravenously at a dose of about 2 mg/kg at 3-week intervals.

8. The composition for use of any of any preceding claim, wherein:
the immunomodulator is Nivolumab, Pembrolizumab, or MSB0010718C used in combination with an IAP inhibitor; or
the immunomodulator is Nivolumab, Pembrolizumab, or MSB0010718C used in combination with LCL161 to treat a cancer or disorder described in Table 1, *e.g.*, a solid tumor,*e.g*., a breast cancer, a colon cancer, or a pancreatic cancer; or a hematological malignancy, *e.g*., multiple myeloma or a hematopoeisis disorder, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide; optionally wherein LCL161 is administered at an oral dose of about 10-3000 mg, *e.g.*, about 20-2400 mg, about 50-1800 mg, about 100-1500 mg, about 200-1200 mg, about 300-900 mg, *e.g*., about 600 mg, about 900 mg, about 1200 mg, about 1500 mg, about 1800 mg, about 2100 mg, or about 2400 mg, or LCL161 is administered once a week or once every two weeks; or
the immunomodulator is Nivolumab, Pembrolizumab, or MSB0010718C used in combination with a TOR kinase inhibitor; or
the immunomodulator is Nivolumab, Pembrolizumab, or MSB0010718C used in combination with Rad-001 to treat a cancer or disorder described in Table 1, *e.g.*, a solid tumor, *e.g.*, a sarcoma, a lung cancer (*e.g.*, a non-small cell lung cancer (NSCLC) (*e.g.*, a NSCLC with squamous and/or non-squamous histology)), a melanoma (*e.g*., an advanced melanoma), a digestive/gastrointestinal cancer, a gastric cancer, a neurologic cancer, a prostate cancer, a bladder cancer, a breast cancer; or a hematological malignancy, *e.g*., a lymphoma or leukemia, wherein Rad-001 is ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone); or
the immunomodulator is Nivolumab, Pembrolizumab, or MSB0010718C used in combination with an HDM2 ligase inhibitor; or
the immunomodulator is Nivolumab, Pembrolizumab, or MSB0010718C used in combination with CGM097 to treat a cancer or disorder described in Table 1, *e.g*., a solid tumor, wherein CGM097 is (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one; or
the immunomodulator is Nivolumab, Pembrolizumab, or MSB0010718C used in combination with a PIM kinase inhibitor; or
the immunomodulator is Nivolumab, Pembrolizumab, or MSB0010718C used in combination with LGH447 to treat a cancer or disorder described in Table 1, *e.g*., hematological malignancy, *e.g*., multiple myeloma, myelodysplastic syndrome, myeloid leukemia, or non-Hodgkin lymphoma, wherein LGH447 is N-(4-((1R,3S,5S)-3-amino-5-methylcyclohexyl)pyridine-3-yl)-6-(2,6-difluorophenyl)-3-fluoropicolinamide; or
the immunomodulator is Nivolumab, Pembrolizumab, or MSB0010718C used in combination with a HER3 kinase inhibitor; or
the immunomodulator is Nivolumab, Pembrolizumab, or MSB0010718C used in combination with a LJM716 to treat a cancer or disorder described in Table 1, *e.g*., a solid tumor, *e.g.* a gastric cancer, an esophageal cancer, a breast cancer, a head and neck cancer, a stomach cancer, or a digestive/gastrointestinal cancer therapy, wherein LJM716 is an anti-HER3 monoclonal antibody or antigen binding fragment thereof, that comprises a VH of SEQ ID NO: 141 and VL of SEQ ID NO: 140, as described in U.S. 8,735,551; or
the immunomodulator is Nivolumab, Pembrolizumab, or MSB0010718C used in combination with an HDAC inhibitor; or
the immunomodulator is Nivolumab, Pembrolizumab, or MSB0010718C used in combination with LBH589 to treat a cancer or disorder described in Table 1, *e.g*., a solid tumor,*e.g*., a bone cancer, a small cell lung cancer, a respiratory/thoracic cancer a prostate cancer, a non-small cell lung cancer (NSCLC), a nerologic cancer, a gastric cancer, a melanoma, a breast cancer, a pancreatic cancer, a colorectal cancer, a renal cancer, or a head and neck cancer, or a liver cancer; or a hematological malignancy, *e.g*., multiple myeloma, a hematopoeisis disorder, myelodysplastic syndrome, lymphoma (*e.g*., non-Hodgkin lymphoma), or leukemia (*e.g*., myeloid leukemia), wherein LBH589 is (E)-N-hydroxy-3-(4-(((2-(2-methyl-1H-indol-3-yl)ethyl)amino)methyl)phenyl)acrylamide; or
the immunomodulator is Nivolumab, Pembrolizumab, or MSB0010718C used in combination with a Janus kinase inhibitor; or
the immunomodulator is Nivolumab, Pembrolizumab, or MSB0010718C used in combination with INC424 to treat a cancer or disorder described in Table 1, *e.g*., a solid tumor,*e.g*., a prostate cancer, a lung cancer, a breast cancer, a pancreatic cancer, a colorectal cancer; or a hematological malignancy, *e.g*., multiple myeloma, lymphoma (*e.g.*, non-Hodgkin lymphoma), or leukemia (*e.g*., myeloid leukemia, lymphocytic leukemia), wherein INC424 is (3R)-3-cyclopentyl-3-[4-(7H-pyrrolo-[2,3-d]pyrimidin-4-yl)-1H-pyrazol-1-yl]propanenitrile; optionally wherein the cancer has, or is identified as having, a JAK mutation, *e.g*., a JAK2 V617F mutation.

9. The composition for use of any of any of claims 1-7, wherein the immunomodulator is Nivolumab, Pembrolizumab, or MSB0010718C used in combination with an FGF receptor inhibitor.

10. The composition for use of any of claims 1-7, wherein the immunomodulator is Nivolumab, Pembrolizumab, or MSB0010718C used in combination with BUW078 to treat a cancer described in Table 1, *e.g.,* a solid tumor, *e.g.,* a digestive/gastrointestinal cancer or a hematological cancer, wherein BUW078 is 8-(2,6-difluoro-3,5-dimethoxy-phenyl)-quinoxaline-5-carboxylic acid (4-dimethylaminomethyl-1H-imidazol-2-yl)-amide.

11. A combination comprising an anti-PD-1 antibody or an anti-TIM-3 antibody and a second therapeutic agent for use in treating a cancer in a subject, wherein:
(i) the PD-1 inhibitor is chosen from Nivolumab, Pembrolizumab, or Pidilizumab; and
(ii) the second therapeutic agent is chosen from one or more of:
1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone);
3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
4) N-(4-((1R,3S,5S)-3-amino-5-methylcyclohexyl)pyridin-3-yl)-6-(2,6-difluorophenyl)-5-fluoropicolinamide;
5) anti-HER3 monoclonal antibody or antigen binding fragment thereof, that comprises a VH of SEQ ID NO: 141 and VL of SEQ ID NO: 140, as described in U.S. 8,735,551;
6) (E)-N-hydroxy-3-(4-(((2-(2-methyl-1H-indol-3-yl)ethyl)amino)methyl)phenyl)acrylamide;
7) (3R)-3-cyclopentyl-3-[4-(7H-pyrrolo-[2,3-d]pyrimidin-4-yl)-1H-pyrazol-1-yl]propanenitrile; and/or
8) 8-(2,6-difluoro-3,5-dimethoxy-phenyl)-quinoxaline-5-carboxylic acid (4-dimethylaminomethyl-1H-imidazol-2-yl)-amide.

12. A composition comprising an immunomodulator and a second therapeutic agent.

13. A composition according to claim 12, wherein the composition comprises one or more compositions or dosage forms.

14. A composition according to claim 12 or claim 13, wherein the immunomodulator is one or more of an activator of a costimulatory molecule or an inhibitor of an immune checkpoint molecule.

15. A composition according to any of claims 12 to 14, wherein the second therapeutic agent chosen from one or more of 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; or 8) an FGF receptor inhibitor, as provided in Table 1.
